# EUROPÄISCHE PATENTSCHRIFT

(11) **EP 1 051 143 B1**
(45) Veröffentlichungstag und Bekanntmachung des Hinweises auf die Patenterteilung: **21.07.2004**
(21) Anmeldenummer: 99913174.1
(22) Anmeldetag: 19.02.1999
(51) Int. Cl.: A61K 7/00

(54) **2,5-DIAMINO-1-PHENYLBENZOL-DERIVATE ENTHALTENDE OXIDATIONSHAARFÄRBEMITTEL SOWIE NEUE 2,5-DIAMINO-1-PHENYLBENZOL-DERIVATE**
OXIDIZING HAIR COLORING AGENTS CONTAINING 2,5-DIAMINO-1-PHENYLBENZENE DERIVATIVES AND NOVEL 2,5-DIAMINO-1-PHENYLBENZENE DERIVATIVES
PRODUITS DE COLORATION OXYDANTE POUR LES CHEVEUX CONTENANT DES DERIVES DE 2,5-DIAMINO-1-PHENYLBENZOL ET NOUVEAUX DERIVES DE 2,5-DIAMINO-1-PHENYLBENZOL

(30) Priorität: 16.05.1998 DE 19822041
(43) Veröffentlichungstag der Anmeldung: 15.11.2000
(73) Patentinhaber: Wella Aktiengesellschaft, 64274 Darmstadt (DE)
(72) Erfinder: BRAUN, Hans-Jürgen, CH-3182 Überstorf (CH); CHASSOT, Laurent, CH-1724 Praroman (CH)
(86) Internationale Anmeldenummer: PCT/EP1999/001084
(87) Internationale Veröffentlichungsnummer: WO 1999/059527

(56) Entgegenhaltungen:
- EP-A- 0 797 980
- DE-A- 2 518 393
- R. KUNZ: "Umlagerung des p-Dimethylamidohydrazobenzols" JUSTUS LIEBIGS ANNALEN DER CHEMIE., Bd. 303, 1898, Seiten 353-361, XP002123659 VERLAG CHEMIE GMBH. WEINHEIM., DE ISSN: 0075-4617

## Beschreibung

Die vorliegende Erfindung betrifft Mittel zur oxidativen Färbung von Keratinfasern, insbesondere menschlichen Haaren auf der Basis einer Entwicklersubstanz/Kupplersubstanz-Kombination, welche als Eritwicklersubstanz 2,5-Diamino-1-phenylbenzol-Derivate enthalten sowie neue 2,5-Diamino-1-phenylbenzol-Derivate.

Auf dem Gebiet der Färbung von Keratinfasern, insbesondere der Haarfärbung, haben Oxidationsfarbstoffe eine wesentliche Bedeutung erlangt. Die Färbung entsteht hierbei durch Reaktion bestimmter Entwicklersubstanzen mit bestimmten Kupplersubstanzen in Gegenwart eines geeigneten Oxidationsmittels. Als Entwicklersubstanzen werden hierbei insbesondere 2,5-Diaminotoluol, 2,5-Diaminophenylethylalkohol, p-Aminophenol und 1,4-Diaminobenzol eingesetzt, während als Kupplersubstanzen beispielsweise Resorcin, 4-Chlorresorcin, 1-Naphthol, 3-Aminophenol und Derivate des m-Phenylendiamins zu nennen sind.

An Oxidationsfarbstoffe, die zur Färbung menschlicher Haare verwendet werden, werden neben der Färbung in der gewünschten Intensität zahlreiche zusätzliche Anforderungen gestellt. So müssen die Farbstoffe in toxikologischer und dermatologischer Hinsicht unbedenklich sein und die erzielten Haarfärbungen eine gute Lichtechtheit, Dauerwellechtheit, Säureechtheit und Reibeechtheit aufweisen. Auf jeden Fall aber müssen solche Färbungen ohne Einwirkung von Licht, Reibung und chemischen Mitteln über einen Zeitraum von mindestens 4 bis 6 Wochen stabil bleiben. Außerdem ist es erforderlich, daß durch Kombination geeigneter Entwicklersubstanzen und Kupplersubstanzen eine breite Palette verschiedener Farbnuancen erzeugt werden kann.
Aus der DE-A 25 18 393 sind Haarfärbemittel auf der Basis von symmetrischen Tetraamino- oder Diaminodihydroxy-diphenylen bzw. -diphenyläthern bekannt.
Mit den derzeit eingesetzten Färbemitteln ist es jedoch nicht möglich, die vorgenannten Anforderungen in allen Punkten zu erfüllen.

Es besteht daher weiterhin ein Bedürfnis nach neuen Entwicklersubstanzen, welche die vorgenannten Anforderung in besonderem Maße erfüllen.

Hierzu wurde nun gefunden, daß bestimmte neue 1,4-Diaminobenzol-Derivate die an Entwicklerkomponenten gestellten Anforderungen in besonders hohem Maße erfüllen. So werden unter Verwendung dieser Entwicklerkomponenten mit den bekannten Kupplerkomponenten farbstarke Farbnuancen erhalten, die außerordentlich lichtecht und waschecht sind.

Gegenstand der vorliegende Erfindung sind Mittel zur oxidativen Färbung von Keratinfasem, wie zum Beispiel Wolle, Pelzen, Federn oder Haaren, insbesondere menschlichen Haaren, auf der Basis einer Entwicklersubstanz-Kupplersubstanz-Kombination, welche als Entwicklersubstanz mindestens eine Verbindung aus der Gruppe bestehend aus 2,5-Diamino-1-phenyl-benzol; 2,5-Diamino-1- (4-bromophenyl)benzol; 2,5-Diamino-1-(4-ethenylphenyl)- benzol; 2,5-Diamino-1-(2,3,4-trimethoxyphenyl)benzol; 2,5-Diamino-1-(2,4-di(2-hydroxyethyl)aminophenyl)benzol; 2,5-Diamino-1-(2,4-diamino-phenyl)benzol; 2,5-Diamino-1-(2,4-dihydroxyphenyl)benzol; 2,5-Diamino-1-(2,4-dimethylaminophenyl)benzol; 2,5-Diamino-1-(2,4-methoxyphenyl)-benzol; 2,5-Diamino-1-(2,5-di(2-hydroxyethyl)aminophenyl)benzol, 2,5-Diamino-1-(2,5-diaminophenyl)-benzol; 2,5-Diamino-1-(2,5-dihydroxy-phenyl)benzol; 2,5-Diamino-1-(2,5-dimethoxyphenyl)benzol; 2,5-Diamino-1-(2,5-dimethylaminophenyl)benzol; 2,5-Diamino-1-(2,6-di(2-hydroxyethyl)-aminophenyl)benzol; 2,5-Diamino-1-(2,6-diaminophenyl)-benzol; 2,5-Diamino-1-(2,6-dihydroxyphenyl)-benzol; 2,5-Diamino-1-(2,6-dimethoxyphenyl)benzol; 2,5-Diamino-1-(2,6-dimethylaminophenyl)benzol; 2,5-Diamino-1-(2-(bromomethyl)phenyl)-benzol; 2,5-Diamino-1-(2-amino-5-hydroxyphenyl)benzol; 2,5-Diamino-1-(2-aminophenyl)benzol; 2,5-Diamino-1-(2-carbonsäurephenyl)benzol; 2,5-Diamino-1-(2-chlor-phenyl)benzol; 2,5-Diamino-1-(2-di(2-hydroxyethyl)aminophenyl)benzol; 2,5-Diamino-1-(2-dimethylaminophenyl)benzol; 2,5-Diamino-1-(2-fluoro-phenyl)benzol; 2,5-Diamino-1-(2-formylphenyl)benzol; 2,5-Diamino-1-(2-hydroxy-4-aminophenyl)benzol; 2,5-Diamino-1-(2-hydroxy-5-amino-phenyl)-benzol; 2,5-Diamino-1-(2-hydroxyphenyl)benzol; 2,5-Diamino-1-(2-methoxyphenyl)benzol; 2,5-Diamino-1-(2-methylphenyl)benzol; 2,5-Diamino-1-(2-nitrophenyl)-benzol; 2,5-Diamino-1-(2-trifluoromethyl-phenyl)benzol; 2,5-Diamino-1-(3,5-di(2-hydroxyethyl)aminophenyl)benzol; 2,5-Diamino-1-(3,5-diaminophenyl)-benzol; 2,5-Diamino-1-(3,5-dihydroxy-phenyl)benzol; 2,5-Diamino-1-(3,5-dimethoxyphenyl)benzol; 2,5-Diamino-1-(3,5-dimethylaminophenyl)benzol; 2,5-Diamino-1-(3-aminophenyl)-benzol; 2,5-Diamino-1-(3-bromophenyl)-benzol; 2,5-Diamino-1-(3-carbonsäurephenyl)benzol; 2,5-Diamino-1-(3-chlorophenyl)benzol; 2,5-Diamino-1-(3-di(2-hydroxyethyl)aminophenyl)-benzol; 2,5-Diamino-1-(3-dimethylaminophenyl)benzol; 2,5-Diamino-1-(3-fluorophenyl)benzol; 2,5-Diamino-1-(3-formylphenyl)benzol; 2,5-Diamino-1-(3-hydroxy-5-aminophenyl)benzol; 2,5-Diamino-1-(3-hydroxyphenyl)-benzol; 2,5-Diamino-1-(3-methoxyphenyl)benzol; 2,5-Diamino-1-(3-nitrophenyl)benzol; 2,5-Diamino-1-(3-trifluoromethylphenyl)benzol; 2,5-Diamino-1-(4-(dimethylamino)phenyl)-benzol; 2,5-Diamino-1-(4-(hydroxymethyl)phenyl)benzol; 2,5-Diamino-1-(4-(methylthio)phenyl)-benzol; 2,5-Diamino-1-(4-(trifluoromethyl)phenyl)-benzol; 2,5-Diamino-1-(4-(trimethylsilyl)phenyl)benzol; 2,5-Diamino-1-(4-acetylphenyl)benzol; 2,5-Diamino-1-(4-aminophenyl)benzol; 2,5-Diamino-1-(4-carbonsäure-phenyl)benzol; 2,5-Diamino-1-(4-chlorophenyl)benzol; 2,5-Diamino-1-(4-di(2-hydroxyethyl)aminophenyl)benzol; 2,5-Diamino-1-(4-dimethylamino-phenyl)benzol; 2,5-Diamino-1-(4-ethoxyphenyl)benzol; 2,5-Diamino-1-(4-fluorophenyl)benzol; 2,5-Diamino-1-(4-formylphenyl)-benzol; 2,5-Diamino-1-(4-hydroxyphenyl)benzol; 2,5-Diamino-1-(4-methoxy-phenyl)benzol; 2,5-Diamino-1-(4-methylphenyl)benzol; 2,5-Diamino-1-phenylbenzol; 2,5-Diamino-4-chlor-1-phenylbenzol; 2,5-Diamino-4-methoxy-1-phenylbenzol; 2,5-Diamino-4-methyl-1-phenylbenzol; 2,5-Diamino-1-(2-cyanmethyl-phenyl)benzol; 2,5-Diamino-1-(4-ethyl-phenyl)benzol; 2,5-Diamino-1-(4-propyl-phenyl)benzol; 2,5-Diamino-1-(4-isopropyl-phenyl)benzol; 2,5-Diamino-1-(4-butylphenyl)benzol; 2,5-Diamino-1-(4-ter-butyl-phenyl)benzol; 2,5-Diamino-1-(4-pentyl-phenyl)benzol; 2,5-Diamino-1-(4-thiomethoxy-phenyl)benzol; 2,5-Diamino-1-(2-ethyl-phenyl)benzol; 2,5-Diamino-1-(2-fluor-4-methylphenyl)benzol; 2,5-Diamino-1-(2-methyl-5-fluor-phenyl)benzol; 2,5-Diamino-1-(2-thiomethoxy-phenyl)benzol; 2,5-Diamino-1-(2,3-dichlorphenyl)benzol; 2,5-Diamino-4-(4'-hydroxy-biphenyl)benzol; 2,5-Diamino-1-(3-ethoxy-phenyl)benzol; 2,5-Diamino-1-(4-(2-pyrrolidin-1-yl-ethoxy)-phenyl)benzol; 2,5-Diamino-1-(4-(1-hydroxy-ethyl)-phenyl)benzol; 2,5-Diamino-1-(2,4-trifluormethyl-phenyl)benzol; 2,5-Diamino-1-(2-fluor-5-acetyl-phenyl)benzol; 2,5-Diamino-1-(3-fluor-4-methoxy-phenyl)benzol; 2,5-Diamino-1-(3-acetyl-4-hydroxy-phenyl)benzol; 2,5-Diamino-1-(4-(2-hydroxyethyl)-phenyl)benzol; 2,5-Diamino-1-(4-(propyl-1-on)-phenyl)-benzol; 2,5-Diamino-1-(4-N,N'diisopropylaminomethyl-phenyl)benzol; 2,5-Diamino-1-(3-acetyl-phenyl)benzol; 2,5-Diamino-1-(2-(2-hydroxyethyl)-phenyl)benzol; 2,5-Diamino-1-(3-aminomethyl-phenyl)benzol; 2-Amino-5-(2,3-dihydroxypropyl)amino-1-(2,3,4-trimethoxyphenyl)benzol; 2-Amino-5-(2,3-dihydroxypropyl)amino-1-(2,4-dihydroxyphenyl)benzol; 2-Amino-5-(2,3-dihydroxypropyl)amino-1-(2,5-diaminophenyl)benzol; 2-Amino-5-(2,3-dihydroxypropyl)amino-1-(2,6-dimethoxyphenyl)benzol; 2-Amino-5-(2,3-dihydroxypropyl)amino-1-(2-amino-5-hydroxyphenyl)- benzol; 2-Amino-5-(2,3-dihydroxypropyl)amino-1-(2-aminophenyl)benzol; 2-Amino-5-(2,3-dihydroxypropyl)amino-1-(2-hydroxy-4-aminophenyl)-benzol; 2-Amino-5-(2,3-dihydroxypropyl)amino-1-(2-hydroxy-5-amino-phenyl)benzol; 2-Amino-5-(2,3-dihydroxypropyl)amino-1-(2-hydroxy-phenyl)benzol; 2-Amino-5-(2,3-dihydroxypropyl)amino-1-(2-methoxy-phenyl)benzol; 2-Amino-5-(2,3-dihydroxypropyl)amino-1-(2-methyl-phenyl)benzol; 2-Amino-5-(2,3-dihydroxypropyl)amino-1-(3,5-diamino-phenyl)benzol; 2-Amino-5-(2,3-dihydroxypropyl)amino-1-(3,5-dihydroxy-phenyl)benzol; 2-Amino-5-(2,3-dihydroxypropyl)amino-1-(3-aminophenyl)-benzol; 2-Amino-5-(2,3-dihydroxypropyl)amino-1-(3-hydroxy-5-aminphenyl)benzol; 2-Amino-5-(2,3-dihydroxypropyl)amino-1-(3-hydroxy-phenyl)benzol; 2-Amino-5-(2,3-dihydroxypropyl)amino-1-(3-methoxy-phenyl)benzol; 2-Amino-5-(2,3-dihydroxypropyl)amino-1-(4-(dimethyl-amino)phenyl)benzol; 2-Amino-5-(2,3-dihydroxypropyl)amino-1-(4-(trifluoromethyl)phenyl)benzol; 2-Amino-5-(2,3-dihydroxypropyl)amino-1-(4-aminophenyl)benzol; 2-Amino-5-(2,3-dihydroxypropyl)amino-1-(4-carbonsäurephenyl)benzol; 2-Amino-5-(2,3-dihydroxypropyl)amino-1-(4-chlorophenyl)benzol; 2-Amino-5-(2,3-dihydroxypropyl)amino-1-(4-hydroxyphenyl)benzol; 2-Amino-5-(2,3-dihydroxypropyl)amino-1-(4-methoxyphenyl)benzol; 2-Amino-5-(2,3-dihydroxypropyl)amino-1-(4-methylphenyl)benzol; 2-Amino-5-(2,3-dihydroxypropyl)amino-1-phenylbenzol; 2-Amino-5-(2-hydroxyethyl)amino-1-(2,3,4-trimethoxy-phenyl)benzol; 2-Amino-5-(2-hydroxyethyl)amino-1-(2,4-diamino-phenyl)benzol; 2-Amino-5-(2-hydroxyethyl)amino-1-(2,4-dihydroxy-phenyl)benzol; 2-Amino-5-(2-hydroxyethyl)amino-1-(2,5-diamino-phenyl)benzol; 2-Amino-5-(2-hydroxyethyl)amino-1-(2,5-dimethoxy-phenyl)benzol; 2-Amino-5-(2-hydroxyethyl)amino-1-(2,6-dimethoxy-phenyl)benzol; 2-Amino-5-(2-hydroxyethyl)amino-1-(2-amino-5-hydroxyphenyl)benzol; 2-Amino-5-(2-hydroxyethyl)amino-1-(2-aminophenyl)benzol; 2-Amino-5-(2-hydroxyethyl)amino-1-(2-hydroxy-4-aminophenyl)benzol; 2-Amino-5-(2-hydroxyethyl)amino-1-(2-hydroxy-5-aminophenyl)benzol; 2-Amino-5-(2-hydroxyethyl)amino-1-(2-hydroxy-phenyl)benzol; 2-Amino-5-(2-hydroxyethyl)amino-1-(2-methoxyphenyl)-benzol; 2-Amino-5-(2-hydroxyethyl)amino-1-(2-methylphenyl)benzol; 2-Amino-5-(2-hydroxyethyl)amino-1-(3,5-diaminophenyl)benzol; 2-Amino-5-(2-hydroxyethyl)amino-1-(3,5-dihydroxyphenyl)benzol; 2-Amino-5-(2-hydroxyethyl)amino-1-(3-aminophenyl)benzol; 2-Amino-5-(2-hydroxyethyl)amino-1-(3-hydroxy-5-aminophenyl)benzol; 2-Amino-5-(2-hydroxyethyl)amino-1-(3-hydroxyphenyl)benzol; 2-Amino-5-(2-hydroxyethyl)amino-1-(3-methoxyphenyl)benzol; 2-Amino-5-(2-hydroxyethyl)amino-1-(4-(dimethylamino)phenyl)benzol; 2-Amino-5-(2-hydroxyethyl)amino-1-(4-(trifluoromethyl)phenyl)benzol; 2-Amino-5-(2-hydroxyethyl)amino-1-(4-aminophenyl)benzol; 2-Amino-5-(2-hydroxyethyl)amino-1-(4-carbonsäurephenyl)benzol; 2-Amino-5-(2-hydroxyethyl)amino-1-(4-chlorophenyl)benzol; 2-Amino-5-(2-hydroxyethyl)amino-1-(4-hydroxyphenyl)benzol; 2-Amino-5-(2-hydroxyethyl)amino-1-(4-methoxyphenyl)benzol; 2-Amino-5-(2-hydroxyethyl)amino-1-(4-methylphenyl)benzol; 2-Amino-5-(2-hydroxyethyl)amino-1-phenylbenzol; 2-Amino-5-(2-hydroxyethyl)methylamino-1-phenylbenzol; 2-Amino-5-(2-methoxy-ethyl)amino-1-(2,3,4-trimethoxyphenyl)benzol; 2-Amino-5-(2-methoxy-ethyl)amino-1-(2,4-diaminophenyl)benzol; 2-Amino-5-(2-methoxyethyl)-amino-1-(2,4-dihydroxyphenyl)benzol; 2-Amino-5-(2-methoxyethyl)amino-1-(2,5-diaminophenyl)benzol; 2-Amino-5-(2-methoxyethyl)amino-1-(2,5-dimethoxyphenyl)benzol; 2-Amino-5-(2-methoxyethyl)amino-1-(2,6-dimethoxyphenyl)benzol; 2-Amino-5-(2-methoxyethyl)amino-1-(2-amino-5-hydroxyphenyl)benzol; 2-Amino-5-(2-methoxyethyl)amino-1-(2-amino-phenyl)benzol; 2-Amino-5-(2-methoxyethyl)amino-1-(2-hydroxy-4-amino-phenyl)benzol; 2-Amino-5-(2-methoxyethyl)amino-1-(2-hydroxy-5-amino-phenyl)benzol; 2-Amino-5-(2-methoxyethyl)amino-1-(2-hydroxyphenyl)-benzol; 2-Amino-5-(2-methoxyethyl)amino-1-(2-methoxyphenyl)benzol; 2-Amino-5-(2-methoxyethyl)amino-1-(2-methylphenyl)benzol; 2-Amino-5-(2-methoxyethyl)amino-1-(3,5-diaminophenyl)benzol; 2-Amino-5-(2-methoxyethyl)amino-1-(3,5-dihydroxyphenyl)benzol; 2-Amino-5-(2-methoxyethyl)amino-1-(3-aminophenyl)benzol; 2-Amino-5-(2-methoxyethyl)amino-1-(3-hydroxy-5-aminophenyl)benzol; 2-Amino-5-(2-methoxyethyl)amino-1-(3-hydroxyphenyl)benzol; 2-Amino-5-(2-methoxyethyl)amino-1-(3-methoxyphenyl)benzol; 2-Amino-5-(2-methoxyethyl)amino-1-(4-(dimethylamino)phenyl)benzol; 2-Amino-5-(2-methoxyethyl)amino-1-(4-(trifluoromethyl)phenyl)benzol; 2-Amino-5-(2-methoxyethyl)amino-1-(4-aminophenyl)benzol; 2-Amino-5-(2-methoxyethyl)amino-1-(4-carbonsäurephenyl)benzol; 2-Amino-5-(2-methoxyethyl)amino-1-(4-chlorophenyl)benzol; 2-Amino-5-(2-methoxyethyl)amino-1-(4-hydroxyphenyl)benzol; 2-Amino-5-(2-methoxyethyl)amino-1-(4-methoxyphenyl)benzol; 2-Amino-5-(2-methoxyethyl)amino-1-(4-methylphenyl)benzol; 2-Amino-5-(2-methoxyethyl)amino-1-phenylbenzol; 2-Amino-5-di(2-hydroxyethyl)amino-1-(2,3,4-trimethoxyphenyl)benzol; 2-Amino-5-di(2-hydroxyethyl)amino-1-(2,3-difluoro-4-heptylphenyl)benzol; 2-Amino-5-di(2-hydroxyethyl)amino-1-(2,3-difluorophenyl)benzol; 2-Amino-5-di(2-hydroxy-ethyl)amino-1-(2,4,6-trimethylphenyl)benzol; 2-Amino-5-di(2-hydroxy-ethyl)amino-1-(2,4-diaminophenyl)benzol; 2-Amino-5-di(2-hydroxyethyl)-amino-1-(2,4-dichlorophenyl)benzol; 2-Amino-5-di(2-hydroxyethyl)amino-1-(2,4-dihydroxyphenyl)benzol; 2-Amino-5-di(2-hydroxyethyl)amino-1-(2,5-diaminophenyl)benzol; 2-Amino-5-di(2-hydroxyethyl)amino-1-(2,5-dimethoxyphenyl)benzol; 2-Amino-5-di(2-hydroxyethyl)amino-1-(2,6-difluorophenylbenzol; 2-Amino-5-di(2-hydroxyethyl)amino-1-(2,6-dimethoxyphenyl)benzol; 2-Amino-5-di(2-hydroxyethyl)amino-1-(2,6-dimethoxyphenyl)benzol; 2-Amino-5-di(2-hydroxyethyl)amino-1-(2-((bis(1-methylethyl)amino)-carbonyl)phenyl)benzol; 2-Amino-5-di(2-hydroxyethyl)-amino-1-(2-((bis(1-methylethyl)amino)-carbonyl)-3-methoxyphenyl)benzol; 2-Amino-5-di(2-hydroxyethyl)amino-1-(2-(bromomethyl)phenyl)benzol; 2-Amino-5-di(2-hydroxyethyl)amino-1-(2-(diethylamino)carbonyl)phenyl)-benzol; 2-Amino-5-di(2-hydroxyethyl)amino-1-(2-amino-5-hydroxyphenyl)- benzol; 2-Amino-5-di(2-hydroxyethyl)amino-1-(2-aminophenyl)benzol; 2-Amino-5-di(2-hydroxyethyl)amino-1-(2-carbonsäurephenyl)benzol; 2-Amino-5-di(2-hydroxyethyl)amino-1-(2-chlorophenyl)benzol; 2-Amino-5-di(2-hydroxyethyl)amino-1-(2-fluorophenyl)benzol; 2-Amino-5-di(2-hydroxyethyl)amino-1-(2-formyl-4-methoxyphenyl)benzol; 2-Amino-5-di(2-hydroxyethyl)amino-1-(2-formyl-4-methylphenyl)benzol; 2-Amino-5-di(2-hydroxyethyl)amino-1-(2-formyl-5-methoxyphenyl)benzol; 2-Amino-5-di(2-hydroxyethyl)amino-1-(2-formylphenyl)benzol; 2-Amino-5-di(2-hydroxy-ethyl)amino-1-(2-hydroxy-4-aminophenyl)benzol; 2-Amino-5-di(2-hydroxy-ethyl)amino-1-(2-hydroxy-5-aminophenyl)benzol; 2-Amino-5-di(2-hydroxyethyl)amino-1-(2-hydroxyphenyl)benzol; 2-Amino-5-di(2-hydroxyethyl)amino-1-(2-methoxyphenyl)benzol; 2-Amino-5-di(2-hydroxyethyl)amino-1-(2-methylphenyl)benzol; 2-Amino-5-di(2-hydroxyethyl)amino-1-(2-nitrophenyl)benzol; 2-Amino-5-di(2-hydroxy-ethyl)amino-1-(2-trifluoromethylphenyl)benzol; 2-Amino-5-di(2-hydroxy-ethyl)amino-1-(3,4-dichlorophenyl)benzol; 2-Amino-5-di(2-hydroxyethyl)-amino-1-(3,5-diaminophenyl)benzol; 2-Amino-5-di(2-hydroxyethyl)amino-1-(3,5-dichlorophenyl)benzol; 2-Amino-5-di(2-hydroxyethyl)amino-1-(3,5-dihydroxyphenyl)benzol; 2-Amino-5-di(2-hydroxyethyl)amino-1-(3-(acetylamino)phenyl)benzol; 2-Amino-5-di(2-hydroxyethyl)amino-1-(3-aminophenyl)benzol; 2-Amino-5-di(2-hydroxyethyl)amino-1-(3-bromo-phenyl)benzol; 2-Amino-5-di(2-hydroxyethyl)amino-1-(3-carbonsäure-phenyl)benzol; 2-Amino-5-di(2-hydroxyethyl)amino-1-(3-chloro-4-fluorophenyl)benzol; 2-Amino-5-di(2-hydroxyethyl)amino-1-(3-chloro-phenyl)benzol; 2-Amino-5-di(2-hydroxyethyl)amino-1-(3-fluorophenyl)-benzol; 2-Amino-5-di(2-hydroxyethyl)amino-1-(3-formylphenyl)benzol; 2-Amino-5-di(2-hydroxyethyl)amino-1-(3-hydroxy-5-aminopheriyl)benzol; 2-Amino-5-di(2-hydroxyethyl)amino-1-(3-hydroxyphenyl)benzol; 2-Amino-5-di(2-hydroxyethyl)amino-1-(3-hydroxyphenyl)benzol; 2-Amino-5-di(2-hydroxyethyl)amino-1-(3-methoxyphenyl)benzol; 2-Amino-5-di(2-hydroxyethyl)amino-1-(3-methylphenyl)benzol; 2-Amino-5-di(2-hydroxyethyl)amino-1-(3-nitrophenyl)benzol; 2-Amino-5-di(2-hydroxyethyl)amino-1-(3-trifluoromethylphenyl)benzol; 2-Amino-5-di(2-hydroxyethyl)amino-1-(4-(bromomethyl)phenyl)benzol; 2-Amino-5-di(2-hydroxyethyl)amino-1-(4-(dimethylamino)phenyl)benzol; 2-Amino-5-di(2-hydroxyethyl)amino-1-(4-(hydroxymethyl)phenyl)benzol; 2-Amino-5-di(2-hydroxyethyl)amino-1-(4-(methylthio)phenyl)benzol; 2-Amino-5-di(2-hydroxyethyl)amino-1-(4-(trifluoromethyl)phenyl)benzol; 2-Amino-5-di(2-hydroxyethyl)amino-1-(4-(trimethylsilyl)phenyl)benzol; 2-Amino-5-di(2-hydroxyethyl)amino-1-(4-acetylphenyl)benzol; 2-Amino-5-di(2-hydroxy-ethyl)amino-1-(4-aminophenyl)benzol; 2-Amino-5-di(2-hydroxyethyl)-amino-1-(4-bromophenyl)benzol; 2-Amino-5-di(2-hydroxyethyl)amino-1-(4-carbonsäurephenyl)benzol; 2-Amino-5-di(2-hydroxyethyl)amino-1-(4-chloro-3-methoxyphenyl)benzol; 2-Amino-5-di(2-hydroxyethyl)amino-1-(4-chlorophenyl)benzol; 2-Amino-5-di(2-hydroxyethyl)amino-1-(4-ethenyl-phenyl)benzol; 2-Amino-5-di(2-hydroxyethyl)amino-1-(4-fluorophenyl)-benzol; 2-Amino-5-di(2-hydroxyethyl)amino-1-(4-formylphenyl)benzol; 2-Amino-5-di(2-hydroxyethyl)amino-1-(4-hydroxyphenyl)benzol; 2-Amino-5-di(2-hydroxyethyl)amino-1-(4-iodophenyl)benzol; 2-Amino-5-di(2-hydroxyethyl)amino-1-(4-methoxyphenyl)benzol; 2-Amino-5-di(2-hydroxy-ethyl)amino-1-(4-methyl-3-nitrophenyl)benzol; 2-Amino-5-di(2-hydroxyö-ethyl)amino-1-(4-methylphenyl)benzol; 2-Amino-5-di(2-hydroxyethyl)-amino-1-(5-bromo-2-methoxyphenyl)benzol; 2-Amino-5-di(2-hydroxy-ethyl)amino-1-(5-chloro-2-methoxyphenyl)benzol; 2-Amino-5-di(2-hydroxyethyl)amino-1-(5-formyl-2-methoxyphenyl)benzol; 2-Amino-5-di(2-hydroxyethyl)amino-1-phenylbenzol; 2-Amino-5-di(2-methoxyethyl)amino-1-(2,3,4-trimethoxyphenyl)benzol; 2-Amino-5-di(2-methoxyethyl)amino-1-(2,3-difluoro-4-heptylphenyl)benzol; 2-Amino-5-di(2-methoxyethyl)amino-1-(2,3-difluorophenyl)benzol; 2-Amino-5-di(2-methoxyethyl)amino-1-(2,4,6-trimethylphenyl)benzol; 2-Amino-5-di(2-methoxyethyl)amino-1-(2,4-diaminophenyl)benzol; 2-Amino-5-di(2-methoxyethyl)amino-1-(2,4-dichlorophenyl)benzol; 2-Amino-5-di(2-methoxyethyl)amino-1-(2,4-dihydroxyphenyl)benzol; 2-Amino-5-di(2-methoxyethyl)amino-1-(2,5-dimethoxyphenyl)benzol; 2-Amino-5-di(2-methoxyethyl)amino-1-(2,6-difluorophenyl)benzol; 2-Amino-5-di(2-methoxyethyl)amino-1-(2,6-dimethoxyphenyl)benzol; 2-Amino-5-di(2-methoxyethyl)amino-1-(2-((bis(1-methylethyl)amino)-carbonyl)phenyl)benzol; 2-Amino-5-di(2-methoxy-ethyl)amino-1-(2-(bromomethyl)phenyl)benzol; 2-Amino-5-di(2-methoxyethyl)amino-1-(2-(diethylamino)carbonyl)phenyl)-benzol; 2-Amino-5-di(2-methoxyethyl)-amino-1-(2-carbonsäurephenyl)benzol; 2-Amino-5-di(2-methoxyethyl)-amino-1-(2-chlorophenyl)benzol; 2-Amino-5-di(2-methoxyethyl)amino-1-(2-fluorophenyl)benzol; 2-Amino-5-di(2-methoxyethyl)amino-1-(2-formyl-4-methoxyphenyl)benzol; 2-Amino-5-di(2-methoxyethyl)amino-1-(2-formyl-4-methylphenyl)benzol; 2-Amino-5-di(2-methoxyethyl)amino-1-(2-formyl-5-methoxyphenyl)benzol; 2-Amino-5-di(2-methoxyethyl)amino-1-(2-formylphenyl)benzol; 2-Amino-5-di(2-methoxy-ethyl)amino-1-(2-hydroxy-4-aminophenyl)benzol; 2-Amino-5-di(2-methoxy-ethyl)amino-1-(2-hydroxyphenyl)benzol; 2-Amino-5-di(2-methoxyethyl)-amino-1-(2-methoxyphenyl)benzol; 2-Amino-5-di(2-methoxyethyl)amino-1-(2-methylphenyl)benzol; 2-Amino-5-di(2-methoxyethyl)amino-1-(2-nitrophenyl)benzol; 2-Amino-5-di(2-methoxyethyl)amino-1-(2-trifluoromethylphenyl)benzol; 2-Amino-5-di(2-methoxyethyl)amino-1-(3,4-dichlorophenyl)benzol; 2-Amino-5-di(2-methoxyethyl)amino-1-(3,5-diaminophenyl)benzol; 2-Amino-5-di(2-methoxyethyl)amino-1-(3,5-dichlorophenyl)benzol; 2-Amino-5-di(2-methoxyethyl)amino-1-(3,5-dihydroxyphenyl)benzol; 2-Amino-5-di(2-methoxyethyl)amino-1-(3-(acetylamino)phenyl)benzol; 2-Amino-5-di(2-methoxyethyl)amino-1-(3-aminophenyl)benzol; 2-Amino-5-di(2-methoxyethyl)amino-1-(3-bromophenyl)benzol; 2-Amino-5-di(2-methoxyethyl)amino-1-(3-carbonsäurephenyl)benzol; 2-Amino-5-di(2-methoxyethyl)amino-1-(3-chloro-4-fluorophenyl)benzol; 2-Amino-5-di(2-methoxyethyl)amino-1-(3-chlorophenyl)benzol; 2-Amino-5-di(2-methoxyethyl)amino-1-(3-fluorophenyl)-benzol; 2-Amino-5-di(2-methoxyethyl)amino-1-(3-formylphenyl)benzol; 2-Amino-5-di(2-methoxyethyl)amino-1-(3-hydroxyphenyl)benzol; 2-Amino-5-di(2-methoxyethyl)amino-1-(3-methoxyphenyl)benzol; 2-Amino-5-di(2-methoxyethyl)amino-1-(3-methylphenyl)benzol; 2-Amino-5-di(2-methoxyethyl)amino-1-(3-nitrophenyl)benzol; 2-Amino-5-di(2-methoxyethyl)amino-1-(3-trifluoromethylphenyl)benzol; 2-Amino-5-di(2-methoxyethyl)amino-1-(4-(bromomethyl)phenyl)benzol; 2-Amino-5-di(2-methoxyethyl)amino-1-(4-(dimethylamino)phenyl)benzol; 2-Amino-5-di(2-methoxyethyl)amino-1-(4-(hydroxymethyl)phenyl)benzol; 2-Amino-5-di(2-methoxyethyl)amino-1-(4-(methylthio)phenyl)benzol; 2-Amino-5-di(2-methoxyethyl)amino-1-(4-(trifluoromethyl)phenyl)benzol; 2-Amino-5-di(2-methoxyethyl)amino-1-(4-(trimethylsilyl)phenyl)benzol; 2-Amino-5-di(2-methoxyethyl)amino-1-(4-acetylphenyl)benzol; 2-Amino-5-di(2-methoxy-ethyl)amino-1-(4-aminophenyl)benzol; 2-Amino-5-di(2-methoxyethyl)-amino-1-(4-bromophenyl)benzol; 2-Amino-5-di(2-methoxyethyl)amino-1-(4-carbonsäurephenyl)benzol; 2-Amino-5-di(2-methoxyethyl)amino-1-(4-chloro-3-methoxyphenyl)benzol; 2-Amino-5-di(2-methoxyethyl)amino-1-(4-chlorophenyl)benzol; 2-Amino-5-di(2-methoxyethyl)amino-1-(4-ethenylphenyl)benzol; 2-Amino-5-di(2-methoxyethyl)amino-1-(4-ethoxyphenyl)benzol; 2-Amino-5-di(2-methoxyethyl)amino-1-(4-fluorophenyl)benzol; 2-Amino-5-di(2-methoxyethyl)amino-1-(4-formylphenyl)benzol; 2-Amino-5-di(2-methoxyethyl)amino-1-(4-hydroxyphenyl)benzol; 2-Amino-5-di(2-methoxyethyl)amino-1-(4-iodophenyl)benzol; 2-Amino-5-di(2-methoxyethyl)amino-1-(4-methoxy-phenyl)benzol; 2-Amino-5-di(2-methoxyethyl)amino-1-(4-methyl-3-nitrophenyl)benzol; 2-Amino-5-di(2-methoxyethyl)amino-1-(4-methyl-phenyl)benzol; 2-Amino-5-di(2-methoxyethyl)amino-1-(5-bromo-2-methoxyphenyl)benzol; 2-Amino-5-di(2-methoxyethyl)amino-1-(5-chloro-2-methoxyphenyl)benzol; 2-Amino-5-di(2-methoxyethyl)amino-1-(5-formyl-2-methoxyphenyl)benzol; 2-Amino-5-dimethylamino-1-phenylbenzol; 2-Amino-5-methylamino-1-(2,3,4-trimethoxyphenyl)benzol; 2-Amino-5-methylamino-1-(2,4-diaminophenyl)benzol; 2-Amino-5-methylamino-1-(2,4-dihydroxyphenyl)benzol; 2-Amino-5-methylamino-1-(2,5-diaminophenyl)benzol; 2-Amino-5-methylamino-1-(2,5-dimethoxyphenyl)benzol; 2-Amino-5-methylamino-1-(2,6-dimethoxyphenyl)benzol; 2-Amino-5-methylamino-1-(2-amino-5-hydroxyphenyl)benzol; 2-Amino-5-methyl-amino-1-(2-aminophenyl)benzol; 2-Amino-5-methylamino-1-(2-hydroxy-4-aminophenyl)benzol; 2-Amino-5-methylamino-1-(2-hydroxy-5-amino-phenyl)benzol; 2-Amino-5-methylamino-1-(2-hydroxyphenyl)benzol;2-Amino-5-methylamino-1-(2-methoxyphenyl)benzol; 2-Amino-5-methylamino-1-(2-methylphenyl)-benzol; 2-Amino-5-methylamino-1-(3,5-diaminophenyl)benzol; 2-Amino-5-methylamino-1-(3,5-dihydroxyphenyl)-benzol; 2-Amino-5-methylamino-1-(3-aminophenyl)benzol; 2-Amino-5-methylamino-1-(3-hydroxy-5-aminophenyl)benzol; 2-Amino-5-methylamino-1-(3-hydroxyphenyl)benzol; 2-Amino-5-methylamino-1-(3-methoxyphenyl)-benzol; 2-Amino-5-methylamino-1-(4-(dimethylamino)-phenyl)benzol; 2-Amino-5-methylamino-1-(4-(trifluoromethyl)-phenyl)benzol; 2-Amino-5-methylamino-1-(4-aminophenyl)benzol; 2-Amino-5-methylamino-1-(4-carbonsäurephenyl)benzol; 2-Amino-5-methylamino-1-(4-chlorophenyl)-benzol; 2-Amino-5-methylamino-1-(4-hydroxyphenyl)benzol; 2-Amino-5-methylamino-1-(4-methoxyphenyl)-benzol; 2-Amino-5-methylamino-1-(4-methylphenyl)benzol; 2-Amino-5-methylamino-1-phenylbenzol; 5-Amino-2-(2,3-dihydroxypropyl)amino-1- (4-bromophenyl)benzol; 5-Amino-2-(2,3-dihydroxypropyl)amino-1- (4-ethenylphenyl)benzol; 5-Amino-2-(2,3-dihydroxypropyl)amino-1-(2,3,4-trimethoxyphenyl)benzol; 5-Amino-2-(2,3-dihydroxypropyl)amino-1-(2,4-di(2-hydroxyethyl)-aminophenyl)benzol; 5-Amino-2-(2,3-dihydroxypropyl)-amino-1-(2,4-diaminophenyl)benzol; 5-Amino-2-(2,3-dihydroxypropyl)-amino-1-(2,4-dihydroxyphenyl)benzol; 5-Amino-2-(2,3-dihydroxypropyl)-amino-1-(2,4-dimethylaminophenyl)-benzol; 5-Amino-2-(2,3-dihydroxy-propyl)amino-1-(2,4-methoxyphenyl)benzol; 5-Amino-2-(2,3-dihydroxy-propyl)amino-1-(2,5-di(2-hydroxyethyl)-aminophenyl)benzol; 5-Amino-2-(2,3-dihydroxypropyl)amino-1-(2,5-diaminophenyl)benzol; 5-Amino-2-(2,3-dihydroxypropyl)amino-1-(2,5-dihydroxyphenyl)benzol; 5-Amino-2-(2,3-dihydroxypropyl)amino-1-(2,5-dimethoxyphenyl)benzol; 5-Amino-2-(2,3-dihydroxypropyl)amino-1-(2,5-dimethylaminophenyl)-benzol; 5-Amino-2-(2,3-dihydroxypropyl)amino-1-(2,6-di(2-hydroxyethyl)-aminophenyl)benzol; 5-Amino-2-(2,3-dihydroxypropyl)amino-1-(2,6-diaminophenyl)benzol; 5-Amino-2-(2,3-dihydroxypropyl)amino-1-(2,6-dihydroxyphenyl)benzol; 5-Amino-2-(2,3-dihydroxypropyl)amino-1-(2,6-dimethoxyphenyl)benzol; 5-Amino-2-(2,3-dihydroxypropyl)amino-1-(2,6-dimethylaminophenyl)-benzol; 5-Amino-2-(2,3-dihydroxypropyl)amino-1-(2-(bromomethyl)-phenyl)-benzol; 5-Amino-2-(2,3-dihydroxypropyl)amino-1-(2-amino-5-hydroxyphenyl)benzol; 5-Amino-2-(2,3-dihydroxypropyl)amino-1-(2-aminophenyl)benzol; 5-Amino-2-(2,3-dihydroxypropyl)amino-1-(2-carbonsäurephenyl)benzol; 5-Amino-2-(2,3-dihydroxypropyl)amino-1-(2-chlorophenyl)benzol; 5-Amino-2-(2,3-dihydroxypropyl)amino-1-(2-di(2-hydroxyethyl)-aminophenyl)-benzol; 5-Amino-2-(2,3-dihydroxypropyl)-amino-1-(2-dimethylaminophenyl)benzol; 5-Amino-2-(2,3-dihydroxy-propyl)amino-1-(2-fluorophenyl)benzol; 5-Amino-2-(2,3-dihydroxy-propyl)amino-1-(2-formylphenyl)benzol; 5-Amino-2-(2,3-dihydroxy-propyl)amino-1-(2-hydroxy-4-aminophenyl)benzol; 5-Amino-2-(2,3-dihydroxypropyl)amino-1-(2-hydroxy-5-aminophenyl)benzol; 5-Amino-2-(2,3-dihydroxypropyl)amino-1-(2-hydroxyphenyl)benzol; 5-Amino-2-(2,3-dihydroxypropyl)amino-1-(2-methoxyphenyl)benzol; 5-Amino-2-(2,3-dihydroxypropyl)amino-1-(2-methylphenyl) benzol; 5-Amino-2-(2,3-dihydroxypropyl)amino-1-(2-nitrophenyl)benzol; 5-Amino-2-(2,3-dihydroxypropyl)amino-1-(2-trifluoromethylphenyl)benzol; 5-Amino-2-(2,3-dihydroxypropyl)amino-1-(3,5-di(2-hydroxyethyl)-aminophenyl)benzol; 5-Amino-2-(2,3-dihydroxypropyl)amino-1-(3,5-diaminophenyl)benzol; 5-Amino-2-(2,3-dihydroxypropyl)amino-1-(3,5-dihydroxyphenyl)benzol; 5-Amino-2-(2,3-dihydroxypropyl)amino-1-(3,5-dimethoxyphenyl)benzol; 5-Amino-2-(2,3-dihydroxypropyl)amino-1-(3,5-dimethylaminophenyl)-benzol; 5-Amino-2-(2,3-dihydroxypropyl)amino-1-(3-aminophenyl)benzol; 5-Amino-2-(2,3-dihydroxypropyl)amino-1-(3-bromophenyl)benzol; 5-Amino-2-(2,3-dihydroxypropyl)amino-1-(3-carbonsäurephenyl)benzol; 5-Amino-2-(2,3-dihydroxypropyl)amino-1-(3-chlorophenyl)-benzol; 5-Amino-2-(2,3-dihydroxypropyl)amino-1-(3-di(2-hydroxyethyl)-amino-phenyl)benzol; 5-Amino-2-(2,3-dihydroxypropyl)amino-1-(3-dimethylaminophenyl)benzol; 5-Amino-2-(2,3-dihydroxypropyl)amino-1-(3-fluorophenyl)benzol; 5-Amino-2-(2,3-dihydroxypropyl)amino-1-(3-formylphenyl)benzol; 5-Amino-2-(2,3-dihydroxypropyl)amino-1-(3-hydroxy-5-aminophenyl)-benzol; 5-Amino-2-(2,3-dihydroxypropyl)amino-1-(3-hydroxyphenyl)benzol; 5-Amino-2-(2,3-dihydroxypropyl)amino-1-(3-methoxyphenyl)benzol; 5-Amino-2-(2,3-dihydroxypropyl)amino-1-(3-nitrophenyl)benzol; 5-Amino-2-(2,3-dihydroxypropyl)amino-1-(3-trifluoromethylphenyl)benzol; 5-Amino-2-(2,3-dihydroxypropyl)amino-1-(4-(dimethylamino)phenyl)-benzol; 5-Amino-2-(2,3-dihydroxypropyl)amino-1-(4-(hydroxymethyl)phenyl)-benzol; 5-Amino-2-(2,3-dihydroxypropyl)amino-1-(4-(methylthio)phenyl)-benzol; 5-Amino-2-(2,3-dihydroxypropyl)amino-1-(4-(trifluoromethyl)phenyl)benzol; 5-Amino-2-(2,3-dihydroxypropyl)amino-1-(4-(trimethylsilyl)phenyl)benzol; 5-Amino-2-(2,3-dihydroxypropyl)amino-1-(4-acetylphenyl)benzol; 5-Amino-2-(2,3-dihydroxypropyl)amino-1-(4-aminophenyl)benzol; 5-Amino-2-(2,3-dihydroxypropyl)amino-1-(4-carbonsäurephenyl)benzol; 5-Amino-2-(2,3-dihydroxypropyl)amino-1-(4-chlorophenyl)benzol; 5-Amino-2-(2,3-dihydroxypropyl)amino-1-(4-di(2-hydroxyethyl)-amino-phenyl)benzol; 5-Amino-2-(2,3-dihydroxypropyl)-amino-1-(4-dimethylaminophenyl)benzol; 5-Amino-2-(2,3-dihydroxypropyl)amino-1-(4-ethoxyphenyl)benzol; 5-Amino-2-(2,3-dihydroxypropyl)amino-1-(4-fluorophenyl)benzol; 5-Amino-2-(2,3-dihydroxypropyl)amino-1-(4-formylphenyl)benzol; 5-Amino-2-(2,3-dihydroxypropyl)-amino-1-(4-hydroxyphenyl)benzol; 5-Amino-2-(2,3-dihydroxypropyl)amino-1-(4-methoxyphenyl)benzol; 5-Amino-2-(2,3-dihydroxypropyl)amino-1-(4-methylphenyl)benzol; 5-Amino-2-(2,3-dihydroxypropyl)amino-1-phenylbenzol; 5-Amino-2-(2-hydroxyethyl)amino-1-(4-bromophenyl)-benzol; 5-Amino-2-(2-hydroxyethyl)amino-1-(4-ethenylphenyl)benzol; 5-Amino-2-(2-hydroxyethyl)amino-1-(2,3,4-trimethoxyphenyl)benzol; 5-Amino-2-(2-hydroxyethyl)amino-1-(2,4-di(2-hydroxyethyl)aminophenyl)-benzol; 5-Amino-2-(2-hydroxyethyl)amino-1-(2,4-diaminophenyl)benzol; 5-Amino-2-(2-hydroxyethyl)amino-1-(2,4-dihydroxyphenyl)benzol; 5-Amino-2-(2-hydroxyethyl)amino-1-(2,4-dimethylaminophenyl)benzol; 5-Amino-2-(2-hydroxyethyl)amino-1-(2,4-methoxyphenyl)benzol; 5-Amino-2-(2-hydroxyethyl)amino-1-(2,5-di(2-hydroxyethyl)aminophenyl)-benzol; 5-Amino-2-(2-hydroxyethyl)amino-1-(2,5-diaminophenyl)benzol; 5-Amino-2-(2-hydroxyethyl)amino-1-(2,5-dihydroxyphenyl)benzol; 5-Amino-2-(2-hydroxyethyl)amino-1-(2,5-dimethoxyphenyl)benzol; 5-Amino-2-(2-hydroxyethyl)amino-1-(2,5-dimethylaminophenyl)benzol; 5-Amino-2-(2-hydroxyethyl)amino-1-(2,6-di(2-hydroxyethyl)aminophenyl)-benzol; 5-Amino-2-(2-hydroxyethyl)amino-1-(2,6-diaminophenyl)benzol; 5-Amino-2-(2-hydroxyethyl)amino-1-(2,6-dihydroxyphenyl)benzol; 5-Amino-2-(2-hydroxyethyl)amino-1-(2,6-dimethoxyphenyl)benzol; 5-Amino-2-(2-hydroxyethyl)amino-1-(2,6-dimethylaminophenyl)benzol; 5-Amino-2-(2-hydroxyethyl)amino-1-(2-(bromomethyl)phenyl)benzol; 5-Amino-2-(2-hydroxyethyl)amino-1-(2-amino-5-hydroxyphenyl)benzol; 5-Amino-2-(2-hydroxyethyl)amino-1-(2-aminophenyl)benzol; 5-Amino-2-(2-hydroxy-ethyl)amino-1-(2-carbonsäurephenyl)benzol; 5-Amino-2-(2-hydroxyethyl)-amino-1-(2-chlorophenyl)benzol; 5-Amino-2-(2-hydroxyethyl)amino-1-(2-di(2-hydroxyethyl)aminophenyl)-benzol; 5-Amino-2-(2-hydroxyethyl)amino-1-(2-dimethylaminophenyl)benzol; 5-Amino-2-(2-hydroxyethyl)amino-1-(2-fluorophenyl)-benzol; 5-Amino-2-(2-hydroxyethyl)amino-1-(2-formylphenyl)-benzol; 5-Amino-2-(2-hydroxyethyl)amino-1-(2-hydroxy-4-aminophenyl)-benzol; 5-Amino-2-(2-hydroxyethyl)amino-1-(2-hydroxy-5-aminophenyl)-benzol; 5-Amino-2-(2-hydroxyethyl)amino-1-(2-hydroxyphenyl)benzol; 5-Amino-2-(2-hydroxyethyl)amino-1-(2-methoxyphenyl)benzol; 5-Amino-2-(2-hydroxyethyl)amino-1-(2-methylphenyl)benzol; 5-Amino-2-(2-hydroxyethyl)amino-1-(2-nitrophenyl)benzol; 5-Amino-2-(2-hydroxyethyl)amino-1-(2-trifluoromethylphenyl)benzol; 5-Amino-2-(2-hydroxyethyl)amino-1-(3,5-di(2-hydroxyethyl)-aminophenyl)-benzol; 5-Amino-2-(2-hydroxyethyl)-amino-1-(3,5-diaminophenyl)benzol; 5-Amino-2-(2-hydroxyethyl)amino-1-(3,5-dihydroxyphenyl)benzol; 5-Amino-2-(2-hydroxyethyl)amino-1-(3,5-dimethoxyphenyl)benzol; 5-Amino-2-(2-hydroxyethyl)amino-1-(3,5-dimethylaminophenyl)benzol; 5-Amino-2-(2-hydroxyethyl)amino-1-(3-aminophenyl)benzol; 5-Amino-2-(2-hydroxyethyl)amino-1-(3-bromophenyl)benzol; 5-Amino-2-(2-hydroxyethyl)amino-1-(3-carbonsäurephenyl)benzol; 5-Amino-2-(2-hydroxyethyl)amino-1-(3-chlorophenyl)-benzol; 5-Amino-2-(2-hydroxyethyl)amino-1-(3-di(2-hydroxyethyl)-aminophenyl)-benzol; 5-Amino-2-(2-hydroxyethyl)amino-1-(3-dimethylaminophenyl)benzol; 5-Amino-2-(2-hydroxyethyl)amino-1-(3-fluorophenyl)benzol; 5-Amino-2-(2-hydroxyethyl)amino-1-(3-formylphenyl)benzol; 5-Amino-2-(2-hydroxyethyl)amino-1-(3-hydroxy-5-aminophenyl)benzol; 5-Amino-2-(2-hydroxyethyl)amino-1-(3-hydroxyphenyl)benzol; 5-Amino-2-(2-hydroxyethyl)amino-1-(3-methoxyphenyl)-benzol; 5-Amino-2-(2-hydroxyethyl)amino-1-(3-nitrophenyl)benzol; 5-Amino-2-(2-hydroxyethyl)amino-1-(3-trifluoromethylphenyl)benzol; 5-Amino-2-(2-hydroxyethyl)amino-1-(4-(dimethylamino)phenyl)benzol; 5-Amino-2-(2-hydroxyethyl)amino-1-(4-(hydroxymethyl)phenyl)benzol; 5-Amino-2-(2-hydroxyethyl)amino-1-(4-(methylthio)phenyl)benzol; 5-Amino-2-(2-hydroxyethyl)amino-1-(4-(trifluoromethyl)phenyl)benzol; 5-Amino-2-(2-hydroxyethyl)amino-1-(4-(trimethylsilyl)phenyl)benzol; 5-Amino-2-(2-hydroxyethyl)amino-1-(4-acetylphenyl)benzol; 5-Amino-2-(2-hydroxyethyl)amino-1-(4-aminophenyl)benzol; 5-Amino-2-(2-hydroxyethyl)amino-1-(4-carbonsäurephenyl)benzol; 5-Amino-2-(2-hydroxyethyl)-amino-1-(4-chlorophenyl)benzol; 5-Amino-2-(2-hydroxyethyl)amino-1-(4-di(2-hydroxyethyl)aminophenyl)-benzol; 5-Amino-2-(2-hydroxyethyl)amino-1-(4-dimethylaminophenyl)benzol; 5-Amino-2-(2-hydroxyethyl)amino-1-(4-ethoxyphenyl)benzol; 5-Amino-2-(2-hydroxyethyl)amino-1-(4-fluorophenyl)benzol; 5-Amino-2-(2-hydroxyethyl)amino-1-(4-formylphenyl)-benzol; 5-Amino-2-(2-hydroxyethyl)amino-1-(4-hydroxyphenyl)benzol; 5-Amino-2-(2-hydroxyethyl)amino-1-(4-methoxyphenyl)benzol; 5-Amino-2-(2-hydroxyethyl)amino-1-(4-methylphenyl)benzol; 5-Amino-2-(2-hydroxyethyl)amino-1-phenylbenzol; 5-Amino-2-(2-methoxyethyl)amino-1-(2,3,4-trimethoxyphenyl)benzol; 5-Amino-2-(2-methoxyethyl)amino-1-(2,4-diaminophenyl)benzol; 5-Amino-2-(2-methoxyethyl)amino-1-(2,4-dihydroxyphenyl)benzol; 5-Amino-2-(2-methoxyethyl)amino-1-(2,5-diaminophenyl)benzol; 5-Amino-2-(2-methoxyethyl)amino-1-(2,5-dimethoxyphenyl)benzol; 5-Amino-2-(2-methoxyethyl)amino-1-(2,6-dimethoxyphenyl)benzol; 5-Amino-2-(2-methoxyethyl)amino-1-(2-amino-5-hydroxyphenyl)benzol; 5-Amino-2-(2-methoxyethyl)amino-1-(2-aminophenyl)benzol; 5-Amino-2-(2-methoxyethyl)amino-1-(2-hydroxy-4-aminophenyl)benzol; 5-Amino-2-(2-methoxyethyl)amino-1-(2-hydroxy-5-aminophenyl)benzol; 5-Amino-2-(2-methoxyethyl)amino-1-(2-hydroxyphenyl)-benzot; 5-Amino-2-(2-methoxyethyl)amino-1-(2-methoxyphenyl)benzol; 5-Amino-2-(2-methoxyethyl)amino-1-(2-methylphenyl)benzol; 5-Amino-2-(2-methoxyethyl)amino-1-(3,5-diaminophenyl)benzol; 5-Amino-2-(2-methoxyethyl)amino-1-(3,5-dihydroxyphenyl)benzol; 5-Amino-2-(2-methoxyethyl)amino-1-(3-aminophenyl)benzol; 5-Amino-2-(2-methoxyethyl)amino-1-(3-hydroxy-5-aminophenyl)benzol; 5-Amino-2-(2-methoxyethyl)amino-1-(3-hydroxyphenyl)benzol; 5-Amino-2-(2-methoxyethyl)-amino-1-(3-methoxyphenyl)benzol; 5-Amino-2-(2-methoxyethyl)amino-1-(4-(dimethylamino)phenyl)benzol; 5-Amino-2-(2-methoxyethyl)amino-1-(4-(trifluoromethyl)phenyl)benzol; 5-Amino-2-(2-methoxyethyl)amino-1-(4-aminophenyl)benzol; 5-Amino-2-(2-methoxyethyl)amino-1-(4-carbonsäurephenyl)benzol; 5-Amino-2-(2-methoxyethyl)amino-1-(4-chlorophenyl)-benzol; 5-Amino-2-(2-methoxyethyl)amino-1-(4-hydroxyphenyl)benzol; 5-Amino-2-(2-methoxyethyl)amino-1-(4-methoxyphenyl)benzol; 5-Amino-2-(2-methoxyethyl)amino-1-(4-methylphenyl)benzol; 5-Amino-2-(2-methoxyethyl)amino-1-phenylbenzol; 5-Amino-2-di(2-hydroxyethyl)amino-1-(2,3,4-trimethoxyphenyl)benzol; 5-Amino-2-di(2-hydroxyethyl)amino-1-(2,3-difluoro-4-heptylphenyl)benzol; 5-Amino-2-di(2-hydroxyethyl)amino-1-(2,3-difluorophenyl)benzol; 5-Amino-2-di(2-hydroxyethyl)amino-1-(2,4,6-trimethylphenyl)benzol; 5-Amino-2-di(2-hydroxyethyl)amino-1-(2,4-diaminophenyl)benzol; 5-Amino-2-di(2-hydroxyethyl)amino-1-(2,4-dichlorophenyl)benzol; 5-Amino-2-di(2-hydroxyethyl)amino-1-(2,4-dihydroxyphenyl)benzol; 5-Amino-2-di(2-hydroxyethyl)amino-1-(2,5-dimethoxyphenyl)benzol; 5-Amino-2-di(2-hydroxyethyl)amino-1-(2,6-difluorophenyl)benzol; 5-Amino-2-di(2-hydroxyethyl)amino-1-(2,6-dimethoxyphenyl)benzol; 5-Amino-2-di(2-hydroxyethyl)amino-1-(2-((bis(1-methylethyl)amino)-carbonyl)phenyl)benzol; 5-Amino-2-di(2-hydroxyethyl)-amino-1-(2-((bis(1-methylethyl)amino)-carbonyl)-3-methoxyphenyl)benzol; 5-Amino-2-di(2-hydroxyethyl)amino-1-(2-(bromomethyl)phenyl)benzol; 5-Amino-2-di(2-hydroxyethyl)amino-1-(2-(diethylamino)carbonyl)phenyl)-benzol; 5-Amino-2-di(2-hydroxyethyl)amino-1-(2-carbonsäurephenyl)-benzol; 5-Amino-2-di(2-hydroxyethyl)amino-1-(2-chlorophenyl)benzol; 5-Amino-2-di(2-hydroxyethyl)amino-1-(2-fluorophenyl)benzol; 5-Amino-2-di(2-hydroxyethyl)amino-1-(2-formyl-4-methoxyphenyl)benzol; 5-Amino-2-di(2-hydroxyethyl)amino-1-(2-formyl-4-methylphenyl)benzol; 5-Amino-2-di(2-hydroxyethyl)amino-1-(2-formyl-5-methoxyphenyl)benzol; 5-Amino-2-di(2-hydroxyethyl)amino-1-(2-formylphenyl)benzol; 5-Amino-2-di(2-hydroxyethyl)amino-1-(2-hydroxy-4-aminophenyl)benzol; 5-Amino-2-di(2-hydroxyethyl)amino-1-(2-hydroxyphenyl)benzol; 5-Amino-2-di(2-hydroxy-ethyl)amino-1-(2-methoxyphenyl)benzol; 5-Amino-2-di(2-hydroxyethyl)-amino-1-(2-methylphenyl)benzol; 5-Amino-2-di(2-hydroxyethyl)amino-1-(2-nitrophenyl)benzol; 5-Amino-2-di(2-hydroxyethyl)amino-1-(2-trifluoro-methylphenyl)benzol; 5-Amino-2-di(2-hydroxyethyl)amino-1-(3,4-dichlorophenyl)benzol; 5-Amino-2-di(2-hydroxyethyl)amino-1-(3,5-diaminophenyl)benzol; 5-Amino-2-di(2-hydroxyethyl)amino-1-(3,5-dichlorophenyl)benzol; 5-Amino-2-di(2-hydroxyethyl)amino-1-(3,5-dihydroxyphenyl)benzol; 5-Amino-2-di(2-hydroxyethyl)amino-1-(3-(acetylamino)phenyl)benzol; 5-Amino-2-di(2-hydroxyethyl)amino-1-(3-aminophenyl)benzol; 5-Amino-2-di(2-hydroxyethyl)amino-1-(3-bromo-phenyl)benzol; 5-Amino-2-di(2-hydroxyethyl)amino-1-(3-carbonsäure-phenyl)benzol; 5-Amino-2-di(2-hydroxyethyl)amino-1-(3-chloro-4-fluorophenyl)benzol; 5-Amino-2-di(2-hydroxyethyl)amino-1-(3-chloro-phenyl)benzol; 5-Amino-2-di(2-hydroxyethyl)amino-1-(3-fluorophenyl)-benzol; 5-Amino-2-di(2-hydroxyethyl)amino-1-(3-formylphenyl)benzol;5-Amino-2-di(2-hydroxyethyl)amino-1-(3-hydroxyphenyl)benzol; 5-Amino-2-di(2-hydroxyethyl)amino-1-(3-methoxyphenyl)benzol; 5-Amino-2-di(2-hydroxyethyl)amino-1-(3-methylphenyl)benzol; 5-Amino-2-di(2-hydroxyethyl)amino-1-(3-nitrophenyl)benzol; 5-Amino-2-di(2-hydroxyethyl)amino-1-(3-trifluoromethylphenyl)benzol; 5-Amino-2-di(2-hydroxyethyl)amino-1-(4-(bromomethyl)phenyl)benzol; 5-Amino-2-di(2-hydroxyethyl)amino-1-(4-(dimethylamino)phenyl)benzol; 5-Amino-2-di(2-hydroxyethyl)amino-1-(4-(hydroxymethyl)phenyl)benzol; 5-Amino-2-di(2-hydroxyethyl)amino-1-(4-(methylthio)phenyl)benzol; 5-Amino-2-di(2-hydroxyethyl)amino-1-(4-(trifluoromethyl)phenyl)benzol; 5-Amino-2-di(2-hydroxyethyl)amino-1-(4-(trimethylsilyl)phenyl)benzol; 5-Amino-2-di(2-hydroxyethyl)amino-1-(4-acetylphenyl)benzol; 5-Amino-2-di(2-hydroxyethyl)amino-1-(4-amino-phenyl)benzol; 5-Amino-2-di(2-hydroxyethyl)amino-1-(4-bromophenyl)-benzol; 5-Amino-2-di(2-hydroxyethyl)amino-1-(4-carbonsäurephenyl)-benzol; 5-Amino-2-di(2-hydroxyethyl)amino-1-(4-chloro-3-methoxyphenyl)-benzol; 5-Amino-2-di(2-hydroxyethyl)amino-1-(4-chlorophenyl)benzol; 5-Amino-2-di(2-hydroxyethyl)amino-1-(4-ethenylphenyl)benzol; 5-Amino-2-di(2-hydroxyethyl)amino-1-(4-ethoxyphenyl)benzol; 5-Amina-2-di(2-hydroxyethyl)amino-1-(4-fluorophenyl)benzol; 5-Amino-2-di(2-hydroxy-ethyl)amino-1-(4-formylphenyl)benzol; 5-Amino-2-di(2-hydroxyethyl)-amino-1-(4-hydroxyphenyl)benzol; 5-Amino-2-di(2-hydroxyethyl)amino-1-(4-iodophenyl)benzol; 5-Amino-2-di(2-hydroxyethyl)amino-1-(4-methoxy-phenyl)benzol; 5-Amino-2-di(2-hydroxyethyl)amino-1-(4-methyl-3-nitrophenyl)benzol; 5-Amino-2-di(2-hydroxyethyl)amino-1-(4-methyl-phenyl)benzol; 5-Amino-2-di(2-hydroxyethyl)amino-1-(5-bromo-2-methoxy-phenyl)benzol; 5-Amino-2-di(2-hydroxyethyl)amino-1-(5-chloro-2-methoxy-phenyl)benzol; 5-Amino-2-di(2-hydroxyethyl)amino-1-(5-formyl-2-methoxy-phenyl)benzol; 5-Amino-2-di(2-methoxyethyl)amino-1-(2,3,4-trimethoxy-phenyl)benzol; 5-Amino-2-di(2-methoxyethyl)amino-1-(2,3-difluoro-4-heptylphenyl)benzol; 5-Amino-2-di(2-methoxyethyl)amino-1-(2,3-difluorophenyl)benzol; 5-Amino-2-di(2-methoxyethyl)amino-1-(2,4,6-trimethylphenyl)benzol; 5-Amino-2-di(2-methoxyethyl)amino-1-(2,4-diaminophenyl)benzol; 5-Amino-2-di(2-methoxyethyl)amino-1-(2,4-dichlorophenyl)benzol; 5-Amino-2-di(2-methoxyethyl)amino-1-(2,4-dihydroxyphenyl)benzol; 5-Amino-2-di(2-methoxyethyl)amino-1-(2,5-dimethoxyphenyl)benzol; 5-Amino-2-di(2-methoxyethyl)amino-1-(2,6-difluorophenyl)benzol; 5-Amino-2-di(2-methoxyethyl)amino-1-(2,6-dimethoxyphenyl)benzol; 5-Amino-2-di(2-methoxyethyl)amino-1-(2-((bis(1-methylethyl)amino)-carbonyl)phenyl)benzol; 5-Amino-2-di(2-methoxy-ethyl)amino-1-(2-((bis(1-methylethyl)amino)-carbonyl)-3-methoxyphenyl)-benzol; 5-Amino-2-di(2-methoxyethyl)amino-1-(2-(bromomethyl)phenyl)-benzol; 5-Amino-2-di(2-methoxyethyl)amino-1-(2-(diethylamino)carbonyl)-phenyl)-benzol; 5-Amino-2-di(2-methoxyethyl)amino-1-(2-carbonsäure-phenyl)benzol; 5-Amino-2-di(2-methoxyethyl)amino-1-(2-chlorophenyl)-benzol; 5-Amino-2-di(2-methoxyethyl)amino-1-(2-fluorophenyl)benzol; 5-Amino-2-di(2-methoxyethyl)amino-1-(2-formyl-4-methoxyphenyl)benzol; 5-Amino-2-di(2-methoxyethyl)amino-1-(2-formyl-4-methylphenyl)benzol; 5-Amino-2-di(2-methoxyethyl)amino-1-(2-formyl-5-methoxyphenyl)benzol; 5-Amino-2-di(2-methoxyethyl)amino-1-(2-formylphenyl)benzol; 5-Amino-2-di(2-methoxyethyl)amino-1-(2-hydroxy-4-aminophenyl)benzol; 5-Amino-2-di(2-methoxyethyl)amino-1-(2-hydroxyphenyl)benzol; 5-Amino-2-di(2-methoxyethyl)amino-1-(2-methoxyphenyl)benzol; 5-Amino-2-di(2-methoxyethyl)amino-1-(2-methylphenyl)benzol; 5-Amino-2-di(2-methoxyethyl)amino-1-(2-nitrophenyl)benzol; 5-Amino-2-di(2-methoxy-ethyl)amino-1-(2-trifluoromethylphenyl)benzol; 5-Amino-2-di(2-methoxy-ethyl)amino-1-(3,4-dichlorophenyl)benzol; 5-Amino-2-di(2-methoxyethyl)-amino-1-(3,5-diaminophenyl)benzol; 5-Amino-2-di(2-methoxyethyl)amino-1-(3,5-dichlorophenyl)benzol; 5-Amino-2-di(2-methoxyethyl)amino-1-(3,5-dihydroxyphenyl)benzol; 5-Amino-2-di(2-methoxyethyl)amino-1-(3-(acetylamino)phenyl)benzol; 5-Amino-2-di(2-methoxyethyl)amino-1-(3-aminophenyl)benzol; 5-Amino-2-di(2-methoxyethyl)amino-1-(3-bromophenyl)benzol; 5-Amino-2-di(2-methoxyethyl)amino-1-(3-carbonsäurephenyl)benzol; 5-Amino-2-di(2-methoxyethyl)amino-1-(3-chloro-4-fluorophenyl)benzol; 5-Amino-2-di(2-methoxyethyl)amino-1-(3-chlorophenyl)benzol; 5-Amino-2-di(2-methoxyethyl)amino-1-(3-fluoro-phenylbenzol; 5-Amino-2-di(2-methoxyethyl)amino-1-(3-formylphenyl)-benzol; 5-Amino-2-di(2-methoxyethyl)amino-1-(3-hydroxyphenyl)benzol; 5-Amino-2-di(2-methoxyethyl)amino-1-(3-methoxyphenyl)benzol; 5-Amino-2-di(2-methoxyethyl)amino-1-(3-methylphenyl)benzol; 5-Amino-2-di(2-methoxyethyl)amino-1-(3-nitrophenyl)benzol; 5-Amino-2-di(2-methoxy-ethyl)amino-1-(3-trifluoromethylphenyl)benzol; 5-Amino-2-di(2-methoxyethyl)amino-1-(4-(bromomethyl)phenyl)benzol; 5-Amino-2-di(2-methoxyethyl)amino-1-(4-(dimethylamino)phenyl)benzol; 5-Amino-2-di(2-methoxyethyl)amino-1-(4-(hydroxymethyl)phenyl)benzol; 5-Amino-2-di(2-methoxyethyl)amino-1-(4-(methylthio)phenyl)benzol; 5-Amino-2-di(2-methoxyethyl)amino-1-(4-(trifluoromethyl)phenyl)benzol; 5-Amino-2-di(2-methoxyethyl)amino-1-(4-(trimethylsilyl)phenyl)benzol; 5-Amino-2-di(2-methoxyethyl)amino-1-(4-acetylphenyl)benzol; 5-Amino-2-di(2-methoxy-ethyl)amino-1-(4-aminophenyl)benzol; 5-Amino-2-di(2-methoxyethyl)-amino-1-(4-bromophenyl)benzol; 5-Amino-2-di(2-methoxyethyl)amino-1-(4-carbonsäurephenyl)benzol; 5-Amino-2-di(2-methoxyethyl)amino-1-(4-chloro-3-methoxyphenyl)benzol; 5-Amino-2-di(2-methoxyethyl)amino-1-(4-chlorophenyl)benzol; 5-Amino-2-di(2-methoxyethyl)amino-1-(4-ethenyl-phenyl)benzol; 5-Amino-2-di(2-methoxyethyl)amino-1-(4-ethoxyphenyl)-benzol; 5-Amino-2-di(2-methoxyethyl)amino-1-(4-fluorophenyl)benzol; 5-Amino-2-di(2-methoxyethyl)amino-1-(4-formylphenyl)benzol; 5-Amino-2-di(2-methoxyethyl)amino-1-(4-hydroxyphenyl)benzol; 5-Amino-2-di(2-methoxyethyl)amino-1-(4-iodophenyl)benzol; 5-Amino-2-di(2-methoxy-ethyl)amino-1-(4-methoxyphenyl)benzol; 5-Amino-2-di(2-methoxyethyl)-amino-1-(4-methyl-3-nitrophenyl)benzol; 5-Amino-2-di(2-methoxyethyl)-amino-1-(4-methylphenyl)benzol; 5-Amino-2-di(2-methoxyethyl)amino-1-(5-bromo-2-methoxyphenyl)benzol; 5-Amino-2-di(2-methoxyethyl)amino-1-(5-chloro-2-methoxyphenyl)benzol; 5-Amino-2-di(2-methoxyethyl)amino-1-(5-formyl-2-methoxyphenyl)benzol; 5-Amino-2-dimethylamino-1-phenyl-benzol; 5-Amino-2-methylamino-1-(2,3,4-trimethoxyphenyl)benzol; 5-Amino-2-methylamino-1-(2,4-diaminophenyl)benzol; 5-Amino-2-methylamino-1-(2,4-dihydroxyphenyl)-benzol; 5-Amino-2-methylamino-1-(2,5-diaminophenyl)benzol; 5-Amino-2-methylamino-1-(2,5-dimethoxy-phenyl)benzol; 5-Amino-2-methylamino-1-(2,6-dimethoxyphenyl)benzol;5-Amino-2-methylamino-1-(2-amino-5-hydroxyphenyl)benzol; 5-Amino-2-methylamino-1-(2-aminophenyl)benzol; 5-Amino-2-methylamino-1-(2-hydroxy-4-aminophenyl)benzol; 5-Amino-2-methylamino-1-(2-hydroxy-5-aminophenyl)benzol; 5-Amino-2-methylamino-1-(2-hydroxyphenyl)benzol; 5-Amino-2-methylamino-1-(2-methoxyphenyl)benzol; 5-Amino-2-methyl-amino-1-(2-methylphenyl)-benzol; 5-Amino-2-methylamino-1-(3,5-diamino-phenyl)benzol; 5-Amino-2-methylamino-1-(3,5-dihydroxyphenyl)benzol; 5-Amino-2-methylamino-1-(3-aminophenyl)benzol; 5-Amino-2-methyl-amino-1-(3-hydroxy-5-aminophenyl)benzol; 5-Amino-2-methylamino-1-(3-hydroxyphenyl)benzol; 5-Amino-2-methylamino-1-(3-methoxyphenyl)-benzol; 5-Amino-2-methylamino-1-(4-(dimethylamino)phenyl)benzol; 5-Amino-2-methylamino-1-(4-(trifluoromethyl)phenyl)benzol; 5-Amino-2-methylamino-1-(4-aminophenyl)benzol; 5-Amino-2-methylamino-1-(4-carbonsäurephenyl)benzol; 5-Amino-2-methylamino-1-(4-chlorophenyl)-benzol; 5-Amino-2-methylamino-1-(4-hydroxyphenyl)benzol; 5-Amino-2-methylamino-1-(4-methoxyphenyl)benzol; 5-Amino-2-methylamino-1-(4-methylphenyl)benzol und5-Amino-2-methylamino-1-phenylbenzol, oder deren physiologisch verträglichen Salzen enthält.

Besonders hervorragend im Sinne der Gesamterfindung geeignete 2,5-Diamino-1-phenylbenzol-Derivate der Formel (I) sind 2,5-Diamino-1-phenyl-benzol; 2,5-Diamino-1-(3-nitro-phenyl)benzol; 2,5-Diamino-1-(4-methoxy-phenyl)benzol; 2,5-Diamino-1-(3-methoxy-phenyl)benzol; 2,5-Diamino-1-(3-amino-phenyl)benzol; 2,5-Diamino-1-(2-methyl-phenyl)-benzol; 2,5-Diamino-1-(3-methyl-phenyl)-benzol; 2,5-Diamino-1-(4-methylphenyl)benzol; 2,5-Diamino-1-(3-chlor-phenyl)benzol und 2,5-Diamino-1-(4-chlor-phenyl)benzol, oder deren physiologisch verträgliche Salze.

Die vorgenannten 2,5-Diamino-1-phenylbenzol-Derivate können sowohl als freie Basen als auch in Form ihrer physiologisch verträglichen Salze mit anorganischen oder organischen Säuren, wie zum Beispiel Salzsäure, Schwefelsäure, Phosphorsäure, Essigsäure, Propionsäure, Milchsäure oder Zitronensäure, eingesetzt werden.

Das erfindungsgemäße 2,5-Diamino-1-phenylbenzol-Derivat ist in dem erfindungsgemäßen Färbemittel in einer Menge von etwa 0,005 bis 20 Gewichtsprozent enthalten, wobei eine Menge von etwa 0,01 bis 5,0 Gewichtsprozent und insbesondere 0,1 bis 2,5 Gewichtsprozent bevorzugt ist.

Als Kupplersubstanzen kommen vorzugsweise 2,6-Diamino-pyridin, 2-Amino-4-[(2-hydroxyethyl)amino]-anisol, 2,4-Diamino-1-fluor 5-methylbenzol, 2,4-Diamino-1-methoxy-5-methyl-benzol, 2,4-Diamino-1-ethoxy-5-methyl-benzol, 2,4-Diamino-1-(2-hydroxyethoxy)-5-methyl-benzol, 2,4-Di[(2-hydroxyethyl)amino]-1,5-dimethoxy-benzol, 2,3-Diamino-6-methoxydimethoxy-pyridin, 3,5-Diamino-2,6-dimethoxy-pyridin, 1,3-Diaminobenzol, 2,4-Diamino-1-(2-hydroxyethoxy)-benzol, 2,4-Diamino-1,5-di(2-hydroxyethoxy)-benzol, 1-(2-Aminoethoxy)-2,4-diamino-benzol, 2-Amino-1-(2-hydroxyethoxy)-4-methylamino-benzol, 2,4-Diaminophenoxyessigsäure, 3-[Di(2-hydroxyethyl)amino]-anilin, 4-Amino-2-di[(2-hydroxyethyl)amino]-1-ethoxy-benzol, 5-Methyl-2-(1-methylethyl)-phenol, 3-[(2-Hydroxyethyl)amino]-anilin, 3-[(2-Aminoethyl)amino]-anilin, 1,3-Di(2,4-diaminophenoxy)-propan, Di(2,4-diaminophenoxy)-methan, 1,3-Diamino-2,4-dimethoxy-benzol, 2,6-Bis(2-hydroxyethyl)amino-toluol, 4-Hydroxyindol, 3-Dimethylamino-phenol, 3-Diethylamino-phenol, 5-Amino-2-methyl-phenol, 5-Amino-4-fluor-2-methyl-phenol, 5-Amino-4-methoxy-2-methyl-phenol, 5-Amino-4-ethoxy-2-methyl-phenol, 3-Amino-2,4-dichlorphenol, 5-Amino-2,4-dichlor-phenol, 3-Amino-2-methyl-phenol, 3-Amino-2-chlor-6-methyl-phenol, 3-Amino-phenol, 2-[(3-Hydroxyphenyl)amino]-acetamid, 5-[(2-Hydroxyethyl)amino]-2-methyl-phenol, 3-[(2-Hydroxyethyl)amino]-phenol, 3-[(2-Methoxyethyl)amino]-phenol, 5-Amino-2-ethyl-phenol, 2-(4-Amino-2-hydroxyphenoxy)-ethanol, 5-[(3-Hydroxypropyl)amino]-2-methyl-phenol, 3-[(2,3-Dihydroxypropyl)amino]-2-methyl-phenol, 3-[(2-Hydroxyethyl)amino]-2-methyl-phenol, 2-Amino-3-hydroxy-pyridin, 5-Amino-4-chlor-2-methyl-phenol, 1-Naphthol, 1,5-Dihydroxy-naphthalin, 1,7-Dihydroxy-naphthalin, 2,3-Dihydroxy-naphthalin, 2,7-Dihydroxy-naphthalin, 2-Methyl-1-naphthol-acetat, 1,3-Dihydroxy-benzol, 1-Chlor-2,4-dihydroxy-benzol, 2-Chlor-1,3-dihydroxybenzol, 1,2-Dichlor-3,5-dihydroxy-4-methyl-benzol, 1,5-Dichlor-2,4-dihydroxy-benzol, 1,3-Dihydroxy-2-methyl-benzol, 3,4-Methylendioxyphenol, 3,4-Methylendioxy-anilin, 5-[(2-Hydroxyethyl)amino]-1,3-benzodioxol, 6-Brom-1-hydroxy-3,4-methylendioxy-benzol, 3,4-Diaminobenzoesäure, 3,4-Dihydro-6-hydroxy-1,4(2H)-benzoxazin, 6-Amino-3,4-dihydro-1,4(2H)-benzoxazin, 3-Methyl-1-phenyl-5-pyrazolon, 5,6-Dihydroxy-indol, 5,6-Dihydroxy-indolin, 5-Hydroxy-indol, 6-Hydroxyindol,7-Hydroxy-indol und 2,3-Indolindion in Betracht.

Obwohl die vorteilhaften Eigenschaften der hier beschriebenen 2,5-Diaminobenzol-Derivate es nahelegen, diese als alleinige Entwicklersubstanz zu verwenden, ist es selbstverständlich auch möglich, diese 2,5-Diaminobenzol-Derivate gemeinsam mit bekannten Entwicklersubstanzen, wie zum Beispiel 1,4-Diaminobenzol, 2,5-Diamino-toluol, 2,5-Diaminophenylethylalkohol, 4-Aminophenol und seinen Derivaten, beispielsweise 4-Amino-3-methylphenol, 4,5-Diamino-1-(2-hydroxyethyl)-pyrazol oder Tetraaminopyrimidinen, einzusetzen.
Die Kupplersubstanzen und Entwicklersubstanzen können in dem erfindungsgemäßen Färbemittel jeweils einzeln oder im Gemisch miteinander enthalten sein, wobei die Gesamtmenge an Kupplersubstanzen und Entwicklersubstanzen in dem erfindungsgemäßen Färbemittel (bezogen auf die Gesamtmenge des Färbemittels) jeweils etwa 0,005 bis 20 Gewichtsprozent, vorzugsweise etwa 0,01 bis 5,0 Gewichtsprozent und insbesondere 0,1 bis 2,5 Gewichtsprozent, beträgt. Die Gesamtmenge der in dem hier beschriebenen Färbemittel enthaltenen Entwicklersubstanz-Kupplersubstanz-Kombination beträgt vorzugsweise etwa 0,01 bis 20 Gewichtsprozent, wobei eine Menge von etwa 0,02 bis 10 Gewichtsprozent und insbesondere 0,2 bis 6,0 Gewichtsprozent besonders bevorzugt ist. Die Entwicklersubstanzen und Kupplersubstanzen werden im allgemeinen in etwa äquimolaren Mengen eingesetzt; es ist jedoch nicht nachteilig, wenn die Entwicklersubstanzen diesbezüglich in einem gewissen Uberschuß oder Unterschuß (beispielsweise in einem Verhältnis (Kuppler: Entwickler) von 1:2 bis 1:0,5) vorhanden sind.

Weiterhin kann das erfindungsgemäße Färbemittel zusätzlich andere Farbkomponenten, beispielsweise 6-Amino-2-methylphenol und 2-Amino-5-methylphenol, sowie ferner übliche direktziehende Farbstoffe, zum Beispiel Triphenylmethanfarbstoffe wie 4-[(4'-aminophenyl)-(4'imino-2",5"cyclohexadien-1"-yliden)-methyl]-2-methylaminobenzol-monohydrochlorid (C.I. 42 510) und 4-[(4'amino-3'-methyl-phenyl)-(4"-imino-3"-methyl-2",5"cyclohexadien-1"-yliden)-methyl]-2-methyl-aminobenzol monohydrochlorid (C.I. 42 520), aromatische Nitrofarbstoffe wie 4-(2'hydroxyethyl)amino-nitrotoluol, 2-Amino-4,6-dinitrophenol, 2-Amino-5-(2'hydroxyethyl)amino-nitrobenzol, 2-Chlor-6-(ethylamino)-4-nitrophenof, 4-Chlor-N-(2-hydroxyethyl-2-nitroanilin, 5-Chlor-2-hydroxy-4-nitroanilin, 2-Amino-4-chlor-6-nitrophenol und 1-[(2'-Ureidoethyl)amino-4-nitrobenzol, Azofarbstoffe wie 6-[(4'-Aminophenyl)azo]-5-hydroxy-naphthalin-1-sulfonsäure-Natriumsalz (C.I. 14 805) und Dispersionsfarbstoffe wie beispielsweise 1,4-Diaminoanthrachinon und 1,4,5,8-Tetraaminoantrachinon, enthalten. Die Färbemittel können diese Farbkomponenten in einer Menge von etwa 0,1 bis 4,0 Gewichtsprozent enthalten.

Selbstverständlich können die Kupplersubstanzen und Entwicklersubstanzen sowie die anderen Farbkomponenten, sofern es Basen sind, auch in Form der physiologisch verträglichen Salze mit organischen oder anorganischen Säuren, wie beispielsweise Salzsäure oder Schwefelsäure, beziehungsweise - sofern sie aromatische OH-Gruppen besitzen - in Form der Salze mit Basen, zum Beispiel als Alkaliphenolate, eingesetzt werden.

Darüber hinaus können in den Färbemitteln, falls diese zur Färbung von Haaren verwendet werden sollen, noch weitere übliche kosmetische Zusätze, beispielsweise Antioxidantien wie Ascorbinsäure, Thioglykolsäure oder Natriumsulfit, sowie Parfümöle, Komplexbildner, Netzmittel, Emulgatoren, Verdicker und Pflegestoffe enthalten sein.
Die Zubereitungsform des erfindungsgemäßen Färbemittels kann beispielsweise eine Lösung, insbesondere eine wäßrige oder wäßrigalkoholische Lösung sein. Die besonders bevorzugten Zubereitungsformen sind jedoch eine Creme, ein Gel oder eine Emulsion. Ihre Zusammensetzung stellt eine Mischung der Farbstoffkomponenten mit den für solche Zubereitungen üblichen Zusätzen dar.

Ubliche Zusätze in Lösungen, Cremes, Emulsionen oder Gelen sind zum Beispiel Lösungsmittel wie Wasser, niedere aliphatische Alkohole, beispielsweise Ethanol, Propanol oder Isopropanol, Glycerin oder Glykole wie 1,2-Propylenglykol, weiterhin Netzmittel oder Emulgatoren aus den Klassen der anionischen, kationischen, amphoteren oder nichtionogenen oberflächenaktiven Substanzen wie zum Beispiel Fettalkoholsulfate, oxethylierte Fettalkoholsulfate, Alkylsulfonate, Alkylbenzolsulfonate, Alkyltrimethylammoniumsalze, Alkylbetaine, oxethylierte Fettalkohole, oxethylierte Nonylphenole, Fettsäurealkanolamide und oxethylierte Fettsäureester ferner Verdicker wie höhere Fettalkohole, Stärke, Cellulosederivate, Petrolatum, Paraffinöl und Fettsäuren, sowie außerdem Pflegestoffe wie kationische Harze, Lanolinderivate, Cholesterin, Pantothensäure und Betain. Die erwähnten Bestandteile werden in den für solche Zwecke üblichen Mengen verwendet, zum Beispiel die Netzmittel und Emulgatoren in Konzentrationen von etwa 0,5 bis 30 Gewichtsprozent, die Verdicker in einer Menge von etwa 0,1 bis 25 Gewichtsprozent und die Pflegestoffe in einer Konzentration von etwa 0,1 bis 5,0 Gewichtsprozent.

Je nach Zusammensetzung kann das erfindungsgemäße Färbemittel schwach sauer, neutral oder alkalisch reagieren. Insbesondere weist es einen pH-Wert von 6,8 bis 11,5 auf, wobei die basische Einstellung vorzugsweise mit Ammoniak erfolgt. Es können aber auch organische Amine, zum Beispiel Monoethanolamin und Triethanolamin, oder auch anorganische Basen wie Natriumhydroxid und Kaliumhydroxid Verwendung finden. Für eine pH-Einstellung im sauren Bereich kommen anorganische oder organische Säuren, zum Beispiel Phosphorsäure, Essigsäure Zitronensäure oder Weinsäure, in Betracht.

Für die Anwendung zur oxidativen Färbung von Haaren vermischt man das vorstehend beschriebene Färbemittel unmittelbar vor dem Gebrauch mit einem Oxidationsmittel und trägt eine für die Haarfärbebehandlung ausreichende Menge, je nach Haarfülle, im allgemeinen etwa 60 bis 200 Gramm, dieses Gemisches auf das Haar auf.

Als Oxidationsmittel zur Entwicklung der Haarfärbung kommen hauptsächlich Wasserstoffperoxid oder dessen Additionsverbindungen an Harnstoff, Melamin, Natriumborat oder Natriumcarbonat in Form einer 3-bis 12prozentigen, vorzugsweise 6prozentigen, wässrigen Lösung, aber auch Luftsauerstoff in Betracht. Wird eine 6prozentige Wasserstoffperoxid-Lösung als Oxidationsmittel verwendet, so beträgt das Gewichtsverhältnis zwischen Haarfärbemittel und Oxidationsmittel 5:1 bis 1:2, vorzugeweise jedoch 1:1. Größere Mengen an Oxidationsmittel werden vor allem bei höheren Farbstoffkonzentrationen im Haarfärbemittel, oder wenn gleichzeitig eine stärkere Bleichung des Haares beabsichtigt ist, verwendet. Man läßt das Gemisch bei 15 bis 50 Grad Celsius etwa 10 bis 45 Minuten lang, vorzugsweise 30 Minuten lang, auf das Haar einwirken, spült sodann das Haar mit Wasser aus und trocknet es. Gegebenenfalls wird im Anschluß an diese Spülung mit einem Shampoo gewaschen und eventuell mit einer schwachen organischen Säure, wie zum Beispiel Zitronensäure oder Weinsäure, nachgespült. Anschließend wird das Haar getrocknet.

Die erfindungsgemäßen Färbemittel mit einem Gehalt an den erfindungsgemäßen 2,5-Diaminobenzol-Derivaten als Entwicklersubstanz ermöglichen Färbungen mit ausgezeichneter Farbechtheit, insbesondere was die Lichtechtheit, Waschechtheit und Reibeechtheit anbetrifft. Hinsichtlich der färberischen Eigenschaften bieten die erfindungsgemäßen Färbemittel je nach Art und Zusammensetzung der Farb-komponenten eine breite Palette verschiedener Farbnuancen, welche sich von blonden über braune, purpume, violette bis hin zu blauen und schwarzen Farbtönen erstreckt. Hierbei zeichnen sich die Farbtöne durch ihre besondere Farbintensität aus. Die sehr guten färberischen Eigenschaften der Färbemittel gemäß der vorliegenden Anmeldung zeigen sich weiterhin darin, daß diese Mittel insbesondere auch eine Anfärbung von ergrauten, chemisch nicht vorgeschädigten Haaren problemlos und mit guter Deckkraft ermöglichen. Die in dem erfindungsgemäßen Mittel verwendeten 2,5-Diamino-1-phenylbenzol-Derivate sind gut in Wasser löslich und ermöglichen Färbungen mit hoher Farbintensität und ausgezeichneter Farbechtheit, insbesondere was die Lichtechtheit, Waschechtheit und Reibeechtheit anbetrifft. Sie weisen weiterhin eine ausgezeichnete Lagerstabilität, insbesondere als Bestandteil der vorstehend beschriebenen Färbemittel, auf.

Ein weiterer Gegenstand der vorliegenden Erfindung sind die in den nachfolgenden Patentansprüchen beschriebenen neuen 2,5-Diamino-1-phenylbenzol-Derivate oder deren physiologisch verträgliche, wasserlösliche Salze.

Die Herstellung der erfindungsgemäßen 2,5-Diamino-1-phenylbenzol-Derivate kann unter Verwendung von bekannten Syntheseverfahren, beispielsweise dem in den Ausführungsbeispielen beschriebenen Verfahren, erfolgen.

Die nachfolgenden Beispiele sollen den Gegenstand der Erfindung näher erläutern, ohne ihn darauf zu beschränken.

### Beispiele

### Beispiel 1: Synthese von 2,5-Diamino-1-phenylbenzol-Derivaten (Allgemeine Synthesevorschrift)

### A. Synthese von 2,5-tert.-Butyloxycarbonylamino-brombenzol

15,65g (0,07 mol) Brom-p-phenylendiamin-Hydrochlorid und 32,7 g (0,15 mol) Di-tert.-butyl-dicarbonat werden in einer Mischung von 250 ml 2N Natriunhydroxide und 250 ml Trifluortoluol gelöst und auf 45 °C erwärmt. Die Reaktionmischung wird 3 Tage gerührt. Schrittweise werden noch insgesamt 30 g (0,14 mol) Di-tert.-butyl-dicarbonat zugegeben. Anschließend wird die organische Schicht abgetrennt und die wäßrige Phase noch zweimal mit 100ml Dichlormethan extrahiert. Die vereinigten Extrakte weren eingedampft und der Rückstand in 200 ml Hexan aufgenommen. Der Niederschlag wird abfiltriert und mit 50 ml Hexan nachgewaschen.
Es werden 18,6 g (82 % der Theorie) 2,5-tert.-Butyloxycarbonylaminobrombenzol mit einem Schmelzpunkt von 130 °C erhalten.

### B. Synthese von 2,5-Diamino-1-phenylbenzol-Derivaten

3,3 g (0,01 mol) 2,5-tert.-Butyloxycarbonylamino-brombenzol aus Stufe **A** und 0,013 mol der entsprechenden Borsäure werden unter Argon in 70 ml 1,2-Dimethoxyethan gelöst. Anschließend werden 0,5 g Tetrakis(triphenylphosphin)-palladium (0,0005 mol) und 13 ml 2N Kaliumcarbonatlösung zugegeben und die Reaktionsmischung auf 80 °C erwärmt. Nach Beendigung der Reaktion wird die Reaktionsmischung in 100 ml Essigsäureethylester gegossen, die organische Phase mit verdünnter Natronlauge extrahiert und sodann mit Magnesiumsulfat getrocknet. Das Lösungsmittel wird am Rotationsverdampfer abdestilliert und der Rückstand an Kieselgel mit Petrolether/Essigsäureethylester (9:1) gereinigt. Das so erhaltene Produkt wird in 40 ml Ethanol auf 50 °C erwärmt. Anschließend werden zur Herstellung des Hydrochlorides 15 ml einer 2,9 molaren ethanolische Salzsäurelösung zugetropft. Der Niederschlag wird abfiltriert, zweimal mit 10 ml Ethanol gewaschen und sodann getrocknet.

### a. 2,5-Diamino-1-phenylbenzol-dihydrochlorid

Verwendete Borsäure: Benzolborsäure
Ausbeute: 1,8 g (= 72 Prozent der Theorie)
Schmelzpunkt: 250 °Celsius (farblose Kristalle)

| CHN-Analyse: | | | |
|---|---|---|---|
| (C₁₂H₁₄N₂Cl₂) | % C | % H | % N |
| berechnet | 56,05 | 5,49 | 10,89 |
| gefunden | 55,95 | 5,46 | 10,58 |

### b. 2,5-Diamino-1-(3-nitro-phenyl)benzol-dihydrochlorid

Verwendete Borsäure: 3-Nitro-benzolborsäure
Ausbeute: 1,9 g (= 65 Prozent der Theorie)
Schmelzpunkt: 245 °Celsius (farblose Kristalle)

| CHN-Analyse: | | | |
|---|---|---|---|
| (C₁₂H₁₃N₃O₂Cl₂) | % C | % H | % N |
| berechnet | 47,70 | 4,34 | 13,91 |
| gefunden | 47,89 | 4,28 | 13,78 |

### c. 2,5-Diamino-1-(3-methoxy-phenyl)benzol-dihydrochlorid

Verwendete Borsäure: 3-Methoxy-benzolborsäure
Ausbeute: 2,01g (= 72 Prozent der Theorie)
Schmelzpunkt: 255 °Celsius (farblose Kristalle)

| CHN-Analyse: | | | |
|---|---|---|---|
| (C₁₃H₁₆N₂OCl₂) | % C | % H | % N |
| berechnet | 54,37 | 5,62 | 9,75 |
| gefunden | 54,25 | 5,59 | 9,60 |

### d. 2,5-Diamino-1-(4-methoxy-phenyl)benzol-dihydrochlorid

Verwendete Borsäure: 4-Methoxy-benzolborsäure
Ausbeute: 2,2 g (= 77 Prozent der Theorie)
Schmelzpunkt: 250 °Celsius (farblose Kristalle)

| CHN-Analyse: | | | |
|---|---|---|---|
| (C₁₃H₁₆N₂OCl₂) | % C | % H | % N |
| berechnet | 54,37 | 5,62 | 9,75 |
| gefunden | 54,51 | 5,32 | 9,64 |

### e. 2,5-Diamino-1-(3-amino-phenyl)benzol-dihydrochlorid

Verwendete Borsäure: 3-Amino-benzolborsäure
Ausbeute: 0,6 g (= 20 Prozent der Theorie)
Schmelzpunkt: 260 °Celsius (farblose Kristalle)

| CHN-Analyse: | | | |
|---|---|---|---|
| (C₁₂H₁₆N₃Cl₃) | % C | % H | % N |
| berechnet | 46,7 | 5,23 | 13,61 |
| gefunden | 46,47 | 5,48 | 11,94 |

### C. Synthese von N,N'-Bis(tert-Butoxycarbonyl)-2,5-diamino-1-phenylborsäure

Die N,N'-Bis(tert-Butoxycarbonyl)-2,5-diamino-1-phenylborsäure werden durch Umsetzung von N,N'-Bis(tert-Butoxycarbonyl)-2,5-diamino-1-brombenzol mit tert-Butyllithium und Trimethylborate dargestellt. Die experimentelle Vorschrift dieser Herstellungsmethode wird von J. M. Tour und J. J. S: Lamba in J. Am. Chem. Soc.1994,116 Seite 11723 beschrieben.

### D. Synthese von 2,5-Diamino-1-(phenyl)-benzolen

0,035 g (0,0001 mol) 2,5-tert.-Butyloxycarbonylamino-1-phenylborsäure aus Stufe **C** und 0,00015 mol des entsprechenden Bromderivates werden unter Argon in 10 ml 1,2-Dimethoxyethan gelöst. Anschließend werden 0,005 g Tetrakis-(triphenylphosphin)-palladium (0,000005 mol) und 0,13 ml 2N Kaliumcarbonat-lösung zugegeben und die Reaktionsmischung auf 80 °C erwärmt. Nach Beendigung der Reaktion wird die Reaktionsmischung in 10 ml Essigsäureethylester gegossen, die organische Phase mit verdünnter Natronlauge extrahiert und sodann mit Magnesiumsulfat getrocknet. Das Lösungsmittel wird am Rotationsverdampfer abdestilliert und der Rückstand an Kieselgel mit Petrolether/Essigsäureethylester (9:1) gereinigt. Das so erhaltene Produkt wird in 4 ml Ethanol auf 50 °C erwärmt.

Anschließend werden zur Herstellung des Hydrochlorides 1,5 ml einer 2,9 molaren ethanolische Salzsäurelösung zugetropft. Der Niederschlag wird abfiltriert, zweimal mit 1 ml Ethanol gewaschen und sodann getrocknet.

### a. 2,5-Diamino-1-(4-nitro-phenyl)benzol-dihydrochlorid

Verwendete Bromderivat: 1-Brom-4-nitro-benzol
Ausbeute: 0,025 g ( 80 % der Theorie)
Masspektren MH⁺ 230 (100)

### b. 2,5-Diamino-1-(4-chlor-phenyl)benzol-dihydrochlorid

Verwendete Bromderivat: 1-Brom-4-chlor-benzol
Ausbeute: 0,025 g (86 % der Theorie)
Masspektren MH⁺ 219 (100)

### c. 2,5-Diamino-1-(4-trifluormethyl-phenyl)benzol-dihydrochlorid

Verwendete Bromderivat: 1-Brom-4-trifluormethyl-benzol
Ausbeute: 0,025 g ( 78 % der Theorie)
Masspektren MH⁺ 253 (100)

### d. 2,5-Diamino-1-(4-cyan-phenyl)benzol-dihydrochlorid

Verwendete Bromderivat: 1-Brom-4-cyan-benzol
Ausbeute: 0,025 g (90 % der Theorie)
Masspektren MH⁺ 210 (100)

### e. 2,5-Diamino-1-(4-methyl-phenyl)benzol-dihydrochlorid

Verwendete Bromderivat: 1-Brom-4-methyl-benzol
Ausbeute: 0,025 g ( 92 % der Theorie)
Masspektren MH⁺ 199 (100)

### f. 2,5-Diamino-1-(4-hydroxy-phenyl)benzol-dihydrochlorid

Verwendete Bromderivat: 4-Brom-phenol
Ausbeute: 0,025 g (92 % der Theorie)
Masspektren MH⁺ 201 (100)

### g. 2,5-Diamino-1-(4-amino-phenyl)benzol-dihydrochlorid

Verwendete Bromderivat: 4-Brom-anilin
Ausbeute: 0,025 g ( 90 % der Theorie)
Masspektren MH⁺ 200 (100)

### h. 2,5-Diamino-1-(4-carbonsäure-ethylester-phenyl)benzol-dihydrochlorid

Verwendete Bromderivat: 1-Brom-benzoesäure-ethylester
Ausbeute: 0,025 g ( 75 % der Theorie)
Masspektren MH⁺ 257 (100)

### i. 2,5-Diamino-1-(3-nitro-4-trifluormetyl-phenyl)benzol-dihydrochlorid

Verwendete Bromderivat: 1-Brom-3-nitro-4-trifluormethyl-benzol
Ausbeute: 0,025 g ( 65 % der Theorie)
Masspektren MH⁺ 298 (100)

### j. 2,5-Diamino-1-(4-phenoxy-phenyl)benzol-dihydrochlorid

Verwendete Bromderivat: 1-Brom-4-phenoxy-benzol
Ausbeute: 0,025 g ( 70 % der Theorie)
Masspektren MH⁺ 277 (100)

### k. 2,5-Diamino-1-(2-methoxy-5-cyan-phenyl)benzol-dihydrochlorid

Verwendete Bromderivat: 1-Brom-2-methoxy-5-cyan-benzol
Ausbeute: 0,025 g ( 80 % der Theorie)
Masspektren MH⁺ 240 (100)

### l. 2,5-Diamino-1-(3-nitro-4-methyl-phenyl)benzol-dihydrochlorid

Verwendete Bromderivat: 1-Brom-3-nitro-4-methyl-benzol
Ausbeute: 0,025 g ( 78 % der Theorie)
Masspektren MH⁺ 244 (100)

### m. 2,5-Diamino-1-(2-nitro-4-methyl-phenyl)benzol-dihydrochlorid

Verwendete Bromderivat: 1-Brom-2-nitro-4-methyl-benzol
Ausbeute: 0,025 g ( 78 % der Theorie)
Masspektren MH⁺ 244 (100)

### n. 2,5-Diamino-1-(3-trifluormethyl-4-nitro-phenyl)benzol-dihydrochlorid

Verwendete Bromderivat: 1-Brom-3-trifluormethyl-4-nitro-benzol
Ausbeute: 0,025 g ( 67% der Theorie)
Masspektren MH⁺ 298 (100)

### o. 2,5-Diamino-1-(2,4-dimethoxy-phenyl)benzol-dihydrochlorid

Verwendete Bromderivat: 1-Brom-2,4-dimethoxy-benzol
Ausbeute: 0,025 g ( 78 % der Theorie)
Masspektren MH⁺ 245 (100)

### p. 2,5-Diamino-1-(2-methyl-3-nitro-phenyl)benzol-dihydrochlorid

Verwendete Bromderivat: 1-Brom-2-methyl-3-nitro-benzol
Ausbeute: 0,025 g ( 78 % der Theorie)
Masspektren MH⁺ 244 (100)

### q. 2,5-Diamino-1-(3,4-dimethoxy-phenyl)benzol-dihydrochlorid

Verwendete Bromderivat: 1-Brom-3,4-dimethoxy-benzol
Ausbeute: 0,025 g ( 78 % der Theorie)
Masspektren MH⁺ 245 (100)

### r. 2,5-Diamino-1-(2,5-dimethoxy-phenyl)benzol-dihydrochlorid

Verwendete Bromderivat: 1-Brom-2,5-dimethoxy-benzol
Ausbeute: 0,025 g ( 78 % der Theorie)
Masspektren MH⁺ 245 (100)

### s. 2,5-Diamino-1-(2-methyl-4-nitro-phenyl)benzol-dihydrochlorid

Verwendete Bromderivat: 1-Brom-2-methyl-4-nitro-benzol
Ausbeute: 0,025 g ( 78 % der Theorie)
Masspektren MH⁺ 244 (100)

### t. 2,5-Diamino-1-(2-methyl-5-nitro-phenyl)benzol-dihydrochlorid

Verwendete Bromderivat: 1-Brom-2-methyl-5-nitro-benzol
Ausbeute: 0,025 g ( 78 % der Theorie)
Masspektren MH⁺ 244 (100)

### u. 2,5-Diamino-1-(4-(1-oxy-pentyl)-phenyl)benzol-dihydrochlorid

Verwendete Bromderivat: 1-(4-Brom-phenyl)-1-pentan-1-one
Ausbeute: 0,025 g (73 % der Theorie)
Masspektren MH⁺ 269 (100)

### v. 2,5-Diamino-3-(biphenyl)benzol-dihydrochlorid

Verwendete Bromderivat: 3-Brom-biphenyl
Ausbeute: 0,025 g (76 % der Theorie)
Masspektren MH⁺ 261 (100)

### w. 2,5-Diamino-1-(2,5-dimethyl-phenyl)benzol-dihydrochlorid

Verwendete Bromderivat: 1-Brom-2,5-dimethyl-benzol
Ausbeute: 0,025 g ( 89 % der Theorie)
Masspektren MH⁺ 213 (100)

### x. 2,5-Diamino-1-(2-chlor-5-nitro-phenyl)benzol-dihydrochlorid

Verwendete Bromderivat: 1-Brom-2-chlor-5-nitro-benzol
Ausbeute: 0,025 g ( 73 % der Theorie)
Masspektren MH⁺ 265 (100)

### y. 2,5-Diamino-1-(2-methyl-4-hydroxy-phenyl)benzol-dihydrochlorid

Verwendete Bromderivat: 1-Brom-2-methyl-4-hydroxy-benzol
Ausbeute: 0,025 g ( 86 % der Theorie)
Masspektren MH⁺ 215 (100)

### z. 2,5-Diamino-1-(indan-1-on-5-yl)benzol-dihydrochlorid

Verwendete Bromderivat: 5-Brom-indan-1-on
Ausbeute: 0,025 g ( 80 % der Theorie)
Masspektren MH⁺ 239 (100)

### a'. 2,5-Diamino-1-(2-methyl-4-methoxy-phenyl)benzol-dihydrochlorid

Verwendete Bromderivat: 1-Brom-2-methyl-4-methoxyy-benzol
Ausbeute: 0,025 g ( 83 % der Theorie)
Masspektren MH⁺ 229 (100)

### b'. 2,5-Diamino-1-(2,4-dichloro-phenyl)benzol-dihydrochlorid

Verwendete Bromderivat: 1-Brom-2,4-dichloro-benzol
Ausbeute: 0,025 g ( 77 % der Theorie)
Masspektren M⁺ 253 (100)

### c'. 2,5-Diamino-1-(2,3-methylendioxy-phenyl)benzol-dihydrochlorid

Verwendete Bromderivat: 1-Brom-2,3-methylendioxy-benzol
Ausbeute: 0,025 g ( 83 % der Theorie)
Masspektren MH⁺ 229 (100)

### d'. 2,5-Diamino-1-(3-trifluormethyl-4-chlor-phenyl)benzol-dihydrochlorid

Verwendete Bromderivat: 1-Brom-3-trifluormethyl-4-chlor-benzol
Ausbeute: 0,025 g ( 70 % der Theorie)
Masspektren MH⁺ 287 (100)

### e'. 2,5-Diamino-1-(3,5-dimethyl-phenyl)benzol-dihydrochlorid

Verwendete Bromderivat: 1-Brom-3,5-dimethyl-benzol
Ausbeute: 0,025 g ( 88 % der Theorie)
Masspektren MH⁺ 213 (100)

### f'. 2,5-Diamino-1-(3,4-dimethyl-phenyl)benzol-dihydrochlorid

Verwendete Bromderivat: 1-Brom-3,4-dimethyl-benzol
Ausbeute: 0,025 g ( 88 % der Theorie)
Masspektren M⁺ 212 (100)

### g'. 2,5-Diamino-1-(3-chlor-phenyl)benzol-dihydrochlorid

Verwendete Bromderivat: 1-Brom-3-chlor-benzol
Ausbeute: 0,025 g ( 86 % der Theorie)
Masspektren MH⁺ 219 (100)

### h'. 2,5-Diamino-1-(3-trifluormethyl-phenyl)benzol-dihydrochlorid

Verwendete Bromderivat: 1-Brom-3-trifluormethyl-benzol
Ausbeute: 0,025 g ( 77 % der Theorie)
Masspektren MH⁺(+ CH₃CN) 294 (100)

### i'. 2,5-Diamino-1-(2-methoxy-phenyl)benzol-dihydrochlorid

Verwendete Bromderivat: 1-Brom-2-methoxy-benzol
Ausbeute: 0,025 g ( 87 % der Theorie)
Masspektren MH⁺ 215 (100)

### j'. 2,5-Diamino-1-(4-ethoxy-phenyl)benzol-dihydrochlorid

Verwendete Bromderivat: 1-Brom-4-ethoxy-benzol
Ausbeute: 0,025 g (83 % der Theorie)
Masspektren MH⁺ 229 (100)

### k'. 2.5-Diamino-1-(4-fluor-phenyl)benzol-dihydrochlorid

Verwendete Bromderivat: 1-Brom-4-fluor-benzol
Ausbeute: 0,025 g ( 91 % der Theorie)
Masspektren MH⁺ 203 (100)

### l'. 2,5-Diamino-1-(4-carboxamid-phenyl)benzol-dihydrochlorid

Verwendete Bromderivat: 4-Brom-benzamid
Ausbeute: 0,025 g ( 83 % der Theorie)
Masspektren MH⁺ 228 (100)

### m'. 2,5-Diamino-1-(2-nitro-4-methoxy-phenyl)benzol-dihydrochlorid

Verwendete Bromderivat: 1-Brom-2-nitro-4-methoxy-benzol
Ausbeute: 0,025 g ( 75 % der Theorie)
Masspektren MH⁺ 260 (100)

### n'. 2,5-Diamino-1-(1-acetyl-2,3-dihydroindol-5-yl)benzol-dihydrochlorid

Verwendete Bromderivat: 1-Acetyl-2,3-dihydro-5-brom-indol
Ausbeute: 0,025 g ( 73 % der Theorie)
Masspektren MH⁺ 268 (100)

### o'. 2,5-Diamino-1-(2,4-dimethoxy-3-carboxamid-phenyl)benzol-dihydrochlorid

Verwendete Bromderivat: 3-Brom-2,6-dimethoxy-benzamid
Ausbeute: 0,025 g ( 69 % der Theorie)
Masspektren MH⁺ 288 (100)

### p'. 2.5-Diamino-1-(2-chlor-4-nitro-phenyl)benzol-dihydrochlorid

Verwendete Bromderivat: 1-Brom-2-chlor-4-nitro-benzol
Ausbeute: 0,025 g ( 74 % der Theorie)
Masspektren MH⁺ 264 (100)

### q'. 2,5-Diamino-1-(2,5-difluoro-4-nitro-phenyl)benzol-dihydrochlorid

Verwendete Bromderivat: 1-Brom-2,5-difluoro-4-nitro-benzol
Ausbeute: 0,025 g ( 74 % der Theorie)
Masspektren MH⁺ 266 (100)

### r'. 2,5-Diamino-1-(2-chlor-4-N-acetyl-amino-phenyl)benzol-dihydrochlorid

Verwendete Bromderivat: 4-Brom-2-chlor-acetanilid
Ausbeute: 0,025 g (72 % der Theorie)
Masspektren MH⁺ 276 (100)

### s'. 2,5-Diamino-1-(indol-5-yl)benzol-dihydrochlorid

Verwendete Bromderivat: 1-Brom-indol
Ausbeute: 0,025 g ( 84 % der Theorie)
Masspektren MH⁺ 224(100)

### t'. 2,5-Diamino-1-(3,5-dichlor-phenyl)benzol-dihydrochlorid

Verwendete Bromderivat: 1-Brom-3,5-dichlor-benzol
Ausbeute: 0,025 g ( 77 % der Theorie)
Masspektren M⁺ 253 (100)

### u'. 2,5-Diamino-1-(2,4,5-trimethyl-phenyl)benzol-dihydrochlorid

Verwendete Bromderivat: 1-Brom-2,4,5-trimethyl-benzol
Ausbeute: 0,025 g ( 84 % der Theorie)
Masspektren MH⁺ 227 (100)

### v'. 2,5-Diamino-1-(2,4-dimethyl-phenyl)benzol-dihydrochlorid

Verwendete Bromderivat: 1-Brom-2,4-dimethyl-benzol
Ausbeute: 0,025 g ( 88 % der Theorie)
Masspektren MH⁺ 213 (100)

### w'. 2,5-Diamino-1-(2,3-dimethyl-phenyl)benzol-dihydrochlorid

Verwendete Bromderivat: 1-Brom-2,3-dimethyl-benzol
Ausbeute: 0,025 g ( 88 % der Theorie)
Masspektren MH⁺ 213 (100)

### x'. 2,5-Diamino-1-(3-fluor-phenyl)benzol-dihydrochlorid

Verwendete Bromderivat: 1-Brom-3-fluor-benzol
Ausbeute: 0,025 g ( 91 % der Theorie)
Masspektren MH⁺ 203 (100)

### y'. 2,5-Diamino-1-(3-cyan-phenyl)benzol-dihydrochlorid

Verwendete Bromderivat: 1-Brom-3-cyan-benzol
Ausbeute: 0,025 g ( 89 % der Theorie)
Masspektren MH⁺ 210 (100)

### z'. 2,5-Diamino-1-(3-methyl-phenyl)benzol-dihydrochlorid

Verwendete Bromderivat: 1-Brom-3-methyl-benzol
Ausbeute: 0,025 g ( 92 % der Theorie)
Masspektren MH⁺ 199 (100)

### a". 2,5-Diamino-1-(3-hydroxy-phenyl)benzol-dihydrochlorid

Verwendete Bromderivat: 1-Brom-3-hydroxy-benzol
Ausbeute: 0,025 g ( 92 % der Theorie)
Masspektren MH⁺ 201 (100)

### b". 2,5-Diamino-1-(3-aminomethyl-phenyl)benzol-dihydrochlorid

Verwendete Bromderivat: 1-Brom-3-aminomethyl-benzol
Ausbeute: 0,025 g ( 77 % der Theorie)
Masspektren MH⁺ 214 (100)

### c". 2,5-Diamino-1-(3-carbonsäure-ethylester-phenyl)benzol-dihydrochlorid

Verwendete Bromderivat: 3-Brom-benzoesäure-ethylester
Ausbeute: 0,025 g ( 76 % der Theorie)
Masspektren MH⁺ 257 (100)

### d". 2,5-Diamino-1-(2-nitro-phenyl)benzol-dihydrochlorid

Verwendete Bromderivat: 1-Brom-2-nitro-benzol
Ausbeute: 0,025 g ( 83% der Theorie)
Masspektren MH⁺ 230 (100)

### e". 2,5-Diamino-1-(2-fluor-phenyl)benzol-dihydrochlorid

Verwendete Bromderivat: 1-Brom-2-fluor-benzol
Ausbeute: 0,025 g ( 91 % der Theorie)
Masspektren MH⁺ 203 (100)

### f". 2,5-Diamino-1-(2-chlor-phenyl)benzol-dihydrochlorid

Verwendete Bromderivat: 1-Brom-2-chlor-benzol
Ausbeute: 0,025 g (86 % der Theorie)
Masspektren MH⁺ 219 (100)

### g". 2,5-Diamino-1-(2-trifluormethyl-phenyl)benzol-dihydrochlorid

Verwendete Bromderivat: 1-Brom-2-trifluormethyl-benzol
Ausbeute: 0,025 g ( 77 % der Theorie)
Masspektren MH⁺ 253 (100)

### h". 2,5-Diamino-1-(2-methyl-phenyl)benzol-dihydrochlorid

Verwendete Bromderivat: 1-Brom-2-methyl-benzol
Ausbeute: 0,025 g (92 % der Theorie)
Masspektren MH⁺ 199 (100)

### i". 2,5-Diamino-1-(2-cyanmethyl-phenyl)benzol-dihydrochlorid

Verwendete Bromderivat: 1-Brom-2-cyanmethyl-benzol
Ausbeute: 0,025 g ( 84 % der Theorie)
Masspektren MH⁺ 224 (100)

### j". 2,5-Diamino-1-(4-ethyl-phenyl)benzol-dihydrochlorid

Verwendete Bromderivat: 1-Brom-4-ethyl-benzol
Ausbeute: 0,025 g ( 88 % der Theorie)
Masspektren MH⁺ 213 (100)

### k". 2,5-Diamino-1-(4-propyl-phenyl)benzol-dihydrochlorid

Verwendete Bromderivat: 1-Brom-4-propyl-benzol
Ausbeute: 0,025 g ( 84 % der Theorie)
Masspektren MH⁺ 227 (100),

### l". 2,5-Diamino-1-(4-isopropyl-phenyl)benzol-dihydrochlorid

Verwendete Bromderivat: 1-Brom-4-Isopropyl-benzol
Ausbeute: 0,025 g ( 84 % der Theorie)
Masspektren MH⁺ 227 (100)

### m". 2,5-Diamino-1-(4-butyl-phenyl)benzol-dihydrochlorid

Verwendete Bromderivat: 1-Brom-4-butyl-benzol
Ausbeute: 0,025 g (80 % der Theorie)
Masspektren MH⁺ 241 (100)

### n". 2,5-Diamino-1-(4-ter-butyl-phenyl)benzol-dihydrochlorid

Verwendete Bromderivat: 1-Brom-4-ter-butyl-benzol
Ausbeute: 0,025 g (80 % der Theorie)
Masspektren MH⁺ 241 (100)

### o". 2,5-Diamino-1-(4-pentyl-phenyl)benzol-dihydrochlorid

Verwendete Bromderivat: 1-Brom-4-pentyl-benzol
Ausbeute: 0,025 g (76 % der Theorie)
Masspektren MH⁺ 255 (100)

### p". 2,5-Diamino-1-(4-acetyl-phenyl)benzol-dihydrochlorid

Verwendete Bromderivat: 1-Brom-4-acetyl-benzol
Ausbeute: 0,025 g ( 84 % der Theorie)
Masspektren MH⁺ 227 (100)

### q". 2,5-Diamino-1-(4-thiomethoxy-phenyl)benzol-dihydrochlorid

Verwendete Bromderivat: 1-Brom-4-thiomethoxy-benzol
Ausbeute: 0,025 g ( 91 % der Theorie)
Masspektren MH⁺ 203 (100)

### r". 2,5-Diamino-1-(2-ethyl-phenyl)benzol-dihydrochlorid

Verwendete Bromderivat: 1-Brom-2-ethyl-benzol
Ausbeute: 0,025 g ( 88 % der Theorie)
Masspektren MH⁺ 213 (100)

### s". 2,5-Diamino-1-(2-fluor-4-methyl-phenyl)benzol-dihydrochlorid

Verwendete Bromderivat: 1-Brom-2-fluor-4-methyl-benzol
Ausbeute: 0,025 g (86 % der Theorie)
Masspektren MH⁺ 217 (100)

### t". 2,5-Diamino-1-(2-methyl-5-fluor-phenyl)benzol-dihydrochlorid

Verwendete Bromderivat: 1-Brom-2-methyl-5-fluor-benzol
Ausbeute: 0,025 g (86 % der Theorie)
Masspektren MH⁺ 217 (100)

### u". 2,5-Diamino-1-(2-thiomethoxy-phenyl)benzol-dihydrochlorid

Verwendete Bromderivat: 1-Brom-2-thiomethoxy-benzol
Ausbeute: 0,025 g (82 % der Theorie)
Masspektren MH⁺ 231 (100)

### v". 2,5-Diamino-1-(2,3-dichlor-phenyl)benzol-dihydrochlorid

Verwendete Bromderivat: 1-Brom-2,3-dichlor-benzol
Ausbeute: 0,025 g ( 77 % der Theorie)
Masspektren M⁺ 253 (100)

### w". 2,5-Diamino-4-(4'-hydroxy-biphenyl)benzol-dihydrochlorid

Verwendete Bromderivat: 4-Brom-4'-hydroxy-biphenyl
Ausbeute: 0,025 g (72 % der Theorie)
Masspektren MH⁺ 277 (100)

### x". 2,5-Diamino-1-(3-ethoxy-phenyl)benzol-dihydrochlorid

Verwendete Bromderivat: 1-Brom-3-ethoxy-benzol
Ausbeute: 0,025 g ( 77 % der Theorie)
Masspektren MH⁺ 229 (100)

### y". 2,5-Diamino-1-(4-(2-pyrrolidin-1-yl-ethoxy)-phenyl)benzoldihydrochlorid

Verwendete Bromderivat: 1-Brom-4-(2-pyrrolidin-1-yl-ethoxy)-benzol
Ausbeute: 0,025 g ( 61 % der Theorie)
Masspektren MH⁺ 298 (100)

### z". 2,5-Diamino-1-(4-(1-hydroxy-ethyl)-phenyl)benzol-dihydrochlorid

Verwendete Bromderivat: 1-Brom-4-(1-hydroxy-ethyl)-benzol
Ausbeute: 0,025 g ( 83 % der Theorie)
Masspektren MH⁺ 229 (100)

### a"'. 2,5-Diamino-1-(2,4-trifluoromethyl-phenyl)benzol-dihydrochlorid

Verwendete Bromderivat: 1-Brom-2,4-trifluormethyl-benzol
Ausbeute: 0,025 g ( 64 % der Theorie)
Masspektren MH⁺ 321 (100)

### b"'. 2,5-Diamino-1-(2-fluor-5-acetyl-phenyl)benzol-dihydrochlorid

Verwendete Bromderivat: 1-Brom-2-fluor-5-acetyl-benzol
Ausbeute: 0,025 g ( 79 % der Theorie)
Masspektren MH⁺ 245 (100)

### c"'. 2,5-Diamino-1-(3-fluor-4-methoxy-phenyl)benzol-dihydrochlorid

Verwendete Bromderivat: 1-Brom-3-fluor-4-methoxy-benzol
Ausbeute: 0,025 g (82 % der Theorie)
Masspektren MH⁺ 233 (100)

### d'". 2,5-Diamino-1-(3-acetyl-4-hydroxy-phenyl)benzol-dihydrochlorid

Verwendete Bromderivat: 1-Brom-3-acetyl-4-hydroxy-benzol
Ausbeute: 0,025 g ( 79 % der Theorie)
Masspektren MH⁺ 243 (100)

### e"'. 2,5-Diamino-1-(4-(2-hydroxyethyl)-phenyl)benzol-dihydrochlorid

Verwendete Bromderivat: 1-Brom-4-(2-hydroxyethyl)-benzol
Ausbeute: 0,025 g ( 79 % der Theorie)
Masspektren MH⁺ 245 (100)

### f'''. 2,5-Diamino-1-(4-(propyl-1-on)-phenyl)benzol-dihydrochlorid

Verwendete Bromderivat: 1-Brom-4(propyl-1-on)-benzol
Ausbeute: 0,025 g ( 80 % der Theorie)
Masspektren MH⁺ 241 (100)

### g'''. 2.5-Diamino-1-(4-N,N'diisopropylaminomethyl-phenyl)benzol-dihydrochlorid

Verwendete Bromderivat: 1-Brom-4-N,N'-diisopropylaminomethyl-benzol
Ausbeute: 0,025 g (61 % der Theorie)
Masspektren MH⁺ 298 (100)

### h'''. 2.5-Diamino-1-(3-acetyl-phenyl)benzol-dihydrochlorid

Verwendete Bromderivat: 1-Brom-3-acetyl-benzol
Ausbeute: 0,025 g ( 84 % der Theorie)
Masspektren MH⁺ 227 (100)

### i'''. 2,5-Diamino-1-(2-(2-hydroxy-ethyl)-phenyl)benzol-dihydrochlorid

Verwendete Bromderivat: 1-Brom-2-(2-hydroxy-ethyl)-benzol
Ausbeute: 0,025 g ( 83 % der Theorie)
Masspektren MH⁺ 229 (100)

### j'''. 2,5-Diamino-1-(4-methoxy-phenyl)benzol-dihydrochlorid

Verwendete Bromderivat: 4-Brom-anisol
Ausbeute: 0,025 g ( 87 % der Theorie)
Masspektren MH⁺ 215 (100)

### Beispiel 2: Synthese von 2,5-Diamino-4-methoxy-1-phenylbenzol*2HCl

### A) Synthese von 2-Amino-4-methoxy-5-nitro-1-phenylbenzol

2,02 g (0,01 mol) 4-Chlor-5-nitro-2-amino-anisol und 0,013 mol Benzolborsäure werden unter Argon in 70 ml 1,2-Dimethoxyethan gelöst. Anschließend werden 0,5 g Tetrakis-(triphenylphosphin)-palladium (0,0005 mol) und 13 ml 2N Kaliumcarbonatlösung zugegeben und die Reaktionsmischung auf 80 °C erwärmt. Nach Beendigung der Reaktion wird die Reaktionsmischung in 100 ml Essigsäureethylester gegossen, die organische Phase mit verdünnter Natronlauge extrahiert und sodann mit Magnesiumsulfat getrocknet. Das Lösungsmittel wird am Rotationsverdampfer abdestilliert und der Rückstand an Kieselgel mit Hexan/Essigsäureethylester (8:1) gereinigt.
Ausbeute: 1,54g (= 63 Prozent der Theorie)
Schmelzpunkt: 105-109 °Celsius (gelbe Kristalle)

### B) Synthese von 2,5-Diamino-4-methoxy-1-phenylbenzol*2HCl

0,5g (2 mmol) 2-Amino-4-methoxy-5-nitro-1-phenylbenzol **(A)** werden in 30 ml Ethanol gelöst und unter Zusatz von 100 mg eines Palladium-Aktivkohle-Katalysators (10%ig) bei 25 °Celsius hydriert. Nach Aufnahme der theoretisch erforderlichen Wasserstoffmenge wird vom Katalysator abfiltriert und mit einem Uberschuß an verdünnter Salzsäure versetzt. Nach dem Einengen der Lösung am Rotationsverdampfer wird das ausgefallene Hydrochlorid abfiltriert und getrocknet.
Ausbeute: 0,53 g (= 93 Prozent der Theorie)
Schmelzpunkt: 257-260 °Celsius (farblose Kristalle)

| CHN-Analyse: | | | |
|---|---|---|---|
| (C₁₃H₁₆N₂OCl₂) | % C | % H | % N |
| berechnet | 54,37 | 5,62 | 9,75 |
| gefunden | 54,09 | 5,42 | 9,62 |

### Beispiel 3: Synthese von 2,5-Diamino-4-methyl-1-phenylbenzol*2HCl

### A) Synthese von 2-Amino-4-methyl-5-nitro-1-phenylbenzol

1,87 g (0,01 mol) 5-Chlor-2-methyl-4-nitroanilin und 0,013 mol Benzolborsäure werden unter Argon in 70 ml 1,2-Dimethoxyethan gelöst. Anschließend werden 0,5 g Tetrakis-(triphenylphosphin)-palladium (0,0005 mol) und 13 ml 2N Kaliumcarbonatlösung zugegeben und die Reaktionsmischung auf 80 °C erwärmt. Nach Beendigung der Reaktion wird die Reaktionsmischung in 100 ml Essigsäureethylester gegossen, die organische Phase mit verdünnter Natronlauge extrahiert und sodann mit Magnesiumsulfat getrocknet. Das Lösungsmittel wird am Rotationsverdampfer abdestilliert und der Rückstand an Kieselgel mit Hexan/Essigsäureethylester (8:1) gereinigt.
Ausbeute: 2,05g (= 90 Prozent der Theorie)
NMR (500 MHz, CDCl₃) 7,82 (s, 1H); 7,4-7,3 (m, 3H); 7,2 (d, 2H); 6,45 (s, 1H); 6,3 (s, 2H) 2,13 (s, 3H)

### B) Synthese von 2,5-Diamino-4-methyl-1-phenylbenzol*2HCl

0,5g (2 mmol) 2-Amino-4-methyl-5-nitro-1-phenylbenzol **(A)** werden in 30 ml Ethanol gelöst und unter Zusatz von 100 mg eines Palladium-Aktivkohle-Katalysators (10%ig) bei 25 °Celsius hydriert. Nach Aufnahme der theoretisch erforderlichen Wasserstoffmenge wird vom Katalysator abfiltriert und mit einem Uberschuß an verdünnter Salzsäure versetzt. Nach dem Einengen der Lösung am Rotationsverdampfer wird das ausgefallene Hydrochlorid abfiltriert und getrocknet.
Ausbeute: 0,50 g (= 93 Prozent der Theorie)
Schmelzpunkt: 263-265 °Celsius (farblose Kristalle)

| CHN-Analyse: | | | |
|---|---|---|---|
| (C₁₃H₁₆N₂Cl₂) | % C | % H | % N |
| berechnet | 54,37 | 5,62 | 9,75 |
| gefunden | 54,09 | 5,42 | 9,62 |

### Beispiel 4: Synthese von 2-Alkylamino-5-amino-1-phenylbenzolen (Allgemeine Synthesevorschrift)

### A) Synthese von 2-Fluor 5-nitro-1-phenylbenzol

1,75 g (0,01 mol) 3-Chlor-4-fluoro-nitrobenzol und 0,013 mol Benzolborsäure werden unter Argon in 70 m) 1,2-Dimethoxyethan gelöst. Anschließend werden 0,5 g Tetrakis-(triphenylphosphin)-palladium (0,0005 mol) und 13 ml 2N Kaliumcarbonatlösung zugegeben und die Reaktionsmischung auf 80 °C erwärmt. Nach Beendigung der Reaktion wird die Reaktionsmischung in 100 ml Essigsäureethylester gegossen, die organische Phase mit verdünnter Natronlauge extrahiert und sodann mit Magnesiumsulfat getrocknet. Das Lösungsmittel wird am Rotationsverdampfer abdestilliert und der Rückstand an Kieselgel mit Hexan/Toluol (10:1) gereinigt.
Ausbeute: 2,05 g (= 94 Prozent der Theorie)
NMR Spektrum (250 Mhz, CDCl₃) : 8,39 (dd, 1H); 8,25-8,21 (m, 1H); 7,57 (d, 2H); 7,5-7,46 (m, 3H);7,31 (t, 1H)

### B) Synthese von 2-Alkylamino-5-nitro-1-phenylbenzolen (Allgemeine Synthesevorschrift)

2,2 g (0,01 mol) 2-Fluor-5-nitro-1-phenylbenzol **(A)** werden in 25 ml Ethanol gelöst. Anschließend werden unter Rückfluß 0,05 mol des entsprechenden Amins zugegeben. Nach Beendigung der Reaktion wird die Reaktionsmischung in Wasser gegossen, die wässerige Phase mit Essigsäureethylester extrahiert und sodann mit Magnesiumsulfat getrocknet. Das Lösungsmittel wird am Rotationsverdampfer abdestilliert und der Rückstand an Kieselgel mit Hexan/ Essigsäureethylester (20:1) gereinigt.

### a. 2-Dimethylamino-5-nitro-1-phenylbenzol

Verwendetes Amin: Dimethylamin
Ausbeute: 2,1 g (= 87 Prozent der Theorie)
Schmelzpunkt: 70-73 °Celsius (gelbe Kristalle)

### b. 2-Di-(2-hydroxyethyl)amino-5-nitro-1-phenylbenzol

Verwendetes Amin: Diethanolamin
Ausbeute: 0,5 g (= 18 Prozent der Theorie)
Oranges Öl
- NMR (500 MHz, CDCl₃):: 8,13 (dd, 1H); 8,06 (d, 1H); 7,47-7,26 (m, 6H); 3,6-3,58 (m, 4H); 3,3-3,27 (m,4H)

### c. 2-Pyrrolidino-5-nitro-1-phenylbenzol

Verwendetes Amin: Pyrrolidin
Ausbeute: 2,5 g (= 93 Prozent der Theorie)
Schmelzpunkt: 109-113 °Celsius (gelbe Kristalle)

### d. 2-(2-Hydroxyethyl)amino-5-nitro-1-phenylbenzol

Verwendetes Amin: Ethanolamin
Ausbeute: 2,5 g (= 96 Prozent der Theorie)
Oranges Öl
- NMR (500 MHz, CDCl₃):: 8,14 (dd, 1H); 8,00 (s, 1H); 7,5-7,4 (m, 5H); 6,67 (d, 1H); 5,1 (s, 1H) 3,84-3,82 (dd, 2H); 3,4-3,37 (dd,2H)

### e. 2-(2-Methoxyethyl)amino-5-nitro-1-phenylbenzol

Verwendetes Amin: 2-Methoxyethylamin
Ausbeute: 2,6 g (= 96 Prozent der Theorie)
Schmelzpunkt: 93-95 °Celsius (gelbe Kristalle)

### f. 2-(2,3-Dihydroxypropyl)amino-5-nitro-1-phenylbenzol

Verwendetes Amin: 2,3-Dihydroxyprpylamin
Ausbeute: 2,6 g (= 90 Prozent der Theorie)
Schmelzpunkt: 127-131 °Celsius (orange Kristalle)

### C) Synthese von 2-Alkylamino-5-amino-1-phenylbenzolen (Allgemeine Synthesevorschrift)

2 mmol 2-Alkylamino-5-nitro-1-phenylbenzol gemäß **(B)** werden in 30 ml Ethanol gelöst und unter Zusatz von 100 mg eines Palladium-Aktivkohle-Katalysators (10%ig) bei 25 °Celsius hydriert. Nach Aufnahme der erforderlichen Wasserstoffmenge wird vom Katalysator abfiltriert und mit einem Uberschuß an verdünnter Salzsäure versetzt. Nach dem Einengen der Lösung am Rotationsverdampfer wird das ausgefallene Hydrochlorid abfiltriert und getrocknet.

### a. 2-Dimethylamino-5-amino-1-phenylbenzol-dihydrochlorid

Ausbeute: 0,33 g (= 77 Prozent der Theorie)
Schmelzpunkt: 220-225°Cetsius (farblose Kristalle)

| CHN-Analyse: | | | |
|---|---|---|---|
| (C₁₄H₁₈N₂Cl₂) | % C | % H | % N |
| berechnet | 58,96 | 6,36 | 9,82 |
| gefunden | 58,75 | 6,43 | 9,61 |

### b. 2-Di-(2-hydroxyethyl)amino-5-amino-1-phenylbenzo-dihydrochlorid

Ausbeute: 0,6 g (= 80 Prozent der Theorie)
Schmelzpunkt: 222-225°Celsius (farblose Kristalle)

| CHN-Analyse: | | | |
|---|---|---|---|
| (C₁₆H₂₂N₂O₂Cl₂) | % C | % H | % N |
| berechnet | 55,66 | 6,42 | 8,11 |
| gefunden | 55,21 | 6,21 | 7,96 |

### c. 2-Pyrrolidino-5-amino-1-phenylbenzol-dihydrochlorid

Ausbeute: 0,41 g (= 87 Prozent der Theorie)
Schmelzpunkt: 256-261 °Celsius (farblose Kristalle)

| CHN-Analyse: | | | |
|---|---|---|---|
| (C₁₆H₂₀N₂Cl₂) | % C | % H | % N |
| berechnet | 61,74 | 6,48 | 9,00 |
| gefunden | 61,00 | 6,81 | 8,65 |

### d. 2-(2-Hydroxyethyl)amino-5-amino-1-phenylbenzol-dihydrochlorid

Ausbeute: 0,47 g (= 92 Prozent der Theorie)
Schmelzpunkt: 210-213°Celsius (farblose Kristalle)

| CHN-Analyse: | | | |
|---|---|---|---|
| (C₁₄H₁₈N₂OCl₂) | % C | % H | % N |
| berechnet | 55,83 | 6,02 | 9,30 |
| gefunden | 55,71 | 6,25 | 9,43 |

### e. 2-(2-Methoxyethyl)amino-5-amino-1-phenylbenzol-dihydrochlorid

Ausbeute: 0,47 g (= 94 Prozent der Theorie)
Schmelzpunkt: 74°Celsius (Zersetzung) (farblose Kristalle)

| CHN-Analyse: | | | |
|---|---|---|---|
| C₁₅H₂₀N₂OCl₂) | % C | % H | % N |
| berechnet | 57,15 | 6,39 | 8,89 |
| gefunden | 56,82 | 7,04 | 8,63 |

### f. 2-(2,3-Dihydroxypropyl)amino-5-amino-1-phenylbenzol-dihydrochlorid

Ausbeute: 0,48 g (= 90 Prozent der Theorie)
Farbloses Öl)

| CHN-Analyse: | | | |
|---|---|---|---|
| (C₁₅H₂₀N₂O₂Cl₂xH₂O) | % C | % H | % N |
| berechnet | 51,58 | 6,35 | 8,02 |
| gefunden | 52,18 | 6,8 | 7,87 |

### Beispiele 5 bis 8: Haarfärbemittel

Es werden Haarfärbelösungen der folgenden Zusammensetzung hergestellt:

| | |
|---|---|
| 0,00125 mol | 2,5-Diaminobenzol-Derivat gemäß Tabelle 1 |
| 0,00125 mol | Kupplersubstanz gemäß Tabelle 1 |
| 10,0 g | Kaliumoleat (8prozentige wäßrige Lösung) |
| 10,0 g | Ammoniak (22prozentige wäßrige Lösung) |
| 10,0 g | Isopropanol |
| 0,3 g | Ascorbinsäure |
| ad 100,0 g | Wasser |

30 g der vorstehenden Färbelösung werden unmittelbar vor der Anwendung mit 30 g einer 6prozentigen Wasserstoffperoxidlösung vermischt. Anschließend wird das Gemisch auf gebleichte Haare aufgetragen. Nach einer Einwirkungszeit von 30 Minuten bei 40 °Celsius wird das Haar mit Wasser gespült, mit einem handelsüblichen Shampoo gewaschen und getrocknet. Die resultierenden Färbungen sind in Tabelle 1 zusammengefaßt.

**Tabelle 1:**

| **Beispiel** | **2,5-Diaminobenzol-Derivat** | **Kupplersubstanz** | **erhaltene Färbung** |
|---|---|---|---|
| **5** | 2,5-Diamino-1-phenylbenzol*2HCl | 2-Amino-4-(2'-hydroxyethyl)amino-anisolsulfat | dunkelblau |
| **6** | 2,5-Diamino-1-phenylbenzol*2HCl | Resorcin | dunkelblond |
| **7** | 2,5-Diamino-4-methyl-1-(phenyl)benzol*2HCl | Resorcin | dunkelblond |
| **8** | 2-Di(2-Hydroxyethyl)amino-5-amino-1-phenylbenzol*2HCl | 2-Amino-4-(2-hydroxyethyl)amino-anisolsulfat | blau |

### Beispiele 9 bis 14: Haarfärbemittel

Es werden Haarfärbelösungen der folgenden Zusammensetzung hergestellt:

| | |
|---|---|
| 0,00125 mol | 2,5-Diaminobenzol-Derivat gemäß Tabelle 2 |
| 0,00125 mol | Kupplersubstanz gemäß Tabelle 2 |
| 1,000 g | Kaliumoleat (8prozentige wäßrige Lösung) |
| 1,000 g | Ammoniak (22prozentige wäßrige Lösung) |
| 1,000 g | Ethanol |
| 0,300 g | Ascorbinsäure |
| ad 100 g | Wasser |

10 g der vorstehenden Färbelösung werden unmittelbar vor der Anwendung mit 10 g einer 6prozentigen Wasserstoffperoxidlösung vermischt. Anschließend wird das Gemisch auf gebleichte Haare aufgetragen. Nach einer Einwirkungszeit von 30 Minuten bei 40 °C wird das Haar mit Wasser gespült, mit einem handelsüblichen Shampoo gewaschen und getrocknet. Die resultierenden Färbungen sind in Tabelle 2 zusammengefaßt.

**Tabelle 2:**

| **Beispiel** | **2,5-Diaminobenzol-Derivat** | **Kupplersubstanz** | **erhaltene Färbung** |
|---|---|---|---|
| **9** | 2,5-Diamino-1-(4-hydroxy-phenyl)benzol *2HCl | 1,3-Diamino-4-(2-hydroxyethoxy)-benzol | dunkelblau |
| **10** | 2,5-Diamino-1-(4-aminophenyl)benzol *2HCl | Resorcin | dunkelblond |
| **11** | 2,5-Diamino-1-(2-methyl-4-hydroxy-phenyl)benzol *2HCl | Resorcin | dunkelblond |
| **12** | 2,5-Diamino-1-(4-fluor-phenyl)benzol *2HCl | 1,3-Diamino-4-(2-hydroxyethoxy)-benzol | dunkelblau |
| **13** | 2,5-Diamino-1-(3-methylphenyl)benzol *2HCl | Resorcin | dunkelblond |
| **14** | 2,5-Diamino-1-(3-fluor-4-methoxy-phenyl)benzol *2HCl | 1,3-Diamino-4-(2-hydroxyethoxy)-benzol | dunkelblau |

### Beispiel 15: Haarfärbemittel

Es werden Haarfärbelösungen der folgenden Zusammensetzung hergestellt:

| | |
|---|---|
| 0,160 g | 2,5-Diamino-1-phenylbenzol*2HCl |
| 0,160 g | 1,4-Diamino-2-(2-hydroxyethyl)benzol*sulfat |
| 0,137 g | 1,3-Dihydroxy-benzol |
| 0,100 g | 1,3-Dihydroxy-2-methylbenzol |
| 0,100 g | 2-Amino-5-methyl-phenol |
| 10,000 g | Kaliumoleat (8prozentige wäßrige Lösung) |
| 10,000 g | Ammoniak (22prozentige wäßrige Lösung) |
| 10,000 g | Isopropanol |
| 0,300 g | Ascorbinsäure |
| ad 100,000 g | Wasser |

30 g der vorstehenden Färbelösung werden unmittelbar vor der Anwendung mit 30 g einer 6prozentigen Wasserstoffperoxidlösung vermischt. Anschließend wird das Gemisch auf gebleichte Haare aufgetragen. Nach einer Einwirkungszeit von 30 Minuten bei 40 °Celsius wird das Haar mit Wasser gespült, mit einem handelsüblichen Shampoo gewaschen und getrocknet. Das Haar hat eine blonde Färbung erhalten.

### Beispiel 16: Haarfärbemittel

Es werden Haarfärbelösungen der folgenden Zusammensetzung hergestellt:

| | |
|---|---|
| 0,320 g | 2,5-Diamino-1-phenylbenzol*2HCl |
| 0,300 g | 5-Amino-2-methylphenol |
| 0,600 g | 4-Amino-3-methylphenol |
| 0,600 g | 4-Amino-phenol |
| 0,100 g | α-Naphtol |
| 0,200 g | 2-Chlor-6-(ethylamino)-4-nitrophenol |
| 10,000 g | Kaliumoleat (8prozentige wäßrige Lösung) |
| 10,000 g | Ammoniak (22prozentige wäßrige Lösung) |
| 10,000 g | Isopropanol |
| 0,300 g | Ascorbinsäure |
| ad 100,000 g | Wasser |

30 g der vorstehenden Färbelösung werden unmittelbar vor der Anwendung mit 30 g einer 6prozentigen Wasserstoffperoxidlösung vermischt. Anschließend wird das Gemisch auf gebleichte Haare aufgetragen. Nach einer Einwirkungszeit von 30 Minuten bei 40 °Celsius wird das Haar mit Wasser gespült, mit einem handelsüblichen Shampoo gewaschen und getrocknet. Das Haar hat eine rote Färbung erhalten.

### Beispiel 17: Haarfärbemittel

Es werden Haarfärbelösungen der folgenden Zusammensetzung hergestellt:

| | |
|---|---|
| 0,320 g | 2,5-Diamino-1-phenylbenzol*2HCl |
| 0,040 g | 5-Amino-2-methylphenol |
| 0,090 g | 2-Amino-4-(2'-hydroxyethyl)-amino-anisolsulfat |
| 0,030 g | 3-Aminophenol |
| 0,030 g | 1,3-Dihydroxy-benzol |
| 0,040 g | 1,3-Dihydroxy-2-methylbenzol |
| 0,100 g | 4-Amino-5-methylphenol |
| 0,200 g | 2-Amino-3-methylphenol |
| 0,100 g | 2-Amino-6-methylphenol-hydrochlorid |
| 0,010 g | 4-Chlor-N-(2-hydroxyethyl)-2-nitroanilin |
| 0,020 g | 2-Amino-4,6-dinitrophenol |
| 0,100 g | 2-Chlor-6-(ethylamino)-4-nitrophenol |
| 10,000 g | Kaliumoleat (8prozentige wäßrige Lösung) |
| 10,000 g | Ammoniak (22prozentige wäßrige Lösung) |
| 10,000 g | Isopropanol |
| 0,300 g | Ascorbinsäure |
| ad 100,000 g | Wasser |

30 g der vorstehenden Färbelösung werden unmittelbar vor der Anwendung mit 30 g einer 6prozentigen Wasserstoffperoxidlösung vermischt. Anschließend wird das Gemisch auf gebleichte Haare aufgetragen. Nach einer Einwirkungszeit von 30 Minuten bei 40 °Celsius wird das Haar mit Wasser gespült, mit einem handelsüblichen Shampoo gewaschen und getrocknet. Das Haar hat eine braune Färbung erhalten.

### Beispiel 18: Haarfärbemittel

Es werden Haarfärbelösungen der folgenden Zusammensetzung hergestellt:

| | |
|---|---|
| 0,320 g | 2,5-Diamino-1-phenylbenzol*2HCl |
| 0,040 g | 5-Amino-2-methylphenol |
| 0,050 g | 1,3-Diamino-4-(2-hydroxyethoxy)-benzol |
| 0,030 g | 3-Aminophenol |
| 0,030 g | 1,3-Dihydroxy-benzol |
| 0.040 g | 1,3-Dihydroxy-2-methylbenzol |
| 0,100 g | 4-Amino-5-methylphenol |
| 0,200 g | 2-Amino-3-methylphenol |
| 0,100 g | 2-Amino-6-methylphenol-hydrochlorid |
| 0,010 g | 4-Chlor-N-(2-hydroxyethyl)-2-nitroanilin |
| 0,020 g | 2-Amino-4,6-dinitrophenol |
| 0,100 g | 2-Chlor-6-(ethylamino)-4-nitrophenol |
| 10,000 g | Ammoniak (22prozentige wäßrige Lösung) |
| 10,000 g | Isopropanol |
| 0,300 g | Ascorbinsäure |
| ad 100,000 g | Wasser |

30 g der vorstehenden Färbelösung werden unmittelbar vor der Anwendung mit 30 g einer 6prozentigen Wasserstoffperoxidlösung vermischt. Anschließend wird das Gemisch auf gebleichte Haare aufgetragen. Nach einer Einwirkungszeit von 30 Minuten bei 40 °Celsius wird das Haar mit Wasser gespült, mit einem handelsüblichen Shampoo gewaschen und getrocknet. Das Haar hat eine braune Färbung erhalten.

### Beispiel 19: Haarfärbemittel

Es werden Haarfärbelösungen der folgenden Zusammensetzung hergestellt:

| | |
|---|---|
| 0,220 g | 2,5-Diamino-1-phenylbenzol*2HCl |
| 0,100 g | 1,4-Diamino-2-(2-hydroxyethyl)benzol*sulfat |
| 0,020 g | 5-Amino-2-methylphenol |
| 0,010 g | 1,3-Diamino-4-(2-hydroxyethoxy)-benzol |
| 0,004 g | 2-Amino-4-(2'-hydroxyethyl)-amino-anisolsulfat |
| 0,020 g | 1,3-Dihydroxy-benzol |
| 0,040 g | 1,3-Dihydroxy-2-methylbenzol |
| 0,008 g | 4-Amino-3-methylphenol |
| 10,000 g | Kaliumoleat (8prozentige wäßrige Lösung) |
| 10,000 g | Ammoniak (22prozentige wäßrige Lösung) |
| 10,000 g | Isopropanol |
| 0,300 g | Ascorbinsäure |
| ad 100,000 g | Wasser |

30 g der vorstehenden Färbelösung werden unmittelbar vor der Anwendung mit 30 g einer 6prozentigen Wasserstoffperoxidlösung vermischt. Anschließend wird das Gemisch auf gebleichte Haare aufgetragen. Nach einer Einwirkungszeit von 30 Minuten bei 40 °Celsius wird das Haar mit Wasser gespült, mit einem handelsüblichen Shampoo gewaschen und getrocknet. Das Haar hat eine braune Färbung erhalten.

### Beispiel 20: Haarfärbemittel

Es werden Haarfärbelösungen der folgenden Zusammensetzung hergestellt:

| | |
|---|---|
| 0,220 g | 2,5-Diamino-1-phenylbenzol*2HCl |
| 0,100 g | 4-Di-(2-hydroxyethyl)amino-anilin-sulfat |
| 0,020 g | 5-Amino-2-methylphenol |
| 0,010 g | 1,3-Diamino-4-(2-hydroxyethoxy)-benzol |
| 0,015 g | 2-Amino-4-(2'-hydroxyethyl)-amino-anisolsulfat |
| 0,020 g | 1,3-Dihydroxy-benzol |
| 0,040 g | 1,3-Dihydroxy-2-methylbenzol |
| 0,008 g | 4,5-Diamino-1-(2-hydroxyethyl)-1H-pyrazol-sulfat |
| 10,000 g | Kaliumoleat (8prozentige wäßrige Lösung) |
| 10,000 g | Ammoniak (22prozentige wäßrige Lösung) |
| 10,000 g | Isopropanol |
| 0,300 g | Ascorbinsäure |
| ad 100,000 g | Wasser |

30 g der vorstehenden Färbelösung werden unmittelbar vor der Anwendung mit 30 g einer 6prozentigen Wasserstoffperoxidlösung vermischt. Anschließend wird das Gemisch auf gebleichte Haare aufgetragen. Nach einer Einwirkungszeit von 30 Minuten bei 40 °Celsius wird das Haar mit Wasser gespült, mit einem handelsüblichen Shampoo gewaschen und getrocknet. Das Haar hat eine braune Färbung erhalten.

### Beispiel 21: Haarfärbemittel

Es werden Haarfärbelösungen der folgenden Zusammensetzung hergestellt:

| | |
|---|---|
| 0,320 g | 2,5-Diamino-1-phenylbenzol*2HCl |
| 0,020 g | 5-Amino-2-methylphenol |
| 0,010 g | 1,3-Diamino-4-(2-hydroxyethoxy)-benzol |
| 0,015 g | 2-Amino-4-(2'-hydroxyethyl)-amino-anisolsulfat |
| 0,020 g | 1,3-Dihydroxy-benzol |
| 0,040 g | 1,3-Dihydroxy-2-methylbenzol |
| 0,008 g | 4-Amino-2-(aminomethyl)phenol-dihydrochlorid |
| 10,000 g | Kaliumoleat (8prozentige wäßrige Lösung) |
| 10,000 g | Ammoniak (22prozentige wäßrige Lösung) |
| 10,000 g | Isopropanol |
| 0,300 g | Ascorbinsäure |
| ad 100,000 g | Wasser |

30 g der vorstehenden Färbelösung werden unmittelbar vor der Anwendung mit 30 g einer 6prozentigen Wasserstoffperoxidlösung vermischt. Anschließend wird das Gemisch auf gebleichte Haare aufgetragen. Nach einer Einwirkungszeit von 30 Minuten bei 40 °Celsius wird das Haar mit Wasser gespült, mit einem handelsüblichen Shampoo gewaschen und getrocknet. Das Haar hat eine braune Färbung erhalten.

Alle Gewichtsangaben stellen, soweit nicht anders angegeben, Gewichtsprozente dar.

## Patentansprüche

1. Mittel zur oxidativen Färbung von Keratinfasem auf der Basis einer Entwicklersubstanz-Kupplersubstanz-Kombination, **dadurch gekennzeichnet, daß** es als Entwicklersubstanz mindestens eine Verbindung aus der Gruppe bestehend aus
2,5-Diamino-1-phenyl-benzol;
2,5-Diamino-1-(4-bromophenyl)benzol;
2,5-Diamino-1-(4-ethenylphenyl)- benzol;
2,5-Diamino-1-(2,3,4-trimethoxy-phenyl)benzol;
2,5-Diamino-1-(2,4-di(2-hydroxyethyl)aminophenyl)benzol;
2,5-Diamino-1-(2,4-diamino-phenyl)benzol;
2,5-Diamino-1-(2,4-dihydroxy-phenyl)benzol;
2,5-Diamino-1-(2,4-dimethylaminophenyl)benzol;
2,5-Diamino-1-(2,4-methoxyphenyl)-benzol;
2,5-Diamino-1-(2,5-di(2-hydroxyethyl)aminophenyl)benzol;
2,5-Diamino-1-(2,5-diaminophenyl)-benzol;
2,5-Diamino-1-(2,5-dihydroxy-phenyl)benzol;
2,5-Diamino-1-(2,5-dimethoxyphenyl)benzol;
2,5-Diamino-1-(2,5-dimethylaminophenyl)benzol;
2,5-Diamino-1-(2,6-di(2-hydroxyethyl)-aminophenyl)benzol;
2,5-Diamino-1-(2,6-diaminophenyl)-benzol;
2,5-Diamino-1-(2,6-dihydroxyphenyl)-benzol;
2,5-Diamino-1-(2,6-dimethoxyphenyl)benzol;
2,5-Diamino-1-(2,6-dimethylaminophenyl)benzol;
2,5-Diamino-1-(2-(bromomethyl)phenyl)-benzol;
2,5-Diamino-1-(2-amino-5-hydroxyphenyl)benzol;
2,5-Diamino-1-(2-aminophenyl)benzol;
2,5-Diamino-1-(2-carbonsäurephenyl)benzol;
2,5-Diamino-1-(2-chlor-phenyl)benzol;
2,5-Diamino-1-(2-di(2-hydroxyethyl)aminophenyl)benzol;
2,5-Diamino-1-(2-dimethylamino-phenyl)benzol;
2,5-Diamino-1-(2-fluoro-phenyl)benzol;
2,5-Diamino-1-(2-formylphenyl)benzol;
2,5-Diamino-1-(2-hydroxy-4-aminophenyl)benzol;
2,5-Diamino-1-(2-hydroxy-5-amino-phenyl)-benzol;
2,5-Diamino-1-(2-hydroxyphenyl)benzol;
2,5-Diamino-1-(2-methoxyphenyl)benzol;
2,5-Diamino-1-(2-methylphenyl)benzol;
2,5-Diamino-1-(2-nitrophenyl)benzol;
2,5-Diamino-1-(2-trifluoromethyl-phenyl)benzol;
2,5-Diamino-1-(3,5-di(2-hydroxyethyl)aminophenyl)benzol;
2,5-Diamino-1-(3,5-diaminophenyl)-benzol;
2,5-Diamino-1-(3,5-dihydroxy-phenyl)benzol;
2,5-Diamino-1-(3,5-dimethoxyphenyl)benzol;
2,5-Diamino-1-(3,5-dimethylaminophenyl)benzol;
2,5-Diamino-1-(3-aminophenyl)-benzol;
2,5-Diamino-1-(3-bromophenyl)-benzol;
2,5-Diamino-1-(3-carbonsäurephenyl)benzol;
2,5-Diamino-1-(3-chlorophenyl)benzol;
2,5-Diamino-1-(3-di(2-hydroxyethyl)aminophenyl)-benzol;
2,5-Diamino-1-(3-dimethylaminophenyl)benzol;
2,5-Diamino-1-(3-fluorophenyl)benzol;
2,5-Diamino-1-(3-formylphenyl)benzol;
2,5-Diamino-1-(3-hydroxy-5-aminophenyl)benzol;
2,5-Diamino-1-(3-hydroxyphenyl)-benzol;
2,5-Diamino-1-(3-methoxyphenyl)benzol;
2,5-Diamino-1-(3-nitrophenyl)benzol;
2,5-Diamino-1-(3-trifluoromethylphenyl)benzol;
2,5-Diamino-1-(4-(dimethylamino)phenyl)benzol;
2,5-Diamino-1-(4-(hydroxymethyl)phenyl)benzol;
2,5-Diamino-1-(4-(methylthio)phenyl)-benzol;
2,5-Diamino-1-(4-(trifluoromethyl)phenyl)benzol;
2,5-Diamino-1-(4-(trimethylsilyl)phenyl)benzol;
2,5-Diamino-1-(4-acetylphenyl)benzol;
2,5-Diamino-1-(4-aminophenyl)benzol;
2,5-Diamino-1-(4-carbonsäure-phenyl)benzol;
2,5-Diamino-1-(4-chlorophenyl)benzol;
2,5-Diamino-1-(4-di(2-hydroxyethyl)aminophenyl)benzol;
2,5-Diamino-1-(4-dimethylamino-phenyl)benzol;
2,5-Diamino-1-(4-ethoxyphenyl)benzol;
2,5-Diamino-1-(4-fluorophenyl)benzol;
2,5-Diamino-1-(4-formylphenyl)benzol;
2,5-Diamino-1-(4-hydroxyphenyl)benzol;
2,5-Diamino-1-(4-methoxy-phenyl)benzol;
2,5-Diamino-1-(4-methylphenyl)benzol;
2,5-Diamino-1-phenylbenzol; 2,5-Diamino-4-chlor-1-phenylbenzol;
2,5-Diamino-4-methoxy-1-phenylbenzol;
2,5-Diamino-4-methyl-1-phenylbenzol;
2,5-Diamino-1-(2-cyanmethyl-phenyl)benzol;
2,5-Diamino-1-(4-ethyl-phenyl)benzol;
2,5-Diamino-1-(4-propyl-phenyl)benzol;
2,5-Diamino-1-(4-isopropyl-phenyl)benzol;
2,5-Diamino-1-(4-butyl-phenyl)benzol;
2,5-Diamino-1-(4-ter-butyl-phenyl)benzol;
2,5-Diamino-1-(4-pentyl-phenyl)benzol;
2,5-Diamino-1-(4-thiomethoxy-phenyl)benzol;
2,5-Diamino-1-(2-ethyl-phenyl)benzol;
2,5-Diamino-1-(2-fluor-4-methyl-phenyl)benzol;
2,5-Diamino-1-(2-methyl-5-fluor-phenyl)benzol;
2,5-Diamino-1-(2-thiomethoxy-phenyl)benzol;
2,5-Diamino-1-(2,3-dichlor-phenyl)benzol;
2,5-Diamino-4-(4'-hydroxy-biphenyl)benzol;
2,5-Diamino-1-(3-ethoxy-phenyl)benzol;
2,5-Diamino-1-(4-(2-pyrrolidin-1-yl-ethoxy)-phenyl)benzol;
2,5-Diamino-1-(4-(1-hydroxy-ethyl)-phenyl)benzol;
2,5-Diamino-1-(2,4-trifluormethyl-phenyl)benzol;
2,5-Diamino-1-(2-fluor-5-acetyl-phenyl)benzol;
2,5-Diamino-1-(3-fluor-4-methoxy-phenyl)benzol;
2,5-Diamino-1-(3-acetyl-4-hydroxy-phenyl)benzol;
2,5-Diamino-1-(4-(2-hydroxyethyl)-phenyl)benzol;
2,5-Diamino-1-(4-(propyl-1-on)-phenyl)benzol;
2,5-Diamino-1-(4-N,N'diisopropylaminomethyl-phenyl)benzol;
2,5-Diamino-1-(3-acetyl-phenyl)benzol;
2,5-Diamino-1-(2-(2-hydroxy-ethyl)-phenyl)benzol;
2,5-Diamino-1-(3-aminomethyl-phenyl)benzol;
2-Amino-5-(2,3-dihydroxypropyl)amino-1-(2,3,4-trimethoxyphenyl)benzol;
2-Amino-5-(2,3-dihydroxypropyl)amino-1-(2,4-dihydroxyphenyl)benzol;
2-Amino-5-(2,3-dihydroxypropyl)amino-1-(2,5-diaminophenyl)benzol;
2-Amino-5-(2,3-dihydroxypropyl)amino-1-(2,6-dimethoxyphenyl)benzol;
2-Amino-5-(2,3-dihydroxypropyl)amino-1-(2-amino-5-hydroxyphenyl)- benzol;
2-Amino-5-(2,3-dihydroxypropyl)amino-1-(2-aminophenyl)benzol;
2-Amino-5-(2,3-dihydroxypropyl)amino-1-(2-hydroxy-4-aminophenyl)-benzol;
2-Amino-5-(2,3-dihydroxypropyl)amino-1-(2-hydroxy-5-amino-phenyl)benzol;
2-Amino-5-(2,3-dihydroxypropyl)amino-1-(2-hydroxy-phenyl)benzol;
2-Amino-5-(2,3-dihydroxypropyl)amino-1-(2-methoxy-phenyl)benzol;
2-Amino-5-(2,3-dihydroxypropyl)amino-1-(2-methyl-phenyl)benzol;
2-Amino-5-(2,3-dihydroxypropyl)amino-1-(3,5-diamino-phenyl)benzol;
2-Amino-5-(2,3-dihydroxypropyl)amino-1-(3,5-dihydroxy-phenyl)benzol;
2-Amino-5-(2,3-dihydroxypropyl)amino-1-(3-aminophenyl)-benzol;
2-Amino-5-(2,3-dihydroxypropyl)amino-1-(3-hydroxy-5-aminphenyl)benzol;
2-Amino-5-(2,3-dihydroxypropyl)amino-1-(3-hydroxy-phenyl)benzol;
2-Amino-5-(2,3-dihydroxypropyl)amino-1-(3-methoxy-phenyl)benzol;
2-Amino-5-(2,3-dihydroxypropyl)amino-1-(4-(dimethyl-amino)phenyl)benzol;
2-Amino-5-(2,3-dihydroxypropyl)amino-1-(4-(trifluoromethyl)phenyl)benzol;
2-Amino-5-(2,3-dihydroxypropyl)amino-1-(4-aminophenyl)benzol;
2-Amino-5-(2,3-dihydroxypropyl)amino-1-(4-carbonsäurephenyl)benzol;
2-Amino-5-(2,3-dihydroxypropyl)amino-1-(4-chlorophenyl)benzol;
2-Amino-5-(2,3-dihydroxypropyl)amino-1-(4-hydroxyphenyl)benzol;
2-Amino-5-(2,3-dihydroxypropyl)amino-1-(4-methoxyphenyl)benzol;
2-Amino-5-(2,3-dihydroxypropyl)amino-1-(4-methylphenyl)benzol;
2-Amino-5-(2,3-dihydroxypropyl)amino-1-phenylbenzol;
2-Amino-5-(2-hydroxyethyl)amino-1-(2,3,4-trimethoxy-phenyl)benzol;
2-Amino-5-(2-hydroxyethyl)amino-1-(2,4-diamino-phenyl)benzol;
2-Amino-5-(2-hydroxyethyl)amino-1-(2,4-dihydroxy-phenyl)benzol;
2-Amino-5-(2-hydroxyethyl)amino-1-(2,5-diamino-phenyl)benzol;
2-Amino-5-(2-hydroxyethyl)amino-1-(2,5-dimethoxy-phenyl)benzol;
2-Amino-5-(2-hydroxyethyl)amino-1-(2,6-dimethoxy-phenyl)benzol;
2-Amino-5-(2-hydroxyethyl)amino-1-(2-amino-5-hydroxyphenyl)benzol;
2-Amino-5-(2-hydroxyethyl)amino-1-(2-aminophenyl)benzol;
2-Amino-5-(2-hydroxyethyl)amino-1-(2-hydroxy-4-aminophenyl)benzol;
2-Amino-5-(2-hydroxyethyl)amino-1-(2-hydroxy-5-aminophenyl)benzol;
2-Amino-5-(2-hydroxyethyl)amino-1-(2-hydroxy-phenyl)benzol;
2-Amino-5-(2-hydroxyethyl)amino-1-(2-methoxyphenyl)-benzol;
2-Amino-5-(2-hydroxyethyl)amino-1-(2-methylphenyl)benzol;
2-Amino-5-(2-hydroxyethyl)amino-1-(3,5-diaminophenyl)benzol;
2-Amino-5-(2-hydroxyethyl)amino-1-(3,5-dihydroxyphenyl)benzol;
2-Amino-5-(2-hydroxyethyl)amino-1-(3-aminophenyl)benzol;
2-Amino-5-(2-hydroxyethyl)amino-1-(3-hydroxy-5-aminophenyl)benzol;
2-Amino-5-(2-hydroxyethyl)amino-1-(3-hydroxyphenyl)benzol;
2-Amino-5-(2-hydroxyethyl)amino-1-(3-methoxyphenyl)benzol;
2-Amino-5-(2-hydroxyethyl)amino-1-(4-(dimethylamino)phenyl)benzol;
2-Amino-5-(2-hydroxyethyl)amino-1-(4-(trifluoromethyl)phenyl)benzol;
2-Amino-5-(2-hydroxyethyl)amino-1-(4-aminophenyl)benzol;
2-Amino-5-(2-hydroxyethyl)amino-1-(4-carbonsäurephenyl)benzol;
2-Amino-5-(2-hydroxyethyl)amino-1-(4-chlorophenyl)benzol;
2-Amino-5-(2-hydroxyethyl)amino-1-(4-hydroxyphenyl)benzol;
2-Amino-5-(2-hydroxyethyl)amino-1-(4-methoxyphenyl)benzol;
2-Amino-5-(2-hydroxyethyl)amino-1-(4-methylphenyl)benzol;
2-Amino-5-(2-hydroxyethyl)amino-1-phenylbenzol;
2-Amino-5-(2-hydroxyethyl)methylamino-1-phenylbenzol;
2-Amino-5-(2-methoxy-ethyl)amino-1-(2,3,4-trimethoxyphenyl)benzol;
2-Amino-5-(2-methoxy-ethyl)amino-1-(2,4-diaminophenyl)benzol;
2-Amino-5-(2-methoxyethyl)-amino-1-(2,4-dihydroxyphenyl)benzol;
2-Amino-5-(2-methoxyethyl)amino-1-(2,5-diaminophenyl)benzol;
2-Amino-5-(2-methoxyethyl)amino-1-(2,5-dimethoxyphenyl)benzol;
2-Amino-5-(2-methoxyethyl)amino-1-(2,6-dimethoxyphenyl)benzol;
2-Amino-5-(2-methoxyethyl)amino-1-(2-amino-5-hydroxyphenyl)benzol;
2-Amino-5-(2-methoxyethyl)amino-1-(2-amino-phenyl)benzol;
2-Amino-5-(2-methoxyethyl)amino-1-(2-hydroxy-4-amino-phenyl)benzol;
2-Amino-5-(2-methoxyethyl)amino-1-(2-hydroxy-5-amino-phenyl)benzol;
2-Amino-5-(2-methoxyethyl)amino-1-(2-hydroxyphenyl)-benzol;
2-Amino-5-(2-methoxyethyl)amino-1-(2-methoxyphenyl)benzol;
2-Amino-5-(2-methoxyethyl)amino-1-(2-methylphenyl)benzol;
2-Amino-5-(2-methoxyethyl)amino-1-(3,5-diaminophenyl)benzol;
2-Amino-5-(2-methoxyethyl)amino-1-(3,5-dihydroxyphenyl)benzol;
2-Amino-5-(2-methoxyethyl)amino-1-(3-aminophenyl)benzol;
2-Amino-5-(2-methoxyethyl)amino-1-(3-hydroxy-5-aminophenyl)benzol;
2-Amino-5-(2-methoxyethyl)amino-1-(3-hydroxyphenyl)benzol;
2-Amino-5-(2-methoxyethyl)amino-1-(3-methoxyphenyl)benzol;
2-Amino-5-(2-methoxyethyl)amino-1-(4-(dimethylamino)phenyl)benzol;
2-Amino-5-(2-methoxyethyl)amino-1-(4-(trifluoromethyl)phenyl)benzol;
2-Amino-5-(2-methoxyethyl)amino-1-(4-aminophenyl)benzol;
2-Amino-5-(2-methoxyethyl)amino-1-(4-carbonsäurephenyl)benzol;
2-Amino-5-(2-methoxyethyl)amino-1-(4-chlorophenyl)benzol;
2-Amino-5-(2-methoxyethyl)amino-1-(4-hydroxyphenyl)benzol;
2-Amino-5-(2-methoxyethyl)amino-1-(4-methoxyphenyl)benzol;
2-Amino-5-(2-methoxyethyl)amino-1-(4-methylphenyl)benzol;
2-Amino-5-(2-methoxyethyl)amino-1-phenylbenzol;
2-Amino-5-di(2-hydroxyethyl)amino-1-(2,3,4-trimethoxyphenyl)benzol;
2-Amino-5-di(2-hydroxyethyl)amino-1-(2,3-difluoro-4-heptylphenyl)benzol;
2-Amino-5-di(2-hydroxyethyl)amino-1-(2,3-difluorophenyl)benzol;
2-Amino-5-di(2-hydroxy-ethyl)amino-1-(2,4,6-trimethylphenyl)benzol;
2-Amino-5-di(2-hydroxy-ethyl)amino-1-(2,4-diaminophenyl)benzol;
2-Amino-5-di(2-hydroxyethyl)-amino-1-(2,4-dichlorophenyl)benzol;
2-Amino-5-di(2-hydroxyethyl)amino-1-(2,4-dihydroxyphenyl)benzol;
2-Amino-5-di(2-hydroxyethyl)amino-1-(2,5-diaminophenyl)benzol;
2-Amino-5-di(2-hydroxyethyl)amino-1-(2,5-dimethoxyphenyl)benzol;
2-Amino-5-di(2-hydroxyethyl)amino-1-(2,6-difluorophenylbenzol;
2-Amino-5-di(2-hydroxyethyl)amino-1-(2,6-dimethoxyphenyl)benzol;
2-Amino-5-di(2-hydroxyethyl)amino-1-(2,6-dimethoxyphenyl)benzol;
2-Amino-5-di(2-hydroxyethyl)amino-1-(2-((bis(1-methylethyl)amino)-carbonyl)phenyl)benzol;
2-Amino-5-di(2-hydroxyethyl)-amino-1-(2-((bis(1-methylethyl)amino)-carbonyl)-3-methoxyphenyl)benzol;
2-Amino-5-di(2-hydroxyethyl)amino-1-(2-(bromomethyl)phenyl)benzol;
2-Amino-5-di(2-hydroxyethyl)amino-1-(2-(diethylamino)carbonyl)phenyl)-benzol;
2-Amino-5-di(2-hydroxyethyl)amino-1-(2-amino-5-hydroxyphenyl)- benzol;
2-Amino-5-di(2-hydroxyethyl)amino-1-(2-aminophenyl)benzol;
2-Amino-5-di(2-hydroxyethyl)amino-1-(2-carbonsäurephenyl)benzol;
2-Amino-5-di(2-hydroxyethyl)amino-1-(2-chlorophenyl)benzol;
2-Amino-5-di(2-hydroxyethyl)amino-1-(2-fluorophenyl)benzol;
2-Amino-5-di(2-hydroxyethyl)amino-1-(2-formyl-4-methoxyphenyl)benzol;
2-Amino-5-di(2-hydroxyethyl)amino-1-(2-formyl-4-methylphenyl)benzol;
2-Amino-5-di(2-hydroxyethyl)amino-1-(2-formyl-5-methoxyphenyl)benzol;
2-Amino-5-di(2-hydroxyethyl)amino-1-(2-formylphenyl)benzol;
2-Amino-5-di(2-hydroxy-ethyl)amino-1-(2-hydroxy-4-aminophenyl)benzol;
2-Amino-5-di(2-hydroxy-ethyl)amino-1-(2-hydroxy-5-aminophenyl)benzol;
2-Amino-5-di(2-hydroxyethyl)amino-1-(2-hydroxyphenyl)benzol;
2-Amino-5-di(2-hydroxyethyl)amino-1-(2-methoxyphenyl)benzol;
2-Amino-5-di(2-hydroxyethyl)amino-1-(2-methylphenyl)benzol;
2-Amino-5-di(2-hydroxyethyl)amino-1-(2-nitrophenyl)benzol;
2-Amino-5-di(2-hydroxy-ethyl)amino-1-(2-trifluoromethylphenyl)benzol;
2-Amino-5-di(2-hydroxy-ethyl)amino-1-(3,4-dichlorophenyl)benzol;
2-Amino-5-di(2-hydroxyethyl)-amino-1-(3,5-diaminophenyl)benzol;
2-Amino-5-di(2-hydroxyethyl)amino-1-(3,5-dichlorophenyl)benzol;
2-Amino-5-di(2-hydroxyethyl)amino-1-(3,5-dihydroxyphenyl)benzol,
2-Amino-5-di(2-hydroxyethyl)amino-1-(3-(acetylamino)phenyl)benzol;
2-Amino-5-di(2-hydroxyethyl)amino-1-(3-aminophenyl)benzol;
2-Amino-5-di(2-hydroxyethyl)amino-1-(3-bromo-phenyl)benzol;
2-Amino-5-di(2-hydroxyethyl)amino-1-(3-carbonsäure-phenyl)benzol;
2-Amino-5-di(2-hydroxyethyl)amino-1-(3-chloro-4-fluorophenyl)benzol;
2-Amino-5-di(2-hydroxyethyl)amino-1-(3-chloro-phenyl)benzol;
2-Amino-5-di(2-hydroxyethyl)amino-1-(3-fluorophenyl)-benzol;
2-Amino-5-di(2-hydroxyethyl)amino-1-(3-formylphenyl)benzol;
2-Amino-5-di(2-hydroxyethyl)amino-1-(3-hydroxy-5-aminophenyl)benzol;
2-Amino-5-di(2-hydroxyethyl)amino-1-(3-hydroxyphenyl)benzol;
2-Amino-5-di(2-hydroxyethyl)amino-1-(3-hydroxyphenyl)benzol;
2-Amino-5-di(2-hydroxyethyl)amino-1-(3-methoxyphenyl)benzol;
2-Amino-5-di(2-hydroxyethyl)amino-1-(3-methylphenyl)benzol;
2-Amino-5-di(2-hydroxyethyl)amino-1-(3-nitrophenyl)benzol;
2-Amino-5-di(2-hydroxyethyl)amino-1-(3-trifluoromethylphenyl)benzol;
2-Amino-5-di(2-hydroxyethyl)amino-1-(4-(bromomethyl)phenyl)benzol;
2-Amino-5-di(2-hydroxyethyl)amino-1-(4-(dimethylamino)phenyl)benzol;
2-Amino-5-di(2-hydroxyethyl)amino-1-(4-(hydroxymethyl)phenyl)benzol;
2-Amino-5-di(2-hydroxyethyl)amino-1-(4-(methylthio)phenyl)benzol;
2-Amino-5-di(2-hydroxyethyl)amino-1-(4-(trifluoromethyl)phenyl)benzol;
2-Amino-5-di(2-hydroxyethyl)amino-1-(4-(trimethylsilyl)phenyl)benzol;
2-Amino-5-di(2-hydroxyethyl)amino-1-(4-acetylphenyl)benzol;
2-Amino-5-di(2-hydroxy-ethyl)amino-1-(4-aminophenyl)benzol;
2-Amino-5-di(2-hydroxyethyl)-amino-1-(4-bromophenyl)benzol;
2-Amino-5-di(2-hydroxyethyl)amino-1-(4-carbonsäurephenyl)benzol;
2-Amino-5-di(2-hydroxyethyl)amino-1-(4-chloro-3-methoxyphenyl)benzol;
2-Amino-5-di(2-hydroxyethyl)amino-1-(4-chlorophenyl)benzol;
2-Amino-5-di(2-hydroxyethyl)amino-1-(4-ethenyl-phenyl)benzol;
2-Amino-5-di(2-hydroxyethyl)amino-1-(4-fluorophenyl)-benzol;
2-Amino-5-di(2-hydroxyethyl)amino-1-(4-formylphenyl)benzol;
2-Amino-5-di(2-hydroxyethyl)amino-1-(4-hydroxyphenyl)benzol;
2-Amino-5-di(2-hydroxyethyl)amino-1-(4-iodophenyl)benzol;
2-Amino-5-di(2-hydroxyethyl)amino-1-(4-methoxyphenyl)benzol;
2-Amino-5-di(2-hydroxy-ethyl)amino-1-(4-methyl-3-nitrophenyl)benzol;
2-Amino-5-di(2-hydroxyö-ethyl)amino-1-(4-methylphenyl)benzol;
2-Amino-5-di(2-hydroxyethyl)-amino-1-(5-bromo-2-methoxyphenyl)benzol;
2-Amino-5-di(2-hydroxy-ethyl)amino-1-(5-chloro-2-methoxyphenyl)benzol;
2-Amino-5-di(2-hydroxyethyl)amino-1-(5-formyl-2-methoxyphenyl)benzol;
2-Amino-5-di(2-hydroxyethyl)amino-1-phenylbenzol;
2-Amino-5-di(2-methoxyethyl)amino-1-(2,3,4-trimethoxyphenyl)benzol;
2-Amino-5-di(2-methoxyethyl)amino-1-(2,3-difluoro-4-heptylphenyl)benzol;
2-Amino-5-di(2-methoxyethyl)amino-1-(2,3-difluorophenyl)benzol;
2-Amino-5-di(2-methoxyethyl)amino-1-(2,4,6-trimethylphenyl)benzol;
2-Amino-5-di(2-methoxyethyl)amino-1-(2,4-diaminophenyl)benzol;
2-Amino-5-di(2-methoxyethyl)amino-1-(2,4-dichlorophenyl)benzol;
2-Amino-5-di(2-methoxyethyl)amino-1-(2,4-dihydroxyphenyl)benzol;
2-Amino-5-di(2-methoxyethyl)amino-1-(2,5-dimethoxyphenyl)benzol;
2-Amino-5-di(2-methoxyethyl)amino-1-(2,6-difluorophenyl)benzol;
2-Amino-5-di(2-methoxyethyl)amino-1-(2,6-dimethoxyphenyl)benzol;
2-Amino-5-di(2-methoxyethyl)amino-1-(2-((bis(1-methylethyl)amino)-carbonyl)phenyl)benzol;
2-Amino-5-di(2-methoxy-ethyl)amino-1-(2-(bromomethyl)phenyl)benzol;
2-Amino-5-di(2-methoxyethyl)amino-1-(2-(diethylamino)carbonyl)phenyl)-benzol;
2-Amino-5-di(2-methoxyethyl)amino-1-(2-carbonsäurephenyl)benzol;
2-Amino-5-di(2-methoxyethyl)amino-1-(2-chlorophenyl)benzol;
2-Amino-5-di(2-methoxyethyl)amino-1-(2-fluorophenyl)benzol;
2-Amino-5-di(2-methoxyethyl)amino-1-(2-formyl-4-methoxyphenyl)benzol;
2-Amino-5-di(2-methoxyethyl)amino-1-(2-formyl-4-methylphenyl)benzol;
2-Amino-5-di(2-methoxyethyl)amino-1-(2-formyl-5-methoxyphenyl)benzol;
2-Amino-5-di(2-methoxyethyl)amino-1-(2-formylphenyl)benzol;
2-Amino-5-di(2-methoxy-ethyl)amino-1-(2-hydroxy-4-aminophenyl)benzol;
2-Amino-5-di(2-methoxy-ethyl)amino-1-(2-hydroxyphenyl)benzol;
2-Amino-5-di(2-methoxyethyl)-amino-1-(2-methoxyphenyl)benzol;
2-Amino-5-di(2-methoxyethyl)amino-1-(2-methylphenyl)benzol;
2-Amino-5-di(2-methoxyethyl)amino-1-(2-nitro-phenyl)benzol;
2-Amino-5-di(2-methoxyethyl)amino-1-(2-trifluoromethyl-phenyl)benzol;
2-Amino-5-di(2-methoxyethyl)amino-1-(3,4-dichloro-phenyl)benzol;
2-Amino-5-di(2-methoxyethyl)amino-1-(3,5-diaminophenyl)benzol;
2-Amino-5-di(2-methoxyethyl)amino-1-(3,5-dichlorophenyl)benzol;
2-Amino-5-di(2-methoxyethyl)amino-1-(3,5-dihydroxyphenyl)benzol;
2-Amino-5-di(2-methoxyethyl)amino-1-(3-(acetylamino)phenyl)benzol;
2-Amino-5-di(2-methoxyethyl)amino-1-(3-aminophenyl)benzol;
2-Amino-5-di(2-methoxyethyl)amino-1-(3-bromo-phenyl)benzol;
2-Amino-5-di(2-methoxyethyl)amino-1-(3-carbonsäure-phenyl)benzol;
2-Amino-5-di(2-methoxyethyl)amino-1-(3-chloro-4-fluorophenyl)benzol;
2-Amino-5-di(2-methoxyethyl)amino-1-(3-chloro-phenyl)benzol;
2-Amino-5-di(2-methoxyethyl)amino-1-(3-fluorophenyl)-benzol;
2-Amino-5-di(2-methoxyethyl)amino-1-(3-formylphenyl)benzol;
2-Amino-5-di(2-methoxyethyl)amino-1-(3-hydroxyphenyl)benzol;
2-Amino-5-di(2-methoxyethyl)amino-1-(3-methoxyphenyl)benzol;
2-Amino-5-di(2-methoxyethyl)amino-1-(3-methylphenyl)benzol;
2-Amino-5-di(2-methoxyethyl)amino-1-(3-nitrophenyl)benzol;
2-Amino-5-di(2-methoxyethyl)amino-1-(3-trifluoromethylphenyl)benzol;
2-Amino-5-di(2-methoxyethyl)amino-1-(4-(bromomethyl)phenyl)benzol;
2-Amino-5-di(2-methoxyethyl)amino-1-(4-(dimethylamino)phenyl)benzol;
2-Amino-5-di(2-methoxyethyl)amino-1-(4-(hydroxymethyl)phenyl)benzol;
2-Amino-5-di(2-methoxyethyl)amino-1-(4-(methylthio)phenyl)benzol;
2-Amino-5-di(2-methoxyethyl)amino-1-(4-(trifluoromethyl)phenyl)benzol;
2-Amino-5-di(2-methoxyethyl)amino-1-(4-(trimethylsilyl)phenyl)benzol;
2-Amino-5-di(2-methoxyethyl)amino-1-(4-acetylphenyl)benzol;
2-Amino-5-di(2-methoxy-ethyl)amino-1-(4-aminophenyl)benzol;
2-Amino-5-di(2-methoxyethyl)-amino-1-(4-bromophenyl)benzol;
2-Amino-5-di(2-methoxyethyl)amino-1-(4-carbonsäurephenyl)benzol;
2-Amino-5-di(2-methoxyethyl)amino-1-(4-chloro-3-methoxyphenyl)benzol;
2-Amino-5-di(2-methoxyethyl)amino-1-(4-chlorophenyl)benzol;
2-Amino-5-di(2-methoxyethyl)amino-1-(4-ethenylphenyl)benzol;
2-Amino-5-di(2-methoxyethyl)amino-1-(4-ethoxyphenyl)benzol;
2-Amino-5-di(2-methoxyethyl)amino-1-(4-fluorophenyl)benzol;
2-Amino-5-di(2-methoxyethyl)amino-1-(4-formylphenyl)benzol;
2-Amino-5-di(2-methoxyethyl)amino-1-(4-hydroxyphenyl)benzol;
2-Amino-5-di(2-methoxyethyl)amino-1-(4-iodophenyl)benzol;
2-Amino-5-di(2-methoxyethyl)amino-1-(4-methoxy-phenyl)benzol;
2-Amino-5-di(2-methoxyethyl)amino-1-(4-methyl-3-nitrophenyl)benzol;
2-Amino-5-di(2-methoxyethyl)amino-1-(4-methyl-phenyl)benzol;
2-Amino-5-di(2-methoxyethyl)amino-1-(5-bromo-2-methoxyphenyl)benzol;
2-Amino-5-di(2-methoxyethyl)amino-1-(5-chloro-2-methoxyphenyl)benzol;
2-Amino-5-di(2-methoxyethyl)amino-1-(5-formyl-2-methoxyphenyl)benzol;
2-Amino-5-dimethylamino-1-phenylbenzol;
2-Amino-5-methylamino-1-(2,3,4-trimethoxyphenyl)benzol;
2-Amino-5-methylamino-1-(2,4-diaminophenyl)benzol;
2-Amino-5-methylamino-1-(2,4-dihydroxyphenyl)benzol;
2-Amino-5-methylamino-1-(2,5-diamino-phenyl)benzol;
2-Amino-5-methylamino-1-(2,5-dimethoxyphenyl)benzol;
2-Amino-5-methylamino-1-(2,6-dimethoxyphenyl)benzol;
2-Amino-5-methylamino-1-(2-amino-5-hydroxyphenyl)benzol;
2-Amino-5-methyl-amino-1-(2-aminophenyl)benzol;
2-Amino-5-methylamino-1-(2-hydroxy-4-aminophenyl)benzol;
2-Amino-5-methylamino-1-(2-hydroxy-5-amino-phenyl)benzol;
2-Amino-5-methylamino-1-(2-hydroxyphenyl)benzol;
2-Amino-5-methylamino-1-(2-methoxyphenyl)benzol;
2-Amino-5-methylamino-1-(2-methylphenyl)benzol;
2-Amino-5-methylamino-1-(3,5-diaminophenyl)benzol;
2-Amino-5-methylamino-1-(3,5-dihydroxyphenyl)-benzol;
2-Amino-5-methylamino-1-(3-aminophenyl)benzol;
2-Amino-5-methylamino-1-(3-hydroxy-5-aminophenyl)benzol;
2-Amino-5-methylamino-1-(3-hydroxyphenyl)benzol;
2-Amino-5-methylamino-1-(3-methoxyphenyl)benzol;
2-Amino-5-methylamino-1-(4-(dimethylamino)-phenyl)benzol;
2-Amino-5-methylamino-1-(4-(trifluoromethyl)-phenyl)benzol;
2-Amino-5-methylamino-1-(4-aminophenyl)benzol;
2-Amino-5-methylamino-1-(4-carbonsäurephenyl)benzol;
2-Amino-5-methylamino-1-(4-chlorophenyl)benzol;
2-Amino-5-methylamino-1-(4-hydroxyphenyl)benzol;
2-Amino-5-methylamino-1-(4-methoxyphenyl)-benzol;
2-Amino-5-methylamino-1-(4-methylphenyl)benzol;
2-Amino-5-methylamino-1-phenylbenzol;
5-Amino-2-(2,3-dihydroxypropyl)amino-1-(4-bromophenyl)benzol;
5-Amino-2-(2,3-dihydroxypropyl)amino-1-(4-ethenylphenyl)benzol;
5-Amino-2-(2,3-dihydroxypropyl)amino-1-(2,3,4-trimethoxyphenyl)benzol;
5-Amino-2-(2,3-dihydroxypropyl)amino-1-(2,4-di(2-hydroxyethyl)-aminophenyl)benzol;
5-Amino-2-(2,3-dihydroxypropyl)-amino-1-(2,4-diaminophenyl)benzol;
5-Amino-2-(2,3-dihydroxypropyl)-amino-1-(2,4-dihydroxyphenyl)benzol;
5-Amino-2-(2,3-dihydroxypropyl)-amino-1-(2,4-dimethylaminophenyl)-benzol;
5-Amino-2-(2,3-dihydroxy-propyl)amino-1-(2,4-methoxyphenyl)benzol;
5-Amino-2-(2,3-dihydroxy-propyl)amino-1-(2,5-di(2-hydroxyethyl)-aminophenyl)benzol;
5-Amino-2-(2,3-dihydroxypropyl)amino-1-(2,5-diaminophenyl)benzol;
5-Amino-2-(2,3-dihydroxypropyl)amino-1-(2,5-dihydroxyphenyl)benzol;
5-Amino-2-(2,3-dihydroxypropyl)amino-1-(2,5-dimethoxyphenyl)benzol;
5-Amino-2-(2,3-dihydroxypropyl)amino-1-(2,5-dimethylaminophenyl)-benzol; 5-Amino-2-(2,3-dihydroxypropyl)amino-1-(2,6-di(2-hydroxyethyl)-aminophenyl)benzol;
5-Amino-2-(2,3-dihydroxypropyl)aminv-1-(2,6-diaminophenyl)benzol;
5-Amino-2-(2,3-dihydroxypropyl)amino-1-(2,6-dihydroxyphenyl)benzol;
5-Amino-2-(2,3-dihydroxypropyl)amino-1-(2,6-dimethoxyphenyl)benzol;
5-Amino-2-(2,3-dihydroxypropyl)amino-1-(2,6-dimethylaminophenyl)-benzol;
5-Amino-2-(2,3-dihydroxypropyl)amino-1-(2-(bromomethyl)-phenyl)-benzol;
5-Amino-2-(2,3-dihydroxypropyl)amino-1-(2-amino-5-hydroxyphenyl)benzol;
5-Amino-2-(2,3-dihydroxypropyl)amino-1-(2-aminophenyl)benzol;
5-Amino-2-(2,3-dihydroxypropyl)amino-1-(2-carbonsäurephenyl)benzol;
5-Amino-2-(2,3-dihydroxypropyl)amino-1-(2-chlorophenyl)benzol;
5-Amino-2-(2,3-dihydroxypropyl)amino-1-(2-di(2-hydroxyethyl)-aminophenyl)-benzol;
5-Amino-2-(2,3-dihydroxypropyl)-amino-1-(2-dimethylaminophenyl)benzol;
5-Amino-2-(2,3-dihydroxy-propyl)amino-1-(2-fluorophenyl)benzol;
5-Amino-2-(2,3-dihydroxy-propyl)amino-1-(2-formylphenyl)benzol;
5-Amino-2-(2,3-dihydroxy-propyl)amino-1-(2-hydroxy-4-aminophenyl)benzol;
5-Amino-2-(2,3-dihydroxypropyl)amino-1-(2-hydroxy-5-aminophenyl)benzol;
5-Amino-2-(2,3-dihydroxypropyl)amino-1-(2-hydroxyphenyl)benzol;
5-Amino-2-(2,3-dihydroxypropyl)amino-1-(2-methoxyphenyl)benzol;
5-Amino-2-(2,3-dihydroxypropyl)amino-1-(2-methylphenyl) benzol;
5-Amino-2-(2,3-dihydroxypropyl)amino-1-(2-nitrophenyl)benzol;
5-Amino-2-(2,3-dihydroxypropyl)amino-1-(2-trifluoromethylphenyl)benzol;
5-Amino-2-(2,3-dihydroxypropyl)amino-1-(3,5-di(2-hydroxyethyl)-aminophenyl)benzol;
5-Amino-2-(2,3-dihydroxypropyl)amino-1-(3,5-diamino-phenyl)benzol;
5-Amino-2-(2,3-dihydroxypropyl)amino-1-(3,5-dihydroxy-phenyl)benzol;
5-Amino-2-(2,3-dihydroxypropyl)amino-1-(3,5-dimethoxy-phenyl)benzol;
5-Amino-2-(2,3-dihydroxypropyl)amino-1-(3,5-dimethyl-aminophenyl)-benzol;
5-Amino-2-(2,3-dihydroxypropyl)amino-1-(3-aminophenyl)benzol;
5-Amino-2-(2,3-dihydroxypropyl)amino-1-(3-bromo-phenyl)benzol;
5-Amino-2-(2,3-dihydroxypropyl)amino-1-(3-carbonsäure-phenyl)benzol;
5-Amino-2-(2,3-dihydroxypropyl)amino-1-(3-chlorophenyl)-benzol;
5-Amino-2-(2,3-dihydroxypropyl)amino-1-(3-di(2-hydroxyethyl)-aminophenyl)benzol;
5-Amino-2-(2,3-dihydroxypropyl)amino-1-(3-dimethylaminophenyl)benzol;
5-Amino-2-(2,3-dihydroxypropyl)amino-1-(3-fluorophenyl)benzol;
5-Amino-2-(2,3-dihydroxypropyl)amino-1-(3-formylphenyl)benzol;
5-Amino-2-(2,3-dihydroxypropyl)amino-1-(3-hydroxy-5-aminophenyl)-benzol;
5-Amino-2-(2,3-dihydroxypropyl)amino-1-(3-hydroxyphenyl)benzol;
5-Amino-2-(2,3-dihydroxypropyl)amino-1-(3-methoxyphenyl)benzol;
5-Amino-2-(2,3-dihydroxypropyl)amino-1-(3-nitrophenyl)benzol;
5-Amino-2-(2,3-dihydroxypropyl)amino-1-(3-trifluoro-methylphenyl)benzol;
5-Amino-2-(2,3-dihydroxypropyl)amino-1-(4-(dimethylamino)phenyl)-benzol;
5-Amino-2-(2,3-dihydroxypropyl)amino-1-(4-(hydroxymethyl)phenyl)-benzol;
5-Amino-2-(2,3-dihydroxypropyl)amino-1-(4-(methylthio)phenyl)-benzol;
5-Amino-2-(2,3-dihydroxypropyl)amino-1-(4-(trifluoromethyl)phenyl)benzol;
5-Amino-2-(2,3-dihydroxypropyl)amino-1-(4-(trimethyisilyl)phenyl)benzol;
5-Amino-2-(2,3-dihydroxypropyl)amino-1-(4-acetylphenyl)benzol;
5-Amino-2-(2,3-dihydroxypropyl)amino-1-(4-aminophenyl)benzol;
5-Amino-2-(2,3-dihydroxypropyl)amino-1-(4-carbonsäurephenyl)benzol;
5-Amino-2-(2,3-dihydroxypropyl)amino-1-(4-chlorophenyl)benzol;
5-Amino-2-(2,3-dihydroxypropyl)amino-1-(4-di(2-hydroxyethyl)-aminophenyl)benzol;
5-Amino-2-(2,3-dihydroxypropyl)-amino-1-(4-dimethylaminophenyl)benzol;
5-Amino-2-(2,3-dihydroxy-propyl)amino-1-(4-ethoxyphenyl)benzol;
5-Amino-2-(2,3-dihydroxy-propyl)amino-1-(4-fluorophenyl)benzol;
5-Amino-2-(2,3-dihydroxy-propyl)amino-1-(4-formylphenyl)benzol;
5-Amino-2-(2,3-dihydroxypropyl)-amino-1-(4-hydroxyphenyl)benzol;
5-Amino-2-(2,3-dihydroxypropyl)amino-1-(4-methoxyphenyl)benzol;
5-Amino-2-(2,3-dihydroxypropyl)amino-1-(4-methylphenyl)benzol;
5-Amino-2-(2,3-dihydroxypropyl)amino-1-phenylbenzol;
5-Amino-2-(2-hydroxyethyl)amino-1-(4-bromophenyl)-benzol;
5-Amino-2-(2-hydroxyethyl)amino-1-(4-ethenylphenyl)benzol;
5-Amino-2-(2-hydroxyethyl)amino-1-(2,3,4-trimethoxyphenyl)benzol;
5-Amino-2-(2-hydroxyethyl)amino-1-(2,4-di(2-hydroxyethyl)aminophenyl)-benzol;
5-Amino-2-(2-hydroxyethyl)amino-1-(2,4-diaminophenyl)benzol;
5-Amino-2-(2-hydroxyethyl)amino-1-(2,4-dihydroxyphenyl)benzol;
5-Amino-2-(2-hydroxyethyl)amino-1-(2,4-dimethylaminophenyl)benzol;
5-Amino-2-(2-hydroxyethyl)amino-1-(2,4-methoxyphenyl)benzol;
5-Amino-2-(2-hydroxyethyl)amino-1-(2,5-di(2-hydroxyethyl)aminophenyl)-benzol;
5-Amino-2-(2-hydroxyethyl)amino-1-(2,5-diaminophenyl)benzol;
5-Amino-2-(2-hydroxyethyl)amino-1-(2,5-dihydroxyphenyl)benzol;
5-Amino-2-(2-hydroxyethyl)amino-1-(2,5-dimethoxyphenyl)benzol;
5-Amino-2-(2-hydroxyethyl)amino-1-(2,5-dimethylaminophenyl)benzol;
5-Amino-2-(2-hydroxyethyl)amino-1-(2,6-di(2-hydroxyethyl)aminophenyl)-benzol;
5-Amino-2-(2-hydroxyethyl)amino-1-(2,6-diaminophenyl)benzol;
5-Amino-2-(2-hydroxyethyl)amino-1-(2,6-dihydroxyphenyl)benzol;
5-Amino-2-(2-hydroxyethyl)amino-1-(2,6-dimethoxyphenyl)benzol;
5-Amino-2-(2-hydroxyethyl)amino-1-(2,6-dimethylaminophenyl)benzol;
5-Amino-2-(2-hydroxyethyl)amino-1-(2-(bromomethyl)phenyl)benzol;
5-Amino-2-(2-hydroxyethyl)amino-1-(2-amino-5-hydroxyphenyl)benzol;
5-Amino-2-(2-hydroxyethyl)amino-1-(2-aminophenyl)benzol;
5-Amino-2-(2-hydroxy-ethyl)amino-1-(2-carbonsäurephenyl)benzol;
5-Amino-2-(2-hydroxyethyl)-amino-1-(2-chlorophenyl)benzol;
5-Amino-2-(2-hydroxyethyl)amino-1-(2-di(2-hydroxyethyl)aminophenyl)-benzol;
5-Amino-2-(2-hydroxyethyl)amino-1-(2-dimethylaminophenyl)benzol;
5-Amino-2-(2-hydroxyethyl)amino-1-(2-fluorophenyl)benzol;
5-Amino-2-(2-hydroxyethyl)amino-1-(2-formylphenyl)-benzol;
5-Amino-2-(2-hydroxyethyl)amino-1-(2-hydroxy-4-aminophenyl)-benzol;
5-Amino-2-(2-hydroxyethyl)amino-1-(2-hydroxy-5-aminophenyl)-benzol;
5-Amino-2-(2-hydroxyethyl)amino-1-(2-hydroxyphenyl)benzol;
5-Amino-2-(2-hydroxyethyl)amino-1-(2-methoxyphenyl)benzol;
5-Amino-2-(2-hydroxyethyl)amino-1-(2-methylphenyl)benzol;
5-Amino-2-(2-hydroxy-ethyl)amino-1-(2-nitrophenyl)benzol;
5-Amino-2-(2-hydroxyethyl)amino-1-(2-trifluoromethylphenyl)benzol;
5-Amino-2-(2-hydroxyethyl)amino-1-(3,5-di(2-hydroxyethyl)-aminophenyl)-benzol;
5-Amino-2-(2-hydroxyethyl)-amino-1-(3,5-diaminophenyl)benzol;
5-Amino-2-(2-hydroxyethyl)amino-1-(3,5-dihydroxyphenyl)benzol;
5-Amino-2-(2-hydroxyethyl)amino-1-(3,5-dimethoxyphenyl)benzol;
5-Amino-2-(2-hydroxyethyl)amino-1-(3,5-dimethylaminophenyl)benzol;
5-Amino-2-(2-hydroxyethyl)amino-1-(3-aminophenyl)benzol;
5-Amino-2-(2-hydroxyethyl)amino-1-(3-bromo-phenyl)benzol;
5-Amino-2-(2-hydroxyethyl)amino-1-(3-carbonsäure-phenyl)benzol;
5-Amino-2-(2-hydroxyethyl)amino-1-(3-chlorophenyl)-benzol;
5-Amino-2-(2-hydroxyethyl)amino-1-(3-di(2-hydroxyethyl)-aminophenyl)-benzol;
5-Amino-2-(2-hydroxyethyl)amino-1-(3-dimethylaminophenyl)benzol;
5-Amino-2-(2-hydroxyethyl)amino-1-(3-fluorophenyl)benzol;
5-Amino-2-(2-hydroxyethyl)amino-1-(3-formylphenyl)benzol;
5-Amino-2-(2-hydroxyethyl)amino-1-(3-hydroxy-5-aminophenyl)benzol;
5-Amino-2-(2-hydroxyethyl)amino-1-(3-hydroxy-phenyl)benzol;
5-Amino-2-(2-hydroxyethyl)amino-1-(3-methoxyphenyl)-benzol;
5-Amino-2-(2-hydroxyethyl)amino-1-(3-nitrophenyl)benzol;
5-Amino-2-(2-hydroxyethyl)amino-1-(3-trifluoromethylphenyl)benzol;
5-Amino-2-(2-hydroxyethyl)amino-1-(4-(dimethylamino)phenyl)benzol;
5-Amino-2-(2-hydroxyethyl)amino-1-(4-(hydroxymethyl)phenyl)benzol;
5-Amino-2-(2-hydroxyethyl)amino-1-(4-(methylthio)phenyl)benzol;
5-Amino-2-(2-hydroxyethyl)amino-1-(4-(trifluoromethyl)phenyl)benzol;
5-Amino-2-(2-hydroxyethyl)amino-1-(4-(trimethylsilyl)phenyl)benzol;
5-Amino-2-(2-hydroxyethyl)amino-1-(4-acetylphenyl)benzol;
5-Amino-2-(2-hydroxyethyl)amino-1-(4-aminophenyl)benzol;
5-Amino-2-(2-hydroxy-ethyl)amino-1-(4-carbonsäurephenyl)benzol;
5-Amino-2-(2-hydroxyethyl)-amino-1-(4-chlorophenyl)benzol;
5-Amino-2-(2-hydroxyethyl)amino-1-(4-di(2-hydroxyethyl)aminophenyl)-benzol;
5-Amino-2-(2-hydroxyethyl)amino-1-(4-dimethylaminophenyl)benzol;
5-Amino-2-(2-hydroxyethyl)amino-1-(4-ethoxyphenyl)benzol;
5-Amino-2-(2-hydroxyethyl)amino-1-(4-fluorophenyl)benzol;
5-Amino-2-(2-hydroxyethyl)amino-1-(4-formylphenyl)- benzol;
5-Amino-2-(2-hydroxyethyl)amino-1-(4-hydroxyphenyl)benzol;
5-Amino-2-(2-hydroxyethyl)amino-1-(4-methoxyphenyl)benzol;
5-Amino-2-(2-hydroxyethyl)amino-1-(4-methylphenyl)benzol;
5-Amino-2-(2-hydroxy-ethyl)amino-1-phenylbenzol;
5-Amino-2-(2-methoxyethyl)amino-1-(2,3,4-trimethoxyphenyl)benzol;
5-Amino-2-(2-methoxyethyl)amino-1-(2,4-diaminophenyl)benzol;
5-Amino-2-(2-methoxyethyl)amino-1-(2,4-dihydroxyphenyl)benzol;
5-Amino-2-(2-methoxyethyl)amino-1-(2,5-diaminophenyl)benzol;
5-Amino-2-(2-methoxyethyl)amino-1-(2,5-dimethoxyphenyl)benzol;
5-Amino-2-(2-methoxyethyl)amino-1-(2,6-dimethoxyphenyl)benzol;
5-Amino-2-(2-methoxyethyl)amino-1-(2-amino-5-hydroxyphenyl)benzol;
5-Amino-2-(2-methoxyethyl)amino-1-(2-amino-phenyl)benzol;
5-Amino-2-(2-methoxyethyl)amino-1-(2-hydroxy-4-amino-phenyl)benzol;
5-Amino-2-(2-methoxyethyl)amino-1-(2-hydroxy-5-amino-phenyl)benzol;
5-Amino-2-(2-methoxyethyl)amino-1-(2-hydroxyphenyl)-benzol;
5-Amino-2-(2-methoxyethyl)amino-1-(2-methoxyphenyl)benzol;
5-Amino-2-(2-methoxyethyl)amino-1-(2-methylphenyl)benzol;
5-Amino-2-(2-methoxyethyl)amino-1-(3,5-diaminophenyl)benzol;
5-Amino-2-(2-methoxyethyl)amino-1-(3,5-dihydroxyphenyl)benzol;
5-Amino-2-(2-methoxyethyl)amino-1-(3-aminophenyl)benzol;
5-Amino-2-(2-methoxy-ethyl)amino-1-(3-hydroxy-5-aminophenyl)benzol;
5-Amino-2-(2-methoxy-ethyl)amino-1-(3-hydroxyphenyl)benzol;
5-Amino-2-(2-methoxyethyl)-amino-1-(3-methoxyphenyl)benzol;
5-Amino-2-(2-methoxyethyl)amino-1-(4-(dimethylamino)phenyl)benzol;
5-Amino-2-(2-methoxyethyl)amino-1-(4-(trifluoromethyl)phenyl)benzol;
5-Amino-2-(2-methoxyethyl)amino-1-(4-aminophenyl)benzol;
5-Amino-2-(2-methoxyethyl)amino-1-(4-carbonsäure-phenyl)benzol;
5-Amino-2-(2-methoxyethyl)amino-1-(4-chlorophenyl)-benzol;
5-Amino-2-(2-methoxyethyl)amino-1-(4-hydroxyphenyl)benzol;
5-Amino-2-(2-methoxyethyl)amino-1-(4-methoxyphenyl)benzol;
5-Amino-2-(2-methoxyethyl)amino-1-(4-methylphenyl)benzol;
5-Amino-2-(2-methoxyethyl)amino-1-phenylbenzol;
5-Amino-2-di(2-hydroxyethyl)amino-1-(2,3,4-trimethoxyphenyl)benzol;
5-Amino-2-di(2-hydroxyethyl)amino-1-(2,3-difluoro-4-heptylphenyl)benzol; 5-Amino-2-di(2-hydroxyethyl)amino-1-(2,3-difluorophenyl)benzol;
5-Amino-2-di(2-hydroxyethyl)amino-1-(2,4,6-trimethylphenyl)benzol;
5-Amino-2-di(2-hydroxyethyl)amino-1-(2,4-diaminophenyl)benzol;
5-Amino-2-di(2-hydroxyethyl)amino-1-(2,4-dichlorophenyl)benzol;
5-Amino-2-di(2-hydroxyethyl)amino-1-(2,4-dihydroxyphenyl)benzol;
5-Amino-2-di(2-hydroxyethyl)amino-1-(2,5-dimethoxyphenyl)benzol;
5-Amino-2-di(2-hydroxyethyl)amino-1-(2,6-difluorophenyl)benzol;
5-Amino-2-di(2-hydroxyethyl)amino-1-(2,6-dimethoxyphenyl)benzol;
5-Amino-2-di(2-hydroxyethyl)amino-1-(2-((bis(1-methylethyl)amino)-carbonyl)phenyl)benzol;
5-Amino-2-di(2-hydroxyethyl)-amino-1-(2-((bis(1-methylethyl)amino)-carbonyl)-3-methoxyphenyl)benzol;
5-Amino-2-di(2-hydroxyethyl)amino-1-(2-(bromomethyl)phenyl)benzol;
5-Amino-2-di(2-hydroxyethyl)amino-1-(2-(diethylamino)carbonyl)phenyl)-benzol;
5-Amino-2-di(2-hydroxyethyl)amino-1-(2-carbonsäurephenyl)-benzol;
5-Amino-2-di(2-hydroxyethyl)amino-1-(2-chlorophenyl)benzol;
5-Amino-2-di(2-hydroxyethyl)amino-1-(2-fluorophenyl)benzol;
5-Amino-2-di(2-hydroxyethyl)amino-1-(2-formyl-4-methoxyphenyl)benzol; 5-Amino-2-di(2-hydroxyethyl)amino-1-(2-formyl-4-methylphenyl)benzol;
5-Amino-2-di(2-hydroxyethyl)amino-1-(2-formyl-5-methoxyphenyl)benzol; 5-Amino-2-di(2-hydroxyethyl)amino-1-(2-formylphenyl)benzol;
5-Amino-2-di(2-hydroxyethyl)amino-1-(2-hydroxy-4-aminophenyl)benzol; 5-Amino-2-di(2-hydroxyethyl)amino-1-(2-hydroxyphenyl)benzol;
5-Amino-2-di(2-hydroxy-ethyl)amino-1-(2-methoxyphenyl)benzol;
5-Amino-2-di(2-hydroxyethyl)-amino-1-(2-methylphenyl)benzol;
5-Amino-2-di(2-hydroxyethyl)amino-1-(2-nitrophenyl)benzol;
5-Amino-2-di(2-hydroxyethyl)amino-1-(2-trifluoro-methylphenyl)benzol;
5-Amino-2-di(2-hydroxyethyl)amino-1-(3,4-dichlorophenyl)benzol;
5-Amino-2-di(2-hydroxyethyl)amino-1-(3,5-diaminophenyl)benzol;
5-Amino-2-di(2-hydroxyethyl)amino-1-(3,5-dichlorophenyl)benzol;
5-Amino-2-di(2-hydroxyethyl)amino-1-(3,5-dihydroxyphenyl)benzol;
5-Amino-2-di(2-hydroxyethyl)amino-1-(3-(acetylamino)phenyl)benzol;
5-Amino-2-di(2-hydroxyethyl)amino-1-(3-aminophenyl)benzol;
5-Amino-2-di(2-hydroxyethyl)amino-1-(3-bromo-phenyl)benzol;
5-Amino-2-di(2-hydroxyethyl)amino-1-(3-carbonsäure-phenyl)benzol;
5-Amino-2-di(2-hydroxyethyl)amino-1-(3-chloro-4-fluorophenyl)benzol;
5-Amino-2-di(2-hydroxyethyl)amino-1-(3-chloro-phenyl)benzol;
5-Amino-2-di(2-hydroxyethyl)amino-1-(3-fluorophenyl)-benzol;
5-Amino-2-di(2-hydroxyethyl)amino-1-(3-formylphenyl)benzol;
5-Amino-2-di(2-hydroxyethyl)amino-1-(3-hydroxyphenyl)benzol;
5-Amino-2-di(2-hydroxyethyl)amino-1-(3-methoxyphenyl)benzol;
5-Amino-2-di(2-hydroxyethyl)amino-1-(3-methylphenyl)benzol;
5-Amino-2-di(2-hydroxy-ethyl)amino-1-(3-nitrophenyl)benzol;
5-Amino-2-di(2-hydroxyethyl)amino-1-(3-trifluoromethylphenyl)benzol;
5-Amino-2-di(2-hydroxyethyl)amino-1-(4-(bromomethyl)phenyl)benzol;
5-Amino-2-di(2-hydroxyethyl)amino-1-(4-(dimethylamino)phenyl)benzol;
5-Amino-2-di(2-hydroxyethyl)amino-1-(4-(hydroxymethyl)phenyl)benzol;
5-Amino-2-di(2-hydroxyethyl)amino-1-(4-(methylthio)phenyl)benzol;
5-Amino-2-di(2-hydroxyethyl)amino-1-(4-(trifluoromethyl)phenyl)benzol;
5-Amino-2-di(2-hydroxyethyl)amino-1-(4-(trimethylsilyl)phenyl)benzol;
5-Amino-2-di(2-hydroxyethyl)amino-1-(4-acetylphenyl)benzol;
5-Amino-2-di(2-hydroxyethyl)amino-1-(4-amino-phenyl)benzol;
5-Amino-2-di(2-hydroxyethyl)amino-1-(4-bromophenyl)-benzol;
5-Amino-2-di(2-hydroxyethyl)amino-1-(4-carbonsäurephenyl)-benzol;
5-Amino-2-di(2-hydroxyethyl)amino-1-(4-chloro-3-methoxyphenyl)-benzol;
5-Amino-2-di(2-hydroxyethyl)amino-1-(4-chlorophenyl)benzol;
5-Amino-2-di(2-hydroxyethyl)amino-1-(4-ethenylphenyl)benzol;
5-Amino-2-di(2-hydroxyethyl)amino-1-(4-ethoxyphenyl)benzol;
5-Amino-2-di(2-hydroxyethyl)amino-1-(4-fluorophenyl)benzol;
5-Amino-2-di(2-hydroxy-ethyl)amino-1-(4-formylphenyl)benzol;
5-Amino-2-di(2-hydroxyethyl)-amino-1-(4-hydroxyphenyl)benzol;
5-Amino-2-di(2-hydroxyethyl)amino-1-(4-iodophenyl)benzol;
5-Amino-2-di(2-hydroxyethyl)amino-1-(4-methoxy-phenyl)benzol;
5-Amino-2-di(2-hydroxyethyl)amino-1-(4-methyl-3-nitrophenyl)benzol;
5-Amino-2-di(2-hydroxyethyl)amino-1-(4-methyl-phenyl)benzol;
5-Amino-2-di(2-hydroxyethyl)amino-1-(5-bromo-2-methoxy-phenyl)benzol;
5-Amino-2-di(2-hydroxyethyl)amino-1-(5-chloro-2-methoxy-phenyl)benzol;
5-Amino-2-di(2-hydroxyethyl)amino-1-(5-formyl-2-methoxy-phenyl)benzol;
5-Amino-2-di(2-methoxyethyl)amino-1-(2,3,4-trimethoxy-phenyl)benzol;
5-Amino-2-di(2-methoxyethyl)amino-1-(2,3-difluoro-4-heptylphenyl)benzol;
5-Amino-2-di(2-methoxyethyl)amino-1-(2,3-difluorophenyl)benzol;
5-Amino-2-di(2-methoxyethyl)amino-1-(2,4,6-trimethylphenyl)benzol;
5-Amino-2-di(2-methoxyethyl)amino-1-(2,4-diaminophenyl)benzol;
5-Amino-2-di(2-methoxyethyl)amino-1-(2,4-dichlorophenyl)benzol;
5-Amino-2-di(2-methoxyethyl)amino-1-(2,4-dihydroxyphenyl)benzol;
5-Amino-2-di(2-methoxyethyl)amino-1-(2,5-dimethoxyphenyl)benzol;
5-Amino-2-di(2-methoxyethyl)amino-1-(2,6-difluorophenyl)benzol;
5-Amino-2-di(2-methoxyethyl)amino-1-(2,6-dimethoxyphenyl)benzol;
5-Amino-2-di(2-methoxyethyl)amino-1-(2-((bis(1-methylethyl)amino)-carbonyl)phenyl)benzol;
5-Amino-2-di(2-methoxy-ethyl)amino-1-(2-((bis(1-methylethyl)amino)-carbonyl)-3-methoxyphenyl)-benzol;
5-Amino-2-di(2-methoxyethyl)amino-1-(2-(bromomethyl)phenyl)-benzol;
5-Amino-2-di(2-methoxyethyl)amino-1-(2-(diethylamino)carbonyl)-phenyl)-benzol;
5-Amino-2-di(2-methoxyethyl)amino-1-(2-carbonsäure-phenyl)benzol;
5-Amino-2-di(2-methoxyethyl)amino-1-(2-chlorophenyl)-benzol;
5-Amino-2-di(2-methoxyethyl)amino-1-(2-fluorophenyl)benzol;
5-Amino-2-di(2-methoxyethyl)amino-1-(2-formyl-4-methoxyphenyl)benzol;
5-Amino-2-di(2-methoxyethyl)amino-1-(2-formyl-4-methylphenyl)benzol;
5-Amino-2-di(2-methoxyethyl)amino-1-(2-formyl-5-methoxyphenyl)benzol;
5-Amino-2-di(2-methoxyethyl)amino-1-(2-formylphenyl)benzol;
5-Amino-2-di(2-methoxyethyl)amino-1-(2-hydroxy-4-aminophenyl)benzol;
5-Amino-2-di(2-methoxyethyl)amino-1-(2-hydroxyphenyl)benzol;
5-Amino-2-di(2-methoxyethyl)amino-1-(2-methoxyphenyl)benzol;
5-Amino-2-di(2-methoxyethyl)amino-1-(2-methylphenyl)benzol;
5-Amino-2-di(2-methoxyethyl)amino-1-(2-nitrophenyl)benzol;
5-Amino-2-di(2-methoxy-ethyl)amino-1-(2-trifluoromethylphenyl)benzol;
5-Amino-2-di(2-methoxy-ethyl)amino-1-(3,4-dichlorophenyl)benzol;
5-Amino-2-di(2-methoxyethyl)-amino-1-(3,5-diaminophenyl)benzol;
5-Amino-2-di(2-methoxyethyl)amino-1-(3,5-dichlorophenyl)benzol;
5-Amino-2-di(2-methoxyethyl)amino-1-(3,5-dihydroxyphenyl)benzol;
5-Amino-2-di(2-methoxyethyl)amino-1-(3-(acetylamino)phenyl)benzol;
5-Amino-2-di(2-methoxyethyl)amino-1-(3-aminophenyl)benzol;
5-Amino-2-di(2-methoxyethyl)amino-1-(3-bromophenyl)benzol;
5-Amino-2-di(2-methoxyethyl)amino-1-(3-carbonsäurephenyl)benzol;
5-Amino-2-di(2-methoxyethyl)amino-1-(3-chloro-4-fluorophenyl)benzol;
5-Amino-2-di(2-methoxyethyl)amino-1-(3-chlorophenyl)benzol;
5-Amino-2-di(2-methoxyethyl)amino-1-(3-fluoro-phenylbenzol;
5-Amino-2-di(2-methoxyethyl)amino-1-(3-formylphenyl)-benzol;
5-Amino-2-di(2-methoxyethyl)amino-1-(3-hydroxyphenyl)benzol;
5-Amino-2-di(2-methoxyethyl)amino-1-(3-methoxyphenyl)benzol;
5-Amino-2-di(2-methoxyethyl)amino-1-(3-methylphenyl)benzol;
5-Amino-2-di(2-methoxyethyl)amino-1-(3-nitrophenyl)benzol;
5-Amino-2-di(2-methoxy-ethyl)amino-1-(3-trifluoromethylphenyl)benzol;
5-Amino-2-di(2-methoxyethyl)amino-1-(4-(bromomethyl)phenyl)benzol;
5-Amino-2-di(2-methoxyethyl)amino-1-(4-(dimethylamino)phenyl)benzol;
5-Amino-2-di(2-methoxyethyl)amino-1-(4-(hydroxymethyl)phenyl)benzol;
5-Amino-2-di(2-methoxyethyl)amino-1-(4-(methylthio)phenyl)benzol;
5-Amino-2-di(2-methoxyethyl)amino-1-(4-(trifluoromethyl)phenyl)benzol;
5-Amino-2-di(2-methoxyethyl)amino-1-(4-(trimethylsilyl)phenyl)benzol;
5-Amino-2-di(2-methoxyethyl)amino-1-(4-acetylphenyl)benzol;
5-Amino-2-di(2-methoxy-ethyl)amino-1-(4-aminophenyl)benzol;
5-Amino-2-di(2-methoxyethyl)-amino-1-(4-bromophenyl)benzol;
5-Amino-2-di(2-methoxyethyl)amino-1-(4-carbonsäurephenyl)benzol;
5-Amino-2-di(2-methoxyethyl)amino-1-(4-chloro-3-methoxyphenyl)benzol;
5-Amino-2-di(2-methoxyethyl)amino-1-(4-chlorophenyl)benzol;
5-Amino-2-di(2-methoxyethyl)amino-1-(4-ethenyl-phenyl)benzol;
5-Amino-2-di(2-methoxyethyl)amino-1-(4-ethoxyphenyl)-benzol;
5-Amino-2-di(2-methoxyethyl)amino-1-(4-fluorophenyl)benzol;
5-Amino-2-di(2-methoxyethyl)amino-1-(4-formylphenyl)benzol;
5-Amino-2-di(2-methoxyethyl)amino-1-(4-hydroxyphenyl)benzol;
5-Amino-2-di(2-methoxyethyl)amino-1-(4-iodophenyl)benzol;
5-Amino-2-di(2-methoxy-ethyl)amino-1-(4-methoxyphenyl)benzol;
5-Amino-2-di(2-methoxyethyl)-amino-1-(4-methyl-3-nitrophenyl)benzol;
5-Amino-2-di(2-methoxyethyl)-amino-1-(4-methylphenyl)benzol;
5-Amino-2-di(2-methoxyethyl)amino-1-(5-bromo-2-methoxyphenyl)benzol;
5-Amino-2-di(2-methoxyethyl)amino-1-(5-chloro-2-methoxyphenyl)benzol;
5-Amino-2-di(2-methoxyethyl)amino-1-(5-formyl-2-methoxyphenyl)benzol;
5-Amino-2-dimethylamino-1-phenyl-benzol;
5-Amino-2-methylamino-1-(2,3,4-trimethoxyphenyl)benzol;
5-Amino-2-methylamino-1-(2,4-diaminophenyl)benzol;
5-Amino-2-methylamino-1-(2,4-dihydroxyphenyl)benzol;
5-Amino-2-methylamino-1-(2,5-diaminophenyl)benzol;
5-Amino-2-methylamino-1-(2,5-dimethoxy-phenyl)benzol;
5-Amino-2-methylamino-1-(2,6-dimethoxyphenyl)benzol;
5-Amino-2-methylamino-1-(2-amino-5-hydroxyphenyl)benzol;
5-Amino-2-methylamino-1-(2-aminophenyl)benzol;
5-Amino-2-methylamino-1-(2-hydroxy-4-aminophenyl)benzol;
5-Amino-2-methylamino-1-(2-hydroxy-5-aminophenyl)benzol;
5-Amino-2-methylamino-1-(2-hydroxyphenyl)benzol;
5-Amino-2-methylamino-1-(2-methoxyphenyl)benzol;
5-Amino-2-methyl-amino-1-(2-methylphenyl)benzol;
5-Amino-2-methylamino-1-(3,5-diamino-phenyl)benzol;
5-Amino-2-methylamino-1-(3,5-dihydroxyphenyl)benzol;
5-Amino-2-methylamino-1-(3-aminophenyl)benzol;
5-Amino-2-methyl-amino-1-(3-hydroxy-5-aminophenyl)benzol;
5-Amino-2-methylamino-1-(3-hydroxyphenyl)benzol;
5-Amino-2-methylamino-1-(3-methoxyphenyl)-benzol;
5-Amino-2-methylamino-1-(4-(dimethylamino)phenyl)benzol;
5-Amino-2-methylamino-1-(4-(trifluoromethyl)phenyl)benzol;
5-Amino-2-methylamino-1-(4-aminophenyl)benzol;
5-Amino-2-methylamino-1-(4-carbonsäurephenyl)benzol;
5-Amino-2-methylamino-1-(4-chlorophenyly-benzol;
5-Amino-2-methylamino-1-(4-hydroxyphenyl)benzol;
5-Amino-2-methylamino-1-(4-methoxyphenyl)benzol;
5-Amino-2-methylamino-1-(4-methylphenyl)benzol und
5-Amino-2-methylamino-1-phenylbenzol, oder deren physiologisch verträglichen Salzen enthält.

2. Mittel nach Anspruch 1, **dadurch gekennzeichnet, dass** die Entwicklersubstanz ausgewählt ist aus 2,5-Diamino-1-phenylbenzol; 2,5-Diamino-1-(3-nitro-phenyl)benzol; 2,5-Diamino-1-(4-methoxyphenyl)benzol; 2,5-Diamino-1-(3-methoxy-phenyl)benzol; 2,5-Diamino-1-(3-amino-phenyl)benzol; 2,5-Diamino-1-(2-methyl-phenyl)-benzol; 2,5-Diamino-1-(3-methyl-phenyl)-benzol; 2,5-Diamino-1-(4-methylphenyl)benzol; 2,5-Diamino-1-(3-chlor-phenyl)benzol und 2,5-Diamino-1-(4-chlor-phenyl)benzol, oder deren physiologisch verträglichen Salzen.

3. Mittel nach Anspruch 1 oder 2, **dadurch gekennzeichnet, dass** das Diaminobenzol-Derivat der Formel (I) in einer Menge von 0,005 bis 20,0 Gewichtsprozent enthält.

4. Mittel nach einem der Ansprüche 1 bis 3, **dadurch gekennzeichnet, daß** es außer der Entwicklersubstanz gemäß Anspruch 1 oder 2 zusätzlich mindestens eine weitere Entwicklersubstanz, welche ausgewählt ist aus 1,4-Diaminobenzol, 2,5-Diaminotoluol, 2,5-Diaminophenylethylalkohol, 4-Aminophenol und seinen Derivaten, 4,5-Diaminopyrazolderivaten und Tetraaminopyrimidinen, enthält.

5. Mittel nach einem der Ansprüche 1 bis 4, **dadurch gekennzeichnet, daß** die Entwicklersubstanzen und Kupplersubstanzen, bezogen auf die Gesamtmenge des Oxidationsfärbemittel jeweils in einer Gesamtmenge von 0,005 bis 20 Gewichtsprozent enthalten sind.,

6. Mittel nach einem der Ansprüche 1 bis 5, **dadurch gekennzeichnet, daß** es zusätzlich mindestens einen direktziehenden Farbstoff enthält.

7. Mittel nach einem der Ansprüche 1 bis 6, **dadurch gekennzeichnet, daß** es einen pH-Wert von 6,8 bis 11,5 aufweist.

8. Mittel nach einem der Ansprüche 1 bis 7, **dadurch gekennzeichnet, daß** es in Form einer wäßrigen oder wäßrig-alkoholischen Lösung, einer Creme, eines Gels oder einer Emulsion vorliegt.

9. Mittel nach einem der Ansprüche 1 bis 8, **dadurch gekennzeichnet, daß** es ein Haarfärbemittel ist.

10. Verbindung, welche ausgewählt ist aus der Gruppe bestehend aus
2,5-Diamino-1-(4-bromophenyl)benzol;
2,5-Diamino-1-(4-ethenylphenyl)-benzol;
2,5-Diamino-1-(2,3,4-trimethoxy-phenyl)benzol;
2,5-Diamino-1-(2,4-di(2-hydroxyethyl)aminophenyl)benzol;
2,5-Diamino-1-(2,4-diamino-phenyl)benzol;
2,5-Diamino-1-(2,4-dihydroxy-phenyl)benzol;
2,5-Diamino-1-(2,4-dimethylaminophenyl)benzol;
2,5-Diamino-1-(2,4-methoxyphenyl)-benzol;
2,5-Diamino-1-(2,5-di(2-hydroxyethyl)aminophenyl)benzol;
2,5-Diamino-1-(2,5-diaminophenyl)-benzol;
2,5-Diamino-1-(2,5-dihydroxy-phenyl)benzol;
2,5-Diamino-1-(2,5-dimethoxyphenyl)benzol;
2,5-Diamino-1-(2,5-dimethylaminophenyl)benzol;
2,5-Diamino-1-(2,6-di(2-hydroxyethyl)-aminophenyl)benzol;
2,5-Diamino-1-(2,6-diaminophenyl)-benzol;
2,5-Diamino-1-(2,6-dihydroxyphenyl)-benzol;
2,5-Diamino-1-(2,6-dimethoxyphenyl)benzol;
2,5-Diamino-1-(2,6-dimethylaminophenyl)benzol;
2,5-Diamino-1-(2-(bromomethyl)phenyl)-benzol;
2,5-Diamino-1-(2-amino-5-hydroxyphenyl)benzol;
2,5-Diamino-1-(2-aminophenyl)benzol;
2,5-Diamino-1-(2-carbonsäurephenyl)benzol;
2,5-Diamino-1-(2-chlor-phenyl)benzol;
2,5-Diamino-1-(2-di(2-hydroxyethyl)aminophenyl)benzol;
2,5-Diamino-1-(2-dimethylamino-phenyl)benzol;
2,5-Diamino-1-(2-fluoro-phenyl)benzol;
2,5-Diamino-1-(2-formylphenyl)benzol;
2,5-Diamino-1-(2-hydroxy-4-aminophenyl)benzol;
2,5-Diamino-1-(2-hydroxy-5-amino-phenyl)-benzol;
2,5-Diamino-1-(2-hydroxyphenyl)benzol;
2,5-Diamino-1-(2-methoxyphenyl)benzol;
2,5-Diamino-1-(2-methylphenyl)benzol;
2,5-Diamino-1-(2-nitrophenyl)benzol;
2,5-Diamino-1-(2-trifluoromethyl-phenyl)benzol;
2,5-Diamino-1-(3,5-di(2-hydroxyethyl)aminophenyl)benzol;
2,5-Diamino-1-(3,5-diaminophenyl)-benzol;
2,5-Diamino-1-(3,5-dihydroxy-phenyl)benzol;
2,5-Diamino-1-(3,5-dimethoxyphenyl)benzol;
2,5-Diamino-1-(3,5-dimethylaminophenyl)benzol;
2,5-Diamino-1-(3-aminophenyl)-benzol;
2,5-Diamino-1-(3-bromophenyl)-benzol;
2,5-Diamino-1-(3-carbonsäurephenyl)benzol;
2,5-Diamino-1-(3-chlorophenyl)benzol;
2,5-Diamino-1-(3-di(2-hydroxyethyl)aminophenyl)-benzol;
2,5-Diamino-1-(3-dimethylaminophenyl)benzol;
2,5-Diamino-1-(3-fluorophenyl)benzol;
2,5-Diamino-1-(3-formylphenyl)benzol;
2,5-Diamino-1-(3-hydroxy-5-aminophenyl)benzol;
2,5-Diamino-1-(3-hydroxyphenyl)-benzol;
2,5-Diamino-1-(3-methoxyphenyl)benzol;
2,5-Diamino-1-(3-nitrophenyl)benzol;
2,5-Diamino-1-(3-trifluoromethylphenyl)benzol;
2,5-Diamino-1-(4-(dimethylamino)phenyl)benzol;
2,5-Diamino-1-(4-(hydroxymethyl)phenyl)benzol;
2,5-Diamino-1-(4-(methylthio)phenyl)-benzol;
2,5-Diamino-1-(4-(trifluoromethyl)phenyl)benzol;
2,5-Diamino-1-(4-(trimethylsilyl)phenyl)benzol;
2,5-Diamino-1-(4-acetylphenyl)benzol;
2,5-Diamino-1-(4-aminophenyl)benzol;
2,5-Diamino-1-(4-carbonsäure-phenyl)benzol;
2,5-Diamino-1-(4-chlorophenyl)benzol;
2,5-Diamino-1-(4-di(2-hydroxyethyl)aminophenyl)benzol;
2,5-Diamino-1-(4-dimethylamino-phenyl)benzol;
2,5-Diamino-1-(4-ethoxyphenyl)benzol;
2,5-Diamino-1-(4-fluorophenyl)benzol;
2,5-Diamino-1-(4-formylphenyl)benzol;
2,5-Diamino-1-(4-hydroxyphenyl)benzol;
2,5-Diamino-1-(4-methoxy-phenyl)benzol;
2,5-Diamino-1-(4-methylphenyl)benzol;
2,5-Diamino-1-phenylbenzol; 2,5-Diamino-4-chlor-1-phenylbenzol;
2,5-Diamino-4-methoxy-1-phenylbenzol;
2,5-Diamino-4-methyl-1-phenylbenzol;
2,5-Diamino-1-(2-cyanmethyl-phenyl)benzol;
2,5-Diamino-1-(4-ethyl-phenyl)benzol;
2,5-Diamino-1-(4-propyl-phenyl)benzol;
2,5-Diamino-1-(4-isopropyl-phenyl)benzol;
2,5-Diamino-1-(4-butyl-phenyl)benzol;
2,5-Diamino-1-(4-ter-butyl-phenyl)benzol;
2,5-Diamino-1-(4-pentyl-phenyl)benzol;
2,5-Diamino-1-(4-thiomethoxy-phenyl)benzol;
2,5-Diamino-1-(2-ethyl-phenyl)benzol;
2,5-Diamino-1-(2-fluor-4-methyl-phenyl)benzol;
2,5-Diamino-1-(2-methyl-5-fluor-phenyl)benzol;
2,5-Diamino-1-(2-thiomethoxy-phenyl)benzol;
2,5-Diamino-1-(2,3-dichlor-phenyl)benzol;
2,5-Diamino-4-(4'-hydroxy-biphenyl)benzol;
2,5-Diamino-1-(3-ethoxy-phenyl)benzol;
2,5-Diamino-1-(4-(2-pyrrolidin-1-yl-ethoxy)-phenyl)benzol;
2,5-Diamino-1-(4-(1-hydroxy-ethyl)-phenyl)benzol;
2,5-Diamino-1-(2,4-trifluormethyl-phenyl)benzol;
2,5-Diamino-1-(2-fluor-5-acetyl-phenyl)benzol;
2,5-Diamino-1-(3-fluor-4-methoxy-phenyl)benzol;
2,5-Diamino-1-(3-acetyl-4-hydroxy-phenyl)benzol;
2,5-Diamino-1-(4-(2-hydroxyethyl)-phenyl)benzol;
2,5-Diamino-1-(4-(propyl-1-on)-phenyl)benzol;
2,5-Diamino-1-(4-N,N'diisopropylaminomethyl-phenyl)benzol;
2,5-Diamino-1-(3-acetyl-phenyl)benzol;
2,5-Diamino-1-(2-(2-hydroxy-ethyl)-phenyl)benzol;
2,5-Diamino-1-(3-aminomethyl-phenyl)benzol;
2-Amino-5-(2,3-dihydroxypropyl)amino-1-(2,3,4-trimethoxyphenyl)benzol;
2-Amino-5-(2,3-dihydroxypropyl)amino-1-(2,4-dihydroxyphenyl)benzol;
2-Amino-5-(2,3-dihydroxypropyl)amino-1-(2,5-diaminophenyl)benzol;
2-Amino-5-(2,3-dihydroxypropyl)amino-1-(2,6-dimethoxyphenyl)benzol;
2-Amino-5-(2,3-dihydroxypropyl)amino-1-(2-amino-5-hydroxyphenyl)- benzol;
2-Amino-5-(2,3-dihydroxypropyl)amino-1-(2-aminophenyl)benzol;
2-Amino-5-(2,3-dihydroxypropyl)amino-1-(2-hydroxy-4-aminophenyl)-benzol;
2-Amino-5-(2,3-dihydroxypropyl)amino-1-(2-hydroxy-5-amino-phenyl)benzol;
2-Amino-5-(2,3-dihydroxypropyl)amino-1-(2-hydroxy-phenyl)benzol;
2-Amino-5-(2,3-dihydroxypropyl)amino-1-(2-methoxy-phenyl)benzol;
2-Amino-5-(2,3-dihydroxypropyl)amino-1-(2-methy!-phenyl)benzol;
2-Amino-5-(2,3-dihydroxypropyl)amino-1-(3,5-diamino-phenyl)benzol;
2-Amino-5-(2,3-dihydroxypropyl)amino-1-(3,5-dihydroxy-phenyl)benzol;
2-Amino-5-(2,3-dihydroxypropyl)amino-1-(3-aminophenyl)-benzol;
2-Amino-5-(2,3-dihydroxypropyl)amino-1-(3-hydroxy-5-aminphenyl)benzol;
2-Amino-5-(2,3-dihydroxypropyl)amino-1-(3-hydroxy-phenyl)benzol;
2-Amino-5-(2,3-dihydroxypropyl)amino-1-(3-methoxy-phenyl)benzol;
2-Amino-5-(2,3-dihydroxypropyl)amino-1-(4-(dimethyl-amino)phenyl)benzol;
2-Amino-5-(2,3-dihydroxypropyl)amino-1-(4-(trifluoromethyl)phenyl)benzol;
2-Amino-5-(2,3-dihydroxypropyl)amino-1-(4-aminophenyl)benzol;
2-Amino-5-(2,3-dihydroxypropyl)amino-1-(4-carbonsäurephenyl)benzol;
2-Amino-5-(2,3-dihydroxypropyl)amino-1-(4-chlorophenyl)benzol;
2-Amino-5-(2,3-dihydroxypropyl)amino-1-(4-hydroxyphenyl)benzol;
2-Amino-5-(2,3-dihydroxypropyl)amino-1-(4-methoxyphenyl)benzol;
2-Amino-5-(2,3-dihydroxypropyl)amino-1-(4-methylphenyl)benzol;
2-Amino-5-(2,3-dihydroxypropyl)amino-1-phenylbenzol;
2-Amino-5-(2-hydroxyethyl)amino-1-(2,3,4-trimethoxy-phenyl)benzol;
2-Amino-5-(2-hydroxyethyl)amino-1-(2,4-diamino-phenyl)benzol;
2-Amino-5-(2-hydroxyethyl)amino-1-(2,4-dihydroxy-phenyl)benzol;
2-Amino-5-(2-hydroxyethyl)amino-1-(2,5-diamino-phenyl)benzol;
2-Amino-5-(2-hydroxyethyl)amino-1-(2,5-dimethoxy-phenyl)benzol;
2-Amino-5-(2-hydroxyethyl)amino-1-(2,6-dimethoxy-phenyl)benzol;
2-Amino-5-(2-hydroxyethyl)amino-1-(2-amino-5-hydroxyphenyl)benzol;
2-Amino-5-(2-hydroxyethyl)amino-1-(2-aminophenyl)benzol;
2-Amino-5-(2-hydroxyethyl)amino-1-(2-hydroxy-4-aminophenyl)benzol;
2-Amino-5-(2-hydroxyethyl)amino-1-(2-hydroxy-5-aminophenyl)benzol;
2-Amino-5-(2-hydroxyethyl)amino-1-(2-hydroxy-phenyl)benzol;
2-Amino-5-(2-hydroxyethyl)amino-1-(2-methoxyphenyl)-benzol;
2-Amino-5-(2-hydroxyethyl)amino-1-(2-methylphenyl)benzol;
2-Amino-5-(2-hydroxyethyl)amino-1-(3,5-diaminophenyl)benzol;
2-Amino-5-(2-hydroxyethyl)amino-1-(3,5-dihydroxyphenyl)benzol;
2-Amino-5-(2-hydroxyethyl)amino-1-(3-aminophenyl)benzol;
2-Amino-5-(2-hydroxyethyl)amino-1-(3-hydroxy-5-aminophenyl)benzol;
2-Amino-5-(2-hydroxyethyl)amino-1-(3-hydroxyphenyl)benzol;
2-Amino-5-(2-hydroxyethyl)amino-1-(3-methoxyphenyl)benzol;
2-Amino-5-(2-hydroxyethyl)amino-1-(4-(dimethylamino)phenyl)benzol;
2-Amino-5-(2-hydroxyethyl)amino-1-(4-(trifluoromethyl)phenyl)benzol;
2-Amino-5-(2-hydroxyethyl)amino-1-(4-aminophenyl)benzol;
2-Amino-5-(2-hydroxyethyl)amino-1-(4-carbonsäurephenyl)benzol;
2-Amino-5-(2-hydroxyethyl)amino-1-(4-chlorophenyl)benzol;
2-Amino-5-(2-hydroxyethyl)amino-1-(4-hydroxyphenyl)benzol;
2-Amino-5-(2-hydroxyethyl)amino-1-(4-methoxyphenyl)benzol;
2-Amino-5-(2-hydroxyethyl)amino-1-(4-methylphenyl)benzol;
2-Amino-5-(2-hydroxyethyl)amino-1-phenylbenzol;
2-Amino-5-(2-hydroxyethyl)methylamino-1-phenylbenzol;
2-Amino-5-(2-methoxy-ethyl)amino-1-(2,3,4-trimethoxyphenyl)benzol;
2-Amino-5-(2-methoxy-ethyl)amino-1-(2,4-diaminophenyl)benzol;
2-Amino-5-(2-methoxyethyl)-amino-1-(2,4-dihydroxyphenyl)benzol;
2-Amino-5-(2-methoxyethyl)amino-1-(2,5-diaminophenyl)benzol;
2-Amino-5-(2-methoxyethyl)amino-1-(2,5-dimethoxyphenyl)benzol;
2-Amino-5-(2-methoxyethyl)amino-1-(2,6-dimethoxyphenyl)benzol;
2-Amino-5-(2-methoxyethyl)amino-1-(2-amino-5-hydroxyphenyl)benzol;
2-Amino-5-(2-methoxyethyl)amino-1-(2-amino-phenyl)benzol;
2-Amino-5-(2-methoxyethyl)amino-1-(2-hydroxy-4-amino-phenyl)benzol;
2-Amino-5-(2-methoxyethyl)amino-1-(2-hydroxy-5-amino-phenyl)benzol;
2-Amino-5-(2-methoxyethyl)amino-1-(2-hydroxyphenyl)-benzol;
2-Amino-5-(2-methoxyethyl)amino-1-(2-methoxyphenyl)benzol;
2-Amino-5-(2-methoxyethyl)amino-1-(2-methylphenyl)benzol;
2-Amino-5-(2-methoxyethyl)amino-1-(3,5-diaminophenyl)benzol;
2-Amino-5-(2-methoxyethyl)amino-1-(3,5-dihydroxyphenyl)benzol;
2-Amino-5-(2-methoxyethyl)amino-1-(3-aminophenyl)benzol;
2-Amino-5-(2-methoxyethyl)amino-1-(3-hydroxy-5-aminophenyl)benzol;
2-Amino-5-(2-methoxyethyl)amino-1-(3-hydroxyphenyl)benzol;
2-Amino-5-(2-methoxyethyl)amino-1-(3-methoxyphenyl)benzol;
2-Amino-5-(2-methoxyethyl)amino-1-(4-(dimethylamino)phenyl)benzol;
2-Amino-5-(2-methoxyethyl)amino-1-(4-(trifluoromethyl)phenyl)benzol;
2-Amino-5-(2-methoxyethyl)amino-1-(4-aminophenyl)benzol;
2-Amino-5-(2-methoxyethyl)amino-1-(4-carbonsäurephenyl)benzol;
2-Amino-5-(2-methoxyethyl)amino-1-(4-chlorophenyl)benzol;
2-Amino-5-(2-methoxyethyl)amino-1-(4-hydroxyphenyl)benzol;
2-Amino-5-(2-methoxyethyl)amino-1-(4-methoxyphenyl)benzol;
2-Amino-5-(2-methoxyethyl)amino-1-(4-methylphenyl)benzol;
2-Amino-5-(2-methoxyethyl)amino-1-phenylbenzol;
2-Amino-5-di(2-hydroxyethyl)amino-1-(2,3,4-trimethoxyphenyl)benzol;
2-Amino-5-di(2-hydroxyethyl)amino-1-(2,3-difluoro-4-heptylphenyl)benzol;
2-Amino-5-di(2-hydroxyethyl)amino-1-(2,3-difluorophenyl)benzol;
2-Amino-5-di(2-hydroxy-ethyl)amino-1-(2,4,6-trimethylphenyl)benzol;
2-Amino-5-di(2-hydroxy-ethyl)amino-1-(2,4-diaminophenyl)benzol;
2-Amino-5-di(2-hydroxyethyl)-amino-1-(2,4-dichlorophenyl)benzol;
2-Amino-5-di(2-hydroxyethyl)amino-1-(2,4-dihydroxyphenyl)benzol;
2-Amino-5-di(2-hydroxyethyl)amino-1-(2,5-diaminophenyl)benzol;
2-Amino-5-di(2-hydroxyethyl)amino-1-(2,5-dimethoxyphenyl)benzol;
2-Amino-5-di(2-hydroxyethyl)amino-1-(2,6-difluorophenylbenzol;
2-Amino-5-di(2-hydroxyethyl)amino-1-(2,6-dimethoxyphenyl)benzol;
2-Amino-5-di(2-hydroxyethyl)amino-1-(2,6-dimethoxyphenyl)benzol;
2-Amino-5-di(2-hydroxyethyl)amino-1-(2-((bis(1-methylethyl)amino)carbonyl)phenyl)benzol;
2-Amino-5-di(2-hydroxyethyl)-amino-1-(2-((bis(1-methylethyl)amino)carbonyl)-3-methoxyphenyl)benzol;
2-Amino-5-di(2-hydroxyethyl)amino-1-(2-(bromomethyl)phenyl)benzol;
2-Amino-5-di(2-hydroxyethyl)amino-1-(2-(diethylamino)carbonyl)phenyl)benzol;
2-Amino-5-di(2-hydroxyethyl)amino-1-(2-amino-5-hydroxyphenyl)- benzol;
2-Amino-5-di(2-hydroxyethyl)amino-1-(2-aminophenyl)benzol;
2-Amino-5-di(2-hydroxyethyl)amino-1-(2-carbonsäurephenyl)benzol;
2-Amino-5-di(2-hydroxyethyl)amino-1-(2-chlorophenyl)benzol;
2-Amino-5-di(2-hydroxyethyl)amino-1-(2-fluorophenyl)benzol;
2-Amino-5-di(2-hydroxyethyl)amino-1-(2-formyl-4-methoxyphenyl)benzol;
2-Amino-5-di(2-hydroxyethyl)amino-1-(2-formyl-4-methylphenyl)benzol;
2-Amino-5-di(2-hydroxyethyl)amino-1-(2-formyl-5-methoxyphenyl)benzol;
2-Amino-5-di(2-hydroxyethyl)amino-1-(2-formylphenyl)benzol;
2-Amino-5-di(2-hydroxy-ethyl)amino-1-(2-hydroxy-4-aminophenyl)benzol;
2-Amino-5-di(2-hydroxy-ethyl)amino-1-(2-hydroxy-5-aminophenyl)benzol;
2-Amino-5-di(2-hydroxyethyl)amino-1-(2-hydroxyphenyl)benzol;
2-Amino-5-di(2-hydroxyethyl)amino-1-(2-methoxyphenyl)benzol;
2-Amino-5-di(2-hydroxyethyl)amino-1-(2-methylphenyl)benzol;
2-Amino-5-di(2-hydroxyethyl)amino-1-(2-nitrophenyl)benzol;
2-Amino-5-di(2-hydroxy-ethyl)amino-1-(2-trifluoromethylphenyl)benzol;
2-Amino-5-di(2-hydroxy-ethyl)amino-1-(3,4-dichlorophenyl)benzol;
2-Amino-5-di(2-hydroxyethyl)-amino-1-(3,5-diaminophenyl)benzol;
2-Amino-5-di(2-hydroxyethyl)amino-1-(3,5-dichlorophenyl)benzol;
2-Amino-5-di(2-hydroxyethyl)amino-1-(3,5-dihydroxyphenyl)benzol;
2-Amino-5-di(2-hydroxyethyl)amino-1-(3-(acetylamino)phenyl)benzol;
2-Amino-5-di(2-hydroxyethyl)amino-1-(3-aminophenyl)benzol;
2-Amino-5-di(2-hydroxyethyl)amino-1-(3-bromo-phenyl)benzol;
2-Amino-5-di(2-hydroxyethyl)amino-1-(3-carbonsäure-phenyl)benzol;
2-Amino-5-di(2-hydroxyethyl)amino-1-(3-chloro-4-fluorophenyl)benzol;
2-Amino-5-di(2-hydroxyethyl)amino-1-(3-chloro-phenyl)benzol;
2-Amino-5-di(2-hydroxyethyl)amino-1-(3-fluorophenyl)-benzol;
2-Amino-5-di(2-hydroxyethyl)amino-1-(3-formylphenyl)benzol;
2-Amino-5-di(2-hydroxyethyl)amino-1-(3-hydroxy-5-aminophenyl)benzol;
2-Amino-5-di(2-hydroxyethyl)amino-1-(3-hydroxyphenyl)benzol;
2-Amino-5-di(2-hydroxyethyl)amino-1-(3-hydroxyphenyl)benzol;
2-Amino-5-di(2-hydroxyethyl)amino-1-(3-methoxyphenyl)benzol;
2-Amino-5-di(2-hydroxyethyl)amino-1-(3-methylphenyl)benzol;
2-Amino-5-di(2-hydroxyethyl)amino-1-(3-nitrophenyl)benzol;
2-Amino-5-di(2-hydroxyethyl)amino-1-(3-trifluoromethylphenyl)benzol;
2-Amino-5-di(2-hydroxyethyl)amino-1-(4-(bromomethyl)phenyl)benzol;
2-Amino-5-di(2-hydroxyethyl)amino-1-(4-(dimethylamino)phenyl)benzol;
2-Amino-5-di(2-hydroxyethyl)amino-1-(4-(hydroxymethyl)phenyl)benzol;
2-Amino-5-di(2-hydroxyethyl)amino-1-(4-(methylthio)phenyl)benzol;
2-Amino-5-di(2-hydroxyethyl)amino-1-(4-(trifluoromethyl)phenyl)benzol;
2-Amino-5-di(2-hydroxyethyl)amino-1-(4-(trimethylsilyl)phenyl)benzol;
2-Amino-5-di(2-hydroxyethyl)amino-1-(4-acetylphenyl)benzol;
2-Amino-5-di(2-hydroxy-ethyl)amino-1-(4-aminophenyl)benzol;
2-Amino-5-di(2-hydroxyethyl)-amino-1-(4-bromophenyl)benzol;
2-Amino-5-di(2-hydroxyethyl)amino-1-(4-carbonsäurephenyl)benzol;
2-Amino-5-di(2-hydroxyethyl)amino-1-(4-chloro-3-methoxyphenyl)benzol;
2-Amino-5-di(2-hydroxyethyl)amino-1-(4-chlorophenyl)benzol;
2-Amino-5-di(2-hydroxyethyl)amino-1-(4-ethenyl-phenyl)benzol;
2-Amino-5-di(2-hydroxyethyl)amino-1-(4-fluorophenyl)-benzol;
2-Amino-5-di(2-hydroxyethyl)amino-1-(4-formylphenyl)benzol;
2-Amino-5-di(2-hydroxyethyl)amino-1-(4-hydroxyphenyl)benzol;
2-Amino-5-di(2-hydroxyethyl)amino-1-(4-iodophenyl)benzol;
2-Amino-5-di(2-hydroxyethyl)amino-1-(4-methoxyphenyl)benzol;
2-Amino-5-di(2-hydroxy-ethyl)amino-1-(4-methyl-3-nitrophenyl)benzol;
2-Amino-5-di(2-hydroxyö-ethyl)amino-1-(4-methylphenyl)benzol;
2-Amino-5-di(2-hydroxyethyl)-amino-1-(5-bromo-2-methoxyphenyl)benzol;
2-Amino-5-di(2-hydroxy-ethyl)amino-1-(5-chloro-2-methoxyphenyl)benzol;
2-Amino-5-di(2-hydroxyethyl)amino-1-(5-formyl-2-methoxyphenyl)benzol;
2-Amino-5-di(2-hydroxyethyl)amino-1-phenylbenzol;
2-Amino-5-di(2-methoxyethyl)amino-1-(2,3,4-trimethoxyphenyl)benzol;
2-Amino-5-di(2-methoxyethyl)amino-1-(2,3-difluoro-4-heptylphenyl)benzol;
2-Amino-5-di(2-methoxyethyl)amino-1-(2,3-difluorophenyl)benzol;
2-Amino-5-di(2-methoxyethyl)amino-1-(2,4,6-trimethylphenyl)benzol;
2-Amino-5-di(2-methoxyethyl)amino-1-(2,4-diaminophenyl)benzol;
2-Amino-5-di(2-methoxyethyl)amino-1-(2,4-dichlorophenyl)benzol;
2-Amino-5-di(2-methoxyethyl)amino-1-(2,4-dihydroxyphenyl)benzol;
2-Amino-5-di(2-methoxyethyl)amino-1-(2,5-dimethoxyphenyl)benzol;
2-Amino-5-di(2-methoxyethyl)amino-1-(2,6-difluorophenyl)benzol;
2-Amino-5-di(2-methoxyethyl)amino-1-(2,6-dimethoxyphenyl)benzol;
2-Amino-5-di(2-methoxyethyl)amino-1-(2-((bis(1-methylethyl)amino)-carbonyl)phenyl)benzol;
2-Amino-5-di(2-methoxy-ethyl)amino-1-(2-(bromomethyl)phenyl)benzol;
2-Amino-5-di(2-methoxyethyl)amino-1-(2-(diethylamino)carbonyl)phenyl)-benzol;
2-Amino-5-di(2-methoxyethyl)amino-1-(2-carbonsäurephenyl)benzol;
2-Amino-5-di(2-methoxyethyl)amino-1-(2-chlorophenyl)benzol;
2-Amino-5-di(2-methoxyethyl)amino-1-(2-fluorophenyl)benzol;
2-Amino-5-di(2-methoxyethyl)amino-1-(2-formyl-4-methoxyphenyl)benzol;
2-Amino-5-di(2-methoxyethyl)amino-1-(2-formyl-4-methylphenyl)benzol;
2-Amino-5-di(2-methoxyethyl)amino-1-(2-formyl-5-methoxyphenyl)benzol;
2-Amino-5-di(2-methoxyethyl)amino-1-(2-formylphenyl)benzol;
2-Amino-5-di(2-methoxy-ethyl)amino-1-(2-hydroxy-4-aminophenyl)benzol;
2-Amino-5-di(2-methoxy-ethyl)amino-1-(2-hydroxyphenyl)benzol;
2-Amino-5-di(2-methoxyethyl)-amino-1-(2-methoxyphenyl)benzol;
2-Amino-5-di(2-methoxyethyl)amino-1-(2-methylphenyl)benzol;
2-Amino-5-di(2-methoxyethyl)amino-1-(2-nitro-phenyl)benzol;
2-Amino-5-di(2-methoxyethyl)amino-1-(2-trifluoromethyl-phenyl)benzol;
2-Amino-5-di(2-methoxyethyl)amino-1-(3,4-dichloro-phenyl)benzol;
2-Amino-5-di(2-methoxyethyl)amino-1-(3,5-diaminophenyl)benzol;
2-Amino-5-di(2-methoxyethyl)amino-1-(3,5-dichlorophenyl)benzol;
2-Amino-5-di(2-methoxyethyl)amino-1-(3,5-dihydroxyphenyl)benzol;
2-Amino-5-di(2-methoxyethyl)amino-1-(3-(acetylamino)phenyl)benzol;
2-Amino-5-di(2-methoxyethyl)amino-1-(3-aminophenyl)benzol;
2-Amino-5-di(2-methoxyethyl)amino-1-(3-bromo-phenyl)benzol;
2-Amino-5-di(2-methoxyethyl)amino-1-(3-carbonsäure-phenyl)benzol;
2-Amino-5-di(2-methoxyethyl)amino-1-(3-chloro-4-fluorophenyl)benzol;
2-Amino-5-di(2-methoxyethyl)amino-1-(3-chloro-phenyl)benzol;
2-Amino-5-di(2-methoxyethyl)amino-1-(3-fluorophenyl)-benzol;
2-Amino-5-di(2-methoxyethyl)amino-1-(3-formylphenyl)benzol;
2-Amino-5-di(2-methoxyethyl)amino-1-(3-hydroxyphenyl)benzol;
2-Amino-5-di(2-methoxyethyl)amino-1-(3-methoxyphenyl)benzol;
2-Amino-5-di(2-methoxyethyl)amino-1-(3-methylphenyl)benzol;
2-Amino-5-di(2-methoxyethyl)amino-1-(3-nitrophenyl)benzol;
2-Amino-5-di(2-methoxyethyl)amino-1-(3-trifluoromethylphenyl)benzol;
2-Amino-5-di(2-methoxyethyl)amino-1-(4-(bromomethyl)phenyl)benzol;
2-Amino-5-di(2-methoxyethyl)amino-1-(4-(dimethylamino)phenyl)benzol;
2-Amino-5-di(2-methoxyethyl)amino-1-(4-(hydroxymethyl)phenyl)benzol;
2-Amino-5-di(2-methoxyethyl)amino-1-(4-(methylthio)phenyl)benzol;
2-Amino-5-di(2-methoxyethyl)amino-1-(4-(trifluoromethyl)phenyl)benzol;
2-Amino-5-di(2-methoxyethyl)amino-1-(4-(trimethylsilyl)phenyl)benzol;
2-Amino-5-di(2-methoxyethyl)amino-1-(4-acetylphenyl)benzol;
2-Amino-5-di(2-methoxy-ethyl)amino-1-(4-aminophenyl)benzol;
2-Amino-5-di(2-methoxyethyl)-amino-1-(4-bromophenyl)benzol;
2-Amino-5-di(2-methoxyethyl)amino-1-(4-carbonsäurephenyl)benzol;
2-Amino-5-di(2-methoxyethyl)amino-1-(4-chloro-3-methoxyphenyl)benzol;
2-Amino-5-di(2-methoxyethyl)amino-1-(4-chlorophenyl)benzol;
2-Amino-5-di(2-methoxyethyl)amino-1-(4-ethenylphenyl)benzol;
2-Amino-5-di(2-methoxyethyl)amino-1-(4-ethoxyphenyl)benzol;
2-Amino-5-di(2-methoxyethyl)amino-1-(4-fluorophenyl)benzol;
2-Amino-5-di(2-methoxyethyl)amino-1-(4-formylphenyl)benzol;
2-Amino-5-di(2-methoxyethyl)amino-1-(4-hydroxyphenyl)benzol;
2-Amino-5-di(2-methoxyethyl)amino-1-(4-iodophenyl)benzol;
2-Amino-5-di(2-methoxyethyl)amino-1-(4-methoxy-phenyl)benzol;
2-Amino-5-di(2-methoxyethyl)amino-1-(4-methyl-3-nitrophenyl)benzol;
2-Amino-5-di(2-methoxyethyl)amino-1-(4-methyl-phenyl)benzol;
2-Amino-5-di(2-methoxyethyl)amino-1-(5-bromo-2-methoxyphenyl)benzol;
2-Amino-5-di(2-methoxyethyl)amino-1-(5-chloro-2-methoxyphenyl)benzol;
2-Amino-5-di(2-methoxyethyl)amino-1-(5-formyl-2-methoxyphenyl)benzol;
2-Amino-5-dimethylamino-1-phenylbenzol;
2-Amino-5-methylamino-1-(2,3,4-trimethoxyphenyl)benzol;
2-Amino-5-methylamino-1-(2,4-diaminophenyl)benzol;
2-Amino-5-methylamino-1-(2,4-dihydroxyphenyl)benzol;
2-Amino-5-methylamino-1-(2,5-diamino-phenyl)benzol;
2-Amino-5-methylamino-1-(2,5-dimethoxyphenyl)benzol;
2-Amino-5-methylamino-1-(2,6-dimethoxyphenyl)benzol;
2-Amino-5-methylamino-1-(2-amino-5-hydroxyphenyl)benzol;
2-Amino-5-methyl-amino-1-(2-aminophenyl)benzol;
2-Amino-5-methylamino-1-(2-hydroxy-4-aminophenyl)benzol;
2-Amino-5-methylamino-1-(2-hydroxy-5-amino-phenyl)benzol;
2-Amino-5-methylamino-1-(2-hydroxyphenyl)benzol;
2-Amino-5-methylamino-1-(2-methoxyphenyl)benzol;
2-Amino-5-methylamino-1-(2-methylphenyl)benzol;
2-Amino-5-methylamino-1-(3,5-diaminophenyl)benzol;
2-Amino-5-methylamino-1-(3,5-dihydroxyphenyl)-benzol;
2-Amino-5-methylamino-1-(3-aminophenyl)benzol;
2-Amino-5-methylamino-1-(3-hydroxy-5-aminophenyl)benzol;
2-Amino-5-methylamino-1-(3-hydroxyphenyl)benzol;
2-Amino-5-methylamino-1-(3-methoxyphenyl)benzol;
2-Amino-5-methylamino-1-(4-(dimethylamino)-phenyl)benzol;
2-Amino-5-methylamino-1-(4-(trifluoromethyl)-phenyl)benzol;
2-Amino-5-methylamino-1-(4-aminophenyl)benzol;
2-Amino-5-methylamino-1-(4-carbonsäurephenyl)benzol;
2-Amino-5-methylamino-1-(4-chlorophenyl)benzol;
2-Amino-5-methylamino-1-(4-hydroxyphenyl)benzol;
2-Amino-5-methylamino-1-(4-methoxyphenyl)-benzol;
2-Amino-5-methylamino-1-(4-methylphenyl)benzol;
2-Amino-5-methylamino-1-phenylbenzol;
5-Amino-2-(2,3-dihydroxypropyl)amino-1-(4-bromophenyl)benzol;
5-Amino-2-(2,3-dihydroxypropyl)amino-1-(4-ethenylphenyl)benzol;
5-Amino-2-(2,3-dihydroxypropyl)amino-1-(2,3,4-trimethoxyphenyl)benzol;
5-Amino-2-(2,3-dihydroxypropyl)amino-1-(2,4-di(2-hydroxyethyl)-aminophenyl)benzol;
5-Amina-2-(2,3-dihydroxypropyl)-amino-1-(2,4-diaminophenyl)benzol;
5-Amino-2-(2,3-dihydroxypropyl)-amino-1-(2,4-dihydroxyphenyl)benzol;
5-Amino-2-(2,3-dihydroxypropyl)-amino-1-(2,4-dimethylaminophenyl)-benzol;
5-Amino-2-(2,3-dihydroxy-propyl)amino-1-(2,4-methoxyphenyl)benzol;
5-Amino-2-(2,3-dihydroxy-propyl)amino-1-(2,5-di(2-hydroxyethyl)-aminophenyl)benzol;
5-Amino-2-(2,3-dihydroxypropyl)amino-1-(2,5-diaminophenyl)benzol;
5-Amino-2-(2,3-dihydroxypropyl)amino-1-(2,5-dihydroxyphenyl)benzol;
5-Amino-2-(2,3-dihydroxypropyl)amino-1-(2,5-dimethoxyphenyl)benzol;
5-Amino-2-(2,3-dihydroxypropyl)amino-1-(2,5-dimethylaminophenyl)-benzol; 5-Amino-2-(2,3-dihydroxypropyl)amino-1-(2,6-di(2-hydroxyethyl)-aminophenyl)benzol;
5-Amino-2-(2,3-dihydroxypropyl)amino-1-(2,6-diaminophenyl)benzol;
5-Amino-2-(2,3-dihydroxypropyl)amino-1-(2,6-dihydroxyphenyl)benzol;
5-Amino-2-(2,3-dihydroxypropyl)amino-1-(2,6-dimethoxyphenyl)benzol;
5-Amino-2-(2,3-dihydroxypropyl)amino-1-(2,6-dimethylaminophenyl)-benzol;
5-Amino-2-(2,3-dihydroxypropyl)amino-1-(2-(bromomethyl)-phenyl)-benzol;
5-Amino-2-(2,3-dihydroxypropyl)amino-1-(2-amino-5-hydroxyphenyl)benzol;
5-Amino-2-(2,3-dihydroxypropyl)amino-1-(2-aminophenyl)benzol;
5-Amino-2-(2,3-dihydroxypropyl)amino-1-(2-carbonsäurephenyl)benzol;
5-Amino-2-(2,3-dihydroxypropyl)amino-1-(2-chlorophenyl)benzol;
5-Amino-2-(2,3-dihydroxypropyl)amino-1-(2-di(2-hydroxyethyl)-aminophenyl)-benzol;
5-Amino-2-(2,3-dihydroxypropyl)-amino-1-(2-dimethylaminophenyl)benzol;
5-Amino-2-(2,3-dihydroxy-propyl)amino-1-(2-fluorophenyl)benzol;
5-Amino-2-(2,3-dihydroxy-propyl)amino-1-(2-formylphenyl)benzol;
5-Amino-2-(2,3-dihydroxy-propyl)amino-1-(2-hydroxy-4-aminophenyl)benzol;
5-Amino-2-(2,3-dihydroxypropyl)amino-1-(2-hydroxy-5-aminophenyl)benzol;
5-Amino-2-(2,3-dihydroxypropyl)amino-1-(2-hydroxyphenyl)benzol;
5-Amino-2-(2,3-dihydroxypropyl)amino-1-(2-methoxyphenyl)benzol;
5-Amino-2-(2,3-dihydroxypropyl)amino-1-(2-methylphenyl) benzol;
5-Amino-2-(2,3-dihydroxypropyl)amino-1-(2-nitrophenyl)benzol;
5-Amino-2-(2,3-dihydroxypropyl)amino-1-(2-trifluoromethylphenyl)benzol;
5-Amino-2-(2,3-dihydroxypropyl)amino-1-(3,5-di(2-hydroxyethyl)-aminophenyl)benzol;
5-Amino-2-(2,3-dihydroxypropyl)amino-1-(3,5-diamino-phenyl)benzol;
5-Amino-2-(2,3-dihydroxypropyl)amino-1-(3,5-dihydroxy-phenyl)benzol;
5-Amino-2-(2,3-dihydroxypropyl)amino-1-(3,5-dimethoxy-phenyl)benzol;
5-Amino-2-(2,3-dihydroxypropyl)amino-1-(3,5-dimethyl-aminophenyl)-benzol;
5-Amino-2-(2,3-dihydroxypropyl)amino-1-(3-aminophenyl)benzol;
5-Amino-2-(2,3-dihydroxypropyl)amino-1-(3-bromo-phenyl)benzol;
5-Amino-2-(2,3-dihydroxypropyl)amino-1-(3-carbonsäure-phenyl)benzol;
5-Amino-2-(2,3-dihydroxypropyl)amino-1-(3-chlorophenyl)-benzol;
5-Amino-2-(2,3-dihydroxypropyl)amino-1-(3-di(2-hydroxyethyl)-aminophenyl)benzol;
5-Amino-2-(2,3-dihydroxypropyl)amino-1-(3-dimethylaminophenyl)benzol;
5-Amino-2-(2,3-dihydroxypropyl)amino-1-(3-fluorophenyl)benzol;
5-Amino-2-(2,3-dihydroxypropyl)amino-1-(3-formylphenyl)benzol;
5-Amino-2-(2,3-dihydroxypropyl)amino-1-(3-hydroxy-5-aminophenyl)-benzol;
5-Amino-2-(2,3-dihydroxypropyl)amino-1-(3-hydroxyphenyl)benzol;
5-Amino-2-(2,3-dihydroxypropyl)amino-1-(3-methoxyphenyl)benzol;
5-Amino-2-(2,3-dihydroxypropyl)amino-1-(3-nitrophenyl)benzol;
5-Amino-2-(2,3-dihydroxypropyl)amino-1-(3-trifluoro-methylphenyl)benzol;
5-Amino-2-(2,3-dihydroxypropyl)amino-1-(4-(dimethylamino)phenyl)-benzol;
5-Amino-2-(2,3-dihydroxypropyl)amino-1-(4-(hydroxymethyl)phenyl)-benzol;
5-Amino-2-(2,3-dihydroxypropyl)amino-1-(4-(methylthio)phenyl)-benzol;
5-Amino-2-(2,3-dihydroxypropyl)amino-1-(4-(trifluoromethyl)phenyl)benzol;
5-Amino-2-(2,3-dihydroxypropyl)amino-1-(4-(trimethylsilyl)phenyl)benzol;
5-Amino-2-(2,3-dihydroxypropyl)amino-1-(4-acetylphenyl)benzol;
5-Amino-2-(2,3-dihydroxypropyl)amino-1-(4-aminophenyl)benzol;
5-Amino-2-(2,3-dihydroxypropyl)amino-1-(4-carbonsäurephenyl)benzol;
5-Amino-2-(2,3-dihydroxypropyl)amino-1-(4-chlorophenyl)benzol;
5-Amino-2-(2,3-dihydroxypropyl)amino-1-(4-di(2-hydroxyethyl)-aminophenyl)benzol;
5-Amino-2-(2,3-dihydroxypropyl)-amino-1-(4-dimethylaminophenyl)benzol;
5-Amino-2-(2,3-dihydroxy-propyl)amino-1-(4-ethoxyphenyl)benzol;
5-Amino-2-(2,3-dihydroxy-propyl)amino-1-(4-fluorophenyl)benzol;
5-Amino-2-(2,3-dihydroxy-propyl)amino-1-(4-formylphenyl)benzol;
5-Amino-2-(2,3-dihydroxypropyl)-amino-1-(4-hydroxyphenyl)benzol;
5-Amino-2-(2,3-dihydroxypropyl)amino-1-(4-methoxyphenyl)benzol;
5-Amino-2-(2,3-dihydroxypropyl)amino-1-(4-methylphenyl)benzol;
5-Amino-2-(2,3-dihydroxypropyl)amino-1-phenylbenzol;
5-Amino-2-(2-hydroxyethyl)amino-1-(4-bromophenyl)-benzol;
5-Amino-2-(2-hydroxyethyl)amino-1-(4-ethenylphenyl)benzol;
5-Amino-2-(2-hydroxyethyl)amino-1-(2,3,4-trimethoxyphenyl)benzol;
5-Amino-2-(2-hydroxyethyl)amino-1-(2,4-di(2-hydroxyethyl)aminophenyl)-benzol;
5-Amino-2-(2-hydroxyethyl)amino-1-(2,4-diaminophenyl)benzol;
5-Amino-2-(2-hydroxyethyl)amino-1-(2,4-dihydroxyphenyl)benzol;
5-Amino-2-(2-hydroxyethyl)amino-1-(2,4-dimethylaminophenyl)benzol;
5-Amino-2-(2-hydroxyethyl)amino-1-(2,4-methoxyphenyl)benzol;
5-Amino-2-(2-hydroxyethyl)amino-1-(2,5-di(2-hydroxyethyl)aminophenyl)-benzol;
5-Amino-2-(2-hydroxyethyl)amino-1-(2,5-diaminophenyl)benzol;
5-Amino-2-(2-hydroxyethyl)amino-1-(2,5-dihydroxyphenyl)benzol;
5-Amino-2-(2-hydroxyethyl)amino-1-(2,5-dimethoxyphenyl)benzol;
5-Amino-2-(2-hydroxyethyl)amino-1-(2,5-dimethylaminophenyl)benzol;
5-Amino-2-(2-hydroxyethyl)amino-1-(2,6-di(2-hydroxyethyl)aminophenyl)-benzol;
5-Amino-2-(2-hydroxyethyl)amino-1-(2,6-diaminophenyl)benzol;
5-Amino-2-(2-hydroxyethyl)amino-1-(2,6-dihydroxyphenyl)benzol;
5-Amino-2-(2-hydroxyethyl)amino-1-(2,6-dimethoxyphenyl)benzol;
5-Amino-2-(2-hydroxyethyl)amino-1-(2,6-dimethylaminophenyl)benzol;
5-Amino-2-(2-hydroxyethyl)amino-1-(2-(bromomethyl)phenyl)benzol;
5-Amino-2-(2-hydroxyethyl)amino-1-(2-amino-5-hydroxyphenyl)benzol;
5-Amino-2-(2-hydroxyethyl)amino-1-(2-aminophenyl)benzol;
5-Amino-2-(2-hydroxy-ethyl)amino-1-(2-carbonsäurephenyl)benzol;
5-Amino-2-(2-hydroxyethyl)-amino-1-(2-chlorophenyl)benzol;
5-Amino-2-(2-hydroxyethyl)amino-1-(2-di(2-hydroxyethyl)aminophenyl)-benzol;
5-Amino-2-(2-hydroxyethyl)amino-1-(2-dimethylaminophenyl)benzol;
5-Amino-2-(2-hydroxyethyl)amino-1-(2-fluorophenyl)benzol;
5-Amino-2-(2-hydroxyethyl)amino-1-(2-formylphenyl)-benzol;
5-Amino-2-(2-hydroxyethyl)amino-1-(2-hydroxy-4-aminophenyl)-benzol;
5-Amino-2-(2-hydroxyethyl)amino-1-(2-hydroxy-5-aminophenyl)-benzol;
5-Amino-2-(2-hydroxyethyl)amino-1-(2-hydroxyphenyl)benzol;
5-Amino-2-(2-hydroxyethyl)amino-1-(2-methoxyphenyl)benzol,
5-Amino-2-(2-hydroxyethyl)amino-1-(2-methylphenyl)benzol;
5-Amino-2-(2-hydroxy-ethyl)amino-1-(2-nitrophenyl)benzol;
5-Amino-2-(2-hydroxyethyl)amino-1-(2-trifluoromethylphenyl)benzol;
5-Amino-2-(2-hydroxyethyl)amino-1-(3,5-di(2-hydroxyethyl)-aminophenyl)-benzol;
5-Amino-2-(2-hydroxyethyl)-amino-1-(3,5-diaminophenyl)benzol;
5-Amino-2-(2-hydroxyethyl)amino-1-(3,5-dihydroxyphenyl)benzol;
5-Amino-2-(2-hydroxyethyl)amino-1-(3,5-dimethoxyphenyl)benzol;
5-Amino-2-(2-hydroxyethyl)amino-1-(3,5-dimethylaminophenyl)benzol;
5-Amino-2-(2-hydroxyethyl)amino-1-(3-aminophenyl)benzol;
5-Amino-2-(2-hydroxyethyl)amino-1-(3-bromo-phenyl)benzol;
5-Amino-2-(2-hydroxyethyl)amino-1-(3-carbonsäure-phenyl)benzol;
5-Amino-2-(2-hydroxyethyl)amino-1-(3-chlorophenyl)-benzol;
5-Amino-2-(2-hydroxyethyl)amino-1-(3-di(2-hydroxyethyl)-aminophenyl)-benzol;
5-Amino-2-(2-hydroxyethyl)amino-1-(3-dimethylaminophenyl)benzol;
5-Amino-2-(2-hydroxyethyl)amino-1-(3-fluorophenyl)benzol;
5-Amino-2-(2-hydroxyethyl)amino-1-(3-formylphenyl)benzol;
5-Amino-2-(2-hydroxyethyl)amino-1-(3-hydroxy-5-aminophenyl)benzol;
5-Amino-2-(2-hydroxyethyl)amino-1-(3-hydroxy-phenyl)benzol,
5-Amino-2-(2-hydroxyethyl)amino-1-(3-methoxyphenyl)-benzol;
5-Amino-2-(2-hydroxyethyl)amino-1-(3-nitrophenyl)benzol;
5-Amino-2-(2-hydroxyethyl)amino-1-(3-trifluoromethylphenyl)benzol;
5-Amino-2-(2-hydroxyethyl)amino-1-(4-(dimethylamino)phenyl)benzol;
5-Amino-2-(2-hydroxyethyl)amino-1-(4-(hydroxymethyl)phenyl)benzol;
5-Amino-2-(2-hydroxyethyl)amino-1-(4-(methylthio)phenyl)benzol;
5-Amino-2-(2-hydroxyethyl)amino-1-(4-(trifluoromethyl)phenyl)benzol;
5-Amino-2-(2-hydroxyethyl)amino-1-(4-(trimethylsilyl)phenyl)benzol;
5-Amino-2-(2-hydroxyethyl)amino-1-(4-acetylphenyl)benzol;
5-Amino-2-(2-hydroxyethyl)amino-1-(4-aminophenyl)benzol;
5-Amino-2-(2-hydroxy-ethyl)amino-1-(4-carbonsäurephenyl)benzol;
5-Amino-2-(2-hydroxyethyl)-amino-1-(4-chlorophenyl)benzol;
5-Amino-2-(2-hydroxyethyl)amino-1-(4-di(2-hydroxyethyl)aminophenyl)benzol;
5-Amino-2-(2-hydroxyethyl)amino-1-(4-dimethylaminophenyl)benzol;
5-Amino-2-(2-hydroxyethyl)amino-1-(4-ethoxyphenyl)benzol;
5-Amino-2-(2-hydroxyethyl)amino-1-(4-fluorophenyl)benzol;
5-Amino-2-(2-hydroxyethyl)amino-1-(4-formylphenyl)- benzol;
5-Amino-2-(2-hydroxyethyl)amino-1-(4-hydroxyphenyl)benzol;
5-Amino-2-(2-hydroxyethyl)amino-1-(4-methoxyphenyl)benzol;
5-Amino-2-(2-hydroxyethyl)amino-1-(4-methylphenyl)benzol;
5-Amino-2-(2-hydroxy-ethyl)amino-1-phenylbenzol;
5-Amino-2-(2-methoxyethyl)amino-1-(2,3,4-trimethoxyphenyl)benzol;
5-Amino-2-(2-methoxyethyl)amino-1-(2,4-diaminophenyl)benzol;
5-Amino-2-(2-methoxyethyl)amino-1-(2,4-dihydroxyphenyl)benzol;
5-Amino-2-(2-methoxyethyl)amino-1-(2,5-diaminophenyl)benzol;
5-Amino-2-(2-methoxyethyl)amino-1-(2,5-dimethoxyphenyl)benzol;
5-Amino-2-(2-methoxyethyl)amino-1-(2,6-dimethoxyphenyl)benzol;
5-Amino-2-(2-methoxyethyl)amino-1-(2-amino-5-hydroxyphenyl)benzol;
5-Amino-2-(2-methoxyethyl)amino-1-(2-amino-phenyl)benzol;
5-Amino-2-(2-methoxyethyl)amino-1-(2-hydroxy-4-amino-phenyl)benzol;
5-Amino-2-(2-methoxyethyl)amino-1-(2-hydroxy-5-amino-phenyl)benzol;
5-Amino-2-(2-methoxyethyl)amino-1-(2-hydroxyphenyl)-benzol;
5-Amino-2-(2-methoxyethyl)amino-1-(2-methoxyphenyl)benzol;
5-Amino-2-(2-methoxyethyl)amino-1-(2-methylphenyl)benzol;
5-Amino-2-(2-methoxyethyl)amino-1-(3,5-diaminophenyl)benzol;
5-Amino-2-(2-methoxyethyl)amino-1-(3,5-dihydroxyphenyl)benzol;
5-Amino-2-(2-methoxyethyl)amino-1-(3-aminophenyl)benzol;
5-Amino-2-(2-methoxy-ethyl)amino-1-(3-hydroxy-5-aminophenyl)benzol;
5-Amino-2-(2-methoxy-ethyl)amino-1-(3-hydroxyphenyl)benzol;
5-Amino-2-(2-methoxyethyl)-amino-1-(3-methoxyphenyl)benzol;
5-Amino-2-(2-methoxyethyl)amino-1-(4-(dimethylamino)phenyl)benzol;
5-Amino-2-(2-methoxyethyl)amino-1-(4-(trifluoromethyl)phenyl)benzol;
5-Amino-2-(2-methoxyethyl)amino-1-(4-aminophenyl)benzol;
5-Amino-2-(2-methoxyethyl)amino-1-(4-carbonsäure-phenyl)benzol;
5-Amino-2-(2-methoxyethyl)amino-1-(4-chlorophenyl)-benzol;
5-Amino-2-(2-methoxyethyl)amino-1-(4-hydroxyphenyl)benzol;
5-Amino-2-(2-methoxyethyl)amino-1-(4-methoxyphenyl)benzol;
5-Amino-2-(2-methoxyethyl)amino-1-(4-methylphenyl)benzol;
5-Amino-2-(2-methoxyethyl)amino-1-phenylbenzol;
5-Amino-2-di(2-hydroxyethyl)amino-1-(2,3,4-trimethoxyphenyl)benzol;
5-Amino-2-di(2-hydroxyethyl)amino-1-(2,3-difluoro-4-heptylphenyl)benzol;
5-Amino-2-di(2-hydroxyethyl)amino-1-(2,3-difluorophenyl)benzol;
5-Amino-2-di(2-hydroxyethyl)amino-1-(2,4,6-trimethylphenyl)benzol;
5-Amino-2-di(2-hydroxyethyl)amino-1-(2,4-diaminophenyl)benzol;
5-Amino-2-di(2-hydroxyethyl)amino-1-(2,4-dichlorophenyl)benzol;
5-Amino-2-di(2-hydroxyethyl)amino-1-(2,4-dihydroxyphenyl)benzol;
5-Amino-2-di(2-hydroxyethyl)amino-1-(2,5-dimethoxyphenyl)benzol;
5-Amino-2-di(2-hydroxyethyl)amino-1-(2,6-difluorophenyl)benzol;
5-Amino-2-di(2-hydroxyethyl)amino-1-(2,6-dimethoxyphenyl)benzol;
5-Amino-2-di(2-hydroxyethyl)amino-1-(2-((bis(1-methylethyl)amino)carbonyl)phenyl)benzol;
5-Amino-2-di(2-hydroxyethyl)-amino-1-(2-((bis(1-methylethyl)amino)carbonyl)-3-methoxyphenyl)benzol;
5-Amino-2-di(2-hydroxyethyl)amino-1-(2-(bromomethyl)phenyl)benzol;
5-Amino-2-di(2-hydroxyethyl)amino-1-(2-(diethylamino)carbonyl)phenyl)benzol;
5-Amino-2-di(2-hydroxyethyl)amino-1-(2-carbonsäurephenyl)-benzol;
5-Amino-2-di(2-hydroxyethyl)amino-1-(2-chlorophenyl)benzol;
5-Amino-2-di(2-hydroxyethyl)amino-1-(2-fluorophenyl)benzol;
5-Amino-2-di(2-hydroxyethyl)amino-1-(2-formyl-4-methoxyphenyl)benzol;
5-Amino-2-di(2-hydroxyethyl)amino-1-(2-formyl-4-methylphenyl)benzol;
5-Amino-2-di(2-hydroxyethyl)amino-1-(2-formyl-5-methoxyphenyl)benzol;
5-Amino-2-di(2-hydroxyethyl)amino-1-(2-formylphenyl)benzol;
5-Amino-2-di(2-hydroxyethyl)amino-1-(2-hydroxy-4-aminophenyl)benzol;
5-Amino-2-di(2-hydroxyethyl)amino-1-(2-hydroxyphenyl)benzol;
5-Amino-2-di(2-hydroxy-ethyl)amino-1-(2-methoxyphenyl)benzol;
5-Amino-2-di(2-hydroxyethyl)-amino-1-(2-methylphenyl)benzol;
5-Amino-2-di(2-hydroxyethyl)amino-1-(2-nitrophenyl)benzol;
5-Amino-2-di(2-hydroxyethyl)amino-1-(2-trifluoro-methylphenyl)benzol;
5-Amino-2-di(2-hydroxyethyl)amino-1-(3,4-dichlorophenyl)benzol;
5-Amino-2-di(2-hydroxyethyl)amino-1-(3,5-diaminophenyl)benzol;
5-Amino-2-di(2-hydroxyethyl)amino-1-(3,5-dichlorophenyl)benzol;
5-Amino-2-di(2-hydroxyethyl)amino-1-(3,5-dihydroxyphenyl)benzol;
5-Amino-2-di(2-hydroxyethyl)amino-1-(3-(acetylamino)phenyl)benzol;
5-Amino-2-di(2-hydroxyethyl)amino-1-(3-aminophenyl)benzol;
5-Amino-2-di(2-hydroxyethyl)amino-1-(3-bromo-phenyl)benzol;
5-Amino-2-di(2-hydroxyethyl)amino-1-(3-carbonsäure-phenyl)benzol;
5-Amino-2-di(2-hydroxyethyl)amino-1-(3-chloro-4-fluorophenyl)benzol;
5-Amino-2-di(2-hydroxyethyl)amino-1-(3-chloro-phenyl)benzol;
5-Amino-2-di(2-hydroxyethyl)amino-1-(3-fluorophenyl)-benzol;
5-Amino-2-di(2-hydroxyethyl)amino-1-(3-formylphenyl)benzol;
5-Amino-2-di(2-hydroxyethyl)amino-1-(3-hydroxyphenyl)benzol;
5-Amino-2-di(2-hydroxyethyl)amino-1-(3-methoxyphenyl)benzol;
5-Amino-2-di(2-hydroxyethyl)amino-1-(3-methylphenyl)benzol;
5-Amino-2-di(2-hydroxy-ethyl)amino-1-(3-nitrophenyl)benzol;
5-Amino-2-di(2-hydroxyethyl)amino-1-(3-trifluoromethylphenyl)benzol;
5-Amino-2-di(2-hydroxyethyl)amino-1-(4-(bromomethyl)phenyl)benzol;
5-Amino-2-di(2-hydroxyethyl)amino-1-(4-(dimethylamino)phenyl)benzol;
5-Amino-2-di(2-hydroxyethyl)amino-1-(4-(hydroxymethyl)phenyl)benzol;
5-Amino-2-di(2-hydroxyethyl)amino-1-(4-(methylthio)phenyl)benzol;
5-Amino-2-di(2-hydroxyethyl)amino-1-(4-(trifluoromethyl)phenyl)benzol;
5-Amino-2-di(2-hydroxyethyl)amino-1-(4-(trimethylsilyl)phenyl)benzol;
5-Amino-2-di(2-hydroxyethyl)amino-1-(4-acetylphenyl)benzol;
5-Amino-2-di(2-hydroxyethyl)amino-1-(4-amino-phenyl)benzol;
5-Amino-2-di(2-hydroxyethyl)amino-1-(4-bromophenyl)-benzol;
5-Amino-2-di(2-hydroxyethyl)amino-1-(4-carbonsäurephenyl)-benzol;
5-Amino-2-di(2-hydroxyethyl)amino-1-(4-chloro-3-methoxyphenyl)-benzol;
5-Amino-2-di(2-hydroxyethyl)amino-1-(4-chlorophenyl)benzol;
5-Amino-2-di(2-hydroxyethyl)amino-1-(4-ethenylphenyl)benzol;
5-Amino-2-di(2-hydroxyethyl)amino-1-(4-ethoxyphenyl)benzol;
5-Amino-2-di(2-hydroxyethyl)amino-1-(4-fluorophenyl)benzol;
5-Amino-2-di(2-hydroxy-ethyl)amino-1-(4-formylphenyl)benzol;
5-Amino-2-di(2-hydroxyethyl)-amino-1-(4-hydroxyphenyl)benzol;
5-Amino-2-di(2-hydroxyethyl)amino-1-(4-iodophenyl)benzol;
5-Amino-2-di(2-hydroxyethyl)amino-1-(4-methoxy-phenyl)benzol;
5-Amino-2-di(2-hydroxyethyl)amino-1-(4-methyl-3-nitrophenyl)benzol;
5-Amino-2-di(2-hydroxyethyl)amino-1-(4-methyl-phenyl)benzol;
5-Amino-2-di(2-hydroxyethyl)amino-1-(5-bromo-2-methoxy-phenyl)benzol;
5-Amino-2-di(2-hydroxyethyl)amino-1-(5-chloro-2-methoxy-phenyl)benzol;
5-Amino-2-di(2-hydroxyethyl)amino-1-(5-formyl-2-methoxy-phenyl)benzol;
5-Amino-2-di(2-methoxyethyl)amino-1-(2,3,4-trimethoxy-phenyl)benzol;
5-Amino-2-di(2-methoxyethyl)amino-1-(2,3-difluoro-4-heptylphenyl)benzol;
5-Amino-2-di(2-methoxyethyl)amino-1-(2,3-difluorophenyl)benzol;
5-Amino-2-di(2-methoxyethyl)amino-1-(2,4,6-trimethylphenyl)benzol;
5-Amino-2-di(2-methoxyethyl)amino-1-(2,4-diaminophenyl)benzol;
5-Amino-2-di(2-methoxyethyl)amino-1-(2,4-dichlorophenyl)benzol;
5-Amino-2-di(2-methoxyethyl)amino-1-(2,4-dihydroxyphenyl)benzol;
5-Amino-2-di(2-methoxyethyl)amino-1-(2,5-dimethoxyphenyl)benzol;
5-Amino-2-di(2-methoxyethyl)amino-1-(2,6-difluorophenyl)benzol;
5-Amino-2-di(2-methoxyethyl)amino-1-(2,6-dimethoxyphenyl)benzol;
5-Amino-2-di(2-methoxyethyl)amino-1-(2-((bis(1-methylethyl)amino)carbonyl)phenyl)benzol;
5-Amino-2-di(2-methoxy-ethyl)amino-1-(2-((bis(1-methylethyl)amino)carbonyl)-3-methoxyphenyl)-benzol;
5-Amino-2-di(2-methoxyethyl)amino-1-(2-(bromomethyl)phenyl)-benzol;
5-Amino-2-di(2-methoxyethyl)amino-1-(2-(diethylamino)carbonyl)-phenyl)-benzol;
5-Amino-2-di(2-methoxyethyl)amino-1-(2-carbonsäure-phenyl)benzol;
5-Amino-2-di(2-methoxyethyl)amino-1-(2-chlorophenyl)-benzol;
5-Amino-2-di(2-methoxyethyl)amino-1-(2-fluorophenyl)benzol;
5-Amino-2-di(2-methoxyethyl)amino-1-(2-formyl-4-methoxyphenyl)benzol;
5-Amino-2-di(2-methoxyethyl)amino-1-(2-formyl-4-methylphenyl)benzol;
5-Amino-2-di(2-methoxyethyl)amino-1-(2-formyl-5-methoxyphenyl)benzol;
5-Amino-2-di(2-methoxyethyl)amino-1-(2-formylphenyl)benzol;
5-Amino-2-di(2-methoxyethyl)amino-1-(2-hydroxy-4-aminophenyl)benzol;
5-Amino-2-di(2-methoxyethyl)amino-1-(2-hydroxyphenyl)benzol;
5-Amino-2-di(2-methoxyethyl)amino-1-(2-methoxyphenyl)benzol;
5-Amino-2-di(2-methoxyethyl)amino-1-(2-methylphenyl)benzol;
5-Amino-2-di(2-methoxyethyl)amino-1-(2-nitrophenyl)benzol;
5-Amino-2-di(2-methoxy-ethyl)amino-1-(2-trifluoromethylphenyl)benzol;
5-Amino-2-di(2-methoxy-ethyl)amino-1-(3,4-dichlorophenyl)benzol;
5-Amino-2-di(2-methoxyethyl)-amino-1-(3,5-diaminophenyl)benzol;
5-Amino-2-di(2-methoxyethyl)amino-1-(3,5-dichlorophenyl)benzol;
5-Amino-2-di(2-methoxyethyl)amino-1-(3,5-dihydroxyphenyl)benzol;
5-Amino-2-di(2-methoxyethyl)amino-1-(3-(acetytamino)phenyl)benzol;
5-Amino-2-di(2-methoxyethyl)amino-1-(3-aminophenyl)benzol;
5-Amino-2-di(2-methoxyethyl)amino-1-(3-bromophenyl)benzol;
5-Amino-2-di(2-methoxyethyl)amino-1-(3-carbonsäurephenyl)benzol;
5-Amino-2-di(2-methoxyethyl)amino-1-(3-chloro-4-fluorophenyl)benzol;
5-Amino-2-di(2-methoxyethyt)amino-1-(3-chlorophenyl)benzol;
5-Amino-2-di(2-methoxyethyl)amino-1-(3-fluoro-phenylbenzol;
5-Amino-2-di(2-methoxyethyl)amino-1-(3-formylphenyl)-benzol;
5-Amino-2-di(2-methoxyethyl)amino-1-(3-hydroxyphenyl)benzol;
5-Amino-2-di(2-methoxyethyl)amino-1-(3-methoxyphenyl)benzol;
5-Amino-2-di(2-methoxyethyl)amino-1-(3-methylphenyl)benzol;
5-Amino-2-di(2-methoxyethyl)amino-1-(3-nitrophenyl)benzol;
5-Amino-2-di(2-methoxy-ethyl)amino-1-(3-trifluoromethylphenyl)benzol;
5-Amino-2-di(2-methoxyethyl)amino-1-(4-(bromomethyl)phenyl)benzol;
5-Amino-2-di(2-methoxyethyl)amino-1-(4-(dimethylamino)phenyl)benzol;
5-Amino-2-di(2-methoxyethyl)amino-1-(4-(hydroxymethyl)phenyl)benzol;
5-Amino-2-di(2-methoxyethyl)amino-1-(4-(methylthio)phenyl)benzol;
5-Amino-2-di(2-methoxyethyl)amino-1-(4-(trifluoromethyl)phenyl)benzol;
5-Amino-2-di(2-methoxyethyl)amino-1-(4-(trimethylsilyl)phenyl)benzol;
5-Amino-2-di(2-methoxyethyl)amino-1-(4-acetylphenyl)benzol;
5-Amino-2-di(2-methoxy-ethyl)amino-1-(4-aminophenyl)benzol;
5-Amino-2-di(2-methoxyethyl)-amino-1-(4-bromophenyl)benzol;
5-Amino-2-di(2-methoxyethyl)amino-1-(4-carbonsäurephenyl)benzol;
5-Amino-2-di(2-methoxyethyl)amino-1-(4-chloro-3-methoxyphenyl)benzol;
5-Amino-2-di(2-methoxyethyl)amino-1-(4-chlorophenyl)benzol;
5-Amino-2-di(2-methoxyethyl)amino-1-(4-ethenyl-phenyl)benzol;
5-Amino-2-di(2-methoxyethyl)amino-1-(4-ethoxyphenyl)-benzol;
5-Amino-2-di(2-methoxyethyl)amino-1-(4-fluorophenyl)benzol;
5-Amino-2-di(2-methoxyethyl)amino-1-(4-formylphenyl)benzol;
5-Amino-2-di(2-methoxyethyl)amino-1-(4-hydroxyphenyl)benzol;
5-Amino-2-di(2-methoxyethyl)amino-1-(4-iodophenyl)benzol;
5-Amino-2-di(2-methoxy-ethyl)amino-1-(4-methoxyphenyl)benzol;
5-Amino-2-di(2-methoxyethyl)-amino-1-(4-methyl-3-nitrophenyl)benzol;
5-Amino-2-di(2-methoxyethyl)-amino-1-(4-methylphenyl)benzol;
5-Amino-2-di(2-methoxyethyl)amino-1-(5-bromo-2-methoxyphenyl)benzol;
5-Amino-2-di(2-methoxyethyl)amino-1-(5-chloro-2-methoxyphenyl)benzol;
5-Amino-2-di(2-methoxyethyl)amino-1-(5-formyl-2-methoxyphenyl)benzol;
5-Amino-2-dimethylamino-1-phenyl-benzol;
5-Amino-2-methylamino-1-(2,3,4-trimethoxyphenyl)benzol;
5-Amino-2-methylamino-1-(2,4-diaminophenyl)benzol;
5-Amino-2-methylamino-1-(2,4-dihydroxyphenyl)benzol;
5-Amino-2-methylamino-1-(2,5-diaminophenyl)benzol;
5-Amino-2-methylamino-1-(2,5-dimethoxy-phenyl)benzol;
5-Amino-2-methylamino-1-(2,6-dimethoxyphenyl)benzol;
5-Amino-2-methylamino-1-(2-amino-5-hydroxyphenyl)benzol;
5-Amino-2-methylamino-1-(2-aminophenyl)benzol;
5-Amino-2-methylamino-1-(2-hydroxy-4-aminophenyl)benzol;
5-Amino-2-methylamino-1-(2-hydroxy-5-aminophenyl)benzol;
5-Amino-2-methylamino-1-(2-hydroxyphenyl)benzol;
5-Amino-2-methylamino-1-(2-methoxyphenyl)benzol;
5-Amino-2-methyl-amino-1-(2-methylphenyl)benzol;
5-Amino-2-methylamino-1-(3,5-diamino-phenyl)benzol;
5-Amino-2-methylamino-1-(3,5-dihydroxyphenyl)benzol;
5-Amino-2-methylamino-1-(3-aminophenyl)benzol;
5-Amino-2-methyl-amino-1-(3-hydroxy-5-aminophenyl)benzol;
5-Amino-2-methylamino-1-(3-hydroxyphenyl)benzol;
5-Amino-2-methylamino-1-(3-methoxyphenyl)-benzol;
5-Amino-2-methylamino-1-(4-(dimethylamino)phenyl)benzol;
5-Amino-2-methylamino-1-(4-(trifluoromethyl)phenyl)benzol;
5-Amino-2-methylamino-1-(4-aminophenyl)benzol;
5-Amino-2-methylamino-1-(4-carbonsäurephenyl)benzol;
5-Amino-2-methylamino-1-(4-chlorophenyl)-benzol;
5-Amino-2-methylamino-1-(4-hydroxyphenyl)benzol;
5-Amino-2-methylamino-1-(4-methoxyphenyl)benzol;
5-Amino-2-methylamino-1-(4-methylphenyl)benzol und
5-Amino-2-methylamino-1-phenylbenzol,
oder deren physiologisch verträgliche Salze.

## Claims

1. Agent for the oxidative colouring of keratin fibres based on a developer substance-coupler substance combination, **characterized in that** it comprises, as developer substance, at least one compound from the group consisting of:
2,5-diamino-1-phenylbenzene;
2,5-diamino-1-(4-bromophenyl)benzene;
2,5-diamino-1-(4-ethenylphenyl)benzene;
2,5-diamino-1-(2,3,4-trimethoxyphenyl)benzene;
2,5-diamino-1-(2,4-di(2-hydroxyethyl)aminophenyl)benzene;
2,5-diamino-1-(2,4-diaminophenyl)benzene;
2,5-diamino-1-(2,4-dihydroxyphenyl)benzene;
2,5-diamino-1-(2,4-dimethylaminophenyl)benzene;
2,5-diamino-1-(2,4-methoxyphenyl)benzene;
2,5-diamino-1-(2,5-di(2-hydroxyethyl)aminophenyl)benzene;
2,5-diamino-1-(2,5-diaminophenyl)benzene;
2,5-diamino-1-(2,5-dihydroxyphenyl)benzene;
2,5-diamino-1-(2,5-dimethoxyphenyl)benzene;
2,5-diamino-1-(2,5-dimethylaminophenyl)benzene;
2,5-diamino-1-(2,6-di(2-hydroxyethyl)aminophenyl)benzene;
2,5-diamino-1-(2,6-diaminophenyl)benzene;
2,5-diamino-1-(2,6-dihydroxyphenyl)benzene;
2,5-diamino-1-(2,6-dimethoxyphenyl)benzene;
2,5-diamino-1-(2,6-dimethylaminophenyl)benzene;
2,5-diamino-1-(2-(bromomethyl)phenyl)benzene;
2,5-diamino-1-(2-amino-5-hydroxyphenyl)benzene;
2,5-diamino-1-(2-aminophenyl)benzene;
2,5-diamino-1-(2-carboxyphenyl)benzene;
2,5-diamino-1-(2-chlorophenyl)benzene;
2,5-diamino-1-(2-di(2-hydroxyethyl)aminophenyl)benzene;
2,5-diamino-1-(2-dimethylaminophenyl)benzene;
2,5-diamino-1-(2-fluorophenyl)benzene;
2,5-diamino-1-(2-formylphenyl)benzene;
2,5-diamino-1-(2-hydroxy-4-aminophenyl)benzene;
2,5-diamino-1-(2-hydroxy-5-aminophenyl)benzene;
2,5-diamino-1-(2-hydroxyphenyl)benzene;
2,5-diamino-1-(2-methoxyphenyl)benzene;
2,5-diamino-1-(2-methylphenyl)benzene;
2,5-diamino-1-(2-nitrophenyl)benzene;
2,5-diamino-1-(2-trifluoromethylphenyl)benzene;
2,5-diamino-1-(3,5-di(2-hydroxyethyl)aminophenyl)-benzene;
2,5-diamino-1-(3,5-diaminophenyl)benzene;
2,5-diamino-1-(3,5-dihydroxyphenyl)benzene;
2,5-diamino-1-(3,5-dimethoxyphenyl)benzene;
2,5-diamino-1-(3,5-dimethylaminophenyl)benzene;
2,5-diamino-1-(3-aminophenyl)benzene;
2,5-diamino-1-(3-bromophenyl)benzene;
2,5-diamino-1-(3-carboxyphenyl)benzene;
2,5-diamino-1-(3-chlorophenyl)benzene;
2,5-diamino-1-(3-di(2-hydroxyethyl)aminophenyl)benzene;
2,5-diamino-1-(3-dimethylaminophenyl)benzene:
2,5-diamino-1-(3-fluorophenyl)benzene;
2,5-diamino-1-(3-formylphenyl)benzene:
2,5-diamino-1-(3-hydroxy-5-aminophenyl)benzene;
2,5-diamino-1-(3-hydroxyphenyl)benzene;
2,5-diamino-1-(3-methoxyphenyl)benzene;
2,5-diamino-1-(3-nitrophenyl)benzene;
2,5-diamino-1-(3-trifluoromethylphenyl)benzene;
2,5-diamino-1-(4-(dimethylamino)phenyl)benzene;
2,5-diamino-1-(4-(hydroxymethyl)phenyl)benzene;
2,5-diamino-1-(4-(methylthio)phenyl)benzene;
2,5-diamino-1-(4-(trifluoromethyl)phenyl)benzene;
2,5-diamino-1-(4-(trimethylsilyl)phenyl)benzene;
2,5-diamino-1-(4-acetylphenyl)benzene;
2,5-diamino-1-(4-aminophenyl)benzene;
2,5-diamino-1-(4-carboxyphenyl)benzene;
2,5-diamino-1-(4-chlorophenyl)benzene;
2,5-diamino-1-(4-di(2-hydroxyethyl)aminophenyl)benzene;
2,5-diamino-1-(4-dimethylaminophenyl)benzene;
2,5-diamino-1-(4-ethoxyphenyl)benzene;
2,5-diamino-1-(4-fluorophenyl)benzene;
2,5-diamino-1-(4-formylphenyl)benzene;
2,5-diamino-1-(4-hydroxyphenyl)benzene;
2,5-diamino-1-(4-methoxyphenyl)benzene;
2,5-diamino-1-(4-methylphenyl)benzene;
2,5-diamino-1-phenylbenzene;
2,5-diamino-4-chloro-1-phenylbenzene;
2,5-diamino-4-methoxy-1-phenylbenzene;
2,5-diamino-4-methyl-1-phenylbenzene;
2,5-diamino-1-(2-cyanomethylphenyl)benzene;
2,5-diamino-1-(4-ethylphenyl)benzene;
2,5-diamino-1-(4-propylphenyl)benzene;
2,5-diamino-1-(4-isopropylphenyl)benzene;
2,5-diamino-1-(4-butylphenyl)benzene;
2,5-diamino-1-(4-tert-butylphenyl)benzene;
2,5-diamino-1-(4-pentylphenyl)benzene;
2,5-diamino-1-(4-thiomethoxyphenyl)benzene;
2,5-diamino-1-(2-ethylphenyl)benzene;
2,5-diamino-1-(2-fluoro-4-methylphenyl)benzene;
2,5-diamino-1-(2-methyl-5-fluorophenyl)benzene;
2,5-diamino-1-(2-thiomethoxyphenyl)benzene;
2,5-diamino-1-(2,3-dichlorophenyl)benzene;
2,5-diamino-4-(4'-hydroxybiphenyl)benzene;
2,5-diamino-1-(3-ethoxyphenyl)benzene;
2,5-diamino-1-(4-(2-pyrrolidin-1-ylethoxy)phenyl)benzene;
2,5-diamino-1-(4-(1-hydroxyethyl)phenyl)benzene;
2,5-diamino-1-(2,4-trifluoromethylphenyl)benzene;
2,5-diamino-1-(2-fluoro-5-acetylphenyl)benzene;
2,5-diamino-1-(3-fluoro-4-methoxyphenyl)benzene;
2,5-diamino-1-(3-acetyl-4-hydroxyphenyl)benzene;
2,5-diamino-1-(4-(2-hydroxyethyl)phenyl)benzene;
2,5-diamino-1-(4-(propyl-1-one)phenyl)benzene;
2,5-diamino-1-(4-N,N'-diisopropylaminomethylphenyl)benzene;
2,5-diamino-1-(3-acetylphenyl)benzene;
2,5-diamino-1-(2-(2-hydroxyethyl)phenyl)benzene;
2,5-diamino-1-(3-aminomethylphenyl)benzene;
2-amino-5-(2,3-dihydroxypropyl)amino-1-(2,3,4-trimethoxyphenyl)benzene;
2-amino-5-(2,3-dihydroxypropyl)amino-1-(2,4-dihydroxyphenyl)benzene;
2-amino-5-(2,3-dihydroxypropyl)amino-1-(2,5-diaminophenyl)benzene;
2-amino-5-(2,3-dihydroxypropyl)amino-1-(2,6-dimethoxyphenyl)benzene;
2-amino-5-(2,3-dihydroxypropyl)amino-1-(2-amino-5-hydroxyphenyl)benzene;
2-amino-5-(2,3-dihydroxypropyl)amino)-1-(2-aminophenyl)benzene;
2-amino-5-(2,3-dihydroxypropyl)amino-1-(2-hydroxy-4-aminophenyl)benzene;
2-amino-5-(2,3-dihydroxypropyl)amino-1-(2-hydroxy-5-aminophenyl)benzene;
2-amino-5-(2,3-dihydroxypropyl)amino-1-(2-hydroxyphenyl)benzene;
2-amino-5-(2,3-dihydroxypropyl)amino-1-(2-methoxyphenyl)benzene;
2-amino-5-(2,3-dihydroxypropyl)amino-1-(2-methylphenyl)benzene;
2-amino-5-(2,3-dihydroxypropyl)amino-1-(3,5-diaminophenyl)benzene;
2-amino-5-(2,3-dihydroxypropyl)amino-1-(3,5-dihydroxyphenyl)benzene;
2-amino-5-(2,3-dihydroxypropyl)amino-1-(3-aminophenyl)benzene;
2-amino-5-(2,3-dihydroxypropyl)amino-1-(3-hydroxy-5-aminophenyl)benzene;
2-amino-5-(2,3-dihydroxypropyl)amino-1-(3-hydroxyphenyl)benzene;
2-amino-5-(2,3-dihydroxypropyl)amino-1-(3-methoxyphenyl)benzene;
2-amino-5-(2,3-dihydroxypropyl)amino-1-(4-(dimethylamino)phenyl)benzene;
2-amino-5-(2,3-dihydroxypropyl)amino-1-(4-(trifluoromethyl)phenyl)benzene;
2-amino-5-(2,3-dihydroxypropyl)amino-1-(4-aminophenyl)benzene;
2-amino-5-(2,3-dihydroxypropyl)amino-1-(4-carboxyphenyl)benzene;
2-amino-5-(2,3-dihydroxypropyl)amino-1-(4-chlorophenyl)benzene;
2-amino-5-(2,3-dihydroxypropyl)amino-1-(4-hydroxyphenyl)benzene;
2-amino-5-(2,3-dihydroxypropyl)amino-1-(4-methoxyphenyl)benzene;
2-amino-5-(2,3-dihydroxypropyl)amino-1-(4-methylphenyl)benzene;
2-amino-5-(2,3-dihydroxypropyl)amino-1-phenylbenzene;
2-amino-5-(2-hydroxyethyl)amino-1-(2,3,4-trimethoxyphenyl)benzene;
2-amino-5-(2-hydroxyethyl)amino-1-(2,4-diaminophenyl)benzene;
2-amino-5-(2-hydroxyethyl)amino-1-(2,4-dihydroxyphenyl)benzene;
2-amino-5-(2-hydroxyethyl)amino-1-(2,5-diaminophenyl)benzene;
2-amino-5-(2-hydroxyethyl)amino-1-(2,5-dimethoxyphenyl)benzene;
2-amino-5-(2-hydroxyethyl)amino-1-(2,6-dimethoxyphenyl)benzene;
2-amino-5-(2-hydroxyethyl)amino-1-(2-amino-5-hydroxyphenyl)benzene;
2-amino-5-(2-hydroxyethyl)amino-1-(2-aminophenyl)benzene;
2-amino-5-(2-hydroxyethyl)amino-1-(2-hydroxy-4-aminophenyl)benzene;
2-amino-5-(2-hydroxyethyl)amino-1-(2-hydroxy-5-aminophenyl)benzene;
2-amino-5-(2-hydroxyethyl)amino-1-(2-hydroxyphenyl)benzene;
2-amino-5-(2-hydroxyethyl)amino-1-(2-methoxyphenyl)benzene;
2-amino-5-(2-hydroxyethyl)amino-1-(2-methylphenyl)benzene;
2-amino-5-(2-hydroxyethyl)amino-1-(3,5-diaminophenyl) benzene;
2-amino-5-(2-hydroxyethyl)amino-1-(3,5-dihydroxyphenyl)benzene;
2-amino-5-(2-hydroxyethyl)amino-1-(3-aminophenyl) benzene;
2-amino-5-(2-hydroxyethyl)amino-1-(3-hydroxy-5-aminophenyl)benzene;
2-amino-5-(2-hydroxyethyl)amino-1-(3-hydroxyphenyl)benzene;
2-amino-5-(2-hydroxyethyl)amino-1-(3-methoxyphenyl) benzene;
2-amino-5-(2-hydroxyethyl)amino-1-(4-(dimethylamino)-phenyl)benzene;
2-amino-5-(2-hydroxyethyl)amino-1-(4-(trifluoromethyl)-phenyl) benzene;
2-amino-5-(2-hydroxyethyl)amino-1-(4-aminophenyl) benzene;
2-amino-5-(2-hydroxyethyl)amino-1-(4-carboxyphenyl)benzene;
2-amino-5-(2-hydroxyethyl)amino-1-(4-chlorophenyl)benzene;
2-amino-5-(2-hydroxyethyl)amino-1-(4-hydroxyphenyl) benzene;
2-amino-5-(2-hydroxyethyl)amino-1-(4-methoxyphenyl)benzene;
2-amino-5-(2-hydroxyethyl)amino-1-(4-methylphenyl)benzene;
2-amino-5-(2-hydroxyethyl)amino-1-phenylbenzene;
2-amino-5-(2-hydroxyethyl)methylamino-1-phenylbenzene;
2-amino-5-(2-methoxyethyl)amino-1-(2,3,4-trimethoxyphenyl)benzene;
2-amino-5-(2-methoxyethyl)amino-1-(2,4-diaminophenyl)benzene;
2-amino-5-(2-methoxyethyl)amino-1-(2,4-dihydroxyphenyl)benzene;
2-amino-5-(2-methoxyethyl)amino-1-(2,5-diaminophenyl)benzene;
2-amino-5-(2-methoxyethyl)amino-1-(2,5-dimethoxyphenyl)benzene;
2-amino-5-(2-methoxyethyl)amino-1-(2,6-dimethoxyphenyl)benzene;
2-amino-5-(2-methoxyethyl)amino-1-(2-amino-5-hydroxyphenyl)benzene;
2-amino-5-(2-methoxyethyl)amino-1-(2-aminophenyl)benzene;
2-amino-5-(2-methoxyethyl)amino-1-(2-hydroxy-4-aminophenyl)benzene;
2-amino-5-(2-methoxyethyl)amino-1-(2-hydroxy-5-aminophenyl)benzene;
2-amino-5-(2-methoxyethyl)amino-1-(2-hydroxyphenyl)benzene;
2-amino-5-(2-methoxyethyl)amino-1-(2-methoxyphenyl)benzene;
2-amino-5-(2-methoxyethyl)amino-1-(2-methylphenyl)benzene;
2-amino-5-(2-methoxyethyl)amino-1-(3,5-diaminophenyl)benzene;
2-amino-5-(2-methoxyethyl)amino-1-(3,5-dihydroxyphenyl)benzene;
2-amino-5-(2-methoxyethyl)amino-1-(3-aminophenyl)benzene;
2-amino-5-(2-methoxyethyl)amino-1-(3-hydroxy-5-aminophenyl)benzene;
2-amino-5-(2-methoxyethyl)amino-1-(3-hydroxyphenyl)benzene;
2-amino-5-(2-methoxyethyl)amino-1-(3-methoxyphenyl)benzene;
2-amino-5-(2-methoxyethyl)amino-1-(4-(dimethylamino)-phenyl)benzene;
2-amino-5-(2-methoxyethyl)amino-1-(4-(trifluoromethyl)-phenyl)benzene;
2-amino-5-(2-methoxyethyl)amino-1-(4-aminophenyl)benzene;
2-amino-5-(2-methoxyethyl)amino-1-(4-carboxyphenyl)benzene;
2-amino-5-(2-methoxyethyl)amino-1-(4-chlorophenyl)benzene;
2-amino-5-(2-methoxyethyl)amino-1-(4-hydroxyphenyl)benzene;
2-amino-5-(2-methoxyethyl)amino-1-(4-methoxyphenyl)benzene;
2-amino-5-(2-methoxyethyl)amino-1-(4-methylphenyl)benzene;
2-amino-5-(2-methoxyethyl)amino-1-phenylbenzene;
2-amino-5-di(2-hydroxyethyl)amino-1-(2,3,4-trimethoxyphenyl)benzene;
2-amino-5-di(2-hydroxyethyl)amino-1-(2,3-difluoro-4-heptylphenyl)benzene;
2-amino-5-di(2-hydroxyethyl)amino-1-(2,3-difluorophenyl)benzene;
2-amino-5-di(2-hydroxyethyl)amino-1-(2,4,6-trimethylphenyl)benzene;
2-amino-5-di(2-hydroxyethyl)amino-1-(2,4-diaminophenyl)benzene;
2-amino-5-di(2-hydroxyethyl)amino-1-(2,4-dichlorophenyl)benzene;
2-amino-5-di(2-hydroxyethyl)amino-1-(2,4-dihydroxyphenyl)benzene;
2-amino-5-di(2-hydroxyethyl)amino-1-(2,5-diaminophenyl)benzene;
2-amino-5-di(2-hydroxyethyl)amino-1-(2,5-dimethoxyphenyl)benzene;
2-amino-5-di(2-hydroxyethyl)amino-1-(2,6-difluorophenyl)benzene;
2-amino-5-di(2-hydroxyethyl)amino-1-(2,6-dimethoxyphenyl)benzene;
2-amino-5-di(2-hydroxyethyl)amino-1-(2,6-dimethoxyphenyl)benzene;
2-amino-5-di(2-hydroxyethyl)amino-1-(2-((bis(1-methylethyl)amino)carbonyl)phenyl)benzene;
2-amino-5-di(2-hydroxyethyl)amino-1-(2-((bis(1-methylethyl)amino)carbonyl)-3-methoxyphenyl)benzene;
2-amino-5-di(2-hydroxyethyl)amino-1-(2-(bromomethyl)phenyl)benzene;
2-amino-5-di(2-hydroxyethyl)amino-1-(2-(diethylamino)-carbonyl)phenyl)benzene;
2-amino-5-di(2-hydroxyethyl)amino-1-(2-amino-5-hydroxyphenyl)benzene;
2-amino-5-di(2-hydroxyethyl)amino-1-(2-aminophenyl) benzene;
2-amino-5-di(2-hydroxyethyl)amino-1-(2-carboxyphenyl)benzene;
2-amino-5-di(2-hydroxyethyl)amino-1-(2-chlorophenyl)benzene;
2-amino-5-di(2-hydroxyethyl)amino-1-(2-fluorophenyl)benzene;
2-amino-5-di(2-hydroxyethyl)amino-1-(2-formyl-4-methoxyphenyl)benzene;
2-amino-5-di(2-hydroxyethyl)amino-1-(2-formyl-4-methylphenyl)benzene;
2-amino-5-di(2-hydroxyethyl)amino-1-(2-formyl-5-methoxyphenyl)benzene;
2-amino-5-di(2-hydroxyethyl)amino-1-(2-formylphenyl)benzene;
2-amino-5-di(2-hydroxyethyl)amino-1-(2-hydroxy-4-aminophenyl)benzene;
2-amino-5-di(2-hydroxyethyl)amino-1-(2-hydroxy-5-aminophenyl)benzene;
2-amino-5-di(2-hydroxyethyl)amino-1-(2-hydroxyphenyl)benzene;
2-amino-5-di(2-hydroxyethyl)amino-1-(2-methoxyphenyl)benzene;
2-amino-5-di(2-hydroxyethyl)amino-1-(2-methylphenyl)benzene;
2-amino-5-di(2-hydroxyethyl)amino-1-(2-nitrophenyl)benzene;
2-amino-5-di(2-hydroxyethyl)amino-1-(2-trifluoromethylphenyl)benzene;
2-amino-5-di(2-hydroxyethyl)amino-1-(3,4-dichlorophenyl)benzene;
2-amino-5-di(2-hydroxyethyl)amino-1-(3,5-diaminophenyl)benzene;
2-amino-5-di(2-hydroxyethyl)amino-1-(3,5-dichlorophenyl)benzene;
2-amino-5-di(2-hydroxyethyl)amino-1-(3,5-dihydroxyphenyl)benzene;
2-amino-5-di(2-hydroxyethyl)amino-1-(3-(acetylamino)phenyl)benzene;
2-amino-5-di(2-hydroxyethyl)amino-1-(3-aminophenyl)benzene;
2-amino-5-di(2-hydroxyethyl)amino-1-(3-bromophenyl)benzene;
2-amino-5-di(2-hydroxyethyl)amino-1-(3-carboxyphenyl)benzene;
2-amino-5-di(2-hydroxyethyl)amino-1-(3-chloro-4-flurophenyl)benzene;
2-amino-5-di(2-hydroxyethyl)amino-1-(3-chlorophenyl)benzene;
2-amino-5-di(2-hydroxyethyl)amino-1(3-fluorophenyl)benzene;
2-amino-5-di(2-hydroxyethyl)amino-1-(3-formylphenyl)benzene;
2-amino-5-di(2-hydroxyethyl)amino-1-(3-hydroxy-5-aminophenyl)benzene;
2-amino-5-di(2-hydroxyethyl)amino-1-(3-hydroxyphenyl) benzene;
2-amino-5-di(2-hydroxyethyl)amino-1-(3-hydroxyphenyl)benzene;
2-amino-5-di(2-hydroxyethyl)amino-1-(3-methoxyphenyl)benzene;
2-amino-5-di(2-hydroxyethyl)amino-1-(3-methylphenyl)benzene;
2-amino-5-di(2-hydroxyethyl)amino-1-(3-nitrophenyl)benzene;
2-amino-5-di(2-hydroxyethyl)amino-1-(3-trifluoromethylphenyl)benzene;
2-amino-5-di(2-hydroxyethyl)amino-1-(4-(bromomethyl)-phenyl)benzene;
2-amino-5-di(2-hydroxyethyl)amino-1-(4-(dimethylamino)-phenyl)benzene;
2-amino-5-di(2-hydroxyethyl)amino-1-(4-(hydroxymethyl)-phenyl)benzene;
2-amino-5-di(2-hydroxyethyl)amino-1-(4-(methylthio)-phenyl)benzene;
2-amino-5-di(2-hydroxyethyl)amino-1-(4-(trifluoromethyl)phenyl)benzene;
2-amino-5-di(2-hydroxyethyl)amino-1-(4-(trimethylsilyl)phenyl)benzene;
2-amino-5-di(2-hydroxyethyl)amino-1-(4-acetylphenyl)benzene;
2-amino-5-di(2-hydroxyethyl)amino-1-(4-aminophenyl)benzene;
2-amino-5-di(2-hydroxyethyl)amino-1-(4-bromophenyl)benzene;
2-amino-5-di(2-hydroxyethyl)amino-1-(4-carboxyphenyl)-benzene;
2-amino-5-di(2-hydroxyethyl)amino-1-(4-chloro-3-methoxyphenyl)benzene;
2-amino-5-di(2-hydroxyethyl)amino-1-(4-chlorophenyl)-benzene;
2-amino-5-di(2-hydroxyethyl)amino-1-(4-ethenylphenyl)-benzene;
2-amino-5-di(2-hydroxyethyl)amino-1-(4-flurophenyl)-benzene;
2-amino-5-di(2-hydroxyethyl)amino-1-(4-formylphenyl)-benzene;
2-amino-5-di(2-hydroxyethyl)amino-1-(4-hydroxyphenyl)-benzene;
2-amino-5-di(2-hydroxyethyl)amino-1-(4-iodophenyl)-benzene;
2-amino-5-di(2-hydroxyethyl)amino-1-(4-methoxyphenyl)-benzene;
2-amino-5-di(2-hydroxyethyl)amino-1-(4-methyl-3-nitrophenyl)benzene;
2-amino-5-di(2-hydroxyethyl)amino-1-(4-methylphenyl)-benzene;
2-amino-5-di(2-hydroxethyl)amino-1-(5-bromo-2-methoxyphenyl)benzene;
2-amino-5-di(2-hydroxyethyl)amino-1-(5-chloro-2-methoxyphenyl)benzene;
2-amino-5-di(2-hydroxethyl)amino-1-(5-formyl-2-methoxyphenyl)benzene;
2-amino-5-di(2-hydroxyethyl)amino-1-phenylbenzene;
2-amino-5-di(2-methoxyethyl)amino-1-(2,3,4-trimethoxyphenyl)benzene;
2-amino-5-di(2-methoxyethyl)amino-1-(2,3-difluoro-4-heptylphenyl)benzene;
2-amino-5-di(2-methoxyethyl)amino-1-(2,3-difluorophenyl)benzene;
2-amino-5-di(2-methoxyethyl)amino-1-(2,4,6-trimethylphenyl)benzene;
2-amino-5-di(2-methoxyethyl)amino-1-(2,4-diaminophenyl)benzene;
2-amino-5-di(2-methoxyethyl)amino-1-(2,4-dichlorophenyl)benzene;
2-amino-5-di(2-methoxyethyl)amino-1-(2,4-dihyroxyphenyl)benzene;
2-amino-5-di(2-methoxyethyl)amino-1-(2,5-dimethoxyphenyl)benzene;
2-amino-5-di(2-methoxyethyl)amino-1-(2,6-difluorophenyl)benzene;
2-amino-5-di(2-methoxyethyl)amino-1-(2,6-dimethoxyphenyl)benzene;
2-amino-5-di(2-methoxyethyl)amino-1-(2-((bis(1-methylethyl)amino)carbonyl)phenyl)benzene;
2-amino-5-di(2-methoxyethyl)amino-1-(2-(bromomethyl)-phenyl)benzene;
2-amino-5-di(2-methoxyethyl)amino-1-(2-(diethylamino)-carbonyl)phenyl)benzene;
2-amino-5-di(2-methoxyethyl)amino-1-(2-carboxyphenyl)benzene;
2-amino-5-di(2-methoxyethyl)amino-1-(2-chlorophenyl)benzene;
2-amino-5-di(2-methoxyethyl)amino-1-(2-fluorophenyl)benzene;
2-amino-5-di(2-methoxyethyl)amino-1-(2-formyl-4-methoxyphenyl)benzene;
2-amino-5-di(2-methoxyethyl)amino-1-(2-formyl-4-methylphenyl)benzene;
2-amino-5-di(2-methoxyethyl)amino-1-(2-formyl-5-methoxyphenyl)benzene;
2-amino-5-di(2-methoxyethyl)amino-1-(2-formylphenyl)-benzene;
2-amino-5-di(2-methoxyethyl)amino-1-(2-hydroxy-4-aminophenyl)benzene;
2-amino-5-di(2-methoxyethyl)amino-1-(2-hydroxyphenyl)-benzene;
2-amino-5-di(2-methoxyethyl)amino-1-(2-methoxyphenyl)-benzene;
2-amino-5-di(2-methoxyethyl)amino-1-(2-methylphenyl)-benzene;
2-amino-5-di(2-methoxyethyl)amino-1-(2-nitrophenyl)-benzene;
2-amino-5-di(2-methoxyethyl)amino-1-(2-trifluoromethylphenyl)benzene;
2-amino-5-di(2-methoxyethyl)amino-1-(3,4-dichlorophenyl)benzene;
2-amino-5-di(2-methoxyethyl)amino-1-(3,5-diaminophenyl)benzene;
2-amino-5-di(2-methoxyethyl)amino-1-(3,5-dichlorophenyl)benzene;
2-amino-5-di(2-methoxyethyl)amino-1-(3,5,dihydroxyphenyl)benzene;
2-amino-5-di(2-methoxyethyl)amino-1-(3-(acetylamino)-phenyl)benzene;
2-amino-5-di(2-methoxyethyl)amino-1-(3-aminophenyl)benzene;
2-amino-5-di(2-methoxyethyl)amino-1-(3-bromophenyl)-benzene;
2-amino-5-di(2-methoxyethyl)amino-1-(3-carboxyphenyl)-benzene;
2-amino-5-di(2-methoxyethyl)amino-1-(3-chloro-4-fluorophenyl)benzene;
2-amino-5-di(2-methoxyethyl)amino-1-(3-chlorophenyl)-benzene;
2-amino-5-di(2-methoxyethyl)amino-1-(3-fluorophenyl)-benzene;
2-amino-5-di(2-methoxyethyl)amino-1-(3-formylphenyl)-benzene;
2-amino-5-di(2-methoxyethyl)amino-1-(3-hydroxyphenyl)-benzene;
2-amino-5-di(2-methoxyethyl)amino-1-(3-methoxyphenyl)-benzene;
2-amino-5-di(2-methoxyethyl)amino-1-(3-methylphenyl)-benzene;
2-amino-5-di(2-methoxyethyl)amino-1-(3-nitrophenyl)-benzene;
2-amino-5-di(2-methoxyethyl)amino-1-(3-trifluoromethylphenyl)benzene;
2-amino-5-di(2-methoxyethyl)amino-1-(4-(bromomethyl)-phenyl)benzene;
2-amino-5-di(2-methoxyethyl)amino-1-(4-(dimethylamino)-phenyl)benzene;
2-amino-5-di(2-methoxyethyl)amino-1-(4-(hydroxymethyl)-phenyl)benzene;
2-amino-5-di(2-methoxyethyl)amino-1-(4-(methylthio)-phenyl)benzene;
2-amino-5-di(2-methoxyethyl)amino-1-(4-(trifluoromethyl)phenyl)benzene;
2-amino-5-di(2-methoxyethyl)amino-1-(4-(trimethylsilyl)phenyl)benzene;
2-amino-5-di(2-methoxyethyl)amino-1-(4-acetylphenyl)-benzene;
2-amino-5-di(2-methoxyethyl)amino-1-(4-aminophenyl)-benzene;
2-amino-5-di(2-methoxyethyl)amino-1-(4-bromophenyl)-benzene;
2-amino-5-di(2-methoxyethyl)amino-1-(4-carboxyphenyl)benzene;
2-amino-5-di(2-methoxyethyl)amino-1-(4-chloro-3-methoxyphenyl)benzene;
2-amino-5-di(2-methoxyethyl)amino-1-(4-chlorophenyl)-benzene;
2-amino-5-di(2-methoxyethyl)amino-1-(4-ethenylphenyl)-benzene;
2-amino-5-di(2-methoxyethyl)amino-1-(4-ethoxyphenyl)-benzene;
2-amino-5-di(2-methoxyethyl)amino-1-(4-fluorophenyl)-benzene;
2-amino-5-di(2-methoxyethyl)amino-1-(4-formylphenyl)-benzene;
2-amino-5-di(2-methoxyethyl)amino-1-(4-hydroxyphenyl)-benzene;
2-amino-5-di(2-methoxyethyl)amino-1-(4-iodophenyl)-benzene;
2-amino-5-di(2-methoxyethyl)amino-1-(4-methoxyphenyl)-benzene;
2-amino-5-di(2-methoxyethyl)amino-1-(4-methyl-3-nitrophenyl)benzene;
2-amino-5-di(2-methoxyethyl)amino-1-(4-methylphenyl)-benzene;
2-amino-5-di(2-methoxyethyl)amino-1-(5-bromo-2-methoxyphenyl)benzene;
2-amino-5-di(2-methoxyethyl)amino-1-(5-chloro-2-methoxyphenyl)benzene;
2-amino-5-di(2-methoxyethyl)amino-1-(5-formyl-2-methoxyphenyl)benzene;
2-amino-5-dimethylamino-1-phenylbenzene;
2-amino-5-methylamino-1-(2,3,4-trimethoxyphenyl)-benzene;
2-amino-5-methylamino-1-(2,4-diaminophenyl)benzene;
2-amino-5-methylamino-1-(2,4-dihydroxyphenyl)benzene;
2-amino-5-methylamino-1-(2,5-diaminophenyl)benzene;
2-amino-5-methylamino-1-(2,5-dimethoxyphenyl)benzene;
2-amino-5-methylamino-1-(2,6-dimethoxyphenyl)benzene;
2-amino-5-methylamino-1-(2-amino-5-hydroxyphenyl)-benzene;
2-amino-5-methylamino-1-(2-aminophenyl)benzene;
2-amino-5-methylamino-1-(2-hydroxy-4-aminophenyl)-benzene;
2-amino-5-methylamino-1-(2-hydroxy-5-aminophenyl)-benzene;
2-amino-5-methylamino-1-(2-hydroxyphenyl)benzene;
2-amino-5-methylamino-1-(2-methoxyphenyl)benzene;
2-amino-5-methylamino-1-(2-methylphenyl)benzene;
2-amino-5-methylamino-1-(3,5-diaminophenyl)benzene;
2-amino-5-methylamino-1-(3,5-dihydroxyphenyl)benzene;
2-amino-5-methylamino-1-(3-aminophenyl)benzene;
2-amino-5-methylamino-1-(3-hydroxy-5-aminophenyl)benzene;
2-amino-5-methylamino-1-(3-hydroxyphenyl)benzene;
2-amino-5-methylamino-1-(3-methoxyphenyl)benzene;
2-amino-5-methylamino-1-(4-(dimethylamino)phenyl)-benzene;
2-amino-5-methylamino-1-(4-(trifluoromethyl)phenyl)-benzene;
2-amino-5-methylamino-1-(4-aminophenyl)benzene;
2-amino-5-methylamino-1-(4-carboxyphenyl)benzene;
2-amino-5-methylamino-1-(4-chlorophenyl)benzene;
2-amino-5-methylamino-1-(4-hydroxyphenyl)benzene;
2-amino-5-methylamino-1-(4-methoxyphenyl)benzene;
2-amino-5-methylamino-1-(4-methylphenyl)benzene;
2-amino-5-methylamino-1-phenylbenzene;
5-amino-2-(2,3-dihydroxypropyl)amino-1-(4-bromophenyl)benzene;
5-amino-2-(2,3-dihydroxypropyl)amino-1-(4-ethenylphenyl)benzene;
5-amino-2-(2,3-dihydroxypropyl)amino-1-(2,3,4-trimethoxyphenyl)benzene;
5-amino-2-(2,3-dihydroxypropyl)amino-1-(2,4-di(2-hydroxyethyl)aminophenyl)benzene;
5-amino-2-(2,3-dihydroxypropyl)amino-1-(2,4-diaminophenyl)benzene;
5-amino-2-(2,3-dihydroxypropyl)amino-1-(2,4-dihydroxyphenyl)benzene;
5-amino-2-(2,3-dihydroxypropyl)amino-1-(2,4-dimethylaminophenyl)benzene;
5-amino-2-(2,3-dihydroxypropyl)amino-1-(2,4-methoxyphenyl)benzene;
5-amino-2-(2,3-dihydroxypropyl)amino-1-(2,5-di(2-hydroxyethyl)aminophenyl)benzene;
5-amino-2-(2,3-dihydroxypropyl)amino-1-(2,5-diaminophenyl)benzene;
5-amino-2-(2,3-dihydroxypropyl)amino-1-(2,5-dihydroxyphenyl)benzene;
5-amino-2-(2,3-dihydroxypropyl)amino-1-(2,5-dimethoxyphenyl)benzene;
5-amino-2-(2,3-dihydroxypropyl)amino-1-(2,5-dimethylaminophenyl)benzene;
5-amino-2-(2,3-dihydroxypropyl)amino-1-(2,6-di(2-hydroxyethyl)aminophenyl)benzene;
5-amino-2-(2,3-dihydroxypropyl)amino-1-(2,6-diaminophenyl)benzene;
5-amino-2-(2,3-dihydroxypropyl)amino-1-(2,6-dihydroxyphenyl)benzene;
5-amino-2-(2,3-dihydroxypropyl)amino-1-(2,6-dimethoxyphenyl)benzene;
5-amino-2-(2,3-dihydroxypropyl)amino-1-(2,6-dimethylaminophenyl)benzene;
5-amino-2-(2,3-dihydroxypropyl)amino-1-(2-(bromomethyl)phenyl)benzene;
5-amino-2-(2,3-dihydroxypropyl)amino-1-(2-amino-5-hydroxyphenyl)benzene;
5-amino-2-(2,3-dihydroxypropyl)amino-1-(2-aminophenyl)benzene;
5-amino-2-(2,3-dihydroxypropyl)amino-1-(2-carboxyphenyl)benzene;
5-amino-2-(2,3-dihydroxypropyl)amino-1-(2-chlorophenyl)benzene;
5-amino-2-(2,3-dihydroxypropyl)amino-1-(2-di(2-hydroxyethyl)aminophenyl)benzene;
5-amino-2-(2,3-dihydroxypropyl)amino-1-(2-dimethylaminophenyl)benzene;
5-amino-2-(2,3-dihydroxypropyl)amino-1-(2-fluorophenyl)benzene;
5-amino-2-(2,3-dihydroxypropyl)amino-1-(2-formylphenyl)benzene;
5-amino-2-(2,3-dihydroxypropyl)amino-1-(2-hydroxy-4-aminophenyl)benzene;
5-amino-2-(2,3-dihydroxypropyl)amino-1-(2-hydroxy-5-aminophenyl)benzene;
5-amino-2-(2,3-dihyroxypropyl)amino-1-(2-hydroxyphenyl)benzene;
5-amino-2-(2,3-dihydroxypropyl)amino-1-(2-methoxyphenyl)benzene;
5-amino-2-(2,3-dihydroxypropyl)amino-1-(2-methylphenyl)benzene;
5-amino-2-(2,3-dihydroxypropyl)amino-1-(2-nitrophenyl)benzene;
5-amino-2-(2,3-dihydroxypropyl)amino-1-(2-trifluoromethylphenyl)benzene;
5-amino-2-(2,3-dihyroxypropyl)amino-1-(3,5-di(2-hydroxyethyl)aminophenyl)benzene;
5-amino-2-(2,3-dihydroxypropyl)amino-1-(3,5-diaminophenyl)benzene;
5-amino-2-(2,3-dihydroxypropyl)amino-1-(3,5-dihydroxyphenyl)benzene;
5-amino-2-(2,3-dihydroxypropyl)amino-1-(3,5-dimethoxyphenyl)benzene;
5-amino-2-(2,3-dihydroxypropyl)amino-1-(3,5-dimethylaminophenyl)benzene;
5-amino-2-(2,3-dihydroxypropyl)amino-1-(3-aminophenyl)benzene;
5-amino-2-(2,3-dihydroxypropyl)amino-1-(3-bromophenyl)benzene;
5-amino-2-(2,3-dihydroxypropyl)amino-1-(3-carboxyphenyl)benzene;
5-amino-2-(2,3-dihydroxypropyl)amino-1-(3-chlorophenyl)benzene;
5-amino-2-(2,3-dihydroxypropyl)amino-1-(3-di(2-hydroxyethyl)aminophenyl)benzene;
5-amino-2-(2,3-dihydroxypropyl)amino-1-(3-dimethylaminophenyl)benzene;
5-amino-2-(2,3-dihydroxypropyl)amino-1-(3-fluorophenyl)benzene;
5-amino-2-(2,3-dihydroxyproply)amino-1-(3-formylphenyl)benzene;
5-amino-2-(2,3-dihydroxypropyl)amino-1-(3-hydroxy-5-aminophenyl)benzene;
5-amino-2-(2,3-dihydroxypropyl)amino-1-(3-hydroxyphenyl)benzene;
5-amino-2-(2,3-dihydroxypropyl)amino-1-(3-methoxyphenyl)benzene;
5-amino-2-(2,3-dihydroxypropyl)amino-1-(3-nitrophenyl)benzene;
5-amino-2-(2,3-dihydroxypropyl)amino-1-(3-trifluoromethylphenyl)benzene;
5-amino-2-(2,3-dihydroxypropyl)amino-1-(4-(dimethylamino)phenyl)benzene;
5-amino-2-(2,3-dihydroxypropyl)amino-1-(4-(hydroxymethyl)phenyl)benzene;
5-amino-2-(2,3-dihydroxypropyl)amino-1-(4-(methylthio)phenyl)benzene;
5-amino-2-(2,3-dihydroxypropyl)amino-1-(4-(trifluoromethyl)phenyl)benzene;
5-amino-2-(2,3-dihydroxypropyl)amino-1-(4-(trimethylsilyl)phenyl)benzene;
5-amino-2-(2,3-dihydroxypropyl)amino-1-(4-acetylphenyl)benzene;
5-amino-2-(2,3-dihydroxypropyl)amino-1-(4-aminophenyl)benzene;
5-amino-2-(2,3-dihydroxpypropyl)amino-1-(4-carboxyphenyl)benzene;
5-amino-2-(2,3-dihydroxypropyl)amino-1-(4-chlorophenyl)benzene;
5-amino-2-(2,3-dihydroxypropyl)amino-1-(4-di(2-hydroxyethyl)aminophenyl)benzene
5-amino-2-(2,3-dihydroxypropyl)amino-1-(4-dimethylaminophenyl)benzene;
5-amino-2-(2,3-dihydroxypropyl)amino-1-(4-ethoxyphenyl)benzene;
5-amino-2-(2,3-dihydroxypropyl)amino-1-(4-fluorophenyl)benzene;
5-amino-2-(2,3-dihydroxypropyl)amino-1-(4-formylphenyl)benzene;
5-amino-2-(2,3-dihydroxypropyl)amino-1-(4-hydroxyphenyl)benzene;
5-amino-2-(2,3-dihydroxypropyl)amino-1-(4-methoxyphenyl)benzene;
5-amino-2-(2,3-dihydroxypropyl)amino-1-(4-methylphenyl)benzene;
5-amino-2-(2,3-dihydroxypropyl)amino-1-phenylbenzene;
5-amino-2-(2-hydroxyethyl)amino-1-(4-bromophenyl)benzene;
5-amino-2-(2-hydroxyethyl)amino-1-(4-ethenylphenyl)benzene;
5-amino-2-(2-hydroxyethyl)amino-1-(2,3,4-trimethoxyphenyl)benzene;
5-amino-2-(2-hydroxyethyl)amino-1-(2,4-di(2-hydroxyethyl)aminophenyl)benzene;
5-amino-2-(2-hydroxyethyl)amino-1-(2,4-diaminophenyl)benzene;
5-amino-2-(2-hydroxyethyl)amino-1-(2,4-dihydroxyphenyl)benzene;
5-amino-2-(2-hydroxyethyl)amino-1-(2,4-dimethylaminophenyl)benzene;
5-amino-2-(2-hydroxyethyl)amino-1-(2,4-methoxyphenyl)benzene;
5-amino-2-(2-hydroxyethyl)amino-1-(2,5-di(2-hydroxyethyl)aminophenyl)benzene;
5-amino-2-(2-hydroxyethyl)amino-1-(2,5-diaminophenyl)benzene;
5-amino-2-(2-hydroxyethyl)amino-1-(2,5-dihydroxyphenyl)benzene;
5-amino-2-(2-hydroxyethyl)amino-1-(2,5-dimethoxyphenyl)benzene;
5-amino-2-(2-hydroxyethyl)amino-1-(2,5-dimethylaminophenyl)benzene;
5-amino-2-(2-hydroxyethyl)amino-1-(2,6-di(2-hydroxyethyl)aminophenyl)benzene;
5-amino-2-(2-hydroxyethyl)amino-1-(2,6-diaminophenyl)benzene;
5-amino-2-(2-hydroxyethyl)amino-1-(2,6-dihydroxyphenyl)benzene;
5-amino-2-(2-hydroxyethyl)amino-1-(2,6-dimethoxyphenyl)benzene;
5-amino-2-(2-hydroxyethyl)amino-1-(2,6-dimethylaminophenyl)benzene;
5-amino-2-(2-hydroxyethyl)amino-1-(2-(bromomethyl)phenyl)benzene;
5-amino-2-(2-hydroxyethyl)amino-1-(2-amino-5-hydroxyphenyl)benzene;
5-amino-2-(2-hydroxyethyl)amino-1-(2-aminophenyl)benzene;
5-amino-2-(2-hydroxyethyl)amino-1-(2-carboxyphenyl)benzene;
5-amino-2-(2-hydroxyethyl)amino-1-(2-chlorophenyl)benzene;
5-amino-2-(2-hydroxyethyl)amino-1-(2-di(2-hydroxyethyl)aminophenyl)benzene;
5-amino-2-(2-hydroxyethyl)amino-1-(2-dimethylaminophenyl)benzene;
5-amino-2-(2-hydroxyethyl)amino-1-(2-fluorophenyl)benzene;
5-amino-2-(2-hydroxyethyl)amino-1-(2-formylphenyl)benzene;
5-amino-2-(2-hydroxyethyl)amino-1-(2-hydroxy-4-aminophenyl)benzene;
5-amino-2-(2-hydroxyethyl)amino-1-(2-hydroxy-5-aminophenyl)benzene;
5-amino-2-(2-hydroxyethyl)amino-1-(2-hydroxyphenyl)benzene;
5-amino-2-(2-hydroxyethyl)amino-1-(2-methoxyphenyl)benzene;
5-amino-2-(2-hydroxyethyl)amino-1-(2-methylphenyl) benzene;
5-amino-2-(2-hydroxyethyl)amino-1-(2-nitrophenyl)benzene;
5-amino-2-(2-hydroxyethyl)amino-1-(2-trifluoromethylphenyl)benzene;
5-amino-2-(2-hydroxyethyl)amino-1-(3,5-di(2-hydroxyethyl)aminophenyl)benzene;
5-amino-2-(2-hydroxyethyl)amino-1-(3,5-diaminophenyl)benzene;
5-amino-2-(2-hydroxyethyl)amino-1-(3,5-dihydroxyphenyl)benzene;
5-amino-2-(2-hydroxyethyl)amino-1-(3,5-dimethoxyphenyl)benzene;
5-amino-2-(2-hydroxyethyl)amino-1-(3,5-dimethylaminophenyl)benzene;
5-amino-2-(2-hydroxyethyl)amino-1-(3-aminophenyl)benzene;
5-amino-2-(2-hydroxyethyl)amino-1-(3-bromophenyl)benzene;
5-amino-2-(2-hydroxyethyl)amino-1-(3-carboxyphenyl)benzene;
5-amino-2-(2-hydroxyethyl)amino-1-(3-chlorophenyl)benzene;
5-amino-2-(2-hydroxyethyl)amino-1-(3-di(2-hydroxyethyl)aminophenyl)benzene;
5-amino-2-(2-hydroxyethyl)amino-1-(3-dimethylaminophenyl)benzene;
5-amino-2-(2-hydroxyethyl)amino-1-(3-fluorophenyl)benzene;
5-amino-2-(2-hydroxyethyl)amino-1-(3-formylphenyl)benzene;
5-amino-2-(2-hydroxyethyl)amino-1-(3-hydroxy-5-aminophenyl)benzene;
5-amino-2-(2-hydroxyethyl)amino-1-(3-hydroxyphenyl)benzene;
5-amino-2-(2-hydroxyethyl)amino-1-(3-methoxyphenyl)benzene;
5-amino-2-(2-hydroxyethyl)amino-1-(3-nitrophenyl)benzene;
5-amino-2-(2-hydroxyethyl)amino-1-(3-trifluoromethylphenyl)benzene;
5-amino-2-(2-hydroxyethyl)amino-1-(4-(dimethylamino)phenyl)benzene;
5-amino-2-(2-hydroxyethyl)amino-1-(4-(hydroxymethyl)phenyl)benzene;
5-amino-2-(2-hydroxyethyl)amino-1-(4-(methylthio)phenyl)benzene;
5-amino-2-(2-hydroxyethyl)amino-1-(4-(trifluoromethyl)phenyl)benzene;
5-amino-2-(2-hydroxyethyl)amino-1-(4-(trimethylsilyl)phenyl)benzene;
5-amino-2-(2-hydroxyethyl)amino-1-(4-acetylphenyl)benzene;
5-amino-2-(2-hydroxyethyl)amino-1-(4-aminophenyl)benzene;
5-amino-2-(2-hydroxyethyl)amino-1-(4-carboxyphenyl)benzene;
5-amino-2-(2-hydroxyethyl)amino-1-(4-chlorophenyl)benzene;
5-amino-2-(2-hydroxyethyl)amino-1-(4-di(2-hydroxyethyl)aminophenyl)benzene;
5-amino-2-(2-hydroxyethyl)amino-1-(4-dimethylaminophenyl)benzene;
5-amino-2-(2-hydroxyethyl)amino-1-(4-ethoxyphenyl)benzene;
5-amino-2-(2-hydroxyethyl)amino-1-(4-fluorophenyl)benzene;
5-amino-2-(2-hydroxyethyl)amino-1-(4-formylphenyl)benzene;
5-amino-2-(2-hydroxyethyl)amino-1-(4-hydroxyphenyl)benzene;
5-amino-2-(2-hydroxyethyl)amino-1-(4-methoxyphenyl)benzene;
5-amino-2-(2-hydroxyethyl)amino-1-(4-methylphenyl)benzene;
5-amino-2-(2-hydroxyethyl)amino-1-phenylbenzene;
5-amino-2-(2-methoxyethyl)amino-1-(2,3,4-trimethoxyphenyl)benzene;
5-amino-2-(2-methoxyethyl)amino-1-(2,4-diaminophenyl)benzene;
5-amino-2-(2-methoxyethyl)amino-1-(2,4-dihydroxyphenyl)benzene;
5-amino-2-(2-methoxyethyl)amino-1-(2,5-diaminophenyl)benzene;
5-amino-2-(2-methoxyethyl)amino-1-(2,5-dimethoxyphenyl)benzene;
5-amino-2-(2-methoxyethyl)amino-1-(2,6-dimethoxyphenyl)benzene;
5-amino-2-(2-methoxyethyl)amino-1-(2-amino-5-hydroxyphenyl)benzene;
5-amino-2-(2-methoxyethyl)amino-1-(2-aminophenyl)benzene;
5-amino-2-(2-methoxyethyl)amino-1-(2-hydroxy-4-aminophenyl)benzene;
5-amino-2-(2-methoxyethyl)amino-1-(2-hydroxy-5-aminophenyl)benzene;
5-amino-2-(2-methoxyethyl)amino-1-(2-hydroxyphenyl)benzene;
5-amino-2-(2-methoxyethyl)amino-1-(2-methoxyphenyl)benzene;
5-amino-2-(2-methoxyethyl)amino-1-(2-methylphenyl)benzene;
5-amino-2-(2-methoxyethyl)amino-1-(3,5-diaminophenyl)benzene;
5-amino-2-(2-methoxyethyl)amino-1-(3,5-dihydroxyphenyl)benzene;
5-amino-2-(2-methoxyethyl)amino-1-(3-aminophenyl)benzene;
5-amino-2-(2-methoxyethyl)amino-1-(3-hydroxy-5-aminophenyl)benzene;
5-amino-2-(2-methoxyethyl)amino-1-(3-hydroxyphenyl)benzene;
5-amino-2-(2-methoxyethyl)amino-1-(3-methoxyphenyl)benzene;
5-amino-2-(2-methoxyethyl)amino-1-(4-(dimethylamino)phenyl)benzene;
5-amino-2-(2-methoxyethyl)amino-1-(4-(trifluoromethyl)phenyl)benzene;
5-amino-2-(2-methoxyethyl)amino-1-(4-aminophenyl)benzene;
5-amino-2-(2-methoxyethyl)amino-1-(4-carboxyphenyl)benzene;
5-amino-2-(2-methoxyethyl)amino-1-(4-chlorophenyl)benzene;
5-amino-2-(2-methoxyethyl)amino-1-(4-hydroxyphenyl)benzene;
5-amino-2-(2-methoxyethyl)amino-1-(4-methoxyphenyl)benzene;
5-amino-2-(2-methoxyethyl)amino-1-(4-methylphenyl)benzene;
5-amino-2-(2-methoxyethyl)amino-1-phenylbenzene;
5-amino-2-di(2-hydroxyethyl)amino-1-(2,3,4-trimethoxyphenyl)benzene;
5-amino-2-di(2-hydroxyethyl)amino-1-(2,3-difluoro-4-heptylphenyl)benzene;
5-amino-2-di(2-hydroxyethyl)amino-1-(2,3-difluorophenyl)benzene;
5-amino-2-di(2-hydroxyethyl)amino-1-(2,4,6-trimethylphenyl)benzene;
5-amino-2-di(2-hydroxyethyl)amino-1-(2,4-diaminophenyl)benzene;
5-amino-2-di(2-hydroxyethyl)amino-1-(2,4-dichlorophenyl)benzene;
5-amino-2-di(2-hydroxyethyl)amino-1-(2,4-dihydroxyphenyl)benzene;
5-amino-2-di(2-hydroxyethyl)amino-1-(2,5-dimethoxyphenyl)benzene;
5-amino-2-di(2-hydroxyethyl)amino-1-(2,6-difluorophenyl)benzene;
5-amino-2-di(2-hydroxyethyl)amino-1-(2,6-dimethoxyphenyl)benzene;
5-amino-2-di(2-hydroxyethyl)amino-1-(2-((bis(1-methylethyl)amino)carbonyl)phenyl)benzene;
5-amino-2-di(2-hydroxyethyl)amino-1-(2-((bis(1-methylethyl)amino)carbonyl)-3-methoxyphenyl)benzene;
5-amino-2-di(2-hydroxyethyl)amino-1-(2-(bromomethyl)phenyl) benzene;
5-amino-2-di(2-hydroxyethyl)amino-1-(2-(diethylamino)carbonyl)phenyl)benzene;
5-amino-2-di(2-hydroxyethyl)amino-1-(2carboxyphenyl)benzene;
5-amino-2-di(2-hydroxyethyl)amino-1-(2-chlorophenyl)benzene;
5-amino-2-di(2-hydroxyethyl)amino-1-(2-fluorophenyl)benzene;
5-amino-2-di(2-hydroxyethyl)amino-1-(2-formyl-4-methoxyphenyl)benzene;
5-amino-2-di(2-hydroxyethyl)amino-1-(2-formyl-4-methylphenyl)benzene;
5-amino-2-di(2-hydroxyethyl)amino-1-(2-formyl-5-methoxyphenyl)benzene;
5-amino-2-di(2-hydroxyethyl)amino-1-(2-formylphenyl)benzene;
5-amino-2-di(2-hydroxyethyl)amino-1-(2-hydroxy-4-aminophenyl)benzene;
5-amino-2-di(2-hydroxyethyl)amino-1-(2-hydroxyphenyl)benzene;
5-amino-2-di(2-hydroxyethyl)amino-1-(2-methoxyphenyl)benzene;
5-amino-2-di(2-hydroxyethyl)amino-1-(2-methylphenyl)benzene;
5-amino-2-di(2-hydroxyethyl)amino-1-(2-nitrophenyl)benzene;
5-amino-2-di(2-hydroxyethyl)amino-1-(2-trifluoromethylphenyl)benzene;
5-amino-2-di(2-hydroxyethyl)amino-1-(3,4-dichlorophenyl)benzene;
5-amino-2-di(2-hydroxyethyl)amino-1-(3,5-diaminophenyl)benzene;
5-amino-2-di(2-hydroxyethyl)amino-1-(3,5-dichlorophenyl)benzene;
5-amino-2-di(2-hydroxyethyl)amino-1-(3,5-dihydroxyphenyl)benzene;
5-amino-2-di(2-hydroxyethyl)amino-1-(3-(acetylamino)phenyl)benzene;
5-amino-2-di(2-hydroxyethyl)amino-1-(3-aminophenyl)benzene;
5-amino-2-di(2-hydroxyethyl)amino-1-(3-bromophenyl)benzene;
5-amino-2-di(2-hydroxyethyl)amino-1-(3carboxyphenyl)benzene;
5-amino-2-di(2-hydroxyethyl)amino-1-(3-chloro-4-fluorophenyl)benzene;
5-amino-2-di(2-hydroxyethyl)amino-1-(3-chlorophenyl)benzene;
5-amino-2-di(2-hydroxyethyl)amino-1-(3-fluorophenyl)benzene;
5-amino-2-di(2-hydroxyethyl)amino-1-(3-formylphenyl)benzene;
5-amino-2-di(2-hydroxyethyl)amino-1-(3-hydroxyphenyl)benzene;
5-amino-2-di(2-hydroxyethyl)amino-1-(3-methoxyphenyl)benzene;
5-amino-2-di(2-hydroxyethyl)amino-1-(3-methylphenyl)benzene;
5-amino-2-di(2-hydroxyethyl)amino-1-(3-nitrophenyl)benzene;
5-amino-2-di(2-hydroxyethyl)amino-1-(3-trifluoromethylphenyl)benzene;
5-amino-2-di(2-hydroxyethyl)amino-1-(4-(bromomethyl)phenyl)benzene;
5-amino-2-di(2-hydroxyethyl)amino-1-(4-(dimethylamino)phenyl)benzene;
5-amino-2-di(2-hydroxyethyl)amino-1-(4-(hydroxymethyl)phenyl)benzene;
5-amino-2-di(2-hydroxyethyl)amino-1-(4-(methylthio)phenyl)benzene;
5-amino-2-di(2-hydroxyethyl)amino-1-(4-(trifluoro methyl) phenyl) benzene;
5-amino-2-di(2-hydroxyethyl)amino-1-(4-(trimethylsilyl)phenyl)benzene;
5-amino-2-di(2-hydroxyethyl)amino-1-(4-acetylphenyl)benzene;
5-amino-2-di(2-hydroxyethyl)amino-1-(4-aminophenyl)benzene;
5-amino-2-di(2-hydroxyethyl)amino-1-(4-bromophenyl)benzene;
5-amino-2-di(2-hydroxyethyl)amino-1-(4carboxyphenyl)benzene;
5-amino-2-di(2-hydroxyethyl)amino-1-(4-chloro-3-methoxyphenyl)benzene;
5-amino-2-di(2-hydroxyethyl)amino-1-(4-chlorophenyl)benzene;
5-amino-2-di(2-hydroxyethyl)amino-1-(4-ethenylphenyl)benzene;
5-amino-2-di(2-hydroxyethyl)amino-1-(4-ethoxyphenyl)benzene;
5-amino-2-di(2-hydroxyethyl)amino-1-(4-fluorophenyl)benzene;
5-amino-2-di(2-hydroxyethyl)amino-1-(4-formylphenyl)benzene;
5-amino-2-di(2-hydroxyethyl)amino-1-(4-hydroxyphenyl)benzene;
5-amino-2-di(2-hydroxyethyl)amino-1-(4-iodophenyl)benzene;
5-amino-2-di(2-hydroxyethyl)amino-1-(4-methoxyphenyl)benzene;
5-amino-2-di(2-hydroxyethyl)amino-1-(4-methyl-3-nitrophenyl)benzene;
5-amino-2-di(2-hydroxyethyl)amino-1-(4-methylphenyl)benzene;
5-amino-2-di(2-hydroxyethyl)amino-1-(5-bromo-2-methoxyphenyl)benzene;
5-amino-2-di(2-hydroxyethyl)amino-1-(5-chloro-2-methoxyphenyl)benzene;
5-amino-2-di(2-hydroxyethyl)amino-1-(5-formyl-2-methoxyphenyl)benzene;
5-amino-2-di(2-methoxyethyl)amino-1-(2,3,4-trimethoxyphenyl)benzene;
5-amino-2-di(2-methoxyethyl)amino-1-(2,3-difluoro-4-heptylphenyl)benzene;
5-amino-2-di(2-methoxyethyl)amino-1-(2,3-difluorophenyl)benzene;
5-amino-2-di(2-methoxyethyl)amino-1-(2,4,6-trimethylphenyl)benzene;
5-amino-2-di(2-methoxyethyl)amino-1-(2,4-diaminophenyl) benzene;
5-amino-2-di(2-methoxyethyl)amino-1-(2,4-dichlorophenyl)benzene;
5-amino-2-di(2-methoxyethyl)amino-1-(2,4-dihydroxyphenyl)benzene;
5-amino-2-di(2-methoxyethyl)amino-1-(2,5-dimethoxyphenyl)benzene;
5-amino-2-di(2-methoxyethyl)amino-1-(2,6-difluorophenyl)benzene;
5-amino-2-di(2-methoxyethyl)amino-1-(2,6-dimethoxyphenyl)benzene;
5-amino-2-di(2-methoxyethyl)amino-1-(2-((bis(1-methylethyl)amino)carbonyl)phenyl)benzene;
5-amino-2-di(2-methoxyethyl)amino-1-(2-((bis(1-methylethyl)amino)carbonyl)3-methoxyphenyl)benzene;
5-amino-2-di(2-methoxyethyl)amino-1-(2-(bromomethyl)phenyl) benzene;
5-amino-2-di(2-methoxyethyl)amino-1-(2-(diethylamino)carbonyl)phenyl)benzene;
5-amino-2-di(2-methoxyethyl)amino-1-(2-carboxyphenyl)benzene;
5-amino-2-di(2-methoxyethyl)amino-1-(2-chlorophenyl)benzene;
5-amino-2-di(2-methoxyethyl)amino-1-(2-fluorophenyl)benzene;
5-amino-2-di(2-methoxyethyl)amino-1-(2-formyl-4-methoxyphenyl)benzene;
5-amino-2-di(2-methoxyethyl)amino-1-(2-formyl-4-methylphenyl)benzene;
5-amino-2-di(2-methoxyethyl)amino-1-(2-formyl-5-methoxyphenyl)benzene;
5-amino-2-di(2-methoxyethyl)amino-1-(2-formylphenyl)benzene;
5-amino-2-di(2-methoxyethyl)amino-1-(2-hydroxy-4-aminophenyl) benzene;
5-amino-2-di(2-methoxyethyl)amino-1-(2-hydroxyphenyl)benzene;
5-amino-2-di(2-methoxyethyl)amino-1-(2-methoxyphenyl)benzene;
5-amino-2-di(2-methoxyethyl)amino-1-(2-methylphenyl)benzene;
5-amino-2-di(2-methoxyethyl)amino-1-(2-nitrophenyl)benzene;
5-amino-2-di(2-methoxyethyl)amino-1-(2-trifluoromethylphenyl)benzene;
5-amino-2-di(2-methoxyethyl)amino-1-(3,4-dichlorophenyl)benzene;
5-amino-2-di(2-methoxyethyl)amino-1-(3,5-diaminophenyl)benzene;
5-amino-2-di(2-methoxyethyl)amino-1-(3,5-dichlorophenyl)benzene;
5-amino-2-di(2-methoxyethyl)amino-1-(3,5-dihydroxyphenyl)benzene;
5-amino-2-di(2-methoxyethyl)amino-1-(3-(acetylamino)phenyl)benzene;
5-amino-2-di(2-methoxyethyl)amino-1-(3-aminophenyl)benzene;
5-amino-2-di(2-methoxyethyl)amino-1-(3-bromophenyl)benzene;
5-amino-2-di(2-methoxyethyl)amino-1-(3-carboxyphenyl)benzene;
5-amino-2-di(2-methoxyethyl)amino-1-(3-chloro-4-fluorophenyl)benzene;
5-amino-2-di(2-methoxyethyl)amino-1-(3-chlorophenyl)benzene;
5-amino-2-di(2-methoxyethyl)amino-1-(3-fluorophenyl)benzene;
5-amino-2-di(2-methoxyethyl)amino-1-(3-formylphenyl)benzene;
5-amino-2-di(2-methoxyethyl)amino-1-(3-hydroxyphenyl)benzene;
5-amino-2-di(2-methoxyethyl)amino-1-(3-methoxyphenyl)benzene;
5-amino-2-di(2-methoxyethyl)amino-1-(3-methylphenyl)benzene;
5-amino-2-di(2-methoxyethyl)amino-1-(3-nitrophenyl)benzene;
5-amino-2-di(2-methoxyethyl)amino-1-(3-trifluoromethylphenyl)benzene;
5-amino-2-di(2-methoxyethyl)amino-1-(4-(bromomethyl)phenyl)benzene;
5-amino-2-di(2-methoxyethyl)amino-1-(4-dimethylamino)phenyl) benzene;
5-amino-2-di(2-methoxyethyl)amino-1-(4-hydroxymethyl)phenyl)benzene;
5-amino-2-di(2-methoxyethyl)amino-1-(4-(methylthio)phenyl)benzene;
5-amino-2-di(2-methoxyethyl)amino-1-(4-(trifluoromethyl)phenyl)benzene;
5-amino-2-di(2-methoxyethyl)amino-1-(4-(trimethylsilyl)phenyl)benzene;
5-amino-2-di(2-methoxyethyl)amino-1-(4-acetylphenyl)benzene;
5-amino-2-di(2-methoxyethyl)amino-1-(4-aminophenyl)benzene;
5-amino-2-di(2-methoxyethyl)amino-1-(4-bromophenyl)benzene;
5-amino-2-di(2-methoxyethyl)amino-1-(4carboxyphenyl)benzene;
5-amino-2-di(2-methoxyethyl)amino-1-(4-chloro-3-methoxyphenyl)benzene;
5-amino-2-di(2-methoxyethyl)amino-1-(4-chlorophenyl)benzene;
5-amino-2-di(2-methoxyethyl)amino-1-(4-ethenylphenyl)benzene;
5-amino-2-di(2-methoxyethyl)amino-1-(4-ethoxyphenyl)benzene;
5-amino-2-di(2-methoxyethyl)amino-1-(4-fluorophenyl)benzene;
5-amino-2-di(2-methoxyethyl)amino-1-(4-formylphenyl)benzene;
5-amino-2-di(2-methoxyethyl)amino-1-(4-hydroxyphenyl)benzene;
5-amino-2-di(2-methoxyethyl)amino-1-(4-iodophenyl)benzene;
5-amino-2-di(2-methoxyethyl)amino-1-(4-methoxyphenyl)benzene;
5-amino-2-di(2-methoxyethyl)amino-1-(4-methyl-3-nitrophenyl)benzene;
5-amina-2-di(2-methoxyethyl)amino-1-(4-methylphenyl)benzene;
5-amino-2-di(2-methoxyethyl)amino-1-(5-bromo-2-methoxyphenyl)benzene;
5-amino-2-di(2-methoxyethyl)amino-1-(5-chloro-2-methoxyphenyl)benzene;
5-amino-2-di(2-methoxyethyl)amino-1-(5-formyl-2-methoxyphenyl)benzene;
5-amino-2-dimethylamino-1-phenylbenzene;
5-amino-2-methylamino-1-(2,3,4-trimethoxyphenyl)-benzene;
5-amino-2-methylamino-1-(2,4-diaminophenyl)benzene;
5-amino-2-methylamino-1-(2,4-dihydroxyphenyl)benzene;
5-amino-2-methylamino-1-(2,5-diaminophenyl)benzene;
5-amino-2-methylamino-1-(2,5-dimethoxyphenyl)benzene;
5-amino-2-methylamino-1-(2,6-dimethoxyphenyl)benzene;
5-amino-2-methylamino-1-(2-amino-5-hydroxphenyl)-benzene;
5-amino-2-methylamino-1-(2-aminophenyl)benzene;
5-amino-2-methylamino-1-(2-hydroxy-4-aminophenyl)benzene;
5-amino-2-methylamino-1-(2-hydroxy-5-aminophenyl)benzene;
5-amino-2-methylamino-1-(2-hydroxyphenyl)benzene;
5-amino-2-methylamino-1-(2-methoxyphenyl)benzene;
5-amino-2-methylamino-1-(2-methylphenyl)benzene;
5-amino-2-methylamino-1-(3,5-diaminophenyl)benzene;
5-amino-2-methylamino-1-(3,5-dihydroxyphenyl)benzene;
5-amino-2-methylamino-1-(3-aminophenyl)benzene;
5-amino-2-methylamino-1-(3-hydroxy-5-aminophenyl)benzene;
5-amino-2-methylamino-1-(3-hydroxyphenyl)benzene;
5-amino-2-methylamino-1-(3-methoxyphenyl)benzene;
5-amino-2-methylamino-1-(4-(dimethylamino)phenyl)benzene;
5-amino-2-methylamino-1-(4-(trifluoromethyl)phenyl)benzene;
5-amino-2-methylamino-1-(4-aminophenyl)benzene;
5-amino-2-methylamino-1-(4-carboxyphenyl)benzene;
5-amino-2-methylamino-1-(4-chlorophenyl)benzene;
5-amino-2-methylamino-1-(4-hydroxyphenyl)benzene;
5-amino-2-methylamino-1-(4-methoxyphenyl)benzene;
5-amino-2-methylamino-1-(4-methylphenyl)benzene and
5-amino-2-methylamino-1-phenylbenzene, or
physiologically compatible salts thereof.

2. Agent according to Claim 1, **characterized in that** the developer substance is chosen from 2,5-diamino-1-phenylbenzene; 2,5-diamino-1-(3-nitrophenyl)benzene; 2,5-diamino-1-(4-methoxyphenyl)benzene; 2,5-diamino-1-(3-methoxyphenyl)benzene 2,5-diamino-1-(3-aminophenyl)benzene; 2,5-diamino-1-(2-methylphenyl)benzene: 2,5-diamino-1-(3-methylphenyl)benzene; 2,5-diamino-1(4-methylphenyl)benzene; 2,5-diamino-1-(3-chlorophenyl)benzene and 2,5-diamino-1-(4-chlorophenyl)benzene, or physiologically compatible salts thereof.

3. Agent according to Claim 1 or 2, **characterized in that** it comprises the diaminobenzene derivative of the formula (I) in an amount of from 0.005 to 20.0% by weight.

4. Agent according to one of Claims 1 to 3, **characterized in that**, apart from the developer substance according to the Claim 1 or 2, it additionally comprises at least one further developer substance which is chosen from 1,4-diaminobenzene, 2,5-diaminotoluene, 2,5-diaminophenylethyl alcohol, 4-aminophenol and its derivatives, 4,5-diaminopyradol derivatives and tetraminopyrimidines.

5. Agent according to one of Claims 1 to 4, **characterized in that** the developer substances and coupler substances are in each case present in a total amount of from 0.005 to 20% by weight, based on the total amount of the oxidizing colouring agent.

6. Agent according to one of Claims 1 to 5, **characterized in that** it additionally comprises at least one direct dye.

7. Agent according to one of Claims 1 to 6, **characterized in that** it has a pH of from 6.8 to 11.5.

8. Agent according to one of Claims 1 to 7, **characterized in that** it is in the form of an aquoues or aqueous-alcoholic solution, a cream, a gel or an emulsion.

9. Agent according to one of Claims 1 to 8, **characterized in that** it is a hair-colouring agent.

10. Compound which is chosen from the group consisting of
2,5-diamino-1-(4-bromophenyl)benzene;
2,5-diamino-1-(4-ethenylphenyl)benzene;
2,5-diamino-1-(2,3,4-trimethoxyphenyl)benzene;
2,5-diamino-1-(2,4-di(2-hydroxyethyl)aminophenyl)benzene;
2,5-diamino-1-(2,4-diaminophenyl)benzene;
2,5-diamino-1-(2,4-dihydroxyphenyl)benzene;
2,5-diamino-1-(2,4-dimethylaminophenyl)benzene;
2,5-diamino-1-(2,4-methoxyphenyl)benzene;
2,5-diamino-1-(2,5-di(2-hydroxyethyl)aminophenyl)benzene;
2,5-diamino-1-(2,5-diaminophenyl)benzene;
2,5-diamino-1-(2,5-dihydroxyphenyl)benzene;
2,5-diamino-1-(2,5-dimethoxyphenyl)benzene;
2,5-diamino-1-(2,5-dimethylaminophenyl)benzene;
2,5-diamino-1-(2,6-di(2-hydroxyethyl)aminophenyl)benzene;
2,5-diamino-1-(2,6-diaminophenyl)benzene;
2,5-diamino-1-(2,6-dihydroxyphenyl)benzene;
2,5-diamino-1-(2,6-dimethoxyphenyl)benzene;
2,5-diamino-1-(2,6-dimethylaminophenyl)benzene;
2,5-diamino-1-(2-(bromomethyl)phenyl)benzene;
2,5-diamino-1-(2-amino-5-hydroxyphenyl)benzene;
2,5-diamino-1-(2-aminophenyl)benzene;
2,5-diamino-1-(2-carboxyphenyl)benzene;
2,5-diamino-1-(2-chlorophenyl)benzene;
2,5-diamino-1-(2-di(2-hydroxyethyl)aminophenyl)benzene;
2,5-diamino-1-(2-dimethylaminophenyl)benzene;
2,5-diamino-1-(2-fluorophenyl)benzene;
2,5-diamino-1-(2-formylphenyl)benzene;
2,5-diamino-1-(2-hydroxy-4-aminophenyl)benzene;
2,5-diamino-1-(2-hydroxy-5-aminophenyl)benzene;
2,5-diamino-1-(2-hydroxyphenyl)benzene;
2,5-diamino-1-(2-methoxyphenyl)benzene;
2,5-diamino-1-(2-methylphenyl)benzene;
2,5-diamino-1-(2-nitrophenyl)benzene;
2,5-diamino-1-(2-trifluoromethylphenyl)benzene;
2,5-diamino-1-(3,5-di(2-hydroxyethyl)aminophenyl)benzene;
2,5-diamino-1-(3,5-diaminophenyl)benzene;
2,5-diamino-1-(3,5-dihydroxyphenyl)benzene;
2,5-diamino-1-(3,5-dimethoxyphenyl)benzene;
2,5-diamino-1-(3,5-dimethylaminophenyl)benzene;
2,5-diamino-1-(3-aminophenyl)benzene;
2,5-diamino-1-(3-bromophenyl)benzene;
2,5-diamino-1-(3-carboxyphenyl)benzene;
2,5-diamino-1-(3-chlorophenyl)benzene;
2,5-diamino-1-(3-di(2-hydroxyethyl)aminophenyl)benzene;
2,5-diamino-1-(3-dimethylaminophenyl)benzene;
2,5-diamino-1-(3-fluorophenyl)benzene;
2,5-diamino-1-(3-formylphenyl)benzene;
2,5-diamino-1-(3-hydroxy-5-aminophenyl)benzene;
2,5-diamino-1-(3-hydroxyphenyl)benzene;
2,5-diamino-1-(3-methoxyphenyl)benzene;
2,5-diamino-1-(3-nitrophenyl)benzene;
2,5-diamino-1-(3-trifluoromethylphenyl)benzene;
2,5-diamino-1-(4-(dimethylamino)phenyl)benzene;
2,5-diamino-1-(4-(hydroxymethyl)phenyl)benzene;
2,5-diamino-1-(4-(methylthio)phenyl)benzene;
2,5-diamino-1-(4-(trifluoromethyl)phenyl)benzene;
2,5-diamino-1-(4-(trimethylsilyl)phenyl)benzene;
2,5-diamino-1-(4-acetylphenyl)benzene;
2,5-diamino-1-(4-aminophenyl)benzene;
2,5-diamino-1-(4-carboxyphenyl)benzene;
2,5-diamino-1-(4-chlorophenyl)benzene;
2,5-diamino-1-(4-di(2-hydroxyethyl)aminophenyl)benzene;
2,5-diamino-1-(4-dimethylaminophenyl)benzene;
2,5-diamino-1-(4-ethoxyphenyl)benzene;
2,5-diamino-1-(4-fluorophenyl)benzene;
2,5-diamino-1-(4-formylphenyl)benzene;
2,5-diamino-1-(4-hydroxyphenyl)benzene;
2,5-diamino-1-(4-methoxyphenyl)benzene;
2,5-diamino-1-(4-methylphenyl)benzene;
2,5-diamino-1-phenylbenzene;
2,5-diamino-4-chloro-1-phenylbenzene;
2,5-diamino-4-methoxy-1-phenylbenzene;
2,5-diamino-4-methyl-1-phenylbenzene;
2,5-diamino-1-(2-cyanomethylphenyl)benzene;
2,5-diamino-1-(4-ethylphenyl)benzene;
2,5-diamino-1-(4-propylphenyl)benzene;
2,5-diamino-1-(4-isopropylphenyl)benzene;
2,5-diamino-1-(4-butylphenyl)benzene;
2,5-diamino-1-(4-tert-butylphenyl)benzene;
2,5-diamino-1-(4-pentylphenyl)benzene;
2,5-diamino-1-(4-thiomethoxyphenyl)benzene;
2,5-diamino-1-(2-ethylphenyl)benzene;
2,5-diamino-1-(2-fluoro-4-methylphenyl)benzene;
2,5-diamino-1-(2-methyl-5-fluorophenyl)benzene;
2,5-diamino-1-(2-thiomethoxyphenyl)benzene;
2,5-diamino-1-(2,3-dichlorophenyl)benzene;
2,5-diamino-4-(4'-hydroxybiphenyl)benzene;
2,5-diamino-1-(3-ethoxyphenyl)benzene;
2,5-diamino-1-(4-(2-pyrrolidin-1-ylethoxy)phenyl)benzene;
2,5-diamino-1-(4-(1-hydroxyethyl)phenyl)benzene;
2,5-diamino-1-(2,4-trifluoromethylphenyl)benzene;
2,5-diamino-1-(2-fluoro-5-acetylphenyl)benzene;
2,5-diamino-1-(3-fluoro-4-methoxyphenyl)benzene;
2,5-diamino-1-(3-acetyl-4-hydroxyphenyl)benzene;
2,5-diamino-1-(4-(2-hydroxyethyl)phenyl)benzene;
2,5-diamino-1-(4-(propyl-1-one)phenyl)benzene;
2,5-diamino-1-(4-N,N'-diisopropylaminomethylphenyl)benzene;
2,5-diamino-1-(3-acetylphenyl)benzene;
2,5-diamino-1-(2-(2-hydroxyethyl)phenyl)benzene;
2,5-diamino-1-(3-aminomethylphenyl)benzene;
2-amino-5-(2,3-dihydroxypropyl)amino-1-(2,3,4-trimethoxyphenyl)benzene;
2-amino-5-(2,3-dihydroxypropyl)amino-1-(2,4-dihydroxyphenyl)benzene;
2-amino-5-(2,3-dihydroxypropyl)amino-1-(2,5-diaminophenyl)benzene;
2-amino-5-(2,3-dihydroxypropyl)amino-1-(2,6-dimethoxyphenyl)benzene;
2-amino-5-(2,3-dihydroxypropyl)amino-1-(2-amino-5-hydroxyphenyl)benzene;
2-amino-5-(2,3-dihydroxypropyl)amino)-1-(2-aminophenyl)benzene;
2-amino-5-(2,3-dihydroxypropyl)amino-1-(2-hydroxy-4-aminophenyl)benzene;
2-amino-5-(2,3-dihydroxypropyl)amino-1-(2-hydroxy-5-aminophenyl)benzene;
2-amino-5-(2,3-dihydroxypropyl)amino-1-(2-hydroxyphenyl)benzene;
2-amino-5-(2,3-dihydroxypropyl)amino-1-(2-methoxyphenyl)benzene;
2-amino-5-(2,3-dihydroxypropyl)amino-1-(2-methylphenyl)benzene;
2-amino-5-(2,3-dihydroxypropyl)amino-1-(3,5-diaminophenyl)benzene;
2-amino-5-(2,3-dihydroxypropyl)amino-1-(3,5-dihydroxyphenyl)benzene;
2-amino-5-(2,3-dihydroxypropyl)amino-1-(3-aminophenyl)benzene;
2-amino-5-(2,3-dihydroxypropyl)amino-1-(3-hydroxy-5-aminophenyl)benzene;
2-amino-5-(2,3-dihydroxypropyl)amino-1-(3-hydroxyphenyl)benzene;
2-amino-5-(2,3-dihydroxypropyl)amino-1-(3-methoxyphenyl)benzene;
2-amino-5-(2,3-dihydroxypropyl)amino-1-(4-(dimethylamino)phenyl)benzene;
2-amino-5-(2,3-dihydroxypropyl)amino-1-(4-(trifluoromethyl)phenyl)benzene;
2-amino-5-(2,3-dihydroxypropyl)amino-1-(4-aminophenyl)benzene;
2-amino-5-(2,3-dihydroxypropyl)amino-1-(4-carboxyphenyl)benzene;
2-amino-5-(2,3-dihydroxypropyl)amino-1-(4-chlorophenyl)benzene;
2-amino-5-(2,3-dihydroxypropyl)amino-1-(4-hydroxyphenyl)benzene;
2-amino-5-(2,3-dihydroxypropyl)amino-1-(4-methoxyphenyl)benzene;
2-amino-5-(2,3-dihydroxypropyl)amino-1-(4-methylphenyl)benzene;
2-amino-5-(2,3-dihydroxypropyl)amino-1-phenylbenzene;
2-amino-5-(2-hydroxyethyl)amino-1-(2,3,4-trimethoxyphenyl)benzene;
2-amino-5-(2-hydroxyethyl)amino-1-(2,4-diaminophenyl)benzene;
2-amino-5-(2-hydroxyethyl)amino-1-(2,4-dihydroxyphenyl)benzene;
2-amino-5-(2-hydroxyethyl)amino-1-(2,5-diaminophenyl)benzene;
2-amino-5-(2-hydroxyethyl)amino-1-(2,5-dimethoxyphenyl)benzene;
2-amino-5-(2-hydroxyethyl)amino-1-(2,6-dimethoxyphenyl)benzene;
2-amino-5-(2-hydroxyethyl)amino-1-(2-amino-5-hydroxyphenyl)benzene;
2-amino-5-(2-hydroxyethyl)amino-1-(2-aminophenyl)benzene;
2-amino-5-(2-hydroxyethyl)amino-1-(2-hydroxy-4-aminophenyl)benzene;
2-amino-5-(2-hydroxyethyl)amino-1-(2-hydroxy-5-aminophenyl)benzene;
2-amino-5-(2-hydroxyethyl)amino-1-(2-hydroxyphenyl)benzene;
2-amino-5-(2-hydroxyethyl)amino-1-(2-methoxyphenyl)benzene;
2-amino-5-(2-hydroxyethyl)amino-1-(2-methylphenyl)benzene;
2-amino-5-(2-hydroxyethyl)amino-1-(3,5-diaminophenyl)benzene;
2-amino-5-(2-hydroxyethyl)amino-1-(3,5-dihydroxyphenyl)benzene;
2-amino-5-(2-hydroxyethyl)amino-1-(3-aminophenyl)benzene;
2-amino-5-(2-hydroxyethyl)amino-1-(3-hydroxy-5-aminophenyl)benzene;
2-amino-5-(2-hydroxyethyl)amino-1-(3-hydroxyphenyl)benzene;
2-amino-5-(2-hydroxyethyl)amino-1-(3-methoxyphenyl)benzene;
2-amino-5-(2-hydroxyethyl)amino-1-(4-(dimethylamino)-phenyl)benzene;
2-amino-5-(2-hydroxyethyl)amino-1-(4-(trifluoromethyl)-phenyl)benzene;
2-amino-5-(2-hydroxyethyl)amino-1-(4-aminophenyl)benzene;
2-amino-5-(2-hydroxyethyl)amino-1-(4-carboxyphenyl)benzene;
2-amino-5-(2-hydroxyethyl)amino-1-(4-chlorophenyl)benzene;
2-amino-5-(2-hydroxyethyl)amino-1-(4-hydroxyphenyl)benzene;
2-amino-5-(2-hydroxyethyl)amino-1-(4-methoxyphenyl)benzene;
2-amino-5-(2-hydroxyethyl)amino-1-(4-methylphenyl) benzene;
2-amino-5-(2-hydroxyethyl)amino-1-phenylbenzene;
2-amino-5-(2-hydroxyethyl)methylamino-1-phenylbenzene;
2-amino-5-(2-methoxyethyl)amino-1-(2,3,4-trimethoxyphenyl)benzene;
2-amino-5-(2-methoxyethyl)amino-1-(2,4-diaminophenyl) benzene;
2-amino-5-(2-methoxyethyl)amino-1-(2,4-dihydroxyphenyl)benzene;
2-amino-5-(2-methoxyethyl)amino-1-(2,5-diaminophenyl)benzene;
2-amino-5-(2-methoxyethyl)amino-1-(2,5-dimethoxyphenyl)benzene;
2-amino-5-(2-methoxyethyl)amino-1-(2,6-dimethoxyphenyl)benzene;
2-amino-5-(2-methoxyethyl)amino-1-(2-amino-5-hydroxyphenyl)benzene;
2-amino-5-(2-methoxyethyl)amino-1-(2-aminophenyl)benzene;
2-amino-5-(2-methoxyethyl)amino-1-(2-hydroxy-4-aminophenyl)benzene;
2-amino-5-(2-methoxyethyl)amino-2-(2-hydroxy-5-aminophenyl)benzene;
2-amino-5-(2-methoxyethyl)amino-1-(2-hydroxyphenyl)benzene;
2-amino-5-(2-methoxyethyl)amino-1-(2-methoxyphenyl)benzene;
2-amino-5-(2-methoxyethyl)amino-1-(2-methylphenyl)benzene;
2-amino-5-(2-methoxyethyl)amino-1-(3,5-diaminophenyl)benzene;
2-amino-5-(2-methoxyethyl)amino-1-(3,5-dihydroxyphenyl)benzene;
2-amino-5-(2-methoxyethyl)amino-1-(3-aminophenyl)benzene;
2-amino-5-(2-methoxyethyl)amino-1-(3-hydroxy-5-aminophenyl)benzene;
2-amino-5-(2-methoxyethyl)amino-1-(3-hydroxyphenyl)benzene;
2-amino-5-(2-methoxyethyl)amino-1-(3-methoxyphenyl)benzene;
2-amino-5-(2-methoxyethyl)amino-1-(4-(dimethylamino)phenyl)benzene;
2-amino-5-(2-methoxyethyl)amino-1-(4-(trifluoromethyl)phenyl)benzene;
2-amino-5-(2-methoxyethyl)amino-1-(4-aminophenyl)benzene;
2-amino-5-(2-methoxyethyl)amino-1-(4-carboxyphenyl)benzene;
2-amino-5-(2-methoxyethyl)amino-1-(4-chlorophenyl)benzene;
2-amino-5-(2-methoxyethyl)amino-1-(4-hydroxyphenyl)benzene;
2-amino-5-(2-methoxyethyl)amino-1-(4-methoxyphenyl)benzene;
2-amino-5-(2-methoxyethyl)amino-1-(4-methylphenyl)benzene;
2-amino-5-(2-methoxyethyl)amino-1-phenylbenzene;
2-amino-5-di(2-hydroxyethyl)amino-1-(2,3,4-trimethoxyphenyl)benzene;
2-amino-5-di(2-hydroxyethyl)amino-1-(2,3-difluoro-4-heptylphenyl)benzene;
2-amino-5-di(2-hydroxyethyl)amino-1-(2,3-difluorophenyl)benzene;
2-amino-5-di(2-hydroxyethyl)amino-1-(2,4,6-trimethylphenyl)benzene;
2-amino-5-di(2-hydroxyethyl)amino-1-(2,4-diaminophenyl)benzene;
2-amino-5-di(2-hydroxyethyl)amino-1-(2,4-dichlorophenyl)benzene;
2-amino-5-di(2-hydroxyethyl)amino-1-(2,4-dihydroxyphenyl)benzene;
2-amino-5-di(2-hydroxyethyl)amino-1-(2,5-diaminophenyl)benzene;
2-amino-5-di(2-hydroxyethyl)amino-1-(2,5-dimethoxyphenyl)benzene;
2-amino-5-di(2-hydroxyethyl)amino-1-(2,6-difluorophenyl)benzene;
2-amino-5-di(2-hydroxyethyl)amino-1-(2,6-dimethoxyphenyl)benzene;
2-amino-5-di(2-hydroxyethyl)amino-1-(2,6-dimethoxyphenyl)benzene;
2-amino-5-di(2-hydroxyethyl)amino-1-(2-((bis(1-methylethyl)amino)carbonyl)phenyl)benzene;
2-amino-5-di(2-hydroxyethyl)amino-1-(2-((bis(1-methylethyl)amino)carbonyl)-3-methoxyphenyl)benzene;
2-amino-5-di(2-hydroxyethyl)amino-1-(2-(bromomethyl)phenyl)benzene;
2-amino-5-di(2-hydroxyethyl)amino-1-(2-(diethylamino)carbonyl)phenyl)benzene;
2-amino-5-di(2-hydroxyethyl)amino-1-(2-amino-5-hydroxyphenyl)benzene;
2-amino-5-di(2-hydroxyethyl)amino-1-(2-aminophenyl)benzene;
2-amino-5-di(2-hydroxyethyl)amino-1-(2-carboxyphenyl)benzene;
2-amino-5-di(2-hydroxyethyl)amino-1-(2-chlorophenyl)benzene;
2-amino-5-di(2-hydroxyethyl)amino-1-(2-fluorophenyl)benzene;
2-amino-5-di(2-hydroxyethyl)amino-1-(2-formyl-4-methoxyphenyl)benzene;
2-amino-5-di(2-hydroxyethyl)amino-1-(2-formyl-4-methylphenyl)benzene;
2-amino-5-di(2 -hydroxyethyl)amino-1-(2-formyl-5-methoxyphenyl)benzene;
2-amino-5-di(2-hydroxyethyl)amino-1-(2-formylphenyl)benzene;
2-amino-5-di(2-hydroxyethyl)amino-1-(2-hydroxy-4-aminophenyl)benzene;
2-amino-5-di(2-hydroxyethyl)amino-1-(2-hydroxy-5-aminophenyl)benzene;
2-amino-5-di(2-hydroxyethyl)amino-1-(2-hydroxyphenyl)benzene;
2-amino-5-di(2-hydroxyethyl)amino-1-(2-methoxyphenyl)benzene;
2-amino-5-di(2-hydroxyethyl)amino-1-(2-methylphenyl)benzene;
2-amino-5-di(2-hydroxyethyl)amino-1-(2-nitrophenyl)benzene;
2-amino-5-di(2-hydroxyethyl)amino-1-(2-trifluoromethylphenyl)benzene;
2-amino-5-di(2-hydroxyethyl)amino-1-(3,4-dichlorophenyl)benzene;
2-amino-5-di(2-hydroxyethyl)amino-1-(3,5-diaminophenyl)benzene;
2-amino-5-di(2-hydroxyethyl)amino-1-(3,5-dichlorophenyl)benzene;
2-amino-5-di(2-hydroxyethyl)amino-1-(3,5-dihydroxyphenyl)benzene;
2-amino-5-di(2-hydroxyethyl)amino-1-(3-(acetylamino)phenyl)benzene;
2-amino-5-di(2-hydroxyethyl)amino-1-(3-aminophenyl)benzene;
2-amino-5-di(2-hydroxyethyl)amino-1-(3-bromophenyl) benzene;
2-amino-5-di(2-hydroxyethyl)amino-1-(3-carboxyphenyl)benzene;
2-amino-5-di(2-hydroxyethyl)amino-1-(3-chloro-4-flurophenyl)benzene;
2-amino-5-di(2-hydroxyethyl)amino-1-(3-chlorophenyl) benzene;
2-amino-5-di(2-hydroxyethyl)amino-1(3-fluorophenyl)benzene;
2-amino-5-di(2-hydroxyethyl)amino-1-(3-formylphenyl) benzene;
2-amino-5-di(2-hydroxyethyl)amino-1-(3-hydroxy-5-aminophenyl)benzene;
2-amino-5-di(2-hydroxyethyl)amino-1-(3-hydroxyphenyl)benzene;
2-amino-5-di(2-hydroxyethyl)amino-1-(3-hydroxyphenyl)benzene;
2-amino-5-di(2-hydroxyethyl)amino-1-(3-methoxyphenyl)benzene;
2-amino-5-di(2-hydroxyethyl)amino-1-(3-methylphenyl)benzene;
2-amino-5-di(2-hydroxyethyl)amino-1-(3-nitrophenyl)benzene;
2-amino-5-di(2-hydroxyethyl)amino-1-(3-trifluoromethylphenyl)benzene;
2-amino-5-di(2-hydroxyethyl)amino-1-(4-(bromomethyl)phenyl)benzene;
2-amino-5-di(2-hydroxyethyl)amino-1-(4-(dimethylamino)phenyl)benzene;
2-amino-5-di(2-hydroxyethyl)amino-1-(4-(hydroxymethyl)phenyl)benzene;
2-amino-5-di(2-hydroxyethyl)amino-1-(4-(methylthio)phenyl)benzene;
2-amino-5-di(2-hydroxyethyl)amino-1-(4-(trifluoromethyl)phenyl)benzene;
2-amino-5-di(2-hydroxyethyl)amino-1-(4-(trimethylsilyl)phenyl)benzene;
2-amino-5-di(2-hydroxyethyl)amino-1-(4-acetylphenyl)benzene;
2-amino-5-di(2-hydroxyethyl)amino-1-(4-aminophenyl)benzene;
2-amino-5-di(2-hydroxyethyl)amino-1-(4-bromophenyl)benzene;
2-amino-5-di(2-hydroxyethyl)amino-1-(4-carboxyphenyl)benzene;
2-amino-5-di(2-hydroxyethyl)amino-1-(4-chloro-3-methoxyphenyl)benzene;
2-amino-5-di(2-hydroxyethyl)amino-1-(4-chlorophenyl)benzene;
2-amino-5-di(2-hydroxyethyl)amino-1-(4-ethenylphenyl)benzene;
2-amino-5-di(2-hydroxyethyl)amino-1-(4-flurophenyl)benzene;
2-amino-5-di(2-hydroxyethyl)amino-1-(4-formylphenyl)benzene;
2-amino-5-di(2-hydroxyethyl)amino-1-(4-hydroxyphenyl)benzene;
2-amino-5-di(2-hydroxyethyl)amino-1-(4-iodophenyl)benzene;
2-amino-5-di(2-hydroxyethyl)amino-1-(4-methoxyphenyl)benzene;
2-amino-5-di(2-hydroxyethyl)amino-1-(4-methyl-3-nitrophenyl)benzene;
2-amino-5-di(2-hydroxyethyl)amino-1-(4-methylphenyl)benzene;
2-amino-5-di(2-hydroxethyl)amino-1-(5-bromo-2-methoxyphenyl)benzene;
2-amino-5-di(2-hydroxyethyl)amino-1-(5-chloro-2-methoxyphenyl)benzene;
2-amino-5-di(2-hydroxethyl)amino-1-(5-formyl-2-methoxyphenyl)benzene;
2-amino-5-di(2-hydroxyethyl)amino-1-phenylbenzene;
2-amino-5-di(2-methoxyethyl)amino-1-(2,3,4-trimethoxyphenyl)benzene;
2-amino-5-di(2-methoxyethyl)amino-1-(2,3-difluoro-4-heptylphenyl)benzene;
2-amino-5-di(2-methoxyethyl)amino-1-(2,3-difluorophenyl)benzene;
2-amino-5-di(2-methoxyethyl)amino-1-(2,4,6-trimethylphenyl)benzene;
2-amino-5-di(2-methoxyethyl)amino-1-(2,4-diaminophenyl)benzene;
2-amino-5-di(2-methoxyethyl)amino-1-(2,4-dichlorophenyl)benzene;
2-amino-5-di(2-methoxyethyl)amino-1-(2,4-dihyroxyphenyl)benzene;
2-amino-5-di(2-methoxyethyl)amino-1-(2,5-dimethoxyphenyl)benzene;
2-amino-5-di(2-methoxyethyl)amino-1-(2,6-difluorophenyl)benzene;
2-amino-5-di(2-methoxyethyl)amino-1-(2,6-dimethoxyphenyl)benzene;
2-amino-5-di(2-methoxyethyl)amino-1-(2-((bis(1-methylethyl)amino)carbonyl)phenyl)benzene;
2-amino-5-di(2-methoxyethyl)amino-1-(2-(bromomethyl)phenyl)benzene;
2-amino-5-di(2-methoxyethyl)amino-1-(2-(diethylamino)carbonyl)phenyl)benzene;
2-amino-5-di(2-methoxyethyl)amino-1-(2-carboxyphenyl)benzene;
2-amino-5-di(2-methoxyethyl)amino-1-(2-chlorophenyl)benzene;
2-amino-5-di(2-methoxyethyl)amino-1-(2-fluorophenyl)benzene;
2-amino-5-di(2-methoxyethyl)amino-1-(2-formyl-4-methoxyphenyl)benzene;
2-amino-5-di(2-methoxyethyl)amino-1-(2-formyl-4-methylphenyl)benzene;
2-amino-5-di(2-methoxyethyl)amino-1-(2-formyl-5-methoxyphenyl)benzene;
2-amino-5-di(2-methoxyethyl)amino-1-(2-formylphenyl)benzene;
2-amino-5-di(2-methoxyethyl)amino-1-(2-hydroxy-4-aminophenyl)benzene;
2-amino-5-di(2-methoxyethyl)amino-1-(2-hydroxyphenyl)benzene;
2-amino-5-di(2-methoxyethyl)amino-1-(2-methoxyphenyl)benzene;
2-amino-5-di(2-methoxyethyl)amino-1-(2-methylphenyl)benzene;
2-amino-5-di(2-methoxyethyl)amino-1-(2-nitrophenyl)benzene;
2-amino-5-di(2-methoxyethyl)amino-1-(2-trifluoromethylphenyl)benzene;
2-amino-5-di(2-methoxyethyl)amino-1-(3,4-dichlorophenyl)benzene;
2-amino-5-di(2-methoxyethyl)amino-1-(3,5-diaminophenyl)benzene;
2-amino-5-di(2-methoxyethyl)amino-1-(3,5-dichlorophenyl)benzene;
2-amino-5-di(2-methoxyethyl)amino-1-(3,5,dihydroxyphenyl)benzene;
2-amino-5-di(2-methoxyethyl)amino-1-(3-(acetylamino)phenyl)benzene;
2-amino-5-di(2-methoxyethyl)amino-1-(3-aminophenyl)benzene;
2-amino-5-di(2-methoxyethyl)amino-1-(3-bromophenyl)benzene;
2-amino-5-di(2-methoxyethyl)amino-1-(3-carboxyphenylphenyl)benzene;
2-amino-5-di(2-methoxyethyl)amino-1-(3-chloro-4-fluorophenyl)benzene;
2-amino-5-di(2-methoxyethyl)amino-1-(3-chlorophenyl)benzene;
2-amino-5-di(2-methoxyethyl)amino-1-(3-fluorophenyl)benzene;
2-amino-5-di(2-methoxyethyl)amino-1-(3-formylphenyl)benzene;
2-amino-5-di(2-methoxyethyl)amino-1-(3-hydroxyphenyl)benzene;
2-amino-5-di(2-methoxyethyl)amino-1-(3-methoxyphenyl)benzene;
2-amino-5-di(2-methoxyethyl)amino-1-(3-methylphenyl)benzene;
2-amino-5-di(2-methoxyethyl)amino-1-(3-nitrophenyl)benzene;
2-amino-5-di(2-methoxyethyl)amino-1-(3-trifluoromethylphenyl)benzene;
2-amino-5-di(2-methoxyethyl)amino-1-(4-(bromomethyl)phenyl)benzene;
2-amino-5-di(2-methoxyethyl)amino-1-(4-(dimethylamino)phenyl)benzene;
2-amino-5-di(2-methoxyethyl)amino-1-(4-(hydroxymethyl)phenyl)benzene;
2-amino-5-di(2-methoxyethyl)amino-1-(4-(methylthio)phenyl)benzene;
2-amino-5-di(2-methoxyethyl)amino-1-(4-(trifluoromethyl)phenyl)benzene;
2-amino-5-di(2-methoxyethyl)amino-1-(4-(trimethylsilyl)phenyl)benzene;
2-amino-5-di(2-methoxyethyl)amino-1-(4-acetylphenyl)benzene;
2-amino-5-di(2-methoxyethyl)amino-1-(4-aminophenyl)benzene;
2-amino-5-di(2-methoxyethyl)amino-1-(4-bromophenyl)benzene;
2-amino-5-di(2-methoxyethyl)amino-1-(4-carboxyphenyl)benzene;
2-amino-5-di(2-methoxyethyl)amino-1-(4-chloro-3-methoxyphenyl)benzene;
2-amino-5-di(2-methoxyethyl)amino-1-(4-chlorophenyl)benzene;
2-amino-5-di(2-methoxyethyl)amino-1-(4-ethenylphenyl)benzene;
2-amino-5-di(2-methoxyethyl)amino-1-(4-ethoxyphenyl)benzene;
2-amino-5-di(2-methoxyethyl)amino-1-(4-fluorophenyl)benzene;
2-amino-5-di(2-methoxyethyl)amino-1-(4-formylphenyl)benzene;
2-amino-5-di(2-methoxyethyl)amino-1-(4-hydroxyphenyl)benzene;
2-amino-5-di(2-methoxyethyl)amino-1-(4-iodophenyl)benzene;
2-amino-5-di(2-methoxyethyl)amino-1-(4-methoxyphenyl)benzene;
2-amino-5-di(2-methoxyethyl)amino-1-(4-methyl-3-nitrophenyl)benzene;
2-amino-5-di(2-methoxyethyl)amino-1-(4-methylphenyl)benzene;
2-amino-5-di(2-methoxyethyl)amino-1-(5-bromo-2-methoxyphenyl)benzene;
2-amino-5-di(2-methoxyethyl)amino-1-(5-chloro-2-methoxyphenyl)benzene;
2-amino-5-di(2-methoxyethyl)amino-1-(5-formyl-2-methoxyphenyl)benzene;
2-amino-5-dimethylamino-1-phenylbenzene;
2-amino-5-methylamino-1-(2,3,4-trimethoxyphenyl)benzene;
2-amino-5-methylamino-1-(2,4-diaminophenyl)benzene;
2-amino-5-methylamino-1-(2,4-dihydroxyphenyl)benzene;
2-amino-5-methylamino-1-(2,5-diaminophenyl)benzene;
2-amino-5-methylamino-1-(2,5-dimethoxyphenyl)benzene;
2-amino-5-methylamino-1-(2,6-dimethoxyphenyl)benzene;
2-amino-5-methylamino-1-(2-amino-5-hydroxyphenyl)benzene;
2-amino-5-methylamino-1-(2-aminophenyl)benzene;
2-amino-5-methylamino-1-(2-hydroxy-4-aminophenyl)benzene;
2-amino-5-methylamino-1-(2-hydroxy-5-aminophenyl)benzene;
2-amino-5-methylamino-1-(2-hydroxyphenyl)benzene;
2-amino-5-methylamino-1-(2-methoxyphenyl)benzene;
2-amino-5-methylamino-1-(2-methylphenyl)benzene;
2-amino-5-methylamino-1-(3,5-diaminophenyl)benzene;
2-amino-5-methylamino-1-(3,5-dihydroxyphenyl)benzene;
2-amino-5-methylamino-1-(3-aminophenyl)benzene;
2-amino-5-methylamino-1-(3-hydroxy-5-aminophenyl)benzene;
2-amino-5-methylamino-1-(3-hydroxyphenyl)benzene;
2-amino-5-methylamino-1-(3-methoxyphenyl)benzene;
2-amino-5-methylamino-1-(4-(dimethylamino)phenyl)benzene;
2-amino-5-methylamino-1-(4-(trifluoromethyl)phenyl)benzene;
2-amino-5-methylamino-1-(4-aminophenyl)benzene;
2-amino-5-methylamino-1-(4-carboxyphenyl)benzene;
2-amino-5-methylamino-1-(4-chlorophenyl)benzene;
2-amino-5-methylamino-1-(4-hydroxyphenyl)benzene;
2-amino-5-methylamino-1-(4-methoxyphenyl)benzene;
2-amino-5-methylamino-1-(4-methylphenyl)benzene;
2-amino-5-methylamino-1-phenylbenzene;
5-amino-2-(2,3-dihydroxypropyl)amino-1-(4-bromophenyl)benzene;
5-amino-2-(2,3-dihydroxypropyl)amino-1-(4-ethenylphenyl)benzene;
5-amino-2-(2,3-dihydroxypropyl)amino-1-(2,3,4-trimethoxyphenyl)benzene;
5-amino-2-(2,3-dihydroxypropyl)amino-1-(2,4-di(2-hydroxyethyl)aminophenyl)benzene;
5-amino-2-(2,3-dihydroxypropyl)amino-1-(2,4-diaminophenyl)benzene;
5-amino-2-(2,3-dihydroxypropyl)amino-1-(2,4-dihydroxyphenyl)benzene;
5-amino-2-(2,3-dihydroxypropyl)amino-1-(2,4-dimethylaminophenyl)benzene;
5-amino-2-(2,3-dihydroxypropyl)amino-1-(2,4-methoxyphenyl)benzene;
5-amino-2-(2,3-dihydroxypropyl)amino-1-(2,5-di(2-hydroxyethyl)aminophenyl)benzene;
5-amino-2-(2,3-dihydroxypropyl)amino-1-(2,5-diaminophenyl)benzene;
5-amino-2-(2,3-dihydroxypropyl)amino-1-(2,5-dihydroxyphenyl)benzene;
5-amino-2-(2,3-dihydroxypropyl)amino-1-(2,5-dimethoxyphenyl)benzene;
5-amino-2-(2,3-dihydroxypropyl)amino-1-(2,5-dimethylaminophenyl)benzene;
5-amino-2-(2,3-dihydroxypropyl)amino-1-(2,6-di(2-hydroxyethyl)aminophenyl)benzene;
5-amino-2-(2,3-dihydroxypropyl)amino-1-(2,6-diaminophenyl)benzene;
5-amino-2-(2,3-dihydroxypropyl)amino-1-(2,6-dihydroxyphenyl)benzene;
5-amino-2-(2,3-dihydroxypropyl)amino-1-(2,6-dimethoxyphenyl)benzene;
5-amino-2-(2,3-dihydroxypropyl)amino-1-(2,6-dimethylaminophenyl)benzene;
5-amino-2-(2,3-dihydroxypropyl)amino-1-(2-(bromomethyl)phenyl)benzene;
5-amino-2-(2,3-dihydroxypropyl)amino-1-(2-amino-5-hydroxyphenyl)benzene;
5-amino-2-(2,3-dihydroxypropyl)amino-1-(2-aminophenyl)benzene;
5-amino-2-(2,3-dihydroxypropyl)amino-1-(2-carboxyphenyl)benzene;
5-amino-2-(2,3-dihydroxypropyl)amino-1-(2-chlorophenyl)benzene;
5-amino-2-(2,3-dihydroxypropyl)amino-1-(2-di(2-hydroxyethyl)aminophenyl)benzene;
5-amino-2-(2,3-dihydroxypropyl)amino-1-(2-dimethylaminophenyl)benzene;
5-amino-2-(2,3-dihydroxypropyl)amino-1-(2-fluorophenyl)benzene;
5-amino-2-(2,3-dihydroxypropyl)amino-1-(2-formylphenyl)benzene;
5-amino-2-(2,3-dihydroxypropyl)amino-1-(2-hydroxy-4-aminophenyl)benzene;
5-amino-2-(2,3-dihydroxypropyl)amino-1-(2-hydroxy-5-aminophenyl)benzene;
5-amino-2-(2,3-dihyroxypropyl)amino-1-(2-hydroxyphenyl)benzene;
5-amino-2-(2,3-dihydroxypropyl)amino-1-(2-methoxyphenyl)benzene;
5-amino-2-(2,3-dihydroxypropyl)amino-1-(2-methylphenyl)benzene;
5-amino-2-(2,3-dihydroxypropyl)amino-1-(2-nitrophenyl)benzene;
5-amino-2-(2,3-dihydroxypropyl)amino-1-(2-trifluoromethylphenyl)benzene;
5-amino-2-(2,3-dihyroxypropyl)amino-1-(3,5-di(2-hydroxyethyl)aminophenyl)benzene;
5-amino-2-(2,3-dihydroxypropyl)amino-1-(3,5-diaminophenyl)benzene;
5-amino-2-(2,3-dihydroxypropyl)amino-1-(3,5-dihydroxyphenyl)benzene;
5-amino-2-(2,3-dihydroxypropyl)amino-1-(3,5-dimethoxyphenyl)benzene;
5-amino-2-(2,3-dihydroxypropyl)amino-1-(3,5-dimethylaminophenyl)benzene;
5-amino-2-(2,3-dihydroxypropyl)amino-1-(3-aminophenyl)benzene;
5-amino-2-(2,3-dihydroxypropyl)amino-1-(3-bromophenyl)benzene;
5-amino-2-(2,3-dihydroxypropyl)amino-1-(3-carboxyphenyl)benzene;
5-amino-2-(2,3-dihydroxypropyl)amino-1-(3-chlorophenyl)benzene;
5-amino-2-(2,3-dihydroxypropyl)amino-1-(3-di(2-hydroxyethyl)aminophenyl)benzene;
5-amino-2-(2,3-dihydroxypropyl)amino-1-(3-dimethylaminophenyl)benzene;
5-amino-2-(2,3-dihydroxypropyl)amino-1-(3-fluorophenyl)benzene;
5-amino-2-(2,3-dihydroxyproply)amino-1-(3-formylphenyl)benzene;
5-amino-2-(2,3-dihydroxypropyl)amino-1-(3-hydroxy-5-aminophenyl)benzene;
5-amino-2-(2,3-dihydroxypropyl)amino-1-(3-hydroxyphenyl)benzene;
5-amino-2-(2,3-dihydroxypropyl)amino-1-(3-methoxyphenyl)benzene;
5-amino-2-(2,3-dihydroxypropyl)amino-1-(3-nitrophenyl)benzene;
5-amino-2-(2,3-dihydroxypropyl)amino-1-(3-trifluoromethylphenyl)benzene;
5-amino-2-(2,3-dihydroxypropyl)amino-1-(4-(dimethylamino)phenyl)benzene;
5-amino-2-(2,3-dihydroxypropyl)amino-1-(4-(hydroxymethyl)phenyl)benzene;
5-amino-2-(2,3-dihydroxypropyl)amino-1-(4-(methylthio)phenyl)benzene;
5-amino-2-(2,3-dihydroxypropyl)amino-1-(4-(trifluoromethyl)phenyl)benzene;
5-amino-2-(2,3-dihydroxypropyl)amino-1-(4-(trimethylsilyl)phenyl)benzene;
5-amino-2-(2,3-dihydroxypropyl)amino-1-(4-acetylphenyl)benzene;
5-amino-2-(2,3-dihydroxypropyl)amino-1-(4-aminophenyl)benzene;
5-amino-2-(2,3-dihydroxpypropyl)amino-1-(4-carboxyphenyl)benzene;
5-amino-2-(2,3-dihydroxypropyl)amino-1-(4-chlorophenyl)benzene;
5-amino-2-(2,3-dihydroxypropyl)amino-1-(4-di(2-hydroxyethyl)aminophenyl)benzene
5-amino-2-(2,3-dihydroxypropyl)amino-1-(4-dimethylaminophenyl)benzene;
5-amino-2-(2,3-dihydroxypropyl)amino-1-(4-ethoxyphenyl)benzene;
5-amino-2-(2,3-dihydroxypropyl)amino-1-(4-fluorophenyl)benzene;
5-amino-2-(2,3-dihydroxypropyl)amino-1-(4-formylphenyl)benzene;
5-amino-2-(2,3-dihydroxypropyl)amino-1-(4-hydroxyphenyl)benzene;
5-amino-2-(2,3-dihydroxypropyl)amino-1-(4-methoxyphenyl)benzene;
5-amino-2-(2,3-dihydroxypropyl)amino-1-(4-methylphenyl)benzene;
5-amino-2-(2,3-dihydroxypropyl)amino-1-phenylbenzene;
5-amino-2-(2-hydroxyethyl)amino-1-(4-bromophenyl)benzene;
5-amino-2-(2-hydroxyethyl)amino-1-(4-ethenylphenyl)benzene;
5-amino-2-(2-hydroxyethyl)amino-1-(2,3,4-trimethoxyphenyl)benzene;
5-amino-2-(2-hydroxyethyl)amino-1-(2,4-di(2-hydroxyethyl)aminophenyl)benzene;
5-amino-2-(2-hydroxyethyl)amino-1-(2,4-diaminophenyl)benzene;
5-amino-2-(2-hydroxyethyl)amino-1-(2,4-dihydroxyphenyl)benzene;
5-amino-2-(2-hydroxyethyl)amino-1-(2,4-dimethylaminophenyl)benzene;
5-amino-2-(2-hydroxyethyl)amino-1-(2,4-methoxyphenyl)benzene;
5-amino-2-(2-hydroxyethyl)amino-1-(2,5-di(2-hydroxyethyl)aminophenyl)benzene;
5-amino-2-(2-hydroxyethyl)amino-1-(2,5-diaminophenyl)benzene;
5-amino-2-(2-hydroxyethyl)amino-1-(2,5-dihydroxyphenyl)benzene;
5-amino-2-(2-hydroxyethyl)amino-1-(2,5-dimethoxyphenyl)benzene;
5-amino-2-(2-hydroxyethyl)amino-1-(2,5-dimethylaminophenyl) benzene;
5-amino-2-(2-hydroxyethyl)amino-1-(2,6-di(2-hydroxyethyl)aminophenyl)benzene;
5-amino-2-(2-hydroxyethyl)amino-1-(2,6-diaminophenyl)benzene;
5-amino-2-(2-hydroxyethyl)amino-1-(2,6-dihydroxyphenyl)benzene;
5-amino-2-(2-hydroxyethyl)amino-1-(2,6-dimethoxyphenyl)benzene;
5-amino-2-(2-hydroxyethyl)amino-1-(2,6-dimethylaminophenyl)benzene;
5-amino-2-(2-hydroxyethyl)amino-1-(2-(bromomethyl)-phenyl)benzene;
5-amino-2-(2-hydroxyethyl)amino-1-(2-amino-5-hydroxyphenyl)benzene;
5-amino-2-(2-hydroxyethyl)amino-1-(2-aminophenyl)benzene;
5-amino-2-(2-hydroxyethyl)amino-1-(2-carboxyphenyl)benzene;
5-amino-2-(2-hydroxyethyl)amino-1-(2-chlorophenyl)benzene;
5-amino-2-(2-hydroxyethyl)amino-1-(2-di(2-hydroxyethyl)aminophenyl)benzene;
5-amino-2-(2-hydroxyethyl)amino-1-(2-dimethylaminophenyl)benzene;
5-amino-2-(2-hydroxyethyl)amino-1-(2-fluorophenyl)benzene;
5-amino-2-(2-hydroxyethyl)amino-1-(2-formylphenyl)benzene;
5-amino-2-(2-hydroxyethyl)amino-1-(2-hydroxy-4-aminophenyl)benzene;
5-amino-2-(2-hydroxyethyl)amino-1-(2-hydroxy-5-aminophenyl)benzene;
5-amino-2-(2-hydroxyethyl)amino-1-(2-hydroxyphenyl)benzene;
5-amino-2-(2-hydroxyethyl)amino-1-(2-methoxyphenyl)benzene;
5-amino-2-(2-hydroxyethyl)amino-1-(2-methylphenyl) benzene;
5-amino-2-(2-hydroxyethyl)amino-1-(2-nitrophenyl)benzene;
5-amino-2-(2-hydroxyethyl)amino-1-(2-trifluoromethylphenyl)benzene;
5-amino-2-(2-hydroxyethyl)amino-1-(3,5-di(2-hydroxyethyl)aminophenyl)benzene;
5-amino-2-(2-hydroxyethyl)amino-1-(3,5-diaminophenyl)benzene;
5-amino-2-(2-hydroxyethyl)amino-1-(3,5-dihydroxyphenyl)benzene;
5-amino-2-(2-hydroxyethyl)amino-1-(3,5-dimethoxyphenyl)benzene;
5-amino-2-(2-hydroxyethyl)amino-1-(3,5-dimethylaminophenyl)benzene;
5-amino-2-(2-hydroxyethyl)amino-1-(3-aminophenyl)benzene;
5-amino-2-(2-hydroxyethyl)amino-1-(3-bromophenyl)benzene;
5-amino-2-(2-hydroxyethyl)amino-1-(3-carboxyphenyl)benzene;
5-amino-2-(2-hydroxyethyl)amino-1-(3-chlorophenyl)benzene;
5-amino-2-(2-hydroxyethyl)amino-1-(3-di(2-hydroxyethyl)aminophenyl)benzene;
5-amino-2-(2-hydroxyethyl)amino-1-(3-dimethylaminophenyl)benzene;
5-amino-2-(2-hydroxyethyl)amino-1-(3-fluorophenyl)benzene;
5-amino-2-(2-hydroxyethyl)amino-1-(3-formylphenyl)benzene;
5-amino-2-(2-hydroxyethyl)amino-1-(3-hydroxy-5-aminophenyl)benzene;
5-amino-2-(2-hydroxyethyl)amino-1-(3-hydroxyphenyl)benzene;
5-amino-2-(2-hydroxyethyl)amino-1-(3-methoxyphenyl)benzene;
5-amino-2-(2-hydroxyethyl)amino-1-(3-nitrophenyl)benzene;
5-amino-2-(2-hydroxyethyl)amino-1-(3-trifluoromethylphenyl)benzene;
5-amino-2-(2-hydroxyethyl)amino-1-(4-(dimethylamino)phenyl)benzene;
5-amino-2-(2-hydroxyethyl)amino-1-(4-(hydroxymethyl)phenyl)benzene;
5-amino-2-(2-hydroxyethyl)amino-1-(4-(methylthio)phenyl)benzene;
5-amino-2-(2-hydroxyethyl)amino-1-(4-(trifluoromethyl)phenyl)benzene;
5-amino-2-(2-hydroxyethyl)amino-1-(4-(trimethylsilyl)phenyl)benzene;
5-amino-2-(2-hydroxyethyl)amino-1-(4-acetylphenyl)benzene;
5-amino-2-(2-hydroxyethyl)amino-1-(4-aminophenyl)benzene;
5-amino-2-(2-hydroxyethyl)amino-1-(4-carboxyphenyl)benzene;
5-amino-2-(2-hydroxyethyl)amino-1-(4-chlorophenyl)benzene;
5-amino-2-(2-hydroxyethyl)amino-1-(4-di(2-hydroxyethyl)aminophenyl)benzene;
5-amino-2-(2-hydroxyethyl)amino-1-(4-dimethylaminophenyl)benzene;
5-amino-2-(2-hydroxyethyl)amino-1-(4-ethoxyphenyl)benzene;
5-amino-2-(2-hydroxyethyl)amino-1-(4-fluorophenyl)benzene;
5-amino-2-(2-hydroxyethyl)amino-1-(4-formylphenyl)benzene;
5-amino-2-(2-hydroxyethyl)amino-1-(4-hydroxyphenyl)benzene;
5-amino-2-(2-hydroxyethyl)amino-1-(4-methoxyphenyl)benzene;
5-amino-2-(2-hydroxyethyl)amino-1-(4-methylphenyl)benzene;
5-amino-2-(2-hydroxyethyl)amino-1-phenylbenzene;
5-amino-2-(2-methoxyethyl)amino-1-(2,3,4-trimethoxyphenyl)benzene;
5-amino-2-(2-methoxyethyl)amino-1-(2,4-diaminophenyl)benzene;
5-amino-2-(2-methoxyethyl)amino-1-(2,4-dihydroxyphenyl)benzene;
5-amino-2-(2-methoxyethyl)amino-1-(2,5-diaminophenyl)benzene;
5-amino-2-(2-methoxyethyl)amino-1-(2,5-dimethoxyphenyl)benzene;
5-amino-2-(2-methoxyethyl)amino-1-(2,6-dimethoxyphenyl)benzene;
5-amino-2-(2-methoxyethyl)amino-1-(2-amino-5-hydroxyphenyl)benzene;
5-amino-2-(2-methoxyethyl)amino-1-(2-aminophenyl)benzene;
5-amino-2-(2-methoxyethyl)amino-1-(2-hydroxy-4-aminophenyl)benzene;
5-amino-2-(2-methoxyethyl)amino-1-(2-hydroxy-5-aminophenyl)benzene;
5-amino-2-(2-methoxyethyl)amino-1-(2-hydroxyphenyl)benzene;
5-amino-2-(2-methoxyethyl)amino-1-(2-methoxyphenyl)benzene;
5-amino-2-(2-methoxyethyl)amino-1-(2-methylphenyl)benzene;
5-amino-2-(2-methoxyethyl)amino-1-(3,5-diaminophenyl)benzene;
5-amino-2-(2-methoxyethyl)amino-1-(3,5-dihydroxyphenyl)benzene;
5-amino-2-(2-methoxyethyl)amino-1-(3-aminophenyl)benzene;
5-amino-2-(2-methoxyethyl)amino-1-(3-hydroxy-5-aminophenyl)benzene;
5-amino-2-(2-methoxyethyl)amino-1-(3-hydroxyphenyl)benzene;
5-amino-2-(2-methoxyethyl)amino-1-(3-methoxyphenyl)benzene;
5-amino-2-(2-methoxyethyl)amino-1-(4-(dimethylamino)phenyl)benzene;
5-amino-2-(2-methoxyethyl)amino-1-(4-(trifluoromethyl)phenyl)benzene;
5-amino-2-(2-methoxyethyl)amino-1-(4-aminophenyl)benzene;
5-amino-2-(2-methoxyethyl)amino-1-(4-carboxyphenyl)benzene;
5-amino-2-(2-methoxyethyl)amino-1-(4-chlorophenyl)benzene;
5-amino-2-(2-methoxyethyl)amino-1-(4-hydroxyphenyl)benzene;
5-amino-2-(2-methoxyethyl)amino-1-(4-methoxyphenyl)benzene;
5-amino-2-(2-methoxyethyl)amino-1-(4-methylphenyl)benzene;
5-amino-2-(2-methoxyethyl)amino-1-phenylbenzene;
5-amino-2-di(2-hydroxyethyl)amino-1-(2,3,4-trimethoxyphenyl)benzene;
5-amino-2-di(2-hydroxyethyl)amino-1-(2,3-difluoro-4-heptylphenyl)benzene;
5-amino-2-di(2-hydroxyethyl)amino-1-(2,3-difluorophenyl)benzene;
5-amino-2-di(2-hydroxyethyl)amino-1-(2,4,6-trimethylphenyl)benzene;
5-amino-2-di(2-hydroxyethyl)amino-1-(2,4-diaminophenyl)benzene;
5-amino-2-di(2-hydroxyethyl)amino-1-(2,4-dichlorophenyl)benzene;
5-amino-2-di(2-hydroxyethyl)amino-1-(2,4-dihydroxyphenyl)benzene;
5-amino-2-di(2-hydroxyethyl)amino-1-(2,5-dimethoxyphenyl)benzene;
5-amino-2-di(2-hydroxyethyl)amino-1-(2,6-difluorophenyl)benzene;
5-amino-2-di(2-hydroxyethyl)amino-1-(2,6-dimethoxyphenyl)benzene;
5-amino-2-di(2-hydroxyethyl)amino-1-(2-((bis(1-methylethyl)amino)carbonyl)phenyl)benzene;
5-amino-2-di(2-hydroxyethyl)amino-1-(2-((bis(1-methylethyl)amino)carbonyl)-3-methoxyphenyl)benzene;
5-amino-2-di(2-hydroxyethyl)amino-1-(2-(bromomethyl)phenyl)benzene;
5-amino-2-di(2-hydroxyethyl)amino-1-(2-(diethylamino)carbonyl)phenyl)benzene;
5-amino-2-di(2-hydroxyethyl)amino-1-(2-carboxyphenyl)benzene;
5-amino-2-di(2-hydroxyethyl)amino-1-(2-chlorophenyl)benzene;
5-amino-2-di(2-hydroxyethyl)amino-1-(2-fluorophenyl)benzene;
5-amino-2-di(2-hydroxyethyl)amino-1-(2-formyl-4-methoxyphenyl)benzene;
5-amino-2-di(2-hydroxyethyl)amino-1-(2-formyl-4-methylphenyl)benzene;
5-amino-2-di(2-hydroxyethyl)amino-1-(2-formyl-5-methoxyphenyl)benzene;
5-amino-2-di(2-hydroxyethyl)amino-1-(2-formylphenyl)benzene;
5-amino-2-di(2-hydroxyethyl)amino-1-(2-hydroxy-4-aminophenyl)benzene;
5-amino-2-di(2-hydroxyethyl)amino-1-(2-hydroxyphenyl)benzene;
5-amino-2-di(2-hydroxyethyl)amino-1-(2-methoxyphenyl)benzene;
5-amino-2-di(2-hydroxyethyl)amino-1-(2-methylphenyl)benzene;
5-amino-2-di(2-hydroxyethyl)amino-1-(2-nitrophenyl)benzene;
5-amino-2-di(2-hydroxyethyl)amino-1-(2-trifluoromethylphenyl)benzene;
5-amino-2-di(2-hydroxyethyl)amino-1-(3,4-dichlorophenyl)benzene;
5-amino-2-di(2-hydroxyethyl)amino-1-(3,5-diaminophenyl)benzene;
5-amino-2-di(2-hydroxyethyl)amino-1-(3,5-dichlorophenyl)benzene;
5-amino-2-di(2-hydroxyethyl)amino-1-(3,5-dihydroxyphenyl)benzene;
5-amino-2-di(2-hydroxyethyl)amino-1-(3-(acetylamino)phenyl)benzene;
5-amino-2-di(2-hydroxyethyl)amino-1-(3-aminophenyl)benzene;
5-amino-2-di(2-hydroxyethyl)amino-1-(3-bromophenyl)benzene;
5-amino-2-di(2-hydroxyethyl)amino-1-(3-carboxyphenyl)benzene;
5-amino-2-di(2-hydroxyethyl)amino-1-(3-chloro-4-fluorophenyl)benzene;
5-amino-2-di(2-hydroxyethyl)amino-1-(3-chlorophenyl)benzene;
5-amino-2-di(2-hydroxyethyl)amino-1-(3-fluorophenyl)benzene;
5-amino-2-di(2-hydroxyethyl)amino-1-(3-formylphenyl)benzene;
5-amino-2-di(2-hydroxyethyl)amino-1-(3-hydroxyphenyl)benzene;
5-amino-2-di(2-hydroxyethyl)amino-1-(3-methoxyphenyl)benzene;
5-amino-2-di(2-hydroxyethyl)amino-1-(3-methylphenyl)benzene;
5-amino-2-di(2-hydroxyethyl)amino-1-(3-nitrophenyl)benzene;
5-amino-2-di(2-hydroxyethyl)amino-1-(3-trifluoromethylphenyl)benzene;
5-amino-2-di(2-hydroxyethyl)amino-1-(4-(bromomethyl)phenyl)benzene;
5-amino-2-di(2-hydroxyethyl)amino-1-(4-(dimethylamino)phenyl)benzene;
5-amino-2-di(2-hydroxyethyl)amino-1-(4-(hydroxymethyl)phenyl)benzene;
5-amino-2-di(2-hydroxyethyl)amino-1-(4-(methylthio)phenyl)benzene;
5-amino-2-di(2-hydroxyethyl)amino-1-(4-(trifluoro methyl)phenyl)benzene;
5-amino-2-di(2-hydroxyethyl)amino-1-(4-(trimethylsilyl)phenyl)benzene;
5-amino-2-di(2-hydroxyethyl)amino-1-(4-acetylphenyl)benzene;
5-amino-2-di(2-hydroxyethyl)amino-1-(4-aminophenyl)benzene;
5-amino-2-di(2-hydroxyethyl)amino-1-(4-bromophenyl)benzene;
5-amino-2-di(2-hydroxyethyl)amino-1-(4-carboxyphenyl)benzene;
5-amino-2-di(2-hydroxyethyl)amino-1-(4-chloro-3-methoxyphenyl)benzene;
5-amino-2-di(2-hydroxyethyl)amino-1-(4-chlorophenyl)benzene;
5-amino-2-di(2-hydroxyethyl)amino-1-(4-ethenylphenyl)benzene;
5-amino-2-di(2-hydroxyethyl)amino-1-(4-ethoxyphenyl)benzene;
5-amino-2-di(2-hydroxyethyl)amino-1-(4-fluorophenyl)benzene;
5-amino-2-di(2-hydroxyethyl)amino-1-(4-formylphenyl)benzene;
5-amino-2-di(2-hydroxyethyl)amino-1-(4-hydroxyphenyl)benzene;
5-amino-2-di(2-hydroxyethyl)amino-1-(4-iodophenyl)benzene;
5-amino-2-di(2-hydroxyethyl)amino-1-(4-methoxyphenyl)benzene;
5-amino-2-di(2-hydroxyethyl)amino-1-(4-methyl-3-nitrophenyl)benzene;
5-amino-2-di(2-hydroxyethyl)amino-1-(4-methylphenyl)benzene;
5-amino-2-di(2-hydroxyethyl)amino-1-(5-bromo-2-methoxyphenyl)benzene;
5-amino-2-di(2-hydroxyethyl)amino-1-(5-chloro-2-methoxyphenyl)benzene;
5-amino-2-di(2-hydroxyethyl)amino-1-(5-formyl-2-methoxyphenyl)benzene;
5-amino-2-di(2-methoxyethyl)amino-1-(2,3,4-trimethoxyphenyl)benzene;
5-amino-2-di(2-methoxyethyl)amino-1-(2,3-difluoro-4-heptylphenyl)benzene;
5-amino-2-di(2-methoxyethyl)amino-1-(2,3-difluorophenyl)benzene;
5-amino-2-di(2-methoxyethyl)amino-1-(2,4,6-trimethylphenyl)benzene;
5-amino-2-di(2-methoxyethyl)amino-1-(2,4-diaminophenyl)benzene;
5-amino-2-di(2-methoxyethyl)amino-1-(2,4-dichlorophenyl)benzene;
5-amino-2-di(2-methoxyethyl)amino-1-(2,4-dihydroxyphenyl)benzene;
5-amino-2-di(2-methoxyethyl)amino-1-(2,5-dimethoxyphenyl)benzene;
5-amino-2-di(2-methoxyethyl)amino-1-(2,6-difluorophenyl)benzene;
5-amino-2-di(2-methoxyethyl)amino-1-(2,6-dimethoxyphenyl)benzene;
5-amino-2-di(2-methoxyethyl)amino-1-(2-((bis(1-methylethyl)amino)carbonyl)phenyl)benzene;
5-amino-2-di(2-methoxyethyl)amino-1-(2-((bis(1-methylethyl)amino)carbonyl)-3-methoxyphenyl)benzene;
5-amino-2-di(2-methoxyethyl)amino-1-(2-(bromomethyl)phenyl)benzene;
5-amino-2-di(2-methoxyethyl)amino-1-(2-(diethylamino)carbonyl)phenyl)benzene;
5-amino-2-di(2-methoxyethyl)amino-1-(2-carboxyphenyl)benzene;
5-amino-2-di(2-methoxyethyl)amino-1-(2-chlorophenyl)benzene;
5-amino-2-di(2-methoxyethyl)amino-1-(2-fluorophenyl)benzene;
5-amino-2-di(2-methoxyethyl)amino-1-(2-formyl-4-methoxyphenyl)benzene;
5-amino-2-di(2-methoxyethyl)amino-1-(2-formyl-4-methylphenyl)benzene;
5-amino-2-di(2-methoxyethyl)amino-1-(2-formyl-5-methoxyphenyl)benzene;
5-amino-2-di(2-methoxyethyl)amino-1-(2-formylphenyl)benzene;
5-amino-2-di(2-methoxyethyl)amino-1-(2-hydroxy-4-aminophenyl)benzene;
5-amino-2-di(2-methoxyethyl)amino-1-(2-hydroxyphenyl)benzene;
5-amino-2-di(2-methoxyethyl)amino-1-(2-methoxyphenyl)benzene;
5-amino-2-di(2-methoxyethyl)amino-1-(2-methylphenyl)benzene;
5-amino-2-di(2-methoxyethyl)amino-1-(2-nitrophenyl)benzene;
5-amino-2-di(2-methoxyethyl)amino-1-(2-trifluoromethylphenyl)benzene;
5-amino-2-di(2-methoxyethyl)amino-1-(3,4-dichlorophenyl)benzene;
5-amino-2-di(2-methoxyethyl)amino-1-(3,5-diaminophenyl) benzene;
5-amino-2-di(2-methoxyethyl)amino-1-(3,5-dichlorophenyl)benzene;
5-amino-2-di(2-methoxyethyl)amino-1-(3,5-dihydroxyphenyl)benzene;
5-amino-2-di(2-methoxyethyl)amino-1-(3-(acetylamino)phenyl)benzene;
5-amino-2-di(2-methoxyethyl)amino-1-(3-aminophenyl)benzene;
5-amino-2-di(2-methoxyethyl)amino-1-(3-bromophenyl)benzene;
5-amino-2-di(2-methoxyethyl)amino-1-(3-carboxyphenyl)benzene;
5-amino-2-di(2-methoxyethyl)amino-1-(3-chloro-4-fluorophenyl)benzene;
5-amino-2-di(2-methoxyethyl)amino-1-(3-chlorophenyl)benzene;
5-amino-2-di(2-methoxyethyl)amino-1-(3-fluorophenyl)benzene;
5-amino-2-di(2-methoxyethyl)amino-1-(3-formylphenyl)benzene;
5-amino-2-di(2-methoxyethyl)amino-1-(3-hydroxyphenyl)benzene;
5-amino-2-di(2-methoxyethyl)amino-1-(3-methoxyphenyl)benzene;
5-amino-2-di(2-methoxyethyl)amino-1-(3-methylphenyl)benzene;
5-amino-2-di(2-methoxyethyl)amino-1-(3-nitrophenyl)benzene;
5-amino-2-di(2-methoxyethyl)amino-1-(3-trifluoromethylphenyl)benzene;
5-amino-2-di(2-methoxyethyl)amino-1-(4-(bromomethyl)phenyl)benzene;
5-amino-2-di(2-methoxyethyl)amino-1-(4-dimethylamino)phenyl)benzene;
5-amino-2-di(2-methoxyethyl)amino-1-(4-hydroxymethyl)phenyl)benzene;
5-amino-2-di(2-methoxyethyl)amino-1-(4-(methylthio)phenyl)benzene;
5-amino-2-di(2-methoxyethyl)amino-1-(4-(trifluoromethyl)phenyl)benzene;
5-amino-2-di(2-methoxyethyl)amino-1-(4-(trimethylsilyl)phenyl)benzene;
5-amino-2-di(2-methoxyethyl)amino-1-(4-acetylphenyl)benzene;
5-amino-2-di(2-methoxyethyl)amino-1-(4-aminophenyl)benzene;
5-amino-2-di(2-methoxyethyl)amino-1-(4-bromophenyl)benzene;
5-amino-2-di(2-methoxyethyl)amino-1-(4-carboxyphenyl)benzene;
5-amino-2-di(2-methoxyethyl)amino-1-(4-chloro-3-methoxyphenyl)benzene;
5-amino-2-di(2-methoxyethyl)amino-1-(4-chlorophenyl)benzene;
5-amino-2-di(2-methoxyethyl)amino-1-(4-ethenylphenyl)benzene;
5-amino-2-di(2-methoxyethyl)amino-1-(4-ethoxyphenyl)benzene;
5-amino-2-di(2-methoxyethyl)amino-1-(4-fluorophenyl)benzene;
5-amino-2-di(2-methoxyethyl)amino-1-(4-formylphenyl)benzene;
5-amino-2-di(2-methoxyethyl)amino-1-(4-hydroxyphenyl)benzene;
5-amino-2-di(2-methoxyethyl)amino-1-(4-iodophenyl)benzene;
5-amino-2-di(2-methoxyethyl)amino-1-(4-methoxyphenyl)benzene;
5-amino-2-di(2-methoxyethyl)amino-1-(4-methyl-3-nitrophenyl)benzene;
5-amino-2-di(2-methoxyethyl)amino-1-(4-methylphenyl)benzene;
5-amino-2-di(2-methoxyethyl)amino-1-(5-bromo-2-methoxyphenyl)benzene;
5-amino-2-di(2-methoxyethyl)amino-1-(5-chloro-2-methoxyphenyl)benzene;
5-amino-2-di(2-methoxyethyl)amino-1-(5-formyl-2-methoxyphenyl)benzene;
5-amino-2-dimethylamino-1-phenylbenzene;
5-amino-2-methylamino-1-(2,3,4-trimethoxyphenyl)benzene;
5-amino-2-methylamino-1-(2,4-diaminophenyl)benzene;
5-amino-2-methylamino-1-(2,4-dihydroxyphenyl)benzene;
5-amino-2-methylamino-1-(2,5-diaminophenyl)benzene;
5-amino-2-methylamino-1-(2,5-dimethoxyphenyl)benzene;
5-amino-2-methylamino-1-(2,6-dimethoxyphenyl)benzene;
5-amino-2-methylamino-1-(2-amino-5-hydroxphenyl)benzene;
5-amino-2-methylamino-1-(2-aminophenyl)benzene;
5-amino-2-methylamino-1-(2-hydroxy-4-aminophenyl)benzene;
5-amino-2-methylamino-1-(2-hydroxy-5-aminophenyl)benzene;
5-amino-2-methylamino-1-(2-hydroxyphenyl)benzene;
5-amino-2-methylamino-1-(2-methoxyphenyl)benzene;
5-amino-2-methylamino-1-(2-methylphenyl)benzene;
5-amino-2-methylamino-1-(3,5-diaminophenyl)benzene;
5-amino-2-methylamino-1-(3,5-dihydroxyphenyl)benzene;
5-amino-2-methylamino-1-(3-aminophenyl)benzene;
5-amino-2-methylamino-1-(3-hydroxy-5-aminophenyl)benzene;
5-amino-2-methylamino-1-(3-hydroxyphenyl)benzene;
5-amino-2-methylamino-1-(3-methoxyphenyl)benzene;
5-amino-2-methylamino-1-(4-(dimethylamino)phenyl)benzene;
5-amino-2-methylamino-1-(4-(trifluoromethyl)phenyl)benzene;
5-amino-2-methylamino-1-(4-aminophenyl)benzene;
5-amino-2-methylamino-1-(4-carboxyphenyl)benzene;
5-amino-2-methylamino-1-(4-chlorophenyl)benzene;
5-amino-2-methylamino-1-(4-hydroxyphenyl)benzene;
5-amino-2-methylamino-1-(4-methoxyphenyl)benzene;
5-amino-2-methylamino-1-(4-methylphenyl)benzene and
5-amino-2-methylamino-1-phenylbenzene, or
physiologically compatible salts thereof.

## Revendications

1. Composition pour la teinture d'oxydation de fibres kératiniques à base d'une combinaison d'une substance révélatrice et d'une substance couplante, **caractérisée en ce qu'**elle comprend, en tant que substance révélatrice, au moins un composé choisi parmi le groupe constitué
du 2,5-diamino-1-phénylbenzène ;
du 2,5-diamino-1-(4-bromophényl)benzène ;
du 2,5-diamino-1-(4-éthénylphényl)benzène ;
du 2,5-diamino-1-(2,3,4-triméthoxyphényl)benzène ;
du 2,5-diamino-1-(2,4-di(2-hydroxyéthyl)aminophényl)benzène ;
du 2,5-diamino-1-(2,4-diaminophényl)benzène ;
du 2,5-diamino-1-(2,4-dihydroxyphényl)benzène ;
du 2,5-diamino-1-(2,4-diméthylaminophényl)benzène ;
du 2,5-diamino-1-(2,4-méthoxyphényl)benzène ;
du 2,5-diamino-1-(2,5-di(2-hydroxyéthyl)aminophényl)benzène ;
du 2,5-diamino-1-(2,5-diaminophényl)benzène ;
du 2,5-diamino-1-(2,5-dihydroxyphényl)benzène ;
du 2,5-diamino-1-(2,5-diméthoxyphényl)benzène ;
du 2,5-diamino-1-(2,5-diméthylaminophényl)benzène ;
du 2,5-diamino-1-(2,6-di(2-hydroxyéthyl)aminophényl)benzène ;
du 2,5-diamino-1-(2,6-diaminophényl)benzène ;
du 2,5-diamino-1-(2,6-dihydroxyphényl)benzène ;
du 2,5-diamino-1-(2,6-diméthoxyphényl)benzène ;
du 2,5-diamino-1-(2,6-diméthylaminophényl)benzène ;
du 2,5-diamino-1-(2-(bromométhyl)phényl)benzène ;
du 2,5-diamino-1-(2-amino-5-hydroxyphényl)benzène ;
du 2,5-diamino-1-(2-aminophényl)benzène ;
du 2,5-diamino-1-(2-carboxyphényl)benzène ;
du 2,5-diamino-1-(2-chlorophényl)benzène ;
du 2,5-diamino-1-(2-di(2-hydroxyéthyl)aminophényl)benzène ;
du 2,5-diamino-1-(2-diméthylaminophényl)benzène ;
du 2,5-diamino-1-(2-fluorophényl)benzène ;
du 2,5-diamino-1-(2-formylphényl)benzène ;
du 2,5-diamino-1-(2-hydroxy-4-aminophényl)benzène ;
du 2,5-diamino-1-(2-hydroxy-5-aminophényl)benzène ;
du 2,5-diamino-1-(2-hydroxyphényl)benzène :
du 2,5-diamino-1-(2-méthoxyphényl)benzène ;
du 2,5-diamino-1-(2-méthylphényl)benzène ;
du 2,5-diamino-1-(2-nitrophényl)benzène ;
du 2,5-diamino-1-(2-trifluorométhylphényl)benzène ;
du 2,5-diamino-1-(3,5-di(2-hydroxyéthyl)aminophényl)benzène ;
du 2,5-diamino-1-(3,5-diaminophényl)benzène ;
du 2,5-diamino-1-(3,5-dihydroxyphényl)benzène ;
du 2,5-diamino-1-(3,5-diméthoxyphényl)benzène ;
du 2,5-diamino-1-(3,5-diméthylaminophényl)benzène ;
du 2,5-diamino-1-(3-aminophényl)benzène ;
du 2,5-diamino-1-(3-bromophényl)benzène ;
du 2,5-diamino-1-(3-carboxyphényl)benzène ;
du 2,5-diamino-1-(3-chlorophényl)benzène ;
du 2,5-diamino-1-(3-di(2-hydroxyéthyl)aminophényl)benzène ;
du 2,5-diamino-1-(3-diméthylaminophényl)benzène ;
du 2,5-diamino-1-(3-fluorophényl)benzène ;
du 2,5-diamino-1-(3-formylphényl)benzène ;
du 2,5-diamino-1-(3-hydroxy-5-aminophényl)benzène ;
du 2,5-diamino-1-(3-hydroxyphényl)benzène ;
du 2,5-diamino-1-(3-méthoxyphényl)benzène ;
du 2,5-diamino-1-(3-nitrophényl)benzène ;
du 2,5-diamino-1-(3-trifluorométhylphényl)benzène ;
du 2,5-diamino-1-(4-(diméthylamino)phényl)benzène ;
du 2,5-diamino-1-(4-(hydroxyméthyl)phényl)benzène ;
du 2,5-diamino-1-(4-(méthylthio)phényl)benzène ;
du 2,5-diamino-1-(4-(trifluorométhyl)phényl)benzène ;
du 2,5-diamino-1-(4-(triméthylsilyl)phényl)benzène ;
du 2,5-diamino-1-(4-acétylphényl)benzène ;
du 2,5-diamino-1-(4-aminophényl)benzène ;
du 2,5-diamino-1-(4-carboxyphényl)benzène ;
du 2,5-diamino-1-(4-chlorophényl)benzène ;
du 2,5-diamino-1-(4-di(2-hydroxyéthyl)aminophényl)benzène ;
du 2,5-diamino-1-(4-diméthylaminophényl)benzène ;
du 2,5-diamino-1-(4-éthoxyphényl)benzène ;
du 2,5-diamino-1-(4-fluorophényl)benzène ;
du 2,5-diamino-1-(4-formylphényl)benzène ;
du 2,5-diamino-1-(4-hydroxyphényl)benzène ;
du 2,5-diamino-1-(4-méthoxyphényl)benzène ;
du 2,5-diamino-1-(4-méthylphényl)benzène ;
du 2,5-diamino-1-phénylbenzène ;
du 2,5-diamino-4-chloro-1-phénylbenzène ;
du 2,5-diamino-4-méthoxy-1-phénylbenzène ;
du 2,5-diamino-4-méthyl-1-phénylbenzène ;
du 2,5-diamino-1-(2-cyanométhylphényl)benzène ;
du 2,5-diamino-1-(4-éthylphényl)benzène ;
du 2,5-diamino-1-(4-propylphényl)benzène ;
du 2,5-diamino-1-(4-isopropylphényl)benzène ;
du 2,5-diamino-1-(4-butylphényl)benzène ;
du 2,5-diamino-1-(4-tert-butylphényl)benzène ;
du 2,5-diamino-1-(4-pentylphényl)benzène ;
du 2,5-diamino-1-(4-thiométhoxyphényl)benzène ;
du 2,5-diamino-1-(2-éthylphényl)benzène ;
du 2,5-diamino-1-(2-fluoro-4-méthylphényl)benzène ;
du 2,5-diamino-1-(2-méthyl-5-fluorophényl)benzène ;
du 2,5-diamino-1-(2-thiométhoxyphényl)benzène ;
du 2,5-diamino-1-(2,3-dichlorophényl)benzène ;
du 2,5-diamino-4-(4'-hydroxybiphényl)benzène ;
du 2,5-diamino-1-(3-éthoxyphényl)benzène ;
du 2,5-diamino-1-(4-(2-pyrrolidin-1-yléthoxy)phényl)benzène ;
du 2,5-diamino-1-(4-(1-hydroxyéthyl)phényl)benzène ;
du 2,5-diamino-1-(2,4-trifluorométhylphényl)benzène ;
du 2,5-diamino-1-(2-fluoro-5-acétylphényl)benzène ;
du 2,5-diamino-1-(3-fluoro-4-méthoxyphényl)benzène ;
du 2,5-diamino-1-(3-acétyl-4-hydroxyphényl)benzène ;
du 2,5-diamino-1-(4-(2-hydroxyéthyl)phényl)benzène ;
du 2,5-diamino-1-(4-(propyl-1-one)phényl)benzène ;
du 2,5-diamino-1-(4-N,N'-diisopropylaminométhylphényl)benzène ;
du 2,5-diamino-1-(3-acétylphényl)benzène ;
du 2,5-diamino-1-(2-(2-hydroxyéthyl)phényl)benzène ;
du 2,5-diamino-1-(3-aminométhylphényl)benzène ;
du 2-amino-5-(2,3-dihydroxypropyl)amino-1-(2,3,4-triméthoxyphényl)benzène ;
du 2-amino-5-(2,3-dihydroxypropyl)amino-1-(2,4-dihydroxyphényl)benzène ;
du 2-amino-5-(2,3-dihydroxypropyl)amino-1-(2,5-diaminophényl)benzène ;
du 2-amino-5-(2,3-dihydroxypropyl)amino-1-(2,6-diméthoxyphényl)benzène ;
du 2-amino-5-(2,3-dihydroxypropyl)amino-1-(2-amino-5-hydroxyphényl)benzène ;
du 2-amino-5-(2,3-dihydroxypropyl)amino-1-(2-aminophényl)benzène ;
du 2-amino-5-(2,3-dihydroxypropyl)amino-1-(2-hydroxy-4-aminophényl)benzène ;
du 2-amino-5-(2,3-dihydroxypropyl)amino-1-(2-hydroxy-5-aminophényl)benzène ;
du 2-amino-5-(2,3-dihydroxypropyl)amino-1-(2-hydroxyphényl)benzène ;
du 2-amino-5-(2,3-dihydroxypropyl)amino-1-(2-méthoxyphényl)benzène ;
du 2-amino-5-(2,3-dihydroxypropyl)amino-1-(2-méthylphényl)benzène ;
du 2-amino-5-(2,3-dihydroxypropyl)amino-1-(3,5-diaminophényl)benzène ;
du 2-amino-5-(2,3-dihydroxypropyl)amino-1-(3,5-dihydroxyphényl)benzène ;
du 2-amino-5-(2,3-dihydroxypropyl)amino-1-(3-aminophényl)benzène ;
du 2-amino-5-(2,3-dihydroxypropyl)amino-1-(3-hydroxy-5-aminophényl)benzène ;
du 2-amino-5-(2,3-dihydroxypropyl)amino-1-(3-hydroxyphényl)benzène ;
du 2-amino-5-(2,3-dihydroxypropyl)amino-1-(3-méthoxyphényl)benzène ;
du 2-amino-5-(2,3-dihydroxypropyl)amino-1-(4-(diméthylamino)phényl)benzène ;
du 2-amino-5-(2,3-dihydroxypropyl)amino-1-(4-(trifluorométhyl)phényl)benzène ;
du 2-amino-5-(2,3-dihydroxypropyl)amino-1-(4-aminophényl)benzène ;
du 2-amino-5-(2,3-dihydroxypropyl)amino-1-(4-carboxyphényl)benzène ;
du 2-amino-5-(2,3-dihydroxypropyl)amino-1-(4-chlorophényl)benzène ;
du 2-amino-5-(2,3-dihydroxypropyl)amino-1-(4-hydroxyphényl)benzène ;
du 2-amino-5-(2,3-dihydroxypropyl)amino-1-(4-méthoxyphényl)benzène ;
du 2-amino-5-(2,3-dihydroxypropyl)amino-1-(4-méthylphényl)benzène ;
du 2-amino-5-(2,3-dihydroxypropyl)amino-1-phénylbenzène ;
du 2-amino-5-(2-hydroxyéthyl)amino-1-(2,3,4-triméthoxyphényl)benzène ;
du 2-amino-5-(2-hydroxyéthyl)amino-1-(2,4-diaminophényl)benzène ;
du 2-amino-5-(2-hydroxyéthyl)amino-1-(2,4-dihydroxyphényl)benzène ;
du 2-amino-5-(2-hydroxyéthyl)amino-1-(2,5-diaminophényl)benzène ;
du 2-amino-5-(2-hydroxyéthyl)amino-1-(2,5-diméthoxyphényl)benzène ;
du 2-amino-5-(2-hydroxyéthyl)amino-1-(2,6-diméthoxyphényl)benzène ;
du 2-amino-5-(2-hydroxyéthyl)amino-1-(2-amino-5-hydroxyphényl)benzène ;
du 2-amino-5-(2-hydroxyéthyl)amino-1-(2-aminophényl)benzène ;
du 2-amino-5-(2-hydroxyéthyl)amino-1-(2-hydroxy-4-aminophényl)benzène ;
du 2-amino-5-(2-hydroxyéthyl)amino-1-(2-hydroxy-5-aminophényl)benzène ;
du 2-amino-5-(2-hydroxyéthyl)amino-1-(2-hydroxyphényl)benzène ;
du 2-amino-5-(2-hydroxyéthyl)amino-1-(2-méthoxyphényl)benzène ;
du 2-amino-5-(2-hydroxyéthyl)amino-1-(2-méthylphényl)benzène ;
du 2-amino-5-(2-hydroxyéthyl)amino-1-(3,5-diaminophényl)benzène ;
du 2-amino-5-(2-hydroxyéthyl)amino-1-(3,5-dihydroxyphényl)benzène ;
du 2-amino-5-(2-hydroxyéthyl)amino-1-(3-aminophényl)benzène ;
du 2-amino-5-(2-hydroxyéthyl)amino-1-(3-hydroxy-5-aminophényl)benzène ;
du 2-amino-5-(2-hydroxyéthyl)amino-1-(3-hydroxyphényl)benzène ;
du 2-amino-5-(2-hydroxyéthyl)amino-1-(3-méthoxyphényl)benzène ;
du 2-amino-5-(2-hydroxyéthyl)amino-1-(4-(diméthylamino)phényl)benzène ;
du 2-amino-5-(2-hydroxyéthyl)amino-1-(4-(trifluorométhyl)phényl)benzène ;
du 2-amino-5-(2-hydroxyéthyl)amino-1-(4-aminophényl)benzène ;
du 2-amino-5-(2-hydroxyéthyl)amino-1-(4-carboxyphényl)benzène ;
du 2-amino-5-(2-hydroxyéthyl)amino-1-(4-chlorophényl)benzène ;
du 2-amino-5-(2-hydroxyéthyl)amino-1-(4-hydroxyphényl)benzène ;
du 2-amino-5-(2-hydroxyéthyl)amino-1-(4-méthoxyphényl)benzène ;
du 2-amino-5-(2-hydroxyéthyl)amino-1-(4-méthylphényl)benzène ;
du 2-amino-5-(2-hydroxyéthyl)amino-1-phénylbenzène ;
du 2-amino-5-(2-hydroxyéthyl)méthylamino-1-phénylbenzène ;
du 2-amino-5-(2-méthoxyéthyl)amino-1-(2,3,4-triméthoxyphényl)benzène ;
du 2-amino-5-(2-méthoxyéthyl)amino-1-(2,4-diaminophényl)benzène ;
du 2-amino-5-(2-méthoxyéthyl)amino-1-(2,4-dihydroxyphényl)benzène ;
du 2-amino-5-(2-méthoxyéthyl)amino-1-(2,5-diaminophényl)benzène ;
du 2-amino-5-(2-méthoxyéthyl)amino-1-(2,5-diméthoxyphényl)benzène ;
du 2-amino-5-(2-méthoxyéthyl)amino-1-(2,6-diméthoxyphényl)benzène ;
du 2-amino-5-(2-méthoxyéthyl)amino-1-(2-amino-5-hydroxyphényl)benzène ;
du 2-amino-5-(2-méthoxyéthyl)amino-1-(2-aminophényl)benzène ;
du 2-amino-5-(2-méthoxyéthyl)amino-1-(2-hydroxy-4-aminophényl)benzène ;
du 2-amino-5-(2-méthoxyéthyl)amino-1-(2-hydroxy-5-aminophényl)benzène ;
du 2-amino-5-(2-méthoxyéthyl)amino-1-(2-hydroxyphényl)benzène ;
du 2-amino-5-(2-méthoxyéthyl)amino-1-(2-méthoxyphényl)benzène ;
du 2-amino-5-(2-méthoxyéthyl)amino-1-(2-méthylphényl)benzène ;
du 2-amino-5-(2-méthoxyéthyl)amino-1-(3,5-diaminophényl)benzène ;
du 2-amino-5-(2-méthoxyéthyl)amino-1-(3,5-dihydroxyphényl)benzène ;
du 2-amino-5-(2-méthoxyéthyl)amino-1-(3-aminophényl)benzène ;
du 2-amino-5-(2-méthoxyéthyl)amino-1-(3-hydroxy-5-aminophényl)benzène ;
du 2-amino-5-(2-méthoxyéthyl)amino-1-(3-hydroxyphényl)benzène ;
du 2-amino-5-(2-méthoxyéthyl)amino-1-(3-méthoxyphényl)benzène ;
du 2-amino-5-(2-méthoxyéthyl)amino-1-(4-(diméthylamino)phényl)benzène ;
du 2-amino-5-(2-méthoxyéthyl)amino-1-(4-(trifluorométhyl)phényl)benzène ;
du 2-amino-5-(2-méthoxyéthyl)amino-1-(4-aminophényl)benzène ;
du 2-amino-5-(2-méthoxyéthyl)amino-1-(4-carboxyphényl)benzène ;
du 2-amino-5-(2-méthoxyéthyl)amino-1-(4-chlorophényl)benzène ;
du 2-amino-5-(2-méthoxyéthyl)amino-1-(4-hydroxyphényl)benzène ;
du 2-amino-5-(2-méthoxyéthyl)amino-1-(4-méthoxyphényl)benzène ;
du 2-amino-5-(2-méthoxyéthyl)amino-1-(4-méthylphényl)benzène ;
du 2-amino-5-(2-méthoxyéthyl)amino-1-phénylbenzène ;
du 2-amino-5-di(2-hydroxyéthyl)amino-1-(2,3,4triméthoxyphényl)benzène ;
du 2-amino-5-di(2-hydroxyéthyl)amino-1-(2,3-difluoro-4-heptylphényl)benzène ;
du 2-amino-5-di(2-hydroxyéthyl)amino-1-(2,3-difluorophényl)benzène ;
du 2-amino-5-di(2-hydroxyéthyl)amino-1-(2,4,6-triméthylphényl)benzène ;
du 2-amino-5-di(2-hydroxyéthyl)amino-1-(2,4-diaminophényl)benzène ;
du 2-amino-5-di(2-hydroxyéthyl)amino-1-(2,4-dichlorophényl)benzène ;
du 2-amino-5-di(2-hydroxyéthyl)amino-1-(2,4-dihydroxyphényl)benzène ;
du 2-amino-5-di(2-hydroxyéthyl)amino-1-(2,5-diaminophényl)benzène ;
du 2-amino-5-di(2-hydroxyéthyl)amino-1-(2,5-diméthoxyphényl)benzène ;
du 2-amino-5-di(2-hydroxyéthyl)amino-1-(2,6-difluorophényl)benzène ;
du 2-amino-5-di(2-hydroxyéthyl)amino-1-(2,6-diméthoxyphényl)benzène ;
du 2-amino-5-di(2-hydroxyéthyl)amino-1-(2,6-diméthoxyphényl)benzène ;
du 2-amino-5-di(2-hydroxyéthyl)amino-1-(2-((bis(1-méthyléthyl)amino)carbonyl)phényl)benzène ;
du 2-amino-5-di(2-hydroxyéthyl)amino-1-(2-((bis(1-méthyléthyl)amino)carbonyl)-3-méthoxyphényl)benzène ;
du 2-amino-5-di(2-hydroxyéthyl)amino-1-(2-(bromométhyl)phényl)benzène ;
du 2-amino-5-di(2-hydroxyéthyl)amino-1-(2-(diéthylamino)carbonyl)phényl)benzène ;
du 2-amino-5-di(2-hydroxyéthyl)amino-1-(2-amino-5-hydroxyphényl)benzène ;
du 2-amino-5-di(2-hydroxyéthyl)amino-1-(2-aminophényl)benzène ;
du 2-amino-5-di(2-hydroxyéthyl)amino-1-(2-carboxyphényl)benzène ;
du 2-amino-5-di(2-hydroxyéthyl)amino-1-(2-chlorophényl)benzène ;
du 2-amino-5-di(2-hydroxyéthyl)amino-1-(2-fluorophényl)benzène ;
du 2-amino-5-di(2-hydroxyéthyl)amino-1-(2-formyl-4-méthoxyphényl)benzène ;
du 2-amino-5-di(2-hydroxyéthyl)amino-1-(2-formyl-4-méthylphényl)benzène ;
du 2-amino-5-di(2-hydroxyéthyl)amino-1-(2-formyl-5-méthoxyphényl)benzène ;
du 2-amino-5-di(2-hydroxyéthyl)amino-1-(2-formylphényl)benzène ;
du 2-amino-5-di(2-hydroxyéthyl)amino-1-(2-hydroxy-4-aminophényl)benzène ;
du 2-amino-5-di(2-hydroxyéthyl)amino-1-(2-hydroxy-5-aminophényl)benzène ;
du 2-amino-5-di(2-hydroxyéthyl)amino-1-(2-hydroxyphényl)benzène ;
du 2-amino-5-di(2-hydroxyéthyl)amino-1-(2-méthoxyphényl)benzène ;
du 2-amino-5-di(2-hydroxyéthyl)amino-1-(2-méthylphényl)benzène ;
du 2-amino-5-di(2-hydroxyéthyl)amino-1-(2-nitrophényl)benzène ;
du 2-amino-5-di(2-hydroxyéthyl)amino-1-(2-trifluorométhylphényl)benzène ;
du 2-amino-5-di(2-hydroxyéthyl)amino-1-(3,4-dichlorophényl)benzène ;
du 2-amino-5-di(2-hydroxyéthyl)amino-1-(3,5-diaminophényl)benzène ;
du 2-amino-5-di(2-hydroxyéthyl)amino-1-(3,5-dichlorophényl)benzène ;
du 2-amino-5-di(2-hydroxyéthyl)amino-1-(3,5-dihydroxyphényl)benzène ;
du 2-amino-5-di(2-hydroxyéthyl)amino-1-(3-(acétylamino)phényl)benzène ;
du 2-amino-5-di(2-hydroxyéthyl)amino-1-(3-aminophényl)benzène ;
du 2-amino-5-di(2-hydroxyéthyl)amino-1-(3-bromophényl)benzène ;
du 2-amino-5-di(2-hydroxyéthyl)amino-1-(3-carboxyphényl)benzène ;
du 2-amino-5-di(2-hydroxyéthyl)amino-1-(3-chloro-4-fluorophényl)benzène ;
du 2-amino-5-di(2-hydroxyéthyl)amino-1-(3-chlorophényl)benzène ;
du 2-amino-5-di(2-hydroxyéthyl)amino-1(3-fluorophényl)benzène ;
du 2-amino-5-di(2-hydroxyéthyl)amino-1-(3-formylphényl)benzène ;
du 2-amino-5-di(2-hydroxyéthyl)amino-1-(3-hydroxy-5-aminophényl)benzène ;
du 2-amino-5-di(2-hydroxyéthyl)amino-1-(3-hydroxyphényl)benzène ;
du 2-amino-5-di(2-hydroxyéthyl)amino-1-(3-hydroxyphényl)benzène ;
du 2-amino-5-di(2-hydroxyéthyl)amino-1-(3-méthoxyphényl)benzène ;
du 2-amino-5-di(2-hydroxyéthyl)amino-1-(3-méthylphényl)benzène ;
du 2-amino-5-di(2-hydroxyéthyl)amino-1-(3-nitrophényl)benzène ;
du 2-amino-5-di(2-hydroxyéthyl)amino-1-(3-trifluorométhylphényl)benzène ;
du 2-amino-5-di(2-hydroxyéthyl)amino-1-(4-(bromométhyl)phényl)benzène ;
du 2-amino-5-di(2-hydroxyéthyl)amino-1-(4-(diméthylamino)phényl)benzène ;
du 2-amino-5-di(2-hydroxyéthyl)amino-1-(4-(hydroxyméthyl)phényl)benzène ;
du 2-amino-5-di(2-hydroxyéthyl)amino-1-(4-(méthylthio)phényl)benzène ;
du 2-amino-5-di(2-hydroxyéthyl)amino-1-(4-(trifluorométhyl)phényl)benzène ;
du 2-amino-5-di(2-hydroxyéthyl)amino-1-(4-(triméthylsilyl)phényl)benzène ;
du 2-amino-5-di(2-hydroxyéthyl)amino-1-(4-acétylphényl)benzène ;
du 2-amino-5-di(2-hydroxyéthyl)amino-1-(4-aminophényl)benzène ;
du 2-amino-5-di(2-hydroxyéthyl)amino-1-(4-bromophényl)benzène ;
du 2-amino-5-di(2-hydroxyéthyl)amino-1-(4-carboxyphényl)benzène ;
du 2-amino-5-di(2-hydroxyéthyl)amino-1-(4-chloro-3méthoxyphényl)benzène ;
du 2-amino-5-di(2-hydroxyéthyl)amino-1-(4-chlorophényl)benzène ;
du 2-amino-5-di(2-hydroxyéthyl)amino-1-(4-éthénylphényl)benzène ;
du 2-amino-5-di(2-hydroxyéthyl)amino-1-(4-fluorophényl)benzène ;
du 2-amino-5-di(2-hydroxyéthyl)amino-1-(4-formylphényl)benzène ;
du 2-amino-5-di(2-hydroxyéthyl)amino-1-(4-hydroxyphényl)benzène ;
du 2-amino-5-di(2-hydroxyéthyl)amino-1-(4-iodophényl)benzène ;
du 2-amino-5-di(2-hydroxyéthyl)amino-1-(4-méthoxyphényl)benzène ;
du 2-amino-5-di(2-hydroxyéthyl)amino-1-(4-méthyl-3-nitrophényl)benzène ;
du 2-amino-5-di(2-hydroxyéthyl)amino-1-(4-méthylphényl)benzène ;
du 2-amino-5-di(2-hydroxyéthyl)amino-1-(5-bromo-2-méthoxyphényl)benzène ;
du 2-amino-5-di(2-hydroxyéthyl)amino-1-(5-chloro-2-méthoxyphényl)benzène ;
du 2-amino-5-di(2-hydroxyéthyl)amino-1-(5-formyl-2-méthoxyphényl)benzène ;
du 2-amino-5-di(2-hydroxyéthyl)amino-1-phénylbenzène ;
du 2-amino-5-di(2-méthoxyéthyl)amino-1-(2,3,4-triméthoxyphényl)benzène ;
du 2-amino-5-di(2-méthoxyéthyl)amino-1-(2,3-difluoro-4-heptylphényl)benzène ;
du 2-amino-5-di(2-méthoxyéthyl)amino-1-(2,3-difluorophényl)benzène ;
du 2-amino-5-di(2-méthoxyéthyl)amino-1-(2,4,6-triméthylphényl)benzène ;
du 2-amino-5-di(2-méthoxyéthyl)amino-1-(2,4,-diaminophényl)benzène ;
du 2-amino-5-di(2-méthoxyéthyl)amino-1-(2,4-dichlorophényl)benzène ;
du 2-amino-5-di(2-méthoxyéthyl)amino-1-(2,4-dihydroxyphényl)benzène ;
du 2-amino-5-di(2-méthoxyéthyl)amino-1-(2,5-diméthoxyphényl)benzène ;
du 2-amino-5-di(2-méthoxyéthyl)amino-1-(2,6-difluorophényl)benzène ;
du 2-amino-5-di(2-méthoxyéthyl)amino-1-(2,6-diméthoxyphényl)benzène ;
du 2-amino-5-di(2-méthoxyéthyl)amino-1-(2-((bis(1-méthyléthyl)amino)carbonyl)phényl)benzène ;
du 2-amino-5-di(2-méthoxyéthyl)amino-1-(2-(bromométhyl)phényl)benzène ;
du 2-amino-5-di(2-méthoxyéthyl)amino-1-(2-(diéthylamino)carbonyl)phényl)benzène ;
du 2-amino-5-di(2-méthoxyéthyl)amino-1-(2-carboxyphényl)benzène ;
du 2-amino-5-di(2-méthoxyéthyl)amino-1-(2-chlorophényl)benzène ;
du 2-amino-5-di(2-méthoxyéthyl)amino-1-(2-fluorophényl)benzène ;
du 2-amino-5-di(2-méthoxyéthyl)amino-1-(2-formyl-4-méthoxyphényl)benzène ;
du 2-amino-5-di(2-méthoxyéthyl)amino-1-(2-formyl-4-méthylphényl)benzène ;
du 2-amino-5-di(2-méthoxyéthyl)amino-1-(2-formyl-5-méthoxyphényl)benzène ;
du 2-amino-5-di(2-méthoxyéthyl)amino-1-(2-formylphényl)benzène ;
du 2-amino-5-di(2-méthoxyéthyl)amino-1-(2-hydroxy-4-aminophényl)benzène ;
du 2-amino-5-di(2-méthoxyéthyl)amino-1-(2-hydroxyphényl)benzène ;
du 2-amino-5-di(2-méthoxyéthyl)amino-1-(2-méthoxyphényl)benzène ;
du 2-amino-5-di(2-méthoxyéthyl)amino-1-(2-méthylphényl)benzène ;
du 2-amino-5-di(2-méthoxyéthyl)amino-1-(2-nitrophényl)benzène ;
du 2-amino-5-di(2-méthoxyéthyl)amino-1-(2-trifluorométhylphényl)benzène ;
du 2-amino-5-di(2-méthoxyéthyl)amino-1-(3,4-dichlorophényl)benzène ;
du 2-amino-5-di(2-méthoxyéthyl)amino-1-(3,5-diaminophényl)benzène ;
du 2-amino-5-di(2-méthoxyéthyl)amino-1-(3,5-dichlorophényl)benzène ;
du 2-amino-5-di(2-méthoxyéthyl)amino-1-(3,5-dihydroxyphényl)benzène ;
du 2-amino-5-di(2-méthoxyéthyl)amino-1-(3-(acétylamino)phényl)benzène ;
du 2-amino-5-di(2-méthoxyéthyl)amino-1-(3-aminophényl)benzène ;
du 2-amino-5-di(2-méthoxyéthyl)amino-1-(3-bromophényl)benzène ;
du 2-amino-5-di(2-méthoxyéthyl)amino-1-(3-carboxyphényl)benzène ;
du 2-amino-5-di(2-méthoxyéthyl)amino-1-(3-chloro-4-fluorophényl)benzène ;
du 2-amino-5-di(2-méthoxyéthyl)amino-1-(3-chlorophényl)benzène ;
du 2-amino-5-di(2-méthoxyéthyl)amino-1-(3-fluorophényl)benzène ;
du 2-amino-5-di(2-méthoxyéthyl)amino-1-(3-formylphényl)benzène ;
du 2-amino-5-di(2-méthoxyéthyl)amino-1-(3-hydroxyphényl)benzène ;
du 2-amino-5-di(2-méthoxyéthyl)amino-1-(3-méthoxyphényl)benzène ;
du 2-amino-5-di(2-méthoxyéthyl)amino-1-(3-méthylphényl)benzène ;
du 2-amino-5-di(2-méthoxyéthyl)amino-1-(3-nitrophényl)benzène ;
du 2-amino-5-di(2-méthoxyéthyl)amino-1-(3-trifluorométhylphényl)benzène ;
du 2-amino-5-di(2-méthoxyéthyl)amino-1-(4-(bromométhyl)phényl)benzène ;
du 2-amino-5-di(2-méthoxyéthyl)amino-1-(4-(diméthylamino)phényl)benzène ;
du 2-amino-5-di(2-méthoxyéthyl)amino-1-(4-(hydroxyméthyl)phényl)benzène ;
du 2-amino-5-di(2-méthoxyéthyl)amino-1-(4-(méthylthio)phényl)benzène ;
du 2-amino-5-di(2-méthoxyéthyl)amino-1-(4-(trifluorométhyl)phényl)benzène ;
du 2-amino-5-di(2-méthoxyéthyl)amino-1-(4-(triméthylsilyl)phényl)benzène ;
du 2-amino-5-di(2-méthoxyéthyl)amino-1-(4-acétylphényl)benzène ;
du 2-amino-5-di(2-méthoxyéthyl)amino-1-(4-aminophényl)benzène ;
du 2-amino-5-di(2-méthoxyéthyl)amino-1-(4-bromophényl)benzène ;
du 2-amino-5-di(2-méthoxyéthyl)amino-1-(4-carboxyphényl)benzène ;
du 2-amino-5-di(2-méthoxyéthyl)amino-1-(4-chloro-3-méthoxyphényl)benzène ;
du 2-amino-5-di(2-méthoxyéthyl)amino-1-(4-chlorophényl)benzène ;
du 2-amino-5-di(2-méthoxyéthyl)amino-1-(4-éthénylphényl)benzène ;
du 2-amino-5-di(2-méthoxyéthyl)amino-1-(4-éthoxyphényl)benzène ;
du 2-amino-5-di(2-méthoxyéthyl)amino-1-(4-fluorophényl)benzène ;
du 2-amino-5-di(2-méthoxyéthyl)amino-1-(4-formylphényl)benzène ;
du 2-amino-5-di(2-méthoxyéthyl)amino-1-(4-hydroxyphényl)benzène ;
du 2-amino-5-di(2-méthoxyéthyl)amino-1-(4-iodophényl)benzène ;
du 2-amino-5-di(2-méthoxyéthyl)amino-1-(4-méthoxyphényl)benzène ;
du 2-amino-5-di(2-méthoxyéthyl)amino-1-(4-méthyl-3-nitrophényl)benzène ;
du 2-amino-5-di(2-méthoxyéthyl)amino-1-(4-méthylphényl)benzène ;
du 2-amino-5-di(2-méthoxyéthyl)amino-1-(5-bromo-2-méthoxyphényl)benzène ;
du 2-amino-5-di(2-méthoxyéthyl)amino-1-(5-chloro-2-méthoxyphényl)benzène ;
du 2-amino-5-di(2-méthoxyéthyl)amino-1-(5-formyl-2-méthoxyphényl)benzène ;
du 2-amino-5-diméthylamino-1-phénylbenzène ;
du 2-amino-5-méthylamino-1-(2,3,4-triméthoxyphényl)benzène ;
du 2-amino-5-méthylamino-1-(2,4-diaminophényl)benzène ;
du 2-amino-5-méthylamino-1-(2,4-dihydroxyphényl)benzène ;
du 2-amino-5-méthylamino-1-(2,5-diaminophényl)benzène ;
du 2-amino-5-méthylamino-1-(2,5-diméthoxyphényl)benzène ;
du 2-amino-5-méthylamino-1-(2,6-diméthoxyphényl)benzène ;
du 2-amino-5-méthylamino-1-(2-amino-5-hydroxyphényl)benzène ;
du 2-amino-5-méthylamino-1-(2-aminophényl)benzène ;
du 2-amino-5-méthylamino-1-(2-hydroxy-4-aminophényl)benzène ;
du 2-amino-5-méthylamino-1-(2-hydroxy-5-aminophényl)benzène ;
du 2-amino-5-méthylamino-1-(2-hydroxyphényl)benzène ;
du 2-amino-5-méthylamino-1-(2-méthoxyphényl)benzène ;
du 2-amino-5-méthylamino-1-(2-méthylphényl)benzène ;
du 2-amino-5-méthylamino-1-(3,5-diaminophényl)benzène ;
du 2-amino-5-méthylamino-1-(3,5-dihydroxyphényl)benzène ;
du 2-amino-5-méthylamino-1-(3-aminophényl)benzène ;
du 2-amino-5-méthylamino-1-(3-hydroxy-5-aminophényl)benzène ;
du 2-amino-5-méthylamino-1-(3-hydroxyphényl)benzène ;
du 2-amino-5-méthylamino-1-(3-méthoxyphényl)benzène ;
du 2-amino-5-méthylamino-1-(4-(diméthylamino)phényl)benzène ;
du 2-amino-5-méthylamino-1-(4-(trifluorométhyl)phényl)benzène ;
du 2-amino-5-méthylamino-1-(4-aminophényl)benzène ;
du 2-amino-5-méthylamino-1-(4-carboxyphényl)benzène ;
du 2-amino-5-méthylamino-1-(4-chlorophényl)benzène ;
du 2-amino-5-méthylamino-1-(4-hydroxyphényl)benzène ;
du 2-amino-5-méthylamino-1-(4-méthoxyphényl)benzène ;
du 2-amino-5-méthylamino-1-(4-méthylphényl)benzène ;
du 2-amino-5-méthylamino-1-phénylbenzène ;
du 5-amino-2-(2,3-dihydroxypropyl)amino-1-(4-bromophényl)benzène ;
du 5-amino-2-(2,3-dihydroxypropyl)amino-1-(4-éthénylphényl)benzène ;
du 5-amino-2-(2,3-dihydroxypropyl)amino-1-(2,3,4-triméthoxyphényl)benzène ;
du 5-amino-2-(2,3-dihydroxypropyl)amino-1-(2,4-di(2-hydroxyéthyl)aminophényl)benzène ;
du 5-amino-2-(2,3-dihydroxypropyl)amino-1-(2,4-diaminophényl)benzène ;
du 5-amino-2-(2,3-dihydroxypropyl)amino-1-(2,4-dihydroxyphényl)benzène ;
du 5-amino-2-(2,3-dihydroxypropyl)amino-1-(2,4-diméthylaminophényl)benzène ;
du 5-amino-2-(2,3-dihydroxypropyl)amino-1-(2,4-méthoxyphényl)benzène ;
du 5-amino-2-(2,3-dihydroxypropyl)amino-1-(2,5-di(2-hydroxyéthyl)aminophényl)benzène ;
du 5-amino-2-(2,3-dihydroxypropyl)amino-1-(2,5-diaminophényl)benzène ;
du 5-amino-2-(2,3-dihydroxypropyl)amino-1-(2,5-dihydroxyphényl)benzène ;
du 5-amino-2-(2,3-dihydroxypropyl)amino-1-(2,5-diméthoxyphényl)benzène ;
du 5-amino-2-(2,3-dihydroxypropyl)amino-1-(2,5-diméthylaminophényl)benzène ;
du 5-amino-2-(2,3-dihydroxypropyl)amino-1-(2,6-di(2-hydroxyéthyl)aminophényl)benzène ;
du 5-amino-2-(2,3-dihydroxypropyl)amino-1-(2,6-diamino-phényl)benzène ;
du 5-amino-2-(2,3-dihydroxypropyl)amino-1-(2,6-dihydroxyphényl)benzène ;
du 5-amino-2-(2,3-dihydroxypropyl)amino-1-(2,6-diméthoxyphényl)benzène ;
du 5-amino-2-(2,3-dihydroxypropyl)amino-1-(2,6-diméthylaminophényl)benzène ;
du 5-amino-2-(2,3-dihydroxypropyl)amino-1-(2-(bromométhyl)phényl)benzène ;
du 5-amino-2-(2,3-dihydroxypropyl)amino-1-(2-amino-5-hydroxyphényl)benzène ;
du 5-amino-2-(2,3-dihydroxypropyl)amino-1-(2-aminophényl)benzène ;
du 5-amino-2-(2,3-dihydroxypropyl)amino-1-(2-carboxyphényl)benzène ;
du 5-amino-2-(2,3-dihydroxypropyl)amino-1-(2-chlorophényl)benzène ;
du 5-amino-2-(2,3-dihydroxypropyl)amino-1-(2-di(2-hydroxyéthyl)aminophényl)benzène ;
du 5-amino-2-(2,3-dihydroxypropyl)amino-1-(2-diméthylaminophényl)benzène ;
du 5-amino-2-(2,3-dihydroxypropyl)amino-1-(2-fluorophényl)benzène ;
du 5-amino-2-(2,3-dihydroxypropyl)amino-1-(2-formylphényl)benzène ;
du 5-amino-2-(2,3-dihydroxypropyl)amino-1-(2-hydroxy-4-aminophényl)benzène ;
du 5-amino-2-(2,3-dihydroxypropyl)amino-1-(2-hydroxy-5-aminophényl)benzène ;
du 5-amino-2-(2,3-dihydroxypropyl)amino-1-(2-hydroxyphényl)benzène ;
du 5-amino-2-(2,3-dihydroxypropyl)amino-1-(2-méthoxyphényl)benzène ;
du 5-amino-2-(2,3-dihydroxypropyl)amino-1-(2-méthylphényl)benzène ;
du 5-amino-2-(2,3-dihydroxypropyl)amino-1-(2-nitrophényl)benzène ;
du 5-amino-2-(2,3-dihydroxypropyl)amino-1-(2-trifluorométhylphényl)benzène ;
du 5-amino-2-(2,3-dihydroxypropyl)amino-1-(3,5-di(2-hydroxyéthyl)aminophényl)benzène ;
du 5-amino-2-(2,3-dihydroxypropyl)amino-1-(3,5-diaminophényl)benzène ;
du 5-amino-2-(2,3-dihydroxypropyl)amino-1-(3,5-dihydroxyphényl)benzène ;
du 5-amino-2-(2,3-dihydroxypropyl)amino-1-(3,5-diméthoxyphényl)benzène ;
du 5-amino-2-(2,3-dihydroxypropyl)amino-1-(3,5-diméthylaminophényl)benzène ;
du 5-amino-2-(2,3-dihydroxypropyl)amino-1-(3-aminophényl)benzène ;
du 5-amino-2-(2,3-dihydroxypropyl)amino-1-(3-bromophényl)benzène ;
du 5-amino-2-(2,3-dihydroxypropyl)amino-1-(3-carboxyphényl)benzène ;
du 5-amino-2-(2,3-dihydroxypropyl)amino-1-(3-chlorophényl)benzène ;
du 5-amino-2-(2,3-dihydroxypropyl)amino-1-(3-di(2-hydroxyéthyl)aminophényl)benzène ;
du 5-amino-2-(2,3-dihydroxypropyl)amino-1-(3-diméthylaminophényl)benzène ;
du 5-amino-2-(2,3-dihydroxypropyl)amino-1-(3-fluorophényl)benzène ;
du 5-amino-2-(2,3-dihydroxypropyl)amino-1-(3-formylphényl)benzène ;
du 5-amino-2-(2,3-dihydroxypropyl)amino-1-(3-hydroxy-5-aminophényl)benzène ;
du 5-amino-2-(2,3-dihydroxypropyl)amino-1-(3-hydroxyphényl)benzène ;
du 5-amino-2-(2,3-dihydroxypropyl)amino-1-(3-méthoxyphényl)benzène ;
du 5-amino-2-(2,3-dihydroxypropyl)amino-1-(3-nitrophényl)benzène ;
du 5-amino-2-(2,3-dihydroxypropyl)amino-1-(3-trifluorométhylphényl)benzène ;
du 5-amino-2-(2,3-dihydroxypropyl)amino-1-(4-(diméthylamino)phényl)benzène ;
du 5-amino-2-(2,3-dihydroxypropyl)amino-1-(4-(hydroxyméthyl)phényl)benzène ;
du 5-amino-2-(2,3-dihydroxypropyl)amino-1-(4-(méthylthio)phényl)benzène ;
du 5-amino-2-(2,3-dihydroxypropyl)amino-1-(4-(trifluorométhyl)phényl)benzène ;
du 5-amino-2-(2,3-dihydroxypropyl)amino-1-(4-(triméthylsilyl)phényl)benzène ;
du 5-amino-2-(2,3-dihydroxypropyl)amino-1-(4-acétylphényl)benzène ;
du 5-amino-2-(2,3-dihydroxypropyl)amino-1-(4-aminophényl)benzène ;
du 5-amino-2-(2,3-dihydroxypropyl)amino-1-(4-carboxyphényl)benzène ;
du 5-amino-2-(2,3-dihydroxypropyl)amino-1-(4-chlorophényl)benzène ;
du 5-amino-2-(2,3-dihydroxypropyl)amino-1-(4-di(2-hydroxyéthyl)aminophényl)benzène ;
du 5-amino-2-(2,3-dihydroxypropyl)amino-1-(4-diméthylaminophényl)benzène ;
du 5-amino-2-(2,3-dihydroxypropyl)amino-1-(4-éthoxyphényl)benzène ;
du 5-amino-2-(2,3-dihydroxypropyl)amino-1-(4-fluorophényl)benzène ;
du 5-amino-2-(2,3-dihydroxypropyl)amino-1-(4-formylphényl)benzène ;
du 5-amino-2-(2,3-dihydroxypropyl)amino-1-(4-hydroxyphényl)benzène ;
du 5-amino-2-(2,3-dihydroxypropyl)amino-1-(4-méthoxyphényl)benzène ;
du 5-amino-2-(2,3-dihydroxypropyl)amino-1-(4-méthylphényl)benzène ;
du 5-amino-2-(2,3-dihydroxypropyl)amino-1-phénylbenzène ;
du 5-amino-2-(2-hydroxyéthyl)amino-1-(4-bromophényl)benzène ;
du 5-amino-2-(2-hydroxyéthyl)amino-1-(4-éthénylphényl)benzène ;
du 5-amino-2-(2-hydroxyéthyl)amino-1-(2,3,4-triméthoxyphényl)benzène ;
du 5-amino-2-(2-hydroxyéthyl)amino-1-(2,4-di(2-hydroxyéthyl)aminophényl)benzène ;
du 5-amino-2-(2-hydroxyéthyl)amino-1-(2,4-diaminophényl)benzène ;
du 5-amino-2-(2-hydroxyéthyl)amino-1-(2,4-dihydroxyphényl)benzène ;
du 5-amino-2-(2-hydroxyéthyl)amino-1-(2,4-diméthylaminophényl)benzène ;
du 5-amino-2-(2-hydroxyéthyl)amino-1-(2,4-méthoxyphényl)benzène ;
du 5-amino-2-(2-hydroxyéthyl)amino-1-(2,5-di(2-hydroxyéthyl)aminophényl)benzène ;
du 5-amino-2-(2-hydroxyéthyl)amino-1-(2,5-diaminophényl)benzène ;
du 5-amino-2-(2-hydroxyéthyl)amino-1-(2,5-dihydroxyphényl)benzène ;
du 5-amino-2-(2-hydroxyéthyl)amino-1-(2,5-diméthoxyphényl)benzène ;
du 5-amino-2-(2-hydroxyéthyl)amino-1-(2,5-diméthylaminophényl)benzène ;
du 5-amino-2-(2-hydroxyéthyl)amino-1-(2,6-di(2-hydroxyéthyl)aminophényl)benzène ;
du 5-amino-2-(2-hydroxyéthyl)amino-1-(2,6-diaminophényl)benzène ;
du 5-amino-2-(2-hydroxyéthyl)amino-1-(2,6-dihydroxyphényl)benzène ;
du 5-amino-2-(2-hydroxyéthyl)amino-1-(2,6-diméthoxyphényl)benzène ;
du 5-amino-2-(2-hydroxyéthyl)amino-1-(2,6-diméthylaminophényl)benzène ;
du 5-amino-2-(2-hydroxyéthyl)amino-1-(2-(bromométhyl)phényl)benzène ;
du 5-amino-2-(2-hydroxyéthyl)amino-1-(2-amino-5-hydroxyphényl)benzène ;
du 5-amino-2-(2-hydroxyéthyl)amino-1-(2-aminophényl)benzène ;
du 5-amino-2-(2-hydroxyéthyl)amino-1-(2-carboxyphényl)benzène ;
du 5-amino-2-(2-hydroxyéthyl)amino-1-(2-chlorophényl)benzène ;
du 5-amino-2-(2-hydroxyéthyl)amino-1-(2-di(2-hydroxyéthyl)aminophényl)benzène ;
du 5-amino-2-(2-hydroxyéthyl)amino-1-(2-diméthylaminophényl)benzène ;
du 5-amino-2-(2-hydroxyéthyl)amino-1-(2-fluorophényl)benzène ;
du 5-amino-2-(2-hydroxyéthyl)amino-1-(2-formylphényl)benzène ;
du 5-amino-2-(2-hydroxyéthyl)amino-1-(2-hydroxy-4-aminophényl)benzène ;
du 5-amino-2-(2-hydroxyéthyl)amino-1-(2-hydroxy-5-aminophényl)benzène ;
du 5-amino-2-(2-hydroxyéthyl)amino-1-(2-hydroxyphényl)benzène ;
du 5-amino-2-(2-hydroxyéthyl)amino-1-(2-méthoxyphényl)benzène ;
du 5-amino-2-(2-hydroxyéthyl)amino-1-(2-méthylphényl)benzène ;
du 5-amino-2-(2-hydroxyéthyl)amino-1-(2-nitrophényl)benzène ;
du 5-amino-2-(2-hydroxyéthyl)amino-1-(2-trifluorométhylphényl)benzène ;
du 5-amino-2-(2-hydroxyéthyl)amino-1-(3,5-di(2-hydroxyéthyl)aminophényl)benzène ;
du 5-amino-2-(2-hydroxyéthyl)amino-1-(3,5-diaminophényl)benzène ;
du 5-amino-2-(2-hydroxyéthyl)amino-1-(3,5-dihydroxyphényl)benzène ;
du 5-amino-2-(2-hydroxyéthyl)amino-1-(3,5-diméthoxyphényl)benzène ;
du 5-amino-2-(2-hydroxyéthyl)amino-1-(3,5-diméthylaminophényl)benzène ;
du 5-amino-2-(2-hydroxyéthyl)amino-1-(3-aminophényl)benzène ;
du 5-amino-2-(2-hydroxyéthyl)amino-1-(3-bromophényl)benzène ;
du 5-amino-2-(2-hydroxyéthyl)amino-1-(3-carboxyphényl)benzène ;
du 5-amino-2-(2-hydroxyéthyl)amino-1-(3-chlorophényl)benzène ;
du 5-amino-2-(2-hydroxyéthyl)amino-1-(3-di(2-hydroxyéthyl)aminophényl)benzène ;
du 5-amino-2-(2-hydroxyéthyl)amino-1-(3-diméthylaminophényl)benzène ;
du 5-amino-2-(2-hydroxyéthyl)amino-1-(3-fluorophényl)benzène ;
du 5-amino-2-(2-hydroxyéthyl)amino-1-(3-formylphényl)benzène ;
du 5-amino-2-(2-hydroxyéthyl)amino-1-(3-hydroxy-5-aminophényl)benzène ;
du 5-amino-2-(2-hydroxyéthyl)amino-1-(3-hydroxyphényl)benzène ;
du 5-amino-2-(2-hydroxyéthyl)amino-1-(3-méthoxyphényl)benzène ;
du 5-amino-2-(2-hydroxyéthyl)amino-1-(3-nitrophényl)benzène ;
du 5-amino-2-(2-hydroxyéthyl)amino-1-(3-trifluorométhylphényl)benzène ;
du 5-amino-2-(2-hydroxyéthyl)amino-1-(4-(diméthylamino)phényl)benzène ;
du 5-amino-2-(2-hydroxyéthyl)amino-1-(4-(hydroxyméthyl)phényl)benzène ;
du 5-amino-2-(2-hydroxyéthyl)amino-1-(4-(méthylthio)phényl)benzène ;
du 5-amino-2-(2-hydroxyéthyl)amino-1-(4-(trifluorométhyl)phényl)benzène ;
du 5-amino-2-(2-hydroxyéthyl)amino-1-(4-(triméthylsilyl)phényl)benzène ;
du 5-amino-2-(2-hydroxyéthyl)amino-1-(4-acétylphényl)benzène ;
du 5-amino-2-(2-hydroxyéthyl)amino-1-(4-aminophényl)benzène ;
du 5-amino-2-(2-hydroxyéthyl)amino-1-(4-carboxyphényl)benzène ;
du 5-amino-2-(2-hydroxyéthyl)amino-1-(4-chlorophényl)benzène ;
du 5-amino-2-(2-hydroxyéthyl)amino-1-(4-di(2-hydroxyéthyl)aminophényl)benzène ;
du 5-amino-2-(2-hydroxyéthyl)amino-1-(4-diméthylaminophényl)benzène ;
du 5-amino-2-(2-hydroxyéthyl)amino-1-(4-éthoxyphényl)benzène ;
du 5-amino-2-(2-hydroxyéthyl)amino-1-(4-fluorophényl)benzène ;
du 5-amino-2-(2-hydroxyéthyl)amino-1-(4-formylphényl)benzène ;
du 5-amino-2-(2-hydroxyéthyl)amino-1-(4-hydroxyphényl)benzène ;
du 5-amino-2-(2-hydroxyéthyl)amino-1-(4-méthoxyphényl)benzène ;
du 5-amino-2-(2-hydroxyéthyl)amino-1-(4-méthylphényl)benzène ;
du 5-amino-2-(2-hydroxyéthyl)amino-1-phénylbenzène ;
du 5-amino-2-(2-méthoxyéthyl)amino-1-(2,3,4-triméthoxyphényl)benzène ;
du 5-amino-2-(2-méthoxyéthyl)amino-1-(2,4-diaminophényl)benzène ;
du 5-amino-2-(2-méthoxyéthyl)amino-1-(2,4-dihydroxyphényl)benzène ;
du 5-amino-2-(2-méthoxyéthyl)amino-1-(2,5-diaminophényl)benzène ;
du 5-amino-2-(2-méthoxyéthyl)amino-1-(2,5-diméthoxyphényl)benzène ;
du 5-amino-2-(2-méthoxyéthyl)amino-1-(2,6-diméthoxyphényl)benzène ;
du 5-amino-2-(2-méthoxyéthyl)amino-1-(2-amino-5-hydroxyphényl)benzène ;
du 5-amino-2-(2-méthoxyéthyl)amino-1-(2-aminophényl)benzène ;
du 5-amino-2-(2-méthoxyéthyl)amino-1-(2-hydroxy-4-aminophényl)benzène ;
du 5-amino-2-(2-méthoxyéthyl)amino-1-(2-hydroxy-5-aminophényl)benzène ;
du 5-amino-2-(2-méthoxyéthyl)amino-1-(2-hydroxyphényl)benzène ;
du 5-amino-2-(2-méthoxyéthyl)amino-1-(2-méthoxyphényl)benzène ;
du 5-amino-2-(2-méthoxyéthyl)amino-1-(2-méthylphényl)benzène ;
du 5-amino-2-(2-méthoxyéthyl)amino-1-(3,5-diaminophényl)benzène ;
du 5-amino-2-(2-méthoxyéthyl)amino-1-(3,5-dihydroxyphényl)benzène ;
du 5-amino-2-(2-méthoxyéthyl)amino-1-(3-aminophényl)benzène ;
du 5-amino-2-(2-méthoxyéthyl)amino-1-(3-hydroxy-5-aminophényl)benzène ;
du 5-amino-2-(2-méthoxyéthyl)amino-1-(3-hydroxyphényl)benzène ;
du 5-amino-2-(2-méthoxyéthyl)amino-1-(3-méthoxyphényl)benzène ;
du 5-amino-2-(2-méthoxyéthyl)amino-1-(4-(diméthylamino)phényl)benzène ;
du 5-amino-2-(2-méthoxyéthyl)amino-1-(4-(trifluorométhyl)phényl)benzène ;
du 5-amino-2-(2-méthoxyéthyl)amino-1-(4-aminophényl)benzène ;
du 5-amino-2-(2-méthoxyéthyl)amino-1-(4-carboxyphényl)benzène ;
du 5-amino-2-(2-méthoxyéthyl)amino-1-(4-chlorophényl)benzène ;
du 5-amino-2-(2-méthoxyéthyl)amino-1-(4-hydroxyphényl)benzène ;
du 5-amino-2-(2-méthoxyéthyl)amino-1-(4-méthoxyphényl)benzène ;
du 5-amino-2-(2-méthoxyéthyl)amino-1-(4-méthylphényl)benzène ;
du 5-amino-2-(2-méthoxyéthyl)amino-1-phénylbenzène ;
du 5-amino-2-di(2-hydroxyéthyl)amino-1-(2,3,4-triméthoxyphényl)benzène ;
du 5-amino-2-di(2-hydroxyéthyl)amino-1-(2,3-difluoro-4-heptylphényl)benzène ;
du 5-amino-2-di(2-hydroxyéthyl)amino-1-(2,3-difluorophényl)benzène ;
du 5-amino-2-di(2-hydroxyéthyl)amino-1-(2,4,6-triméthylphényl)benzène ;
du 5-amino-2-di(2-hydroxyéthyl)amino-1-(2,4-diaminophényl)benzène ;
du 5-amino-2-di(2-hydroxyéthyl)amino-1-(2,4-dichlorophényl)benzène ;
du 5-amino-2-di(2-hydroxyéthyl)amino-1-(2,4-dihydroxyphényl)benzène ;
du 5-amino-2-di(2-hydroxyéthyl)amino-1-(2,5-diméthoxyphényl)benzène ;
du 5-amino-2-di(2-hydroxyéthyl)amino-1-(2,6-difluorophényl)benzène ;
du 5-amino-2-di(2-hydroxyéthyl)amino-1-(2,6-diméthoxyphényl)benzène ;
du 5-amino-2-di(2-hydroxyéthyl)amino-1-(2-((bis(1-méthyléthyl)amino)carbonyl)phényl)benzène ;
du 5-amino-2-di(2-hydroxyéthyl)amino-1-(2-((bis(1-méthyléthyl)amino)carbonyl)-3-méthoxyphényl)benzène ;
du 5-amino-2-di(2-hydroxyéthyl)amino-1-(2-(bromométhyl)phényl)benzène ;
du 5-amino-2-di(2-hydroxyéthyl)amino-1-(2-(diéthylamino)carbonyl)phényl)benzène ;
du 5-amino-2-di(2-hydroxyéthyl)amino-1-(2-carboxyphényl)benzène ;
du 5-amino-2-di(2-hydroxyéthyl)amino-1-(2-chlorophényl)benzène ;
du 5-amino-2-di(2-hydroxyéthyl)amino-1-(2-fluorophényl)benzène ;
du 5-amino-2-di(2-hydroxyéthyl)amino-1-(2-formyl-4-méthoxyphényl)benzène ;
du 5-amino-2-di(2-hydroxyéthyl)amino-1-(2-formyl-4-méthylphényl)benzène ;
du 5-amino-2-di(2-hydroxyéthyl)amino-1-(2-formyl-5-méthoxyphényl)benzène ;
du 5-amino-2-di(2-hydroxyéthyl)amino-1-(2-formylphényl)benzène ;
du 5-amino-2-di(2-hydroxyéthyl)amino-1-(2-hydroxy-4-aminophényl)benzène ;
du 5-amino-2-di(2-hydroxyéthyl)amino-1-(2-hydroxyphényl)benzène ;
du 5-amino-2-di(2-hydroxyéthyl)amino-1-(2-méthoxyphényl)benzène ;
du 5-amino-2-di(2-hydroxyéthyl)amino-1-(2-méthylphényl)benzène ;
du 5-amino-2-di(2-hydroxyéthyl)amino-1-(2-nitrophényl)benzène ;
du 5-amino-2-di(2-hydroxyéthyl)amino-1-(2-trifluorométhylphényl)benzène ;
du 5-amino-2-di(2-hydroxyéthyl)amino-1-(3,4-dichlorophényl)benzène ;
du 5-amino-2-di(2-hydroxyéthyl)amino-1-(3,5-diaminophényl)benzène ;
du 5-amino-2-di(2-hydroxyéthyl)amino-1-(3,5-dichlorophényl)benzène ;
du 5-amino-2-di(2-hydroxyéthyl)amino-1-(3,5-dihydroxyphényl)benzène ;
du 5-amino-2-di(2-hydroxyéthyl)amino-1-(3-(acétylamino)phényl)benzène ;
du 5-amino-2-di(2-hydroxyéthyl)amino-1-(3-aminophényl)benzène ;
du 5-amino-2-di(2-hydroxyéthyl)amino-1-(3-bromophényl)benzène ;
du 5-amino-2-di(2-hydroxyéthyl)amino-1-(3-carboxyphényl)benzène ;
du 5-amino-2-di(2-hydroxyéthyl)amino-1-(3-chloro-4-fluorophényl)benzène ;
du 5-amino-2-di(2-hydroxyéthyl)amino-1-(3-chlorophényl)benzène ;
du 5-amino-2-di(2-hydroxyéthyl)amino-1-(3-fluorophényl)benzène ;
du 5-amino-2-di(2-hydroxyéthyl)amino-1-(3-formylphényl)benzène ;
du 5-amino-2-di(2-hydroxyéthyl)amino-1-(3-hydroxyphényl)benzène ;
du 5-amino-2-di(2-hydroxyéthyl)amino-1-(3-méthoxyphényl)benzène ;
du 5-amino-2-di(2-hydroxyéthyl)amino-1-(3-méthylphényl)benzène ;
du 5-amino-2-di(2-hydroxyéthyl)amino-1-(3-nitrophényl)benzène ;
du 5-amino-2-di(2-hydroxyéthyl)amino-1-(3-trifluorométhylphényl)benzène ;
du 5-amino-2-di(2-hydroxyéthyl)amino-1-(4-(bromométhyl)phényl)benzène ;
du 5-amino-2-di(2-hydroxyéthyl)amino-1-(4-(diméthylamino)phényl)benzène ;
du 5-amino-2-di(2-hydroxyéthyl)amino-1-(4-(hydroxyméthyl)phényl)benzène ;
du 5-amino-2-di(2-hydroxyéthyl)amino-1-(4-(méthylthio)phényl)benzène ;
du 5-amino-2-di(2-hydroxyéthyl)amino-1-(4-(trifluorométhyl)phényl)benzène ;
du 5-amino-2-di(2-hydroxyéthyl)amino-1-(4-(triméthylsilyl)phényl)benzène ;
du 5-amino-2-di(2-hydroxyéthyl)amino-1-(4-acétylphényl)benzène ;
du 5-amino-2-di(2-hydroxyéthyl)amino-1-(4-aminophényl)benzène ;
du 5-amino-2-di(2-hydroxyéthyl)amino-1-(4-bromophényl)benzène ;
du 5-amino-2-di(2-hydroxyéthyl)amino-1-(4-carboxyphényl)benzène ;
du 5-amino-2-di(2-hydroxyéthyl)amino-1-(4-chloro-3-méthoxyphényl)benzène ;
du 5-amino-2-di(2-hydroxyéthyl)amino-1-(4-chlorophényl)benzène ;
du 5-amino-2-di(2-hydroxyéthyl)amino-1-(4-éthénylphényl)benzène ;
du 5-amino-2-di(2-hydroxyéthyl)amino-1-(4-éthoxyphényl)benzène ;
du 5-amino-2-di(2-hydroxyéthyl)amino-1-(4-fluorophényl)benzène ;
du 5-amino-2-di(2-hydroxyéthyl)amino-1-(4-formylphényl)benzène ;
du 5-amino-2-di(2-hydroxyéthyl)amino-1-(4-hydroxyphényl)benzène ;
du 5-amino-2-di(2-hydroxyéthyl)amino-1-(4-iodophényl)benzène ;
du 5-amino-2-di(2-hydroxyéthyl)amino-1-(4-méthoxyphényl)benzène ;
du 5-amino-2-di(2-hydroxyéthyl)amino-1-(4-méthyl-3-nitrophényl)benzène ;
du 5-amino-2-di(2-hydroxyéthyl)amino-1-(4-méthylphényl)benzène ;
du 5-amino-2-di(2-hydroxyéthyl)amino-1-(5-bromo-2-méthoxyphényl)benzène ;
du 5-amino-2-di(2-hydroxyéthyl)amino-1-(5-chloro-2-méthoxyphényl)benzène ;
du 5-amino-2-di(2-hydroxyéthyl)amino-1-(5-formyl-2-méthoxyphényl)benzène ;
du 5-amino-2-di(2-méthoxyéthyl)amino-1-(2,3,4-triméthoxyphényl)benzène ;
du 5-amino-2-di(2-méthoxyéthyl)amino-1-(2,3-difluoro-4-heptylphényl)benzène ;
du 5-amino-2-di(2-méthoxyéthyl)amino-1-(2,3-difluorophényl)benzène ;
du 5-amino-2-di(2-méthoxyéthyl)amino-1-(2,4,6-triméthylphényl)benzène ;
du 5-amino-2-di(2-méthoxyéthyl)amino-1-(2,4-diaminophényl)benzène ;
du 5-amino-2-di(2-méthoxyéthyl)amino-1-(2,4-dichlorophényl)benzène ;
du 5-amino-2-di(2-méthoxyéthyl)amino-1-(2,4-dihydroxyphényl)benzène ;
du 5-amino-2-di(2-méthoxyéthyl)amino-1-(2,5-diméthoxyphényl)benzène ;
du 5-amino-2-di(2-méthoxyéthyl)amino-1-(2,6-difluorophényl)benzène ;
du 5-amino-2-di(2-méthoxyéthyl)amino-1-(2,6-diméthoxyphényl)benzène ;
du 5-amino-2-di(2-méthoxyéthyl)amino-1-(2-((bis(1-méthyléthyl)amino)carbonyl)phényl)benzène ;
du 5-amino-2-di(2-méthoxyéthyl)amino-1-(2-((bis(1-méthyléthyl)amino)-carbonyl)-3-méthoxyphényl)benzène ;
du 5-amino-2-di(2-méthoxyéthyl)amino-1-(2-(bromométhyl)phényl)benzène ;
du 5-amino-2-di(2-méthoxyéthyl)amino-1-(2-(diéthylamino)carbonyl)phényl)benzène ;
du 5-amino-2-di(2-méthoxyéthyl)amino-1-(2-carboxyphényl)benzène ;
du 5-amino-2-di(2-méthoxyéthyl)amino-1-(2-chlorophényl)benzène ;
du 5-amino-2-di(2-méthoxyéthyl)amino-1-(2-fluorophényl)benzène ;
du 5-amino-2-di(2-méthoxyéthyl)amino-1-(2-formyl-4-méthoxyphényl)benzène ;
du 5-amino-2-di(2-méthoxyéthyl)amino-1-(2-formyl-4-méthylphényl)benzène ;
du 5-amino-2-di(2-méthoxyéthyl)amino-1-(2-formyl-5-méthoxyphényl)benzène ;
du 5-amino-2-di(2-méthoxyéthyl)amino-1-(2-formylphényl)benzène ;
du 5-amino-2-di(2-méthoxyéthyl)amino-1-(2-hydroxy-4-aminophényl)benzène ;
du 5-amino-2-di(2-méthoxyéthyl)amino-1-(2-hydroxyphényl)benzène ;
du 5-amino-2-di(2-méthoxyéthyl)amino-1-(2-méthoxyphényl)benzène ;
du 5-amino-2-di(2-méthoxyéthyl)amino-1-(2-méthylphényl)benzène ;
du 5-amino-2-di(2-méthoxyéthyl)amino-1-(2-nitrophényl)benzène ;
du 5-amino-2-di(2-méthoxyéthyl)amino-1-(2-trifluorométhylphényl)benzène ;
du 5-amino-2-di(2-méthoxyéthyl)amino-1-(3,4-dichlorophényl)benzène ;
du 5-amino-2-di(2-méthoxyéthyl)amino-1-(3,5-diaminophényl)benzène ;
du 5-amino-2-di(2-méthoxyéthyl)amino-1-(3,5-dichlorophényl)benzène ;
du 5-amino-2-di(2-méthoxyéthyl)amino-1-(3,5-dihydroxyphényl)benzène ;
du 5-amino-2-di(2-méthoxyéthyl)amino-1-(3-(acétylamino)phényl)benzène ;
du 5-amino-2-di(2-méthoxyéthyl)amino-1-(3-aminophényl)benzène ;
du 5-amino-2-di(2-méthoxyéthyl)amino-1-(3-bromophényl)benzène ;
du 5-amino-2-di(2-méthoxyéthyl)amino-1-(3-carboxyphényl)benzène ;
du 5-amino-2-di(2-méthoxyéthyl)amino-1-(3-chloro-4-fluorophényl)benzène ;
du 5-amino-2-di(2-méthoxyéthyl)amino-1-(3-chlorophényl)benzène ;
du 5-amino-2-di(2-méthoxyéthyl)amino-1-(3-fluorophényl)benzène ;
du 5-amino-2-di(2-méthoxyéthyl)amino-1-(3-formylphényl)benzène ;
du 5-amino-2-di(2-méthoxyéthyl)amino-1-(3-hydroxyphényl)benzène ;
du 5-amino-2-di(2-méthoxyéthyl)amino-1-(3-méthoxyphényl)benzène ;
du 5-amino-2-di(2-méthoxyéthyl)amino-1-(3-méthylphényl)benzène ;
du 5-amino-2-di(2-méthoxyéthyl)amino-1-(3-nitrophényl)benzène ;
du 5-amino-2-di(2-méthoxyéthyl)amino-1-(3-trifluorométhylphényl)benzène ;
du 5-amino-2-di(2-méthoxyéthyl)amino-1-(4-(bromométhyl)phényl)benzène ;
du 5-amino-2-di(2-méthoxyéthyl)amino-1-(4-(diméthylamino)phényl)benzène ;
du 5-amino-2-di(2-méthoxyéthyl)amino-1-(4-(hydroxyméthyl)phényl)benzène ;
du 5-amino-2-di(2-méthoxyéthyl)amino-1-(4-(méthylthio)phényl)benzène ;
du 5-amino-2-di(2-méthoxyéthyl)amino-1-(4-(trifluorométhyl)phényl)benzène ;
du 5-amino-2-di(2-méthoxyéthyl)amino-1-(4-(triméthylsilyl)phényl)benzène ;
du 5-amino-2-di(2-méthoxyéthyl)amino-1-(4-acétylphényl)benzène ;
du 5-amino-2-di(2-méthoxyéthyl)amino-1-(4-aminophényl)benzène ;
du 5-amino-2-di(2-méthoxyéthyl)amino-1-(4-bromophényl)benzène ;
du 5-amino-2-di(2-méthoxyéthyl)amino-1-(4-carboxyphényl)benzène ;
du 5-amino-2-di(2-méthoxyéthyl)amino-1-(4-chloro-3-méthoxyphényl)benzène ;
du 5-amino-2-di(2-méthoxyéthyl)amino-1-(4-chlorophényl)benzène ;
du 5-amino-2-di(2-méthoxyéthyl)amino-1-(4-éthénylphényl)benzène ;
du 5-amino-2-di(2-méthoxyéthyl)amino-1-(4-éthoxyphényl)benzène ;
du 5-amino-2-di(2-méthoxyéthyl)amino-1-(4-fluorophényl)benzène ;
du 5-amino-2-di(2-méthoxyéthyl)amino-1-(4-formylphényl)benzène ;
du 5-amino-2-di(2-méthoxyéthyl)amino-1-(4-hydroxyphényl)benzène ;
du 5-amino-2-di(2-méthoxyéthyl)amino-1-(4-iodophényl)benzène ;
du 5-amino-2-di(2-méthoxyéthyl)amino-1-(4-méthoxy-phényl)benzène ;
du 5-amino-2-di(2-méthoxyéthyl)amino-1-(4-méthyl-3-nitrophényl)benzène ;
du 5-amino-2-di(2-méthoxyéthyl)amino-1-(4-méthylphényl)benzène ;
du 5-amino-2-di(2-méthoxyéthyl)amino-1-(5-bromo-2-méthoxyphényl)benzène ;
du 5-amino-2-di(2-méthoxyéthyl)amino-1-(5-chloro-2-méthoxyphényl)benzène ;
du 5-amino-2-di(2-méthoxyéthyl)amino-1-(5-formyl-2-méthoxyphényl)benzène ;
du 5-amino-2-diméthylamino-1-phénylbenzène ;
du 5-amino-2-méthylamino-1-(2,3,4-triméthoxyphényl)benzène ;
du 5-amino-2-méthylamino-1-(2,4-diaminophényl)benzène ;
du 5-amino-2-méthylamino-1-(2,4-dihydroxyphényl)benzène ;
du 5-amino-2-méthylamino-1-(2,5-diaminophényl)benzène ;
du 5-amino-2-méthylamino-1-(2,5-diméthoxyphényl)benzène ;
du 5-amino-2-méthylamino-1-(2,6-diméthoxyphényl)benzène ;
du 5-amino-2-méthylamino-1-(2-amino-5-hydroxyphényl)benzène ;
du 5-amino-2-méthylamino-1-(2-aminophényl)benzène ;
du 5-amino-2-méthylamino-1-(2-hydroxy-4-aminophényl)benzène ;
du 5-amino-2-méthylamino-1-(2-hydroxy-5-aminophényl)benzène ;
du 5-amino-2-méthylamino-1-(2-hydroxyphényl)benzène ;
du 5-amino-2-méthylamino-1-(2-méthoxyphényl)benzène ;
du 5-amino-2-méthylamino-1-(2-méthylphényl)benzène ;
du 5-amino-2-méthylamino-1-(3,5-diaminophényl)benzène ;
du 5-amino-2-méthylamino-1-(3,5-dihydroxyphényl)-benzène ;
du 5-amino-2-méthylamino-1-(3-aminophényl)benzène ;
du 5-amino-2-méthylamino-1-(3-hydroxy-5-aminophényl)benzène ;
du 5-amino-2-méthylamino-1-(3-hydroxyphényl)benzène ;
du 5-amino-2-méthylamino-1-(3-méthoxyphényl)benzène ;
du 5-amino-2-méthylamino-1-(4-(diméthylamino)phényl)benzène ;
du 5-amino-2-méthylamino-1-(4-(trifluorométhyl)phényl)-benzène ;
du 5-amino-2-méthylamino-1-(4-aminophényl)benzène ;
du 5-amino-2-méthylamino-1-(4-carboxyphényl)benzène ;
du 5-amino-2-méthylamino-1-(4-chlorophényl)benzène ;
du 5-amino-2-méthylamino-1-(4-hydroxyphényl)benzène ;
du 5-amino-2-méthylamino-1-(4-méthoxyphényl)benzène ;
du 5-amino-2-méthylamino-1-(4-méthylphényl)benzène et
du 5-amino-2-méthylamino-1-phénylbenzène, ou leurs sels physiologiquement acceptables.

2. Composition selon la revendication 1, **caractérisée en ce que** la substance révélatrice est choisie parmi le 2,5-diamino-1-phénylbenzène ; le 2,5-diamino-1-(3-nitrophényl)benzène ; le 2,5-diamino-1-(4-méthoxyphényl)benzène ; le 2,5-diamino-1-(3-méthoxyphényl)-benzène ; le 2,5-diamino-1-(3-aminophényl)benzène ; le 2,5-diamino-1-(2-méthylphényl)benzène ; le 2,5-diamino-1-(3-méthylphényl)benzène ; le 2,5-diamino-1-(4-méthylphényl)benzène ; le 2,5-diamino-1-(3-chlorophényl)benzène et le 2,5-diamino-1-(4-chlorophényl)benzène, ou leurs sels physiologiquement acceptables.

3. Composition selon la revendication 1 ou 2,
**caractérisée en ce que** le dérivé diaminobenzène de formule (I) est présent en une quantité de 0,005 à 20,0 pour cent en poids.

4. Composition selon l'une quelconque des revendications 1 à 3, **caractérisée en ce qu'**elle comprend, en plus de la substance révélatrice selon la revendication 1 ou 2, en outre au moins une substance révélatrice supplémentaire qui est choisie parmi le 1,4-diaminobenzène, le 2,5-diaminotoluène, l'alcool 2,5-diaminophényléthylique, le 4-aminophénol et ses dérivés, des dérivés de 4,5-diaminopyrazole et des tétra-aminopyrimidines.

5. Composition selon l'une quelconque des revendications 1 à 4, **caractérisée en ce que** les substances révélatrices et les substances couplantes sont présentes dans chaque cas en une quantité totale de 0,005 à 20 pour cent en poids, par rapport à la quantité totale de la composition de teinture d'oxydation.

6. Composition selon l'une quelconque des revendications 1 à 5, **caractérisée en ce qu'**elle comprend en outre au moins un colorant direct.

7. Composition selon l'une quelconque des revendications 1 à 6, **caractérisée en ce qu'**elle présente un pH de 6,8 à 11,5.

8. Composition selon l'une quelconque des revendications 1 à 7, **caractérisée en ce qu'**elle se présente sous forme d'une solution aqueuse ou aqueuse-alcoolique, d'une crème, d'un gel ou d'une émulsion.

9. Composition selon l'une quelconque des revendications 1 à 8, **caractérisée en ce qu'**il s'agit d'une composition de teinture capillaire.

10. Composé qui est choisi parmi le groupe constitué
du 2,5-diamino-1-(4-bromophényl)benzène ;
du 2,5-diamino-1-(4-éthénylphényl)benzène ;
du 2,5-diamino-1-(2,3,4-triméthoxyphényl)benzène ;
du 2,5-diamino-1-(2,4-di(2-hydroxyéthyl)aminophényl)benzène ;
du 2,5-diamino-1-(2,4-diaminophényl)benzène ;
du 2,5-diamino-1-(2,4-dihydroxyphényl)benzène ;
du 2,5-diamino-1-(2,4-diméthylaminophényl)benzène ;
du 2,5-diamino-1-(2,4-méthoxyphényl)benzène ;
du 2,5-diamino-1-(2,5-di(2-hydroxyéthyl)aminophényl)benzène ;
du 2,5-diamino-1-(2,5-diaminophényl)benzène ;
du 2,5-diamino-1-(2,5-dihydroxyphényl)benzène ;
du 2,5-diamino-1-(2,5-diméthoxyphényl)benzène ;
du 2,5-diamino-1-(2,5-diméthylaminophényl)benzène ;
du 2,5-diamino-1-(2,6-di(2-hydroxyéthyl)aminophényl)benzène ;
du 2,5-diamino-1-(2,6-diaminophényl)benzène ;
du 2,5-diamino-1-(2,6-dihydroxyphényl)benzène ;
du 2,5-diamino-1-(2,6-diméthoxyphényl)benzène ;
du 2,5-diamino-1-(2,6-diméthylaminophényl)benzène ;
du 2,5-diamino-1-(2-(bromométhyl)phényl)benzène ;
du 2,5-diamino-1-(2-amino-5-hydroxyphényl)benzène ;
du 2,5-diamino-1-(2-aminophényl)benzène ;
du 2,5-diamino-1-(2-carboxy-phényl)benzène ;
du 2,5-diamino-1-(2-chlorophényl)benzène ;
du 2,5-diamino-1-(2-di(2-hydroxyéthyl)aminophényl)benzène ;
du 2,5-diamino-1-(2-diméthylaminophényl)benzène ;
du 2,5-diamino-1-(2-fluorophényl)benzène ;
du 2,5-diamino-1-(2-formylphényl)benzène ;
du 2,5-diamino-1-(2-hydroxy-4-aminophényl)benzène ;
du 2,5-diamino-1-(2-hydroxy-5-aminophényl)benzène ;
du 2,5-diamino-1-(2-hydroxyphényl)benzène ;
du 2,5-diamino-1-(2-méthoxyphényl)benzène ;
du 2,5-diamino-1-(2-méthylphényl)benzène ;
du 2,5-diamino-1-(2-nitrophényl)benzène ;
du 2,5-diamino-1-(2-trifluorométhylphényl)benzène ;
du 2,5-diamino-1-(3,5-di(2-hydroxyéthyl)aminophényl)benzène ;
du 2,5-diamino-1-(3,5-diaminophényl)benzène ;
du 2,5-diamino-1-(3,5-dihydroxyphényl)benzène ;
du 2,5-diamino-1-(3,5-diméthoxyphényl)benzène ;
du 2,5-diamino-1-(3,5-diméthylaminophényl)benzène ;
du 2,5-diamino-1-(3-aminophényl)benzène ;
du 2,5-diamino-1-(3-bromophényl)benzène ;
du 2,5-diamino-1-(3-carboxy-phényl)benzène ;
du 2,5-diamino-1-(3-chlorophényl)benzène ;
du 2,5-diamino-1-(3-di(2-hydroxyéthyl)aminophényl)benzène ;
du 2,5-diamino-1-(3-diméthylaminophényl)benzène ;
du 2,5-diamino-1-(3-fluorophényl)benzène ;
du 2,5-diamino-1-(3-formylphényl)benzène ;
du 2,5-diamino-1-(3-hydroxy-5-aminophényl)benzène ;
du 2,5-diamino-1-(3-hydroxyphényl)benzène ;
du 2,5-diamino-1-(3-méthoxyphényl)benzène ;
du 2,5-diamino-1-(3-nitrophényl)benzène ;
du 2,5-diamino-1-(3-trifluorométhylphényl)benzène ;
du 2,5-diamino-1-(4-(diméthylamino)phényl)benzène ;
du 2,5-diamino-1-(4-(hydroxyméthyl)phényl)benzène ;
du 2,5-diamino-1-(4-(méthylthio)phényl)benzène ;
du 2,5-diamino-1-(4-(trifluorométhyl)phényl)benzène ;
du 2,5-diamino-1-(4-(triméthylsilyl)phényl)benzène ;
du 2,5-diamino-1-(4-acétylphényl)benzène ;
du 2,5-diamino-1-(4-aminophényl)benzène ;
du 2,5-diamino-1-(4-carboxyphényl)benzène ;
du 2,5-diamino-1-(4-chlorophényl)benzène ;
du 2,5-diamino-1-(4-di(2-hydroxyéthyl)aminophényl)benzène ;
du 2,5-diamino-1-(4-diméthylaminophényl)benzène ;
du 2,5-diamino-1-(4-éthoxyphényl)benzène ;
du 2,5-diamino-1-(4-fluorophényl)benzène ;
du 2,5-diamino-1-(4-formylphényl)benzène ;
du 2,5-diamino-1-(4-hydroxyphényl)benzène ;
du 2,5-diamino-1-(4-méthoxyphényl)benzène ;
du 2,5-diamino-1-(4-méthylphényl)benzène ;
du 2,5-diamino-1-phénylbenzène ;
du 2,5-diamino-4-chloro-1-phénylbenzène ;
du 2,5-diamino-4-méthoxy-1-phénylbenzène ;
du 2,5-diamino-4-méthyl-1-phénylbenzène ;
du 2,5-diamino-1-(2-cyanométhylphényl)benzène ;
du 2,5-diamino-1-(4-éthylphényl)benzène ;
du 2,5-diamino-1-(9-propylphényl)benzène ;
du 2,5-diamino-1-(4-isopropylphényl)benzène ;
du 2,5-diamino-1-(4-butylphényl)benzène ;
du 2,5-diamino-1-(4-tert-butylphényl)benzène ;
du 2,5-diamino-1-(4-pentylphényl)benzène ;
du 2,5-diamino-1-(4-thiométhoxyphényl)benzène :
du 2,5-diamino-1-(2-éthylphényl)benzène ;
du 2,5-diamino-1-(2-fluoro-4-méthylphényl)benzène ;
du 2,5-diamino-1-(2-méthyl-5-fluorophényl)benzène ;
du 2,5-diamino-1-(2-thiométhoxyphényl)benzène ;
du 2,5-diamino-1-(2,3-dichlorophényl)benzène ;
du 2,5-diamino-4-(4'-hydroxybiphényl)benzène ;
du 2,5-diamino-1-(3-éthoxyphényl)benzène ;
du 2,5-diamino-1-(4-(2-pyrrolidin-1-yléthoxy)phényl)benzène ;
du 2,5-diamino-1-(4-(1-hydroxyéthyl)phényl)benzène ;
du 2,5-diamino-1-(2,4-trifluorométhylphényl)benzène ;
du 2,5-diamino-1-(2-fluoro-5-acétylphényl)benzène ;
du 2,5-diamino-1-(3-fluoro-4-méthoxyphényl)benzène ;
du 2,5-diamino-1-(3-acétyl-4-hydroxyphényl)benzène ;
du 2,5-diamino-1-(4-(2-hydroxyéthyl)phényl)benzène ;
du 2,5-diamino-1-(4-(propyl-1-one)phényl)benzène ;
du 2,5-diamino-1-(4-N,N'-diisopropylaminométhylphényl)benzène ;
du 2,5-diamino-1-(3-acétylphényl)benzène ;
du 2,5-diamino-1-(2-(2-hydroxyéthyl)phényl)benzène ;
du 2,5-diamino-1-(3-aminométhylphényl)benzène ;
du 2-amino-5-(2,3-dihydroxypropyl)amino-1-(2,3,4-triméthoxyphényl)benzène ;
du 2-amino-5-(2,3-dihydroxypropyl)amino-1-(2,4-dihydroxyphényl)benzène ;
du 2-amino-5-(2,3-dihydroxypropyl)amino-1-(2,5-diaminophényl)benzène ;
du 2-amino-5-(2,3-dihydroxypropyl)amino-1-(2,6-diméthoxyphényl)benzène ;
du 2-amino-5-(2,3-dihydroxypropyl)amino-1-(2-amino-5-hydroxyphényl)benzène ;
du 2-amino-5-(2,3-dihydroxypropyl)amino-1-(2-aminophényl)benzène ;
du 2-amino-5-(2,3-dihydroxypropyl)amino-1-(2-hydroxy-4-aminophényl)benzène ;
du 2-amino-5-(2,3-dihydroxypropyl)amino-1-(2-hydroxy-5-aminophényl)benzène ;
du 2-amino-5-(2,3-dihydroxypropyl)amino-1-(2-hydroxyphényl)benzène ;
du 2-amino-5-(2,3-dihydroxypropyl)amino-1-(2-méthoxyphényl)benzène ;
du 2-amino-5-(2,3-dihydroxypropyl)amino-1-(2-méthylphényl)benzène ;
du 2-amino-5-(2,3-dihydroxypropyl)amino-1-(3,5-diaminophényl)benzène ;
du 2-amino-5-(2,3-dihydroxypropyl)amino-1-(3,5-dihydroxyphényl)benzène ;
du 2-amino-5-(2,3-dihydroxypropyl)amino-1-(3-aminophényl)benzène ;
du 2-amino-5-(2,3-dihydroxypropyl)amino-1-(3-hydroxy-5-aminophényl)benzène ;
du 2-amino-5-(2,3-dihydroxypropyl)amino-1-(3-hydroxyphényl)benzène ;
du 2-amino-5-(2,3-dihydroxypropyl)amino-1-(3-méthoxyphényl)benzène ;
du 2-amino-5-(2,3-dihydroxypropyl)amino-1-(4-(diméthylamino)phényl)benzène ;
du 2-amino-5-(2,3-dihydroxypropyl)amino-1-(4-(trifluorométhyl)phényl)benzène ;
du 2-amino-5-(2,3-dihydroxypropyl)amino-1-(4-aminophényl)benzène ;
du 2-amino-5-(2,3-dihydroxypropyl)amino-1-(4-carboxyphényl)benzène ;
du 2-amino-5-(2,3-dihydroxypropyl)amino-1-(4-chlorophényl)benzène ;
du 2-amino-5-(2,3-dihydroxypropyl)amino-1-(4-hydroxyphényl)benzène ;
du 2-amino-5-(2,3-dihydroxypropyl)amino-1-(4-méthoxyphényl)benzène ;
du 2-amino-5-(2,3-dihydroxypropyl)amino-1-(4-méthylphényl)benzène ;
du 2-amino-5-(2,3-dihydroxypropyl)amino-1-phénylbenzène ;
du 2-amino-5-(2-hydroxyéthyl)amino-1-(2,3,4-triméthoxyphényl)benzène ;
du 2-amino-5-(2-hydroxyéthyl)amino-1-(2,4-diaminophényl)benzène ;
du 2-amino-5-(2-hydroxyéthyl)amino-1-(2,4-dihydroxyphényl)benzène ;
du 2-amino-5-(2-hydroxyéthyl)amino-1-(2,5-diaminophényl)benzène ;
du 2-amino-5-(2-hydroxyéthyl)amino-1-(2,5-diméthoxyphényl)benzène ;
du 2-amino-5-(2-hydroxyéthyl)amino-1-(2,6-diméthoxyphényl)benzène ;
du 2-amino-5-(2-hydroxyéthyl)amino-1-(2-amino-5-hydroxyphényl)benzène ;
du 2-amino-5-(2-hydroxyéthyl)amino-1-(2-aminophényl)benzène ;
du 2-amino-5-(2-hydroxyéthyl)amino-1-(2-hydroxy-4-aminophényl)benzène ;
du 2-amino-5-(2-hydroxyéthyl)amino-1-(2-hydroxy-5-aminophényl)benzène ;
du 2-amino-5-(2-hydroxyéthyl)amino-1-(2-hydroxyphényl)benzène ;
du 2-amino-5-(2-hydroxyéthyl)amino-1-(2-méthoxyphényl)benzène ;
du 2-amino-5-(2-hydroxyéthyl)amino-1-(2-méthylphényl)benzène ;
du 2-amino-5-(2-hydroxyéthyl)amino-1-(3,5-diaminophényl)benzène ;
du 2-amino-5-(2-hydroxyéthyl)amino-1-(3,5-dihydroxyphényl)benzène ;
du 2-amino-5-(2-hydroxyéthyl)amino-1-(3-aminophényl)benzène ;
du 2-amino-5-(2-hydroxyéthyl)amino-1-(3-hydroxy-5-aminophényl)benzène ;
du 2-amino-5-(2-hydroxyéthyl)amino-1-(3-hydroxyphényl)benzène ;
du 2-amino-5-(2-hydroxyéthyl)amino-1-(3-méthoxyphényl)benzène ;
du 2-amino-5-(2-hydroxyéthyl)amino-1-(4-(diméthylamino)phényl)benzène ;
du 2-amino-5-(2-hydroxyéthyl)amino-1-(4-(trifluorométhyl)phényl)benzène ;
du 2-amino-5-(2-hydroxyéthyl)amino-1-(4-aminophényl)benzène ;
du 2-amino-5-(2-hydroxyéthyl)amino-1-(4-carboxyphényl)benzène ;
du 2-amino-5-(2-hydroxyéthyl)amino-1-(4-chlorophényl)benzène ;
du 2-amino-5-(2-hydroxyéthyl)amino-1-(4-hydroxyphényl)benzène ;
du 2-amino-5-(2-hydroxyéthyl)amino-1-(4-méthoxyphényl)benzène ;
du 2-amino-5-(2-hydroxyéthyl)amino-1-(4-méthylphényl)benzène ;
du 2-amino-5-(2-hydroxyéthyl)amino-1-phénylbenzène ;
du 2-amino-5-(2-hydroxyéthyl)méthylamino-1-phénylbenzène ;
du 2-amino-5-(2-méthoxyéthyl)amino-1-(2,3,4-triméthoxyphényl)benzène ;
du 2-amino-5-(2-méthoxyéthyl)amino-1-(2,4-diaminophényl)benzène ;
du 2-amino-5-(2-méthoxyéthyl)amino-1-(2,4-dihydroxyphényl)benzène ;
du 2-amino-5-(2-méthoxyéthyl)amino-1-(2,5-diaminophényl)benzène ;
du 2-amino-5-(2-méthoxyéthyl)amino-1-(2,5-diméthoxyphényl)benzène ;
du 2-amino-5-(2-méthoxyéthyl)amino-1-(2,6-diméthoxyphényl)benzène ;
du 2-amino-5-(2-méthoxyéthyl)amino-1-(2-amino-5-hydroxyphényl)benzène ;
du 2-amino-5-(2-méthoxyéthyl)amino-1-(2-aminophényl)benzène ;
du 2-amino-5-(2-méthoxyéthyl)amino-1-(2-hydroxy-4-aminophényl)benzène ;
du 2-amino-5-(2-méthoxyéthyl)amino-1-(2-hydroxy-5-aminophényl)benzène ;
du 2-amino-5-(2-méthoxyéthyl)amino-1-(2-hydroxyphényl)benzène ;
du 2-amino-5-(2-méthoxyéthyl)amino-1-(2-méthoxyphényl)benzène ;
du 2-amino-5-(2-méthoxyéthyl)amino-1-(2-méthylphényl)benzène ;
du 2-amino-5-(2-méthoxyéthyl)amino-1-(3,5-diaminophényl)benzène ;
du 2-amino-5-(2-méthoxyéthyl)amino-1-(3,5-dihydroxyphényl)benzène ;
du 2-amino-5-(2-méthoxyéthyl)amino-1-(3-aminophényl)benzène ;
du 2-amino-5-(2-méthoxyéthyl)amino-1-(3-hydroxy-5-aminophényl)benzène ;
du 2-amino-5-(2-méthoxyéthyl)amino-1-(3-hydroxyphényl)benzène ;
du 2-amino-5-(2-méthoxyéthyl)amino-1-(3-méthoxyphényl)benzène ;
du 2-amino-5-(2-méthoxyéthyl)amino-1-(4-(diméthylamino)phényl)benzène ;
du 2-amino-5-(2-méthoxyéthyl)amino-1-(4-(trifluorométhyl)phényl)benzène ;
du 2-amino-5-(2-méthoxyéthyl)amino-1-(4-aminophényl)benzène ;
du 2-amino-5-(2-méthoxyéthyl)amino-1-(4-carboxyphényl)benzène ;
du 2-amino-5-(2-méthoxyéthyl)amino-1-(4-chlorophényl)benzène ;
du 2-amino-5-(2-méthoxyéthyl)amino-1-(4-hydroxyphényl)benzène ;
du 2-amino-5-(2-méthoxyéthyl)amino-1-(4-méthoxyphényl)benzène ;
du 2-amino-5-(2-méthoxyéthyl)amino-1-(4-méthylphényl)benzène ;
du 2-amino-5-(2-méthoxyéthyl)amino-1-phénylbenzène ;
du 2-amino-5-di(2-hydroxyéthyl)amino-1-(2,3,4-triméthoxyphényl)benzène ;
du 2-amino-5-di(2-hydroxyéthyl)amino-1-(2,3-difluoro-4-heptylphényl)benzène ;
du 2-amino-5-di(2-hydroxyéthyl)amino-1-(2,3-difluorophényl)benzène ;
du 2-amino-5-di(2-hydroxyéthyl)amino-1-(2,4,6-triméthylphényl)benzène ;
du 2-amino-5-di(2-hydroxyéthyl)amino-1-(2,4-diaminophényl)benzène ;
du 2-amino-5-di(2-hydroxyéthyl)amino-1-(2,4-dichlorophényl)benzène ;
du 2-amino-5-di(2-hydroxyéthyl)amino-1-(2,4-dihydroxyphényl)benzène ;
du 2-amino-5-di(2-hydroxyéthyl)amino-1-(2,5-diaminophényl)benzène ;
du 2-amino-5-di(2-hydroxyéthyl)amino-1-(2,5-diméthoxyphényl)benzène ;
du 2-amino-5-di(2-hydroxyéthyl)amino-1-(2,6-difluorophényl)benzène ;
du 2-amino-5-di(2-hydroxyéthyl)amino-1-(2,6-diméthoxyphényl)benzène ;
du 2-amino-5-di(2-hydroxyéthyl)amino-1-(2,6-diméthoxyphényl)benzène ;
du 2-amino-5-di(2-hydroxyéthyl)amino-1-(2-((bis(1-méthyléthyl)amino)carbonyl)phényl)benzène ;
du 2-amino-5-di(2-hydroxyéthyl)amino-1-(2-((bis(1-méthyléthyl)amino)carbonyl)-3-méthoxyphényl)benzène ;
du 2-amino-5-di(2-hydroxyéthyl)amino-1-(2-(bromométhyl)phényl)benzène ;
du 2-amino-5-di(2-hydroxyéthyl)amino-1-(2-(diéthylamino)carbonyl)phényl)benzène ;
du 2-amino-5-di(2-hydroxyéthyl)amino-1-(2-amino-5-hydroxyphényl)benzène ;
du 2-amino-5-di(2-hydroxyéthyl)amino-1-(2-aminophényl)benzène ;
du 2-amino-5-di(2-hydroxyéthyl)amino-1-(2-carboxyphényl)benzène ;
du 2-amino-5-di(2-hydroxyéthyl)amino-1-(2-chlorophényl)benzène ;
du 2-amino-5-di(2-hydroxyéthyl)amino-1-(2-fluorophényl)benzène ;
du 2-amino-5-di(2-hydroxyéthyl)amino-1-(2-formyl-4-méthoxyphényl)benzène ;
du 2-amino-5-di(2-hydroxyéthyl)amino-1-(2-formyl-4-méthylphényl)benzène ;
du 2-amino-5-di(2-hydroxyéthyl)amino-1-(2-formyl-5-méthoxyphényl)benzène ;
du 2-amino-5-di(2-hydroxyéthyl)amino-1-(2-formylphényl)benzène ;
du 2-amino-5-di(2-hydroxyéthyl)amino-1-(2-hydroxy-4-aminophényl)benzène ;
du 2-amino-5-di(2-hydroxyéthyl)amino-1-(2-hydroxy-5-aminophényl)benzène ;
du 2-amino-5-di(2-hydroxyéthyl)amino-1-(2-hydroxyphényl)benzène ;
du 2-amino-5-di(2-hydroxyéthyl)amino-1-(2-méthoxyphényl)benzène ;
du 2-amino-5-di(2-hydroxyéthyl)amino-1-(2-méthylphényl)benzène ;
du 2-amino-5-di(2-hydroxyéthyl)amino-1-(2-nitrophényl)benzène ;
du 2-amino-5-di(2-hydroxyéthyl)amino-1-(2-trifluorométhylphényl)benzène ;
du 2-amino-5-di(2-hydroxyéthyl)amino-1-(3,4-dichlorophényl)benzène ;
du 2-amino-5-di(2-hydroxyéthyl)amino-1-(3,5-diaminophényl)benzène ;
du 2-amino-5-di(2-hydroxyéthyl)amino-1-(3,5-dichlorophényl)benzène ;
du 2-amino-5-di(2-hydroxyéthyl)amino-1-(3,5-dihydroxyphényl)benzène ;
du 2-amino-5-di(2-hydroxyéthyl)amino-1-(3-(acétylamino)phényl)benzène ;
du 2-amino-5-di(2-hydroxyéthyl)amino-1-(3-aminophényl)benzène ;
du 2-amino-5-di(2-hydroxyéthyl)amino-1-(3-bromophényl)benzène ;
du 2-amino-5-di(2-hydroxyéthyl)amino-1-(3-carboxyphényl)benzène ;
du 2-amino-5-di(2-hydroxyéthyl)amino-1-(3-chloro-4-fluorophényl)benzène ;
du 2-amino-5-di(2-hydroxyéthyl)amino-1-(3-chlorophényl)benzène ;
du 2-amino-5-di(2-hydroxyéthyl)amino-1(3-fluorophényl)benzène ;
du 2-amino-5-di(2-hydroxyéthyl)amino-1-(3-formylphényl)benzène ;
du 2-amino-5-di(2-hydroxyéthyl)amino-1-(3-hydroxy-5-aminophényl)benzène ;
du 2-amino-5-di(2-hydroxyéthyl)amino-1-(3-hydroxyphényl)benzène ;
du 2-amino-5-di(2-hydroxyéthyl)amino-1-(3-hydroxyphényl)benzène ;
du 2-amino-5-di(2-hydroxyéthyl)amino-1-(3-méthoxyphényl)benzène ;
du 2-amino-5-di(2-hydroxyéthyl)amino-1-(3-méthylphényl)benzène ;
du 2-amino-5-di(2-hydroxyéthyl)amino-1-(3-nitrophényl)benzène ;
du 2-amino-5-di(2-hydroxyéthyl)amino-1-(3-trifluorométhylphényl)benzène ;
du 2-amino-5-di(2-hydroxyéthyl)amino-1-(4-(bromométhyl)phényl)benzène ;
du 2-amino-5-di(2-hydroxyéthyl)amino-1-(4-(diméthylamino)phényl)benzène ;
du 2-amino-5-di(2-hydroxyéthyl)amino-1-(4-(hydroxyméthyl)phényl)benzène ;
du 2-amino-5-di(2-hydroxyéthyl)amino-1-(4-(méthylthio)phényl)benzène ;
du 2-amino-5-di(2-hydroxyéthyl)amino-1-(4-(trifluorométhyl)phényl)benzène ;
du 2-amino-5-di(2-hydroxyéthyl)amino-1-(4-(triméthylsilyl)phényl)benzène ;
du 2-amino-5-di(2-hydroxyéthyl)amino-1-(4-acétylphényl)benzène ;
du 2-amino-5-di(2-hydroxyéthyl)amino-1-(4-aminophényl)benzène ;
du 2-amino-5-di(2-hydroxyéthyl)amino-1-(4-bromophényl)benzène ;
du 2-amino-5-di(2-hydroxyéthyl)amino-1-(4-carboxyphényl)benzène ;
du 2-amino-5-di(2-hydroxyéthyl)amino-1-(4-chloro-3-méthoxyphényl)benzène ;
du 2-amino-5-di(2-hydroxyéthyl)amino-1-(4-chlorophényl)benzène ;
du 2-amino-5-di(2-hydroxyéthyl)amino-1-(4-éthénylphényl)benzène ;
du 2-amino-5-di(2-hydroxyéthyl)amino-1-(4-fluorophényl)benzène ;
du 2-amino-5-di(2-hydroxyéthyl)amino-1-(4-formylphényl)benzène ;
du 2-amino-5-di(2-hydroxyéthyl)amino-1-(4-hydroxyphényl)benzène ;
du 2-amino-5-di(2-hydroxyéthyl)amino-1-(4-iodophényl)benzène ;
du 2-amino-5-di(2-hydroxyéthyl)amino-1-(4-méthoxyphényl)benzène ;
du 2-amino-5-di(2-hydroxyéthyl)amino-1-(4-méthyl-3-nitrophényl)benzène ;
du 2-amino-5-di(2-hydroxyéthyl)amino-1-(4-méthylphényl)benzène ;
du 2-amino-5-di(2-hydroxyéthyl)amino-1-(5-bromo-2-méthoxyphényl)benzène ;
du 2-amino-5-di(2-hydroxyéthyl)amino-1-(5-chloro-2-méthoxyphényl)benzène ;
du 2-amino-5-di(2-hydroxyéthyl)amino-1-(5-formyl-2-méthoxyphényl)benzène ;
du 2-amino-5-di(2-hydroxyéthyl)amino-1-phénylbenzène ;
du 2-amino-5-di(2-méthoxyéthyl)amino-1-(2,3,4-triméthoxyphényl)benzène ;
du 2-amino-5-di(2-méthoxyéthyl)amino-1-(2,3-difluoro-4-heptylphényl)benzène ;
du 2-amino-5-di(2-méthoxyéthyl)amino-1-(2,3-difluorophényl)benzène ;
du 2-amino-5-di(2-méthoxyéthyl)amino-1-(2,4,6-triméthylphényl)benzène ;
du 2-amino-5-di(2-méthoxyéthyl)amino-1-(2,4-diaminophényl)benzène ;
du 2-amino-5-di(2-méthoxyéthyl)amino-1-(2,4-dichlorophényl)benzène ;
du 2-amino-5-di(2-méthoxyéthyl)amino-1-(2,4-dihydroxyphényl)benzène ;
du 2-amino-5-di(2-méthoxyéthyl)amino-1-(2,5-diméthoxyphényl)benzène ;
du 2-amino-5-di(2-méthoxyéthyl)amino-1-(2,6-difluorophényl)benzène ;
du 2-amino-5-di(2-méthoxyéthyl)amino-1-(2,6-diméthoxyphényl)benzène ;
du 2-amino-5-di(2-méthoxyéthyl)amino-1-(2-((bis(1-méthyléthyl)amino)carbonyl)phényl)benzène ;
du 2-amino-5-di(2-méthoxyéthyl)amino-1-(2-(bromométhyl)phényl)benzène ;
du 2-amino-5-di(2-méthoxyéthyl)amino-1-(2-(diéthylamino)carbonyl)phényl)benzène ;
du 2-amino-5-di(2-méthoxyéthyl)amino-1-(2-carboxyphényl)benzène ;
du 2-amino-5-di(2-méthoxyéthyl)amino-1-(2-chlorophényl)benzène ;
du 2-amino-5-di(2-méthoxyéthyl)amino-1-(2-fluorophényl)benzène ;
du 2-amino-5-di(2-méthoxyéthyl)amino-1-(2-formyl-4-méthoxyphényl)benzène ;
du 2-amino-5-di(2-méthoxyéthyl)amino-1-(2-formyl-4-méthylphényl)benzène ;
du 2-amino-5-di(2-méthoxyéthyl)amino-1-(2-formyl-5-méthoxyphényl)benzène ;
du 2-amino-5-di(2-méthoxyéthyl)amino-1-(2-formylphényl)benzène ;
du 2-amino-5-di(2-méthoxyéthyl)amino-1-(2-hydroxy-4-aminophényl)benzène ;
du 2-amino-5-di(2-méthoxyéthyl)amino-1-(2-hydroxyphényl)benzène ;
du 2-amino-5-di(2-méthoxyéthyl)amino-1-(2-méthoxyphényl)benzène ;
du 2-amino-5-di(2-méthoxyéthyl)amino-1-(2-méthylphényl)benzène ;
du 2-amino-5-di(2-méthoxyéthyl)amino-1-(2-nitrophényl)benzène ;
du 2-amino-5-di(2-méthoxyéthyl)amino-1-(2-trifluorométhylphényl)benzène ;
du 2-amino-5-di(2-méthoxyéthyl)amino-1-(3,4-dichlorophényl)benzène ;
du 2-amino-5-di(2-méthoxyéthyl)amino-1-(3,5-diaminophényl)benzène ;
du 2-amino-5-di(2-méthoxyéthyl)amino-1-(3,5-dichlorophényl)benzène ;
du 2-amino-5-di(2-méthoxyéthyl)amino-1-(3,5-dihydroxyphényl)benzène ;
du 2-amino-5-di(2-méthoxyéthyl)amino-1-(3-(acétylamino)phényl)benzène ;
du 2-amino-5-di(2-méthoxyéthyl)amino-1-(3-aminophényl)benzène ;
du 2-amino-5-di(2-méthoxyéthyl)amino-1-(3-bromophényl)benzène ;
du 2-amino-5-di(2-méthoxyéthyl)amino-1-(3-carboxyphényl)benzène ;
du 2-amino-5-di(2-méthoxyéthyl)amino-1-(3-chloro-4-fluorophényl)benzène ;
du 2-amino-5-di(2-méthoxyéthyl)amino-1-(3-chlorophényl)benzène ;
du 2-amino-5-di(2-méthoxyéthyl)amino-1-(3-fluorophényl)benzène ;
du 2-amino-5-di(2-méthoxyéthyl)amino-1-(3-formylphényl)benzène ;
du 2-amino-5-di(2-méthoxyéthyl)amino-1-(3-hydroxyphényl)benzène ;
du 2-amino-5-di(2-méthoxyéthyl)amino-1-(3-méthoxyphényl)benzène ;
du 2-amino-5-di(2-méthoxyéthyl)amino-1-(3-méthylphényl)benzène ;
du 2-amino-5-di(2-méthoxyéthyl)amino-1-(3-nitrophényl)benzène ;
du 2-amino-5-di(2-méthoxyéthyl)amino-1-(3-trifluorométhylphényl)benzène ;
du 2-amino-5-di(2-méthoxyéthyl)amino-1-(4-(bromométhyl)phényl)benzène ;
du 2-amino-5-di(2-méthoxyéthyl)amino-1-(4-(diméthylamino)phényl)benzène ;
du 2-amino-5-di(2-méthoxyéthyl)amino-1-(4-(hydroxyméthyl)phényl)benzène ;
du 2-amino-5-di(2-méthoxyéthyl)amino-1-(4-(méthylthio)phényl)benzène ;
du 2-amino-5-di(2-méthoxyéthyl)amino-1-(4-(trifluorométhyl)phényl)benzène ;
du 2-amino-5-di(2-méthoxyéthyl)amino-1-(4-(triméthylsilyl)phényl)benzène ;
du 2-amino-5-di(2-méthoxyéthyl)amino-1-(4-acétylphényl)benzène ;
du 2-amino-5-di(2-méthoxyéthyl)amino-1-(4-aminophényl)benzène ;
du 2-amino-5-di(2-méthoxyéthyl)amino-1-(4-bromophényl)benzène ;
du 2-amino-5-di(2-méthoxyéthyl)amino-1-(4-carboxyphényl)benzène ;
du 2-amino-5-di(2-méthoxyéthyl)amino-1-(4-chloro-3-méthoxyphényl)benzène ;
du 2-amino-5-di(2-méthoxyéthyl)amino-1-(4-chlorophényl)benzène ;
du 2-amino-5-di(2-méthoxyéthyl)amino-1-(4-éthénylphényl)benzène ;
du 2-amino-5-di(2-méthoxyéthyl)amino-1-(4-éthoxyphényl)benzène ;
du 2-amino-5-di(2-méthoxyéthyl)amino-1-(4-fluorophényl)benzène ;
du 2-amino-5-di(2-méthoxyéthyl)amino-1-(4-formylphényl)benzène ;
du 2-amino-5-di(2-méthoxyéthyl)amino-1-(4-hydroxyphényl)benzène ;
du 2-amino-5-di(2-méthoxyéthyl)amino-1-(4-iodophényl)benzène ;
du 2-amino-5-di(2-méthoxyéthyl)amino-1-(4-méthoxyphényl)benzène ;
du 2-amino-5-di(2-méthoxyéthyl)amino-1-(4-méthyl-3-nitrophényl)benzène ;
du 2-amino-5-di(2-méthoxyéthyl)amino-1-(4-méthylphényl)benzène ;
du 2-amino-5-di(2-méthoxyéthyl)amino-1-(5-bromo-2-méthoxyphényl)benzène ;
du 2-amino-5-di(2-méthoxyéthyl)amino-1-(5-chloro-2-méthoxyphényl)benzène ;
du 2-amino-5-di(2-méthoxyéthyl)amino-1-(5-formyl-2-méthoxyphényl)benzène ;
du 2-amino-5-diméthylamino-1-phénylbenzène ;
du 2-amino-5-méthylamino-1-(2,3,4-triméthoxyphényl)benzène ;
du 2-amino-5-méthylamino-1-(2,4-diaminophényl)benzène ; du 2-amino-5-méthylamino-1-(2,4-dihydroxyphényl)benzène ;
du 2-amino-5-méthylamino-1-(2,5-diaminophényl)benzène ;
du 2-amino-5-méthylamino-1-(2,5-diméthoxyphényl)benzène ;
du 2-amino-5-méthylamino-1-(2,6-diméthoxyphényl)benzène ;
du 2-amino-5-méthylamino-1-(2-amino-5-hydroxyphényl)benzène ;
du 2-amino-5-méthylamino-1-(2-aminophényl)benzène ;
du 2-amino-5-méthylamino-1-(2-hydroxy-4-aminophényl)benzène ;
du 2-amino-5-méthylamino-1-(2-hydroxy-5-aminophényl)benzène ;
du 2-amino-5-méthylamino-1-(2-hydroxyphényl)benzène ;
du 2-amino-5-méthylamino-1-(2-méthoxyphényl)benzène ;
du 2-amino-5-méthylamino-1-(2-méthylphényl)benzène ;
du 2-amino-5-méthylamino-1-(3,5-diaminophényl)benzène ;
du 2-amino-5-méthylamino-1-(3,5-dihydroxyphényl)benzène ;
du 2-amino-5-méthylamino-1-(3-aminophényl)benzène ;
du 2-amino-5-méthylamino-1-(3-hydroxy-5-aminophényl)benzène ;
du 2-amino-5-méthylamino-1-(3-hydroxyphényl)benzène ;
du 2-amino-5-méthylamino-1-(3-méthoxyphényl)benzène ;
du 2-amino-5-méthylamino-1-(4-(diméthylamino)phényl)benzène ;
du 2-amino-5-méthylamino-1-(4-(trifluorométhyl)phényl)benzène ;
du 2-amino-5-méthylamino-1-(4-aminophényl)benzène ;
du 2-amino-5-méthylamino-1-(4-carboxyphényl)benzène ;
du 2-amino-5-méthylamino-1-(4-chlorophényl)benzène ;
du 2-amino-5-méthylamino-1-(4-hydroxyphényl)benzène ;
du 2-amino-5-méthylamino-1-(4-méthoxyphényl)benzène ;
du 2-amino-5-méthylamino-1-(4-méthylphényl)benzène ;
du 2-amino-5-méthylamino-1-phénylbenzène ;
du 5-amino-2-(2,3-dihydroxypropyl)amino-1-(4-bromophényl)benzène ;
du 5-amino-2-(2,3-dihydroxypropyl)amino-1-(4-éthénylphényl)benzène ;
du 5-amino-2-(2,3-dihydroxypropyl)amino-1-(2,3,4-triméthoxyphényl)benzène ;
du 5-amino-2-(2,3-dihydroxypropyl)amino-1-(2,4-di(2-hydroxyéthyl)aminophényl)benzène ;
du 5-amino-2-(2,3-dihydroxypropyl)amino-1-(2,4-diaminophényl)benzène ;
du 5-amino-2-(2,3-dihydroxypropyl)amino-1-(2,4-dihydroxyphényl)benzène ;
du 5-amino-2-(2,3-dihydroxypropyl)amino-1-(2,4-diméthylaminophényl)benzène ;
du 5-amino-2-(2,3-dihydroxypropyl)amino-1-(2,4-méthoxyphényl)benzène ;
du 5-amino-2-(2,3-dihydroxypropyl)amino-1-(2,5-di(2-hydroxyéthyl)aminophényl)benzène ;
du 5-amino-2-(2,3-dihydroxypropyl)amino-1-(2,5-diaminophényl)benzène ;
du 5-amino-2-(2,3-dihydroxypropyl)amino-1-(2,5-dihydroxyphényl)benzène ;
du 5-amino-2-(2,3-dihydroxypropyl)amino-1-(2,5-diméthoxyphényl)benzène ;
du 5-amino-2-(2,3-dihydroxypropyl)amino-1-(2,5-diméthylaminophényl)benzène ;
du 5-amino-2-(2,3-dihydroxypropyl)amino-1-(2,6-di(2-hydroxyéthyl)aminophényl)benzène ;
du 5-amino-2-(2,3-dihydroxypropyl)amino-1-(2,6-diaminophényl)benzène ;
du 5-amino-2-(2,3-dihydroxypropyl)amino-1-(2,6-dihydroxyphényl)benzène ;
du 5-amino-2-(2,3-dihydroxypropyl)amino-1-(2,6-diméthoxyphényl)benzène ;
du 5-amino-2-(2,3-dihydroxypropyl)amino-1-(2,6-diméthylaminophényl)benzène ;
du 5-amino-2-(2,3-dihydroxypropyl)amino-1-(2-(bromométhyl)phényl)benzène ;
du 5-amino-2-(2,3-dihydroxypropyl)amino-1-(2-amino-5-hydroxyphényl)benzène ;
du 5-amino-2-(2,3-dihydroxypropyl)amino-1-(2-aminophényl)benzène ;
du 5-amino-2-(2,3-dihydroxypropyl)amino-1-(2-carboxyphényl)benzène ;
du 5-amino-2-(2,3-dihydroxypropyl)amino-1-(2-chlorophényl)benzène ;
du 5-amino-2-(2,3-dihydroxypropyl)amino-1-(2-di(2-hydroxyéthyl)aminophényl)benzène ;
du 5-amino-2-(2,3-dihydroxypropyl)amino-1-(2-diméthylaminophényl)benzène ;
du 5-amino-2-(2,3-dihydroxypropyl)amino-1-(2-fluorophényl)benzène ;
du 5-amino-2-(2,3-dihydroxypropyl)amino-1-(2-formylphényl)benzène ;
du 5-amino-2-(2,3-dihydroxypropyl)amino-1-(2-hydroxy-4-aminophényl)benzène ;
du 5-amino-2-(2,3-dihydroxypropyl)amino-1-(2-hydroxy-5-aminophényl)benzène ;
du 5-amino-2-(2,3-dihydroxypropyl)amino-1-(2-hydroxyphényl)benzène ;
du 5-amino-2-(2,3-dihydroxypropyl)amino-1-(2-méthoxyphényl)benzène ;
du 5-amino-2-(2,3-dihydroxypropyl)amino-1-(2-méthylphényl)benzène ;
du 5-amino-2-(2,3-dihydroxypropyl)amino-1-(2-nitrophényl)benzène ;
du 5-amino-2-(2,3-dihydroxypropyl)amino-1-(2-trifluorométhylphényl)benzène ;
du 5-amino-2-(2,3-dihydroxypropyl)amino-1-(3,5-di(2-hydroxyéthyl)aminophényl)benzène ;
du 5-amino-2-(2,3-dihydroxypropyl)amino-1-(3,5-diaminophényl)benzène ;
du 5-amino-2-(2,3-dihydroxypropyl)amino-1-(3,5-dihydroxyphényl)benzène ;
du 5-amino-2-(2,3-dihydroxypropyl)amino-1-(3,5-diméthoxyphényl)benzène ;
du 5-amino-2-(2,3-dihydroxypropyl)amino-1-(3,5-diméthylaminophényl)benzène ;
du 5-amino-2-(2,3-dihydroxypropyl)amino-1-(3-aminophényl)benzène ;
du 5-amino-2-(2,3-dihydroxypropyl)amino-1-(3-bromophényl)benzène ;
du 5-amino-2-(2,3-dihydroxypropyl)amino-1-(3-carboxyphényl)benzène ;
du 5-amino-2-(2,3-dihydroxypropyl)amino-1-(3-chlorophényl)benzène ;
du 5-amino-2-(2,3-dihydroxypropyl)amino-1-(3-di(2-hydroxyéthyl)aminophényl)benzène ;
du 5-amino-2-(2,3-dihydroxypropyl)amino-1-(3-diméthylaminophényl)benzène ;
du 5-amino-2-(2,3-dihydroxypropyl)amino-1-(3-fluorophényl)benzène ;
du 5-amino-2-(2,3-dihydroxypropyl)amino-1-(3-formylphényl)benzène ;
du 5-amino-2-(2,3-dihydroxypropyl)amino-1-(3-hydroxy-5-aminophényl)benzène ;
du 5-amino-2-(2,3-dihydroxypropyl)amino-1-(3-hydroxyphényl)benzène ;
du 5-amino-2-(2,3-dihydroxypropyl)amino-1-(3-méthoxyphényl)benzène ;
du 5-amino-2-(2,3-dihydroxypropyl)amino-1-(3-nitrophényl)benzène ;
du 5-amino-2-(2,3-dihydroxypropyl)amino-1-(3-trifluorométhylphényl)benzène ;
du 5-amino-2-(2,3-dihydroxypropyl)amino-1-(4-(diméthylamino)phényl)benzène ;
du 5-amino-2-(2,3-dihydroxypropyl)amino-1-(4-(hydroxyméthyl)phényl)benzène ;
du 5-amino-2-(2,3-dihydroxypropyl)amino-1-(4-(méthylthio)phényl)benzène ;
du 5-amino-2-(2,3-dihydroxypropyl)amino-1-(4-(trifluorométhyl)phényl)benzène ;
du 5-amino-2-(2,3-dihydroxypropyl)amino-1-(4-(triméthylsilyl)phényl)benzène ;
du 5-amino-2-(2,3-dihydroxypropyl)amino-1-(4-acétylphényl)benzène ;
du 5-amino-2-(2,3-dihydroxypropyl)amino-1-(4-aminophényl)benzène ;
du 5-amino-2-(2,3-dihydroxypropyl)amino-1-(4-carboxyphényl)benzène ;
du 5-amino-2-(2,3-dihydroxypropyl)amino-1-(4-chlorophényl)benzène ;
du 5-amino-2-(2,3-dihydroxypropyl)amino-1-(4-di(2-hydroxyéthyl)aminophényl)benzène ;
du 5-amino-2-(2,3-dihydroxypropyl)amino-1-(4-diméthylaminophényl)benzène ;
du 5-amino-2-(2,3-dihydroxypropyl)amino-1-(4-éthoxyphényl)benzène ;
du 5-amino-2-(2,3-dihydroxypropyl)amino-1-(4-fluorophényl)benzène ;
du 5-amino-2-(2,3-dihydroxypropyl)amino-1-(4-formylphényl)benzène ;
du 5-amino-2-(2,3-dihydroxypropyl)amino-1-(4-hydroxyphényl)benzène ;
du 5-amino-2-(2,3-dihydroxypropyl)amino-1-(4-méthoxyphényl)benzène ;
du 5-amino-2-(2,3-dihydroxypropyl)amino-1-(4-méthylphényl)benzène ;
du 5-amino-2-(2,3-dihydroxypropyl)amino-1-phénylbenzène ;
du 5-amino-2-(2-hydroxyéthyl)amino-1-(4-bromophényl)benzène ;
du 5-amino-2-(2-hydroxyéthyl)amino-1-(4-éthénylphényl)benzène ;
du 5-amino-2-(2-hydroxyéthyl)amino-1-(2,3,4-triméthoxyphényl)benzène ;
du 5-amino-2-(2-hydroxyéthyl)amino-1-(2,4-di(2-hydroxyéthyl)aminophényl)benzène ;
du 5-amino-2-(2-hydroxyéthyl)amino-1-(2,4-diaminophényl)benzène ;
du 5-amino-2-(2-hydroxyéthyl)amino-1-(2,4-dihydroxyphényl)benzène ;
du 5-amino-2-(2-hydroxyéthyl)amino-1-(2,4-diméthylaminophényl)benzène ;
du 5-amino-2-(2-hydroxyéthyl)amino-1-(2,4-méthoxyphényl)benzène ;
du 5-amino-2-(2-hydroxyéthyl)amino-1-(2,5-di(2-hydroxyéthyl)aminophényl)benzène ;
du 5-amino-2-(2-hydroxyéthyl)amino-1-(2,5-diaminophényl)benzène ;
du 5-amino-2-(2-hydroxyéthyl)amino-1-(2,5-dihydroxyphényl)benzène ;
du 5-amino-2-(2-hydroxyéthyl)amino-1-(2,5-diméthoxyphényl)benzène ;
du 5-amino-2-(2-hydroxyéthyl)amino-1-(2,5-diméthylaminophényl)benzène ;
du 5-amino-2-(2-hydroxyéthyl)amino-1-(2,6-di(2-hydroxyéthyl)aminophényl)benzène ;
du 5-amino-2-(2-hydroxyéthyl)amino-1-(2,6-diaminophényl)benzène ;
du 5-amino-2-(2-hydroxyéthyl)amino-1-(2,6-dihydroxyphényl)benzène ;
du 5-amino-2-(2-hydroxyéthyl)amino-1-(2,6-diméthoxyphényl)benzène ;
du 5-amino-2-(2-hydroxyéthyl)amino-1-(2,6-diméthylaminophényl)benzène ;
du 5-amino-2-(2-hydroxyéthyl)amino-1-(2-(bromométhyl)phényl)benzène ;
du 5-amino-2-(2-hydroxyéthyl)amino-1-(2-amino-5-hydroxyphényl)benzène ;
du 5-amino-2-(2-hydroxyéthyl)amino-1-(2-aminophényl)benzène ;
du 5-amino-2-(2-hydroxyéthyl)amino-1-(2-carboxyphényl)benzène ;
du 5-amino-2-(2-hydroxyéthyl)amino-1-(2-chlorophényl)benzène ;
du 5-amino-2-(2-hydroxyéthyl)amino-1-(2-di(2-hydroxyéthyl)aminophényl)benzène ;
du 5-amino-2-(2-hydroxyéthyl)amino-1-(2-diméthylaminophényl)benzène ;
du 5-amino-2-(2-hydroxyéthyl)amino-1-(2-fluorophényl)benzène ;
du 5-amino-2-(2-hydroxyéthyl)amino-1-(2-formylphényl)benzène ;
du 5-amino-2-(2-hydroxyéthyl)amino-1-(2-hydroxy-4-aminophényl)benzène ;
du 5-amino-2-(2-hydroxyéthyl)amino-1-(2-hydroxy-5-aminophényl)benzène ;
du 5-amino-2-(2-hydroxyéthyl)amino-1-(2-hydroxyphényl)benzène ;
du 5-amino-2-(2-hydroxyéthyl)amino-1-(2-méthoxyphényl)benzène ;
du 5-amino-2-(2-hydroxyéthyl)amino-1-(2-méthylphényl)benzène ;
du 5-amino-2-(2-hydroxyéthyl)amino-1-(2-nitrophényl)benzène ;
du 5-amino-2-(2-hydroxyéthyl)amino-1-(2-trifluorométhylphényl)benzène ;
du 5-amino-2-(2-hydroxyéthyl)amino-1-(3,5-di(2-hydroxyéthyl)aminophényl)benzène ;
du 5-amino-2-(2-hydroxyéthyl)amino-1-(3,5-diaminophényl)benzène ;
du 5-amino-2-(2-hydroxyéthyl)amino-1-(3,5-dihydroxyphényl)benzène ;
du 5-amino-2-(2-hydroxyéthyl)amino-1-(3,5-diméthoxyphényl)benzène ;
du 5-amino-2-(2-hydroxyéthyl)amino-1-(3,5-diméthylaminophényl)benzène ;
du 5-amino-2-(2-hydroxyéthyl)amino-1-(3-aminophényl)benzène ;
du 5-amino-2-(2-hydroxyéthyl)amino-1-(3-bromophényl)benzène ;
du 5-amino-2-(2-hydroxyéthyl)amino-1-(3-carboxyphényl)benzène ;
du 5-amino-2-(2-hydroxyéthyl)amino-1-(3-chlorophényl)benzène ;
du 5-amino-2-(2-hydroxyéthyl)amino-1-(3-di(2-hydroxyéthyl)aminophényl)benzène ;
du 5-amino-2-(2-hydroxyéthyl)amino-1-(3-diméthylaminophényl)benzène ;
du 5-amino-2-(2-hydroxyéthyl)amino-1-(3-fluorophényl)benzène ;
du 5-amino-2-(2-hydroxyéthyl)amino-1-(3-formylphényl)benzène ;
du 5-amino-2-(2-hydroxyéthyl)amino-1-(3-hydroxy-5-aminophényl)benzène ;
du 5-amino-2-(2-hydroxyéthyl)amino-1-(3-hydroxyphényl)benzène ;
du 5-amino-2-(2-hydroxyéthyl)amino-1-(3-méthoxyphényl)benzène ;
du 5-amino-2-(2-hydroxyéthyl)amino-1-(3-nitrophényl)benzène ;
du 5-amino-2-(2-hydroxyéthyl)amino-1-(3-trifluorométhylphényl)benzène ;
du 5-amino-2-(2-hydroxyéthyl)amino-1-(4-(diméthylamino)phényl)benzène ;
du 5-amino-2-(2-hydroxyéthyl)amino-1-(4-(hydroxyméthyl)phényl)benzène ;
du 5-amino-2-(2-hydroxyéthyl)amino-1-(4-(méthylthio)phényl)benzène ;
du 5-amino-2-(2-hydroxyéthyl)amino-1-(4-(trifluorométhyl)phényl)benzène ;
du 5-amino-2-(2-hydroxyéthyl)amino-1-(4-(triméthylsilyl)phényl)benzène ;
du 5-amino-2-(2-hydroxyéthyl)amino-1-(4-acétylphényl)benzène ;
du 5-amino-2-(2-hydroxyéthyl)amino-1-(4-aminophényl)benzène ;
du 5-amino-2-(2-hydroxyéthyl)amino-1-(4-carboxyphényl)benzène ;
du 5-amino-2-(2-hydroxyéthyl)amino-1-(4-chlorophényl)benzène ;
du 5-amino-2-(2-hydroxyéthyl)amino-1-(4-di(2-hydroxyéthyl)aminophényl)benzène ;
du 5-amino-2-(2-hydroxyéthyl)amino-1-(4-diméthylaminophényl)benzène ;
du 5-amino-2-(2-hydroxyéthyl)amino-1-(4-éthoxyphényl)benzène ;
du 5-amino-2-(2-hydroxyéthyl)amino-1-(4-fluorophényl)benzène ;
du 5-amino-2-(2-hydroxyéthyl)amino-1-(4-formylphényl)benzène ;
du 5-amino-2-(2-hydroxyéthyl)amino-1-(4-hydroxyphényl)benzène ;
du 5-amino-2-(2-hydroxyéthyl)amino-1-(4-méthoxyphényl)benzène ;
du 5-amino-2-(2-hydroxyéthyl)amino-1-(4-méthylphényl)benzène ;
du 5-amino-2-(2-hydroxyéthyl)amino-1-phénylbenzène ;
du 5-amino-2-(2-méthoxyéthyl)amino-1-(2,3,4-triméthoxyphényl)benzène ;
du 5-amino-2-(2-méthoxyéthyl)amino-1-(2,4-diaminophényl)benzène ;
du 5-amino-2-(2-méthoxyéthyl)amino-1-(2,4-dihydroxyphényl)benzène ;
du 5-amino-2-(2-méthoxyéthyl)amino-1-(2,5-diaminophényl)benzène ;
du 5-amino-2-(2-méthoxyéthyl)amino-1-(2,5-diméthoxyphényl)benzène ;
du 5-amino-2-(2-méthoxyéthyl)amino-1-(2,6-diméthoxyphényl)benzène ;
du 5-amino-2-(2-méthoxyéthyl)amino-1-(2-amino-5-hydroxyphényl)benzène ;
du 5-amino-2-(2-méthoxyéthyl)amino-1-(2-aminophényl)benzène ;
du 5-amino-2-(2-méthoxyéthyl)amino-1-(2-hydroxy-4-aminophényl)benzène ;
du 5-amino-2-(2-méthoxyéthyl)amino-1-(2-hydroxy-5-aminophényl)benzène ;
du 5-amino-2-(2-méthoxyéthyl)amino-1-(2-hydroxyphényl)benzène ;
du 5-amino-2-(2-méthoxyéthyl)amino-1-(2-méthoxyphényl)benzène ;
du 5-amino-2-(2-méthoxyéthyl)amino-1-(2-méthylphényl)benzène ;
du 5-amino-2-(2-méthoxyéthyl)amino-1-(3,5-diaminophényl)benzène ;
du 5-amino-2-(2-méthoxyéthyl)amino-1-(3,5-dihydroxyphényl)benzène ;
du 5-amino-2-(2-méthoxyéthyl)amino-1-(3-aminophényl)benzène ;
du 5-amino-2-(2-méthoxyéthyl)amino-1-(3-hydroxy-5-aminophényl)benzène ;
du 5-amino-2-(2-méthoxyéthyl)amino-1-(3-hydroxyphényl)benzène ;
du 5-amino-2-(2-méthoxyéthyl)amino-1-(3-méthoxyphényl)benzène ;
du 5-amino-2-(2-méthoxyéthyl)amino-1-(4-(diméthylamino)phényl)benzène ;
du 5-amino-2-(2-méthoxyéthyl)amino-1-(4-(trifluorométhyl)phényl)benzène ;
du 5-amino-2-(2-méthoxyéthyl)amino-1-(4-aminophényl)benzène ;
du 5-amino-2-(2-méthoxyéthyl)amino-1-(4-carboxyphényl)benzène ;
du 5-amino-2-(2-méthoxyéthyl)amino-1-(4-chlorophényl)benzène ;
du 5-amino-2-(2-méthoxyéthyl)amino-1-(4-hydroxyphényl)benzène ;
du 5-amino-2-(2-méthoxyéthyl)amino-1-(4-méthoxyphényl)benzène ;
du 5-amino-2-(2-méthoxyéthyl)amino-1-(4-méthylphényl)benzène ;
du 5-amino-2-(2-méthoxyéthyl)amino-1-phénylbenzène ;
du 5-amino-2-di(2-hydroxyéthyl)amino-1-(2,3,4triméthoxyphényl)benzène ;
du 5-amino-2-di(2-hydroxyéthyl)amino-1-(2,3-difluoro-4-heptylphényl)benzène ;
du 5-amino-2-di(2-hydroxyéthyl)amino-1-(2,3-difluorophényl)-benzène ;
du 5-amino-2-di(2-hydroxyéthyl)amino-1-(2,4,6-triméthylphényl)benzène ;
du 5-amino-2-di(2-hydroxyéthyl)amino-1-(2,4-diaminophényl)benzène ;
du 5-amino-2-di(2-hydroxyéthyl)amino-1-(2,4-dichlorophényl)benzène ;
du 5-amino-2-di(2-hydroxyéthyl)amino-1-(2,4-dihydroxyphényl)benzène ;
du 5-amino-2-di(2-hydroxyéthyl)amino-1-(2,5-diméthoxyphényl)benzène ;
du 5-amino-2-di(2-hydroxyéthyl)amino-1-(2,6-difluorophényl)benzène ;
du 5-amino-2-di(2-hydroxyéthyl)amino-1-(2,6-diméthoxyphényl)benzène ;
du 5-amino-2-di(2-hydroxyéthyl)amino-1-(2-((bis(1-méthyléthyl)amino)carbonyl)phényl)benzène ;
du 5-amino-2-di(2-hydroxyéthyl)amino-1-(2-((bis(1-méthyléthyl)amino)carbonyl)-3-méthoxyphényl)benzène ;
du 5-amino-2-di(2-hydroxyéthyl)amino-1-(2-(bromométhyl)phényl)benzène ;
du 5-amino-2-di(2-hydroxyéthyl)amino-1-(2-(diéthylamino)carbonyl)phényl)benzène ;
du 5-amino-2-di(2-hydroxyéthyl)amino-1-(2-carboxyphényl)benzène ;
du 5-amino-2-di(2-hydroxyéthyl)amino-1-(2-chlorophényl)benzène ;
du 5-amino-2-di(2-hydroxyéthyl)amino-1-(2-fluorophényl)benzène ;
du 5-amino-2-di(2-hydroxyéthyl)amino-1-(2-formyl-4-méthoxyphényl)benzène ;
du 5-amino-2-di(2-hydroxyéthyl)amino-1-(2-formyl-4-méthylphényl)benzène ;
du 5-amino-2-di(2-hydroxyéthyl)amino-1-(2-formyl-5-méthoxyphényl)benzène ;
du 5-amino-2-di(2-hydroxyéthyl)amino-1-(2-formylphényl)benzène ;
du 5-amino-2-di(2-hydroxyéthyl)amino-1-(2-hydroxy-4-aminophényl)benzène ;
du 5-amino-2-di(2-hydroxyéthyl)amino-1-(2-hydroxyphényl)benzène ;
du 5-amino-2-di(2-hydroxyéthyl)amino-1-(2-méthoxyphényl)benzène ;
du 5-amino-2-di(2-hydroxyéthyl)amino-1-(2-méthylphényl)benzène ;
du 5-amino-2-di(2-hydroxyéthyl)amino-1-(2-nitrophényl)benzène ;
du 5-amino-2-di(2-hydroxyéthyl)amino-1-(2-trifluorométhylphényl)benzène ;
du 5-amino-2-di(2-hydroxyéthyl)amino-1-(3,4-dichlorophényl)benzène ;
du 5-amino-2-di(2-hydroxyéthyl)amino-1-(3,5-diaminophényl)benzène ;
du 5-amino-2-di(2-hydroxyéthyl)amino-1-(3,5-dichlorophényl)benzène ;
du 5-amino-2-di(2-hydroxyéthyl)amino-1-(3,5-dihydroxyphényl)benzène ;
du 5-amino-2-di(2-hydroxyéthyl)amino-1-(3-(acétylamino)phényl)benzène ;
du 5-amino-2-di(2-hydroxyéthyl)amino-1-(3-aminophényl)benzène ;
du 5-amino-2-di(2-hydroxyéthyl)amino-1-(3-bromophényl)benzène ;
du 5-amino-2-di(2-hydroxyéthyl)amino-1-(3-carboxyphényl)benzène ;
du 5-amino-2-di(2-hydroxyéthyl)amino-1-(3-chloro-4-fluorophényl)benzène ;
du 5-amino-2-di(2-hydroxyéthyl)amino-1-(3-chlorophényl)benzène ;
du 5-amino-2-di(2-hydroxyéthyl)amino-1-(3-fluorophényl)benzène ;
du 5-amino-2-di(2-hydroxyéthyl)amino-1-(3-formylphényl)benzène ;
du 5-amino-2-di(2-hydroxyéthyl)amino-1-(3-hydroxyphényl)benzène ;
du 5-amino-2-di(2-hydroxyéthyl)amino-1-(3-méthoxyphényl)benzène ;
du 5-amino-2-di(2-hydroxyéthyl)amino-1-(3-méthylphényl)benzène ;
du 5-amino-2-di(2-hydroxyéthyl)amino-1-(3-nitrophényl)benzène ;
du 5-amino-2-di(2-hydroxyéthyl)amino-1-(3-trifluorométhylphényl)benzène ;
du 5-amino-2-di(2-hydroxyéthyl)amino-1-(4-(bromométhyl)phényl)benzène ;
du 5-amino-2-di(2-hydroxyéthyl)amino-1-(4-(diméthylamino)phényl)benzène ;
du 5-amino-2-di(2-hydroxyéthyl)amino-1-(4-(hydroxyméthyl)phényl)benzène ;
du 5-amino-2-di(2-hydroxyéthyl)amino-1-(4-(méthylthio)phényl)benzène ;
du 5-amino-2-di(2-hydroxyéthyl)amino-1-(4-(trifluorométhyl)phényl)benzène ;
du 5-amino-2-di(2-hydroxyéthyl)amino-1-(4-(triméthylsilyl)phényl)benzène ;
du 5-amino-2-di(2-hydroxyéthyl)amino-1-(4-acétylphényl)benzène ;
du 5-amino-2-di(2-hydroxyéthyl)amino-1-(4-aminophényl)benzène ;
du 5-amino-2-di(2-hydroxyéthyl)amino-1-(4-bromophényl)benzène ;
du 5-amino-2-di(2-hydroxyéthyl)amino-1-(4-carboxyphényl)benzène ;
du 5-amino-2-di(2-hydroxyéthyl)amino-1-(4-chloro-3-méthoxyphényl)benzène ;
du 5-amino-2-di(2-hydroxyéthyl)amino-1-(4-chlorophényl)benzène ;
du 5-amino-2-di(2-hydroxyéthyl)amino-1-(4-éthénylphényl)benzène ;
du 5-amino-2-di(2-hydroxyéthyl)amino-1-(4-éthoxyphényl)benzène ;
du 5-amino-2-di(2-hydroxyéthyl)amino-1-(4-fluorophényl)benzène ;
du 5-amino-2-di(2-hydroxyéthyl)amino-1-(4-formylphényl)benzène ;
du 5-amino-2-di(2-hydroxyéthyl)amino-1-(4-hydroxyphényl)benzène ;
du 5-amino-2-di(2-hydroxyéthyl)amino-1-(4-iodophényl)benzène ;
du 5-amino-2-di(2-hydroxyéthyl)amino-1-(4-méthoxyphényl)benzène ;
du 5-amino-2-di(2-hydroxyéthyl)amino-1-(4-méthyl-3-nitrophényl)benzène ;
du 5-amino-2-di(2-hydroxyéthyl)amino-1-(4-méthylphényl)benzène ;
du 5-amino-2-di(2-hydroxyéthyl)amino-1-(5-bromo-2-méthoxyphényl)benzène ;
du 5-amino-2-di(2-hydroxyéthyl)amino-1-(5-chloro-2-méthoxyphényl)benzène ;
du 5-amino-2-di(2-hydroxyéthyl)amino-1-(5-formyl-2-méthoxyphényl)benzène ;
du 5-amino-2-di(2-méthoxyéthyl)amino-1-(2,3,4-triméthoxyphényl)benzène ;
du 5-amino-2-di(2-méthoxyéthyl)amino-1-(2,3-difluoro-4-heptylphényl)benzène ;
du 5-amino-2-di(2-méthoxyéthyl)amino-1-(2,3-difluorophényl)benzène ;
du 5-amino-2-di(2-méthoxyéthyl)amino-1-(2,4,6-triméthylphényl)benzène ;
du 5-amino-2-di(2-méthoxyéthyl)amino-1-(2,4-diaminophényl)benzène ;
du 5-amino-2-di(2-méthoxyéthyl)amino-1-(2,4-dichlorophényl)benzène ;
du 5-amino-2-di(2-méthoxyéthyl)amino-1-(2,4-dihydroxyphényl)benzène ;
du 5-amino-2-di(2-méthoxyéthyl)amino-1-(2,5-diméthoxyphényl)benzène ;
du 5-amino-2-di(2-méthoxyéthyl)amino-1-(2,6-difluorophényl)benzène ;
du 5-amino-2-di(2-méthoxyéthyl)amino-1-(2,6-diméthoxyphényl)benzène ;
du 5-amino-2-di(2-méthoxyéthyl)amino-1-(2-((bis(1-méthyléthyl)amino)carbonyl)phényl)benzène ;
du 5-amino-2-di(2-méthoxyéthyl)amino-1-(2-((bis(1-méthyléthyl)amino)-carbonyl)-3-méthoxyphényl)benzène ;
du 5-amino-2-di(2-méthoxyéthyl)amino-1-(2-(bromométhyl)phényl)benzène ;
du 5-amino-2-di(2-méthoxyéthyl)amino-1-(2-(diéthylamino)carbonyl)phényl)benzène ;
du 5-amino-2-di(2-méthoxyéthyl)amino-1-(2-carboxyphényl)benzène ;
du 5-amino-2-di(2-méthoxyéthyl)amino-1-(2-chlorophényl)benzène ;
du 5-amino-2-di(2-méthoxyéthyl)amino-1-(2-fluorophényl)benzène ;
du 5-amino-2-di(2-méthoxyéthyl)amino-1-(2-formyl-4-méthoxyphényl)benzène ;
du 5-amino-2-di(2-méthoxyéthyl)amino-1-(2-formyl-4-méthylphényl)benzène ;
du 5-amino-2-di(2-méthoxyéthyl)amino-1-(2-formyl-5-méthoxyphényl)benzène ;
du 5-amino-2-di(2-méthoxyéthyl)amino-1-(2-formylphényl)benzène ;
du 5-amino-2-di(2-méthoxyéthyl)amino-1-(2-hydroxy-4-aminophényl)benzène ;
du 5-amino-2-di(2-méthoxyéthyl)amino-1-(2-hydroxyphényl)benzène ;
du 5-amino-2-di(2-méthoxyéthyl)amino-1-(2-méthoxyphényl)benzène ;
du 5-amino-2-di(2-méthoxyéthyl)amino-1-(2-méthylphényl)benzène ;
du 5-amino-2-di(2-méthoxyéthyl)amino-1-(2-nitrophényl)benzène ;
du 5-amino-2-di(2-méthoxyéthyl)amino-1-(2-trifluorométhylphényl)benzène ;
du 5-amino-2-di(2-méthoxyéthyl)amino-1-(3,4-dichlorophényl)benzène ;
du 5-amino-2-di(2-méthoxyéthyl)amino-1-(3,5-diaminophényl)benzène ;
du 5-amino-2-di(2-méthoxyéthyl)amino-1-(3,5-dichlorophényl)benzène ;
du 5-amino-2-di(2-méthoxyéthyl)amino-1-(3,5-dihydroxyphényl)benzène ;
du 5-amino-2-di(2-méthoxyéthyl)amino-1-(3-(acétylamino)phényl)benzène ;
du 5-amino-2-di(2-méthoxyéthyl)amino-1-(3-aminophényl)benzène ;
du 5-amino-2-di(2-méthoxyéthyl)amino-1-(3-bromophényl)benzène ;
du 5-amino-2-di(2-méthoxyéthyl)amino-1-(3-carboxyphényl)benzène ;
du 5-amino-2-di(2-méthoxyéthyl)amino-1-(3-chloro-4-fluorophényl)benzène ;
du 5-amino-2-di(2-méthoxyéthyl)amino-1-(3-chlorophényl)benzène ;
du 5-amino-2-di(2-méthoxyéthyl)amino-1-(3-fluorophényl)benzène ;
du 5-amino-2-di(2-méthoxyéthyl)amino-1-(3-formylphényl)benzène ;
du 5-amino-2-di(2-méthoxyéthyl)amino-1-(3-hydroxyphényl)benzène ;
du 5-amino-2-di(2-méthoxyéthyl)amino-1-(3-méthoxyphényl) benzène ;
du 5-amino-2-di(2-méthoxyéthyl)amino-1-(3-méthylphényl)benzène ;
du 5-amino-2-di(2-méthoxyéthyl)amino-1-(3-nitrophényl)benzène ;
du 5-amino-2-di(2-méthoxyéthyl)amino-1-(3-trifluorométhylphényl)benzène ;
du 5-amino-2-di(2-méthoxyéthyl)amino-1-(4-(bromométhyl)phényl)benzène ;
du 5-amino-2-di(2-méthoxyéthyl)amino-1-(4-(diméthylamino)phényl)benzène ;
du 5-amino-2-di(2-méthoxyéthyl)amino-1-(4-(hydroxyméthyl)phényl)benzène ;
du 5-amino-2-di(2-méthoxyéthyl)amino-1-(4-(méthylthio)phényl)benzène ;
du 5-amino-2-di(2-méthoxyéthyl)amino-1-(4-(trifluorométhyl)phényl)benzène ;
du 5-amino-2-di(2-méthoxyéthyl)amino-1-(4-(triméthylsilyl)phényl)benzène ;
du 5-amino-2-di(2-méthoxyéthyl)amino-1-(4-acétylphényl)benzène ;
du 5-amino-2-di(2-méthoxyéthyl)amino-1-(4-aminophényl)benzène ;
du 5-amino-2-di(2-méthoxyéthyl)amino-1-(4-bromophényl)benzène ;
du 5-amino-2-di(2-méthoxyéthyl)amino-1-(4-carboxyphényl)benzène ;
du 5-amino-2-di(2-méthoxyéthyl)amino-1-(4-chloro-3-méthoxyphényl)benzène ;
du 5-amino-2-di(2-méthoxyéthyl)amino-1-(4-chlorophényl)benzène ;
du 5-amino-2-di(2-méthoxyéthyl)amino-1-(4-éthénylphényl)benzène ;
du 5-amino-2-di(2-méthoxyéthyl)amino-1-(4-éthoxyphényl)benzène ;
du 5-amino-2-di(2-méthoxyéthyl)amino-1-(4-fluorophényl)benzène ;
du 5-amino-2-di(2-méthoxyéthyl)amino-1-(4-formylphényl)benzène ;
du 5-amino-2-di(2-méthoxyéthyl)amino-1-(4-hydroxyphényl)benzène ;
du 5-amino-2-di(2-méthoxyéthyl)amino-1-(4-iodophényl)benzène ;
du 5-amino-2-di(2-méthoxyéthyl)amino-1-(4-méthoxyphényl)benzène ;
du 5-amino-2-di(2-méthoxyéthyl)amino-1-(4-méthyl-3-nitrophényl)benzène ;
du 5-amino-2-di(2-méthoxyéthyl)amino-1-(4-méthylphényl)benzène ;
du 5-amino-2-di(2-méthoxyéthyl)amino-1-(5-bromo-2-méthoxyphényl)benzène ;
du 5-amino-2-di(2-méthoxyéthyl)amino-1-(5-chloro-2-méthoxyphényl)benzène ;
du 5-amino-2-di(2-méthoxyéthyl)amino-1-(5-formyl-2-méthoxyphényl)benzène ;
du 5-amino-2-diméthylamino-1-phénylbenzène ;
du 5-amino-2-méthylamino-1-(2,3,4-triméthoxyphényl)benzène ;
du 5-amino-2-méthylamino-1-(2,4-diaminophényl)benzène ;
du 5-amino-2-méthylamino-1-(2,4-dihydroxyphényl)benzène ;
du 5-amino-2-méthylamino-1-(2,5-diaminophényl)benzène ;
du 5-amino-2-méthylamino-1-(2,5-diméthoxyphényl)benzène ;
du 5-amino-2-méthylamino-1-(2,6-diméthoxyphényl)benzène ;
du 5-amino-2-méthylamino-1-(2-amino-5-hydroxyphényl)benzène ;
du 5-amino-2-méthylamino-1-(2-aminophényl)benzène ;
du 5-amino-2-méthylamino-1-(2-hydroxy-4-aminophényl)benzène ;
du 5-amino-2-méthylamino-1-(2-hydroxy-5-aminophényl)benzène ;
du 5-amino-2-méthylamino-1-(2-hydroxyphényl)benzène ;
du 5-amino-2-méthylamino-1-(2-méthoxyphényl)benzène ;
du 5-amino-2-méthylamino-1-(2-méthylphényl)benzène ;
du 5-amino-2-méthylamino-1-(3,5-diaminophényl)benzène ;
du 5-amino-2-méthylamino-1-(3,5-dihydroxyphényl)benzène ;
du 5-amino-2-méthylamino-1-(3-aminophényl)benzène ;
du 5-amino-2-méthylamino-1-(3-hydroxy-5-aminophényl)benzène ;
du 5-amino-2-méthylamino-1-(3-hydroxyphényl)benzène ;
du 5-amino-2-méthylamino-1-(3-méthoxyphényl)benzène ;
du 5-amino-2-méthylamino-1-(4-(diméthylamino)phényl)benzène ;
du 5-amino-2-méthylamino-1-(4-(trifluorométhyl)phényl)benzène ;
du 5-amino-2-méthylamino-1-(4-aminophényl)benzène ;
du 5-amino-2-méthylamino-1-(4-carboxyphényl)benzène ;
du 5-amino-2-méthylamino-1-(4-chlorophényl)benzène ;
du 5-amino-2-méthylamino-1-(4-hydroxyphényl)benzène ;
du 5-amino-2-méthylamino-1-(4-méthoxyphényl)benzène ;
du 5-amino-2-méthylamino-1-(4-méthylphényl)benzène et
du 5-amino-2-méthylamino-1-phénylbenzène,
ou leurs sels physiologiquement acceptables.
